(19) Europäisches Patentamt / European Patent Office / Office européen des brevets

(11) **EP 4 116 411 A1**

(12) **EUROPEAN PATENT APPLICATION**
published in accordance with Art. 153(4) EPC

(43) Date of publication:
**11.01.2023 Bulletin 2023/02**

(21) Application number: **21761596.2**

(22) Date of filing: **26.02.2021**

(51) International Patent Classification (IPC):
**C12N 5/071** (2010.01)    **C12Q 1/04** (2006.01)
**G01N 33/48** (2006.01)

(52) Cooperative Patent Classification (CPC):
**C12N 5/06; C12Q 1/04; G01N 33/48**

(86) International application number:
**PCT/JP2021/007490**

(87) International publication number:
**WO 2021/172554 (02.09.2021 Gazette 2021/35)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **27.02.2020 JP 2020032139**

(71) Applicant: **Kyoto Prefectural Public University Corporation**
**Kyoto-shi, Kyoto 602-8566 (JP)**

(72) Inventors:
• **KINOSHITA, Shigeru**
  **Kyoto-shi, Kyoto 602-8566 (JP)**

• **HAMURO, Junji**
  **Kyoto-shi, Kyoto 602-8566 (JP)**
• **SOTOZONO, Chie**
  **Kyoto-shi, Kyoto 602-8566 (JP)**
• **UENO, Morio**
  **Kyoto-shi, Kyoto 602-8566 (JP)**
• **TODA, Munetoyo**
  **Kyoto-shi, Kyoto 602-8566 (JP)**

(74) Representative: **Engelhard, Markus**
**Boehmert & Boehmert**
**Anwaltspartnerschaft mbB**
**Pettenkoferstrasse 22**
**80336 München (DE)**

(54) **FUNCTIONAL HUMAN CORNEAL ENDOTHELIAL CELLS AND APPLICATION THEREOF**

(57)    To provide a cell trait assay technique for identifying cultured human corneal endothelium cells of which early clinical effect manifestation and a long-term stable clinical effect are confirmed, in clinical trials. Provided is a method of manufacturing a functional human corneal endothelial cell capable of eliciting a human corneal function when infused into an anterior chamber of a human eye, the method comprising the step of proliferating and/or differentiating or maturing a corneal endothelial progenitor cell under a culture condition capable of minimizing culture stress, such as proliferation stress. Further, provided is a functional human corneal endothelial cell in which expression of a functional protein leading to a corneal endothelial (cell) functional property leading to improvement on corneal opacity and hydrous edema, resulting in continuous and long-term retention of corneal endothelial tissue cell density and improvement on visual acuity is recognized or in which a protein that inhibits the corneal endothelial (cell) functional property is not elicited or is reduced.

**Description**

[Technical Field]

The present disclosure relates to a human functional corneal endothelial cell capable of eliciting a human corneal endothelial functional property when infused into an anterior chamber of a human eye, medicament comprising the cell, manufacturing method thereof, and the like.

[Background Art]

Currently, the only therapeutic method for corneal endothelial disorders including bullous keratopathy is corneal transplantation surgery using a donor cornea, although the long-term clinical result of this surgery is poor. Furthermore, the visual acuity after corneal transplantation is not sufficient in terms of patient satisfaction either, due to induced corneal irregular astigmatism. About 60% or more of corneal transplantation patients suffer from the corneal endothelial dysfunction including bullous keratopathy and early Fuchs corneal dystrophy. The primary causes of bullous keratopathy are corneal endothelial disorders due to ophthalmic surgery such as cataract surgery, glaucoma surgery, vitreo-retinal surgery, or laser iridotomy, corneal trauma, pseudoexfoliation syndrome, and Fuchs endothelial corneal dystrophy. The potential prevalence rate of Fuchs endothelial corneal dystrophy involving a genetic factor in Europe and US is reportedly about 5% or higher. Corneal transplantation surgery requires one donor cornea for treating one diseased eye, such that transplantation surgery cannot be a means for solving the sustained shortage of donors. In view of the large number of latent patients worldwide, there is an intense worldwide demand, as an urgent issue to be solved, for the provision of innovative medical treatment with versatility and convenience that can be applied at a wide range of medical institutions compared to corneal transplantation techniques. In addition, the cell infusion therapy reproduce the normal shape of the cornea without distortion associated with corneal transplantation, resulting in long-term recovery with good visual function.

[Summary of Invention]

[Solution to Problem]

The inventors have found for the first time in the world that the cultured human corneal endothelial cell is contaminated with a cell subpopulation that has different traits from those of the corneal endothelial cells held by healthy corneal endothelial tissue, due to cell state transition (fibrosis, epithelial mesenchymal transition, endothelial-mesenchymal transition, senescence, dedifferentiation or the like) in culture; and by devising a technique for selectively propagating a subpopulation in culture, the inventors have confirmed that a specific subpopulation, i.e., functional cell (herein also called effector cell) which sufficiently shares a function(s) with mature differentiated human corneal endothelial cell, forms a small hexagonal cobble-stone shape optimal for cell infusion therapy and has traits similar to those of the corneal endothelial cells held by healthy corneal endothelial tissue.

As for the present disclosure, in clinical trials, the inventors diligently investigated a cell trait assay technique for identifying cultured human corneal endothelial cells of which early clinical effect manifestation and a long-term stable clinical effect were confirmed, and the inventors have achieved the invention of this technique for the first time in the world. The inventors have achieved the invention for identifying and providing a cultured human corneal endothelial cell that ensures early clinical effect manifestation and a long-term stable clinical effect by defining mitochondrial function with various metabolism-related enzymes, based on the newly invented technique.

For the first time in the world, the present disclosure provides breakthrough and patient-friendly medical care for reconstructing corneal endothelial tissue which is homogeneous and small and which has a high cell density after transplantation over a long period of time, by a significantly less expensive manufacturing method in terms of cost, the method being capable of stable production, which is an absolute requirement for providing the same medical care at all times, and enabling widespread application all over the world. It is a cultured human corneal endothelial cell with enhanced mitochondrial-dependent oxidative respiration. It has also been found to be effective in patients with a rejection response in corneal transplantation.

Furthermore, with the manufacturing method according to the present disclosure, activation of epigenetic polygenes such as metabolites by enzymes involved in the TCA metabolic pathways acting in the cytoplasm and nucleus, especially histone acetylation by acetylcoenzyme A (AcCoA), is avoided, so that phase transition of cultured cells does not occur and metabolic reprogramming leans towards retention of mitochondrial function. By such an innovative invention, the production of the above-mentioned contaminating subpopulation cells is minimized, and high-performance endothelial cells that are extremely useful in the long term for improving corneal opacity and hydrous edema in patients with corneal endothelial dysfunction are provided.

The present disclosure therefore provides the following.

(Item 1)

A method of manufacturing a human functional corneal endothelial cell capable of eliciting a human corneal function when infused into an anterior chamber of a human eye, the method comprising the step of:
(b) proliferating and/or differentiating or maturing a corneal endothelial progenitor cell under a culture condition capable of minimizing culture stress, such as proliferation stress.

(Item 2)

The method according to (Item 1), wherein the human corneal function comprises a human corneal endothelial functional property.

(Item 3)

The method according to (Item 1) or (Item 2), further comprising the step of: (a) dedifferentiating a human corneal endothelial tissue-derived cell to obtain the corneal endothelial progenitor cell.

(Item 4)

The method of manufacturing a human functional corneal endothelial cell capable of eliciting a human corneal function, and especially a human corneal endothelial functional property, when infused into an anterior chamber of a human eye, according to any one of (Item 1) to ((Item 3), the method proliferating and/or differentiating or maturing a corneal endothelial progenitor cell in the presence of a cell growth factor with an amount less than the amount at which transformation occurs.

(Item 5)

The method according to any one of (Item 1) to (Item 4), wherein the cell growth factor comprises an epidermal growth factor (EGF).

(Item 6)

The method according to any one of (Item 1) to (Item 5), wherein the transformation comprises endothelial-mesenchymal transformation.

(Item 7)

The method according to any one of (Item 1) to (Item 6), wherein the step of proliferating and/or differentiating or maturing is performed in the presence of a ROCK inhibitor.

(Item 8)

The method according to any one of (Item 1) to (Item 6), comprising the step of confirming that the cell obtained in the step (b) is a cell in which mitochondria-dependent oxidative phosphorylation is increased in mitochondria and acetyl-CoA expression in the cytoplasm or nucleus is not increased, and in which epigenetic multigene expression through histone acetylation by acetyl-CoA is not induced.

(Item 9)

The method according to any one of (Item 1) to (Item 8), comprising the step of confirming that the cell obtained in the step (b) is a cell in which one or more metabolic-related enzymes selected from the group consisting of citrate synthase (CS), aconitase 2 (ACO2), isocitrate dehydrogenase 2 (IDH2), malate dehydrogenase 2 (MDH2), malic enzyme 3 (ME3), ACSS1, acetyl-CoA acetyltransferase 1 (ACAT1), pyruvate dehydrogenase (PDH), BCAT2, and branched-chain ketoacid dehydrogenase 2 (BCKDH2) are expressed in mitochondria.

(Item 10)

The method according to any one of (Item 1) to (Item 9), comprising the step of confirming that the cell obtained in the step (b) is a cell in which ATP citrate lyase (ACLY), aconitase 1 (ACO1), isocitrate dehydrogenase 1 (IDH1), malate dehydrogenase 1 (MDH1), malic enzyme 1 (ME1), ACSS2, acetyl-CoA acetyltransferase 2 (ACAT2), and/or lactate dehydrogenase (LDH) is not expressed, or is hardly expressed.

(Item 10A)

The method according to any one of the above Items, wherein expression of IDH1 and/or ACSS2 in the cell obtained in the step (b) is attenuated compared to the expression in a non-human functional corneal endothelial cell.

(Item 10B)

The method according to any one of the above Items, wherein ATP citrate lyase (ACLY) is not expressed in the cell obtained in the step (b).

(Item 10C)

The method according to any one of the above Items, wherein expression of IDH2 in the cell obtained in the step (b) is enhanced compared to the expression in a non-human functional corneal endothelial cell.

(Item 11)

The method according to any one of (Item 1) to (Item 10), comprising the step of confirming that expression of ion channel and/or monocarboxylic acid transporter leading to a corneal endothelial (cell) functional property leading to improvement on corneal opacity and hydrous edema, resulting in continuous and long-term retention of corneal endothelial tissue cell density and improvement on visual acuity is recognized in the cell obtained in the step (b).

(Item 12)

The method according to any one of (Item 1) to (Item 11), comprising the step of confirming that expression of sodium/hydrogen exchanger 1 (NHE1) and/or aquaporin 1 (AQP-1) is increased in the cell obtained in the step (b).

(Item 13)

The method according to (Item 9) or (Item 10), comprising the step of confirming that expression of bicarbonic anhydrase 5B (CA5B) is increased in the cell obtained in the step (b).

(Item 14)

The method according to any one of (Item 1) to (Item 12), comprising the step of confirming that the cell obtained in the step (b) has a property that a metabolic enzyme related to a TCA cycle, etc., and a metabolite, such as AcetylCoA, are not present in the cytoplasm or nucleus so as not to lead to the production of contaminant phase transition cells and are organelle-selectively localized in mitochondria.

(Item 15)

The method according to any one of (Item 1) to (Item 14), wherein the human functional corneal endothelial cell is made from a cell, as the origin thereof, selected from the group consisting of: a corneal endothelial tissue-derived cell; a pluripotent stem cell; a mesenchymal stem cell; a corneal endothelial progenitor cell collected from a corneal endothelium; a cell collected form a corneal endothelium; and a corneal endothelial precursor cell and a corneal endothelial-like cell made by a direct programming method.
The present disclosure also provides the following inventions.

(Item 16)

A human functional corneal endothelial cell in which expression of a functional protein leading to a corneal endothelial (cell) functional property leading to improvement on corneal opacity and hydrous edema, resulting in continuous and long-term retention of corneal endothelial tissue cell density and improvement on visual acuity is recognized or in which a protein that inhibits the corneal endothelial (cell) functional property is not elicited or is reduced.

(Item 17)

The cell according to (Item 16), wherein the cell is a human functional corneal endothelial cell capable of eliciting a human corneal endothelial functional property when infused into an anterior chamber of a human eye, in which one or more metabolic-related enzymes selected from the group consisting of citrate synthase (CS), aconitase 2 (ACO2), isocitrate dehydrogenase 2 (IDH2), malate dehydrogenase 2 (MDH2), malic enzyme 3 (ME3), ACSS1, acetyl-CoA acetyltransferase 1 (ACAT1), pyruvate dehydrogenase (PDH), BCAT2, and branched-chain ketoacid dehydrogenase 2 (BCKDH2) are expressed in mitochondria.

(Item 18)

The cell according to (Item 16) or (Item 17), wherein the cell is a human functional corneal endothelial cell capable of eliciting a human corneal endothelial functional property when infused into an anterior chamber of a human eye, including at least one selected from the group consisting of: a cell in which mitochondria-dependent oxidative phosphorylation is increased in mitochondria or acetyl-CoA expression in the cytoplasm or nucleus is not increased; and a cell in which epigenetic multigene expression through histone acetylation by acetyl-CoA is not induced.

(Item 19)

The cell according to any one of (Item 16) to (Item 18), wherein the cell is a human corneal endothelial cell, in which ATP citrate lyase (ACLY), aconitase 1 (ACO1), isocitrate dehydrogenase 1 (IDH1), malate dehydrogenase 1 (MDH1), malic enzyme 1 (ME1), ACSS2, acetyl-CoA acetyltransferase 2 (ACAT2), and/or lactate dehydrogenase (LDH) is not expressed or is not substantially expressed.

(Item 20)

The cell according to any one of (Item 16) to (Item 19), wherein expression of sodium/hydrogen exchanger 1 (NHE1) and/or aquaporin 1 (AQP-1) is increased in the human functional corneal endothelial cell.

(Item 21)

The cell according to any one of (Item 16) to (Item 20), wherein expression of bicarbonic anhydrase 5B (CA5B) is increased in the human functional corneal endothelial cell.

(Item 22)

The cell according to any one of (Item 16) to (Item 21), wherein the human functional corneal endothelial cell comprises all selected from the group consisting of: (i) a property that a metabolic enzyme related to the TCA cycle, etc., and a metabolite, such as AcetylCoA, are not present in the cytoplasm or nucleus so as not to lead to the production of contaminant phase transition cells and are organelle-selectively localized in mitochondria; (ii) increase in mitochondria-dependent oxidative phosphorylation in mitochondria; (iii) reduction in epigenetic multigene expression through histone acetylation by acetyl-CoA (including no elicitation); (iv) increase in expression of sodium/hydrogen exchanger 1 (NHE1) and/or aquaporin 1 (AQP-1); and (v) increase in expression of bicarbonic anhydrase 5B (CA5B).

(Item 23)

The cell according to any one of (Item 16) to (Item 22), wherein endothelial-mesenchymal transition has not occurred or has not substantially occurred.

(Item 24)

A human functional corneal endothelial cell capable of eliciting a human corneal endothelial functional property when infused into an anterior chamber of a human eye, wherein endothelial-mesenchymal transition has not occurred or has not substantially occurred.

(Item 25)

The cell according to any one of (Item 16) to (Item 24), wherein the human functional corneal endothelial cell is made from a cell, as the origin thereof, selected from the group consisting of: a corneal endothelial tissue-derived cell; a pluripotent stem cell; a mesenchymal stem cell; a corneal endothelial progenitor cell collected from a corneal endothelium; a cell collected form a corneal endothelium; and a corneal endothelial precursor cell and a corneal endothelial-like cell made by a direct programming method.

(Item 26)

A cell population comprising a cell manufactured by the method according to any one of (Item 1) to (Item 15) and/or a cell according to any one of (Item 16) to (Item 25).

(Item 27)

A method of quality control or process control of a human functional corneal endothelial cell capable of eliciting a human corneal endothelial functional property when infused into an anterior chamber of a human eye, the method comprising the step of confirming that one or more metabolic-related enzymes selected from the group consisting of citrate synthase (CS), aconitase 2 (ACO2), isocitrate dehydrogenase 2 (IDH2), malate dehydrogenase 2 (MDH2), malic enzyme 3 (ME3), ACSS1, acetyl-CoA acetyltransferase 1 (ACAT1), pyruvate dehydrogenase (PDH), BCAT2, and branched-chain ketoacid dehydrogenase 2 (BCKDH2) are expressed in mitochondria of the cell.

(Item 28)

The method according to (Item 27), further comprising the step of confirming that expression of acetyl-CoA in the cytoplasm and nucleus, and epigenetic multigene expression through histone acetylation by acetyl-CoA, are not elicited in the cell.

(Item 29)

The method according to (Item 27) or (Item 28), further comprising the step of confirming that expression of sodium/hydrogen exchanger 1 (NHE1) and/or aquaporin 1 (AQP-1) is increased in the cell.

(Item 30)

The method according to any one of (Item 27) to (Item 29), further comprising the step of confirming that expression of bicarbonic anhydrase 5B (CA5B) is increased in the cell.

(Item 31)

A method of quality control or process control of a human functional corneal endothelial cell capable of eliciting a human corneal endothelial functional property when infused into an anterior chamber of a human eye, or a method of detecting a corneal endothelial non-functional cell mixed with a human functional corneal endothelial cell, the method comprising the step of confirming one or more of the following items:

(1) no fibroblast, foreign body, discoloration, or other abnormalities on visual inspection by phase-contrast imaging on the day of transplantation;
(2) number of cell of $1.5 \times 10^6$ cells/450 $\mu$L two weeks prior to and/or on the day of transplantation;
(3) 85% or more cell viability by trypan blue staining;
(4) PDGF-BB: 100 pg/mL or more in a purity test by ELISA of cell supernatant;
(5) in a purity test by FACS of cell supernatant collected two weeks prior to and/or on the day of transplantation,

$CD166^+ > 99\%$
$CD24^+ < 5\%$
$CD26^+ < 5\%$
$CD200^+ < 5\%$
$CD44^{high} < 5\%$
$CD44^{low} > 90\%$
$CD105^{-\sim weak} > 90\%$
$CD90^+ < 5\%$;

(6) effector cell (E-ratio) > 90%;
(7) pump function (Na+/K+ATPase) two days prior to transplantation: positive;
(8) barrier function (ZO-1) two days prior to transplantation: positive;
(9) less than 125 ng/$\mu$L in BSA negative test;
(10) ECD on the day of transplantation to be 1500 cells/mm$^2$ or more;
(11) expression of miR184;
(12) lactic acid production; and
(13) cell size of less than 250 $\mu$m.

(Item 32)

A cell population of a human functional corneal endothelial cell capable of eliciting a human corneal endothelial functional property when infused into an anterior chamber of a human eye, the cell population satisfying one or more of the following items:

(1) no fibroblast, foreign body, discoloration, or other abnormalities on visual inspection by phase-contrast imaging on the day of transplantation;
(2) number of cell of $1.5\times10^6$ cells/450 $\mu$L two weeks prior to and/or on the day of transplantation;
(3) 85% or more cell viability by trypan blue staining;
(4) PDGF-BB: 100 pg/mL or more in a purity test by ELISA of cell supernatant;
(5) in a purity test by FACS of cell supernatant collected two weeks prior to and/or on the day of transplantation,

CD166$^+$ > 99%
CD24$^+$ < 5%
CD26$^+$ < 5%
CD200$^+$ < 5%
CD44$^{high}$ < 5%
CD44$^{low}$ > 90%
CD105$^{-\sim weak}$ > 90%
CD90$^+$ < 5%;

(6) effector cell (E-ratio) > 90%;
(7) pump function (Na+/K+ATPase) two days prior to transplantation: positive;
(8) barrier function (ZO-1) two days prior to transplantation: positive;
(9) less than 125 ng/$\mu$L in BSA negative test;
(10) ECD on the day of transplantation to be 1500 cells/mm$^2$ or more;
(11) expression of miR184;
(12) lactic acid production; and
(13) cell size of less than 250 $\mu$m.

The present disclosure also provides the following inventions.

(Medicament)

(Item A1)

A medicament comprising a human functional corneal endothelial cell in which expression of a functional protein leading to a corneal endothelial (cell) functional property leading to improvement on corneal opacity and hydrous edema, resulting in continuous and long-term retention of corneal endothelial tissue cell density and improvement on visual acuity is recognized or in which a protein that inhibits the corneal endothelial (cell) functional property is not elicited or is reduced.

(Item A12)

The medicament according to (Item A1), wherein the cell is a human functional corneal endothelial cell capable of eliciting a human corneal endothelial functional property when infused into an anterior chamber of a human eye, in which one or more metabolic-related enzymes selected from the group consisting of citrate synthase (CS), aconitase 2 (ACO2), isocitrate dehydrogenase 2 (IDH2), malate dehydrogenase 2 (MDH2), malic enzyme 3 (ME3), ACSS1, acetyl-CoA acetyltransferase 1 (ACAT1), pyruvate dehydrogenase (PDH), BCAT2, and branched-chain ketoacid dehydrogenase 2 (BCKDH2) are expressed in mitochondria.

(Item A3)

The medicament according to (Item A1) or (Item A2), wherein the cell is a human functional corneal endothelial cell capable of eliciting a human corneal endothelial functional property when infused into an anterior chamber of a human eye, including at least one selected from the group consisting of: a cell in which mitochondria-dependent oxidative phosphorylation is increased in mitochondria or acetyl-CoA expression in the cytoplasm or nucleus is not increased; and a cell in which epigenetic multigene expression through histone acetylation by acetyl-CoA is not induced.

(Item A4)

The medicament according to any one of (Item A1) to (Item A3), wherein the cell is a human corneal endothelial cell, in which ATP citrate lyase (ACLY), aconitase 1 (ACO1), isocitrate dehydrogenase 1 (IDH1), malate dehydrogenase 1 (MDH1), malic enzyme 1 (ME1), ACSS2, acetyl-CoA acetyltransferase 2 (ACAT2), and/or lactate dehydrogenase (LDH) is not expressed or is not substantially expressed.

(Item A5)

The medicament according to any one of (Item A1) to (Item A4), wherein expression of sodium/hydrogen exchanger 1 (NHE1) and/or aquaporin 1 (AQP-1) is increased in the human functional corneal endothelial cell.

(Item A6)

The medicament according to any one of (Item A1) to (Item A5), wherein expression of bicarbonic anhydrase 5B (CA5B) is increased in the human functional corneal endothelial cell.

(Item A7)

The medicament according to any one of (Item A1) to (Item A6), wherein the human functional corneal endothelial cell comprises all selected from the group consisting of: (i) a property that a metabolic enzyme related to the TCA cycle, etc., and a metabolite, such as AcetylCoA, are not present in the cytoplasm or nucleus so as not to lead to the production of contaminant phase transition cells and are organelle-selectively localized in mitochondria; (ii) increase in mitochondria-dependent oxidative phosphorylation in mitochondria; (iii) reduction in epigenetic multigene expression through histone acetylation by acetyl-CoA (including no elicitation); (iv) increase in expression of sodium/hydrogen exchanger 1 (NHE1) and/or aquaporin 1 (AQP-1); and (v) increase in expression of bicarbonic anhydrase 5B (CA5B).

(Item A8)

The medicament according to any one of (Item A1) to (Item A7), having the cell in which endothelial-mesenchymal transition has not occurred or has not substantially occurred.

(Item A9)

A medicament having a human functional corneal endothelial cell capable of eliciting a human corneal endothelial functional property when infused into an anterior chamber of a human eye, wherein endothelial-mesenchymal transition has not occurred or has not substantially occurred in the cell.

(Item A10)

The medicament according to any one of (Item A1) to (Item A9), wherein the human functional corneal endothelial cell is made from a cell, as the origin thereof, selected from the group consisting of: a corneal endothelial tissue-derived cell; a pluripotent stem cell; a mesenchymal stem cell; a corneal endothelial progenitor cell collected from a corneal endothelium; a cell collected form a corneal endothelium; and a corneal endothelial precursor cell and a corneal endothelial-like cell made by a direct programming method.

(Item A11)

A medicament comprising a cell population comprising a cell manufactured by the method according to any one of (Item 1) to (Item 15) and/or a cell according to any one of (Item 16) to (Item 25).

(Item A12)

A medicament comprising a cell population of a human functional corneal endothelial cell capable of eliciting a human corneal endothelial functional property when infused into an anterior chamber of a human eye, the cell population satisfying one or more of the following items:

(1) no fibroblast, foreign body, discoloration, or other abnormalities on visual inspection by phase-contrast imaging on the day of transplantation;
(2) number of cell of $1.5 \times 10^6$ cells/450 μL two weeks prior to and/or on the day of transplantation;
(3) 85% or more cell viability by trypan blue staining;
(4) PDGF-BB: 100 pg/mL or more in a purity test by ELISA of cell supernatant;
(5) in a purity test by FACS of cell supernatant collected two weeks prior to and/or on the day of transplantation,

CD166$^+$ > 99%
CD24$^+$ < 5%
CD26$^+$ < 5%
CD200$^+$ < 5%
CD44$^{high}$ < 5%
CD44$^{low}$ > 90%
CD105$^{-\sim weak}$ > 90%
CD90$^+$ < 5%;

(6) effector cell (E-ratio) > 90%;
(7) pump function (Na+/K+ATPase) two days prior to transplantation: positive;
(8) barrier function (ZO-1) two days prior to transplantation: positive;
(9) less than 125 ng/μL in BSA negative test;
(10) ECD on the day of transplantation to be 1500 cells/mm$^2$ or more;
(11) expression of miR184;
(12) lactic acid production; and
(13) cell size of less than 250 μm.

It is understood that one or more of the aforementioned features can further be provided as a combination thereof in addition to the explicitly shown combinations in the present disclosure. Additional embodiments and advantages of the present disclosure are recognized by those skilled in the art who read and understand the following detailed description as needed.

[Brief Description of Drawings]

[Fig. 1] Fig. 1 is a conceptual diagram showing the energy metabolism control action of mitochondria through CD44.
[Fig. 2] Fig. 2 is a conceptual diagram showing action mechanisms of various clinical effects in human functional corneal endothelial cells (standard cells) according to one embodiment of the present disclosure.
[Fig. 3] Fig. 3 is a graph showing the results of examining the effects of changing the method for manufacturing a human functional corneal endothelial cell according to one embodiment of the present disclosure.
[Fig. 4] Fig. 4 is a conceptual diagram showing induction of a differentiated and matured, human functional corneal endothelial cell according to one embodiment of the present disclosure, through a dedifferentiation pathway from a somatic (stem) cell.
[Fig. 5] Fig. 5 is a conceptual diagram showing the antagonism that occurs when phase transitions including differentiation and EMT are caused in parallel.
[Fig. 6] Fig. 6 shows photographs of cells at P2, at which the EGF concentration, added during the manufacture of the human functional corneal endothelial cell of the present disclosure, is set to no addition (-), 0.5 ng / mL, 1 ng / mL, or 5 ng / mL, in one embodiment of the present disclosure.

[Fig. 7] Fig. 7 shows FACS results at P3 at which the EGF concentration added during the manufacture of the human functional corneal endothelial cell of the present disclosure is set to no addition (-) or 0.5 ng/mL, in one embodiment of the present disclosure.

[Fig. 8] Fig. 8 shows FACS results at P3 at which the EGF concentration added during the manufacture of the human functional corneal endothelial cell of the present disclosure is set to 1 ng/mL or 5 ng/mL, in one embodiment of the present disclosure.

[Fig. 9] Fig. 9 shows FACS results at P4 at which the EGF concentration added during the manufacture of the human functional corneal endothelial cell of the present disclosure is set to no addition (-) or 0.5 ng/mL, in one embodiment of the present disclosure.

[Fig. 10] Fig. 10 shows FACS results at P4 at which the EGF concentration added during the manufacture of the human functional corneal endothelial cell of the present disclosure is set to 1 ng/mL or 5 ng/mL, in one embodiment of the present disclosure.

[Fig. 11] Fig. 11 shows FACS results for non-addition (-) or 0.5 ng/mL at P0, in order to investigate the effect of adding EGF from the primary culture during the manufacture of the human functional corneal endothelial cell of the present disclosure, in one embodiment of the present disclosure.

[Fig. 12] Fig. 12 shows FACS results for non-addition (-) at P0 and non-addition (-) or 0.5 ng/mL at P1, in order to investigate the effect of adding EGF from the primary culture during the manufacture of the human functional corneal endothelial cell of the present disclosure, in one embodiment of the present disclosure.

[Fig. 13] Fig. 13 shows FACS results for 0.5 ng/mL at P0 and non-addition (-) or 0.5 ng/mL at P1, in order to investigate the effect of adding EGF from the primary culture during the manufacture of the human functional corneal endothelial cell of the present disclosure, in one embodiment of the present disclosure.

[Fig. 14] Fig. 14 shows FACS results for non-addition (-) at P0 and non-addition (-) or 0.5 ng/mL at P2, in order to investigate the effect of adding EGF from the primary culture during the manufacture of the human functional corneal endothelial cell of the present disclosure, in one embodiment of the present disclosure.

[Fig. 15] Fig. 15 is a schematic diagram showing cases for measuring intracellular gene variation of miR378, miR146, miR34, and miR184.

[Fig. 16] Fig. 16 is a table showing the results of FACS measurement and photographic evaluation in each of the cases with and without EGF and with and without Y27632.

[Fig. 17] Fig. 17 is a graph showing the results of miR184 gene expression variations in cases with and without EGF and with and without Y.

[Fig. 18] Fig. 18 is a graph showing the results of miR34a-5p gene expression variations in cases with and without EGF and with and without Y.

[Fig. 19] Fig. 19 is a schematic diagram showing an example of the hierarchy of metabolites.

[Fig. 20] Fig. 20 is a graph showing the results of confirming the metabolite properties between the human functional corneal endothelial cells of the present disclosure (cells of interest) and cells that are not (non-intended cells), in one embodiment of the present disclosure.

[Fig. 21] Fig. 21 is a graph showing the results of confirming the metabolite properties between the human functional corneal endothelial cells of the present disclosure (cells of interest) and cells that are not (non-intended cells), in one embodiment of the present disclosure.

[Fig. 22] Fig. 22 is a graph showing the results of confirming the metabolite properties between the human functional corneal endothelial cells of the present disclosure (cells of interest) and cells that are not (non-intended cells), in one embodiment of the present disclosure.

[Fig. 23] Fig. 23 is a table showing culture conditions for testing the effects of various additives in one embodiment of the present disclosure.

[Fig. 24] Fig. 24 shows photographs of cells of CT09 at P5 under the conditions 1 and 2 of Fig. 23.

[Fig. 25] Fig. 25 shows photographs of cells of CT09 at P5 under the conditions 3 and 4 of Fig. 23.

[Fig. 26] Fig. 26 shows photographs of cells of CT09 at P5 under the condition 5 of Fig. 23.

[Fig. 27] Fig. 27 shows FACS results under the conditions 1 and 2 of Fig. 23.

[Fig. 28] Fig. 28 shows FACS results under the conditions 3 and 4 of Fig. 23.

[Fig. 29] Fig. 29 shows FACS results under the condition 5 of Fig. 23.

[Fig. 30] Fig. 30 is a culture supernatant and sample list of ELISA PDGF-bb and IL-8, CT09, P4 and P5.

[Fig. 31] Fig. 31 is a graph showing the results of classifying PDGF-bb by additive in one embodiment of the present disclosure.

[Fig. 32] Fig. 32 is a graph showing the results of classifying PDGF-bb by week in one embodiment of the present disclosure.

[Fig. 33] Fig. 33 is a graph showing the results of classifying IL-8 by additive in one embodiment of the present disclosure.

[Fig. 34] Fig. 34 is a graph showing the results of classifying IL-8 by week in one embodiment of the present disclosure.

[Fig. 35] Fig. **35** shows the results of examining mitochondrial respiratory capacity in human functional corneal endothelial cells of the present disclosure, in one embodiment of the present disclosure.

[Fig. 36] Fig. **36** shows the results of examining mitochondrial respiratory capacity in human functional corneal endothelial cells of the present disclosure, in one embodiment of the present disclosure.

[Fig. 37] Fig. **37** shows the results of examining mitochondrial respiratory capacity in human functional corneal endothelial cells of the present disclosure, in one embodiment of the present disclosure.

[Fig. 38] Fig. **38** is a table showing donor information for examining the effects of adding a ROCK inhibitor on human functional corneal endothelial cells of the present disclosure, in one embodiment of the present disclosure.

[Fig. 39] Fig. **39** is a table showing culture conditions for examining the effects of adding a ROCK inhibitor on human functional corneal endothelial cells of the present disclosure, in one embodiment of the present disclosure.

[Fig. 40] Fig. **40** is a table showing additives and timing for supernatant collection, for examining the effects of adding a ROCK inhibitor on human functional corneal endothelial cells of the present disclosure, in one embodiment of the present disclosure.

[Fig. 41] Fig. **41** is a table summarizing FACS results examining the effects of adding a ROCK inhibitor on human functional corneal endothelial cells of the present disclosure, in one embodiment of the present disclosure.

[Fig. 42] Fig. **42** shows photographs of cells examined for the effect of adding a ROCK inhibitor to human functional corneal endothelial cells of the present disclosure, in one embodiment of the present disclosure.

[Fig. 43] Fig. **43** shows FACS results examining the effects of adding a ROCK inhibitor to human functional corneal endothelial cells of the present disclosure, in one embodiment of the present disclosure.

[Fig. 44] Fig. **44** shows FACS results examining the effects of adding a ROCK inhibitor to human functional corneal endothelial cells of the present disclosure, in one embodiment of the present disclosure.

[Fig. 45] Fig. **45** shows FACS results examining the effects of adding a ROCK inhibitor to human functional corneal endothelial cells of the present disclosure, in one embodiment of the present disclosure.

[Fig. 46] Fig. **46** is a graph showing ELISA PDGF-bb measurement results (for each item) in the culture supernatant for #190719 in an embodiment of the present disclosure.

[Fig. 47] Fig. **47** is a graph showing ELISA PDGF-bb measurement results (for each item) in the culture supernatant for #190318 in an embodiment of the present disclosure.

[Fig. 48] Fig. **48** is a graph showing ELISA PDGF-bb measurement results (weekly) in the culture supernatant for #190719 in an embodiment of the present disclosure.

[Fig. 49] Fig. **49** is a graph showing ELISA PDGF-bb measurement results (weekly) in the culture supernatant for #190318 in an embodiment of the present disclosure.

[Fig. 50] Fig. **50** is a graph showing ELISA IL-8 measurement results (weekly) in the culture supernatant for #190719 in one embodiment of the present disclosure.

[Fig. 51] Fig. **51** is a graph showing ELISA IL-8 measurement results (weekly) in the culture supernatant for #190318 in one embodiment of the present disclosure.

[Fig. 52] Fig. **52** is a graph showing ELISA PDGF-bb and IL-8 measurement results (for each item) in the culture supernatant for #190802 in one embodiment of the present disclosure.

[Fig. 53] Fig. **53** is a graph showing the results (for each item) of cytokine measurement (BioPlex) in the culture supernatant for #190318.

[Fig. 54] Fig. **54** is a table showing additive conditions for examining the effects of adding a ROCK inhibitor on human functional corneal endothelial cells of the present disclosure, in one embodiment of the present disclosure.

[Fig. 55] Fig. **55** shows photographs of cells at P4 with and without a ROCK inhibitor for #190719 in one embodiment of the present disclosure.

[Fig. 56] Fig. **56** shows FACS results at P4 with and without a ROCK inhibitor for #190719 in one embodiment of the present disclosure.

[Fig. 57] Fig. **57** shows FACS results at P4 with and without a ROCK inhibitor for #190719 in one embodiment of the present disclosure.

[Fig. 58] Fig. **58** is a graph showing the results for each item of PDGF-bb and IL-8 by ELISA in one embodiment of the present disclosure.

[Fig. 59] Fig. **59** is a graph showing weekly results of PDGF-bb and IL-8 by ELISA in one embodiment of the present disclosure.

[Fig. 60] Fig. **60** shows cell photographs of the results of examining whether adhesion enhancement by ROCK inhibitors is related to cell manufacturing of the present disclosure, in an embodiment of the present disclosure.

[Fig. 61] Fig. **61** shows cell photographs of the results of examining whether adhesion enhancement by ROCK inhibitors is related to cell manufacturing of the present disclosure, in an embodiment of the present disclosure.

[Fig. 62] Fig. **62** shows cell photographs of the results of examining whether adhesion enhancement by ROCK inhibitors is related to cell manufacturing of the present disclosure, in an embodiment of the present disclosure.

[Fig. 63] Fig. **63** shows cell photographs of the results of examining whether adhesion enhancement by ROCK inhibitors is related to cell manufacturing of the present disclosure, in an embodiment of the present disclosure.

[Fig. 64] Fig. **64** is a schematic diagram showing epigenetics regulation by metabolites, cell senescence and disruption of cell differentiation.

[Fig. 65] Fig. **65** is a list of enzymes expressed in human functional corneal endothelial cells (differentiated mature cells) of the present disclosure, in one embodiment of the present disclosure.

[Fig. 66] Fig. **66** is a schematic diagram showing HCEC culture conditions for DAVID analysis in one embodiment of the present disclosure.

[Fig. 67] Fig. **67** shows FACS results at P1 of HCEC for DAVID analysis in one embodiment of the present disclosure.

[Fig. 68] Fig. **68** shows FACS results at P4 of HCEC for DAVID analysis in one embodiment of the present disclosure.

[Fig. 69] Fig. **69** shows photographs of cells at P4 of HCEC for DAVID analysis in one embodiment of the present disclosure.

[Fig. 70] Fig. **70** is a procedure for DAVID analysis of proteomics in one embodiment of the present disclosure.

[Fig. 71] Fig. **71** shows the results of a three-group analysis in one embodiment of the present disclosure.

[Fig. 72] Fig. **72** shows results of GOTERM analysis in one embodiment of the present disclosure.

[Fig. 73] Fig. **73** is a table showing comparative results for mitochondria after DAVID analysis in one embodiment of the present disclosure.

[Fig. 74] Fig. **74** is a table showing comparative clustering results for mitochondria after DAVID analysis in one embodiment of the present disclosure.

[Fig. 75] Fig. **75** is a table showing comparative clustering results for mitochondria after DAVID analysis in one embodiment of the present disclosure.

[Fig. 76] Fig. **76** is a schematic diagram comparing protein expression intensities of enzymes and substrates involved in metabolic pathways between human functional corneal endothelial cells (standard cells) and non-standard cells, of the present disclosure, in one embodiment of the present disclosure.

[Fig. 77] Fig. **77** is a schematic diagram comparing protein expression intensities of enzymes and substrates involved in metabolic pathways between human functional corneal endothelial cells (standard cells) and non-standard cells, of the present disclosure, in one embodiment of the present disclosure.

[Fig. 78] Fig. **78** is a schematic diagram comparing protein expression intensities of enzymes and substrates involved in metabolic pathways between human functional corneal endothelial cells (standard cells) and non-standard cells, of the present disclosure, in one embodiment of the present disclosure.

[Fig. 79] Fig. **79** is a schematic diagram comparing protein expression intensities of enzymes and substrates involved in metabolic pathways between human functional corneal endothelial cells (standard cells) and non-standard cells, of the present disclosure, in one embodiment of the present disclosure.

[Fig. 80] Fig. **80** is a schematic diagram comparing protein expression intensities of enzymes and substrates involved in metabolic pathways between human functional corneal endothelial cells (standard cells) and non-standard cells, of the present disclosure, in one embodiment of the present disclosure.

[Fig. 81] Fig. **81** is a schematic diagram comparing protein expression intensities of enzymes and substrates involved in metabolic pathways between human functional corneal endothelial cells (standard cells) and non-standard cells, of the present disclosure, in one embodiment of the present disclosure.

[Fig. 82] Fig. **82** is a schematic diagram comparing protein expression intensities of enzymes and substrates involved in metabolic pathways between human functional corneal endothelial cells (standard cells) and non-standard cells, of the present disclosure, in one embodiment of the present disclosure.

[Fig. 83] Fig. **83** is a schematic diagram comparing protein expression intensities of enzymes and substrates involved in metabolic pathways between human functional corneal endothelial cells (standard cells) and non-standard cells, of the present disclosure, in one embodiment of the present disclosure.

[Fig. 84] Fig. **84** is a list of antigens used to compare the protein expression intensities of enzymes and substrates involved in metabolic pathways between human functional corneal endothelial cells (standard cells) and non-standard cells, of the present disclosure, in one embodiment of the present disclosure.

[Fig. 85] Fig. **85** shows photographs showing the results of cell staining for investigating the ion channel and/or monocarboxylic acid transport system, in one embodiment of the present disclosure.

[Fig. 86] Fig. **86** shows photographs showing the results of cell staining for investigating the ion channel and/or monocarboxylic acid transport system, in one embodiment of the present disclosure.

[Fig. 87] Fig. **87** shows photographs showing the results of cell staining for investigating the ion channel and/or monocarboxylic acid transport system, in one embodiment of the present disclosure.

[Fig. 88] Fig. **88** shows photographs showing the results of cell staining for investigating the ion channel and/or monocarboxylic acid transport system, in one embodiment of the present disclosure.

[Fig. 89] Fig. **89** is a graph showing FACS results and intracellular pH at P2 for #190719 (standard cells) in one embodiment of the present disclosure.

[Fig. 90] Fig. **90** shows a photograph of cells at P2 for #190719 (standard cells) in one embodiment of the present disclosure.

[Fig. 91] Fig. **91** is a graph showing FACS results and intracellular pH at P3 for #190802 (non-standard cells) in one embodiment of the present disclosure.

[Fig. 92] Fig. **92** shows a photograph of cells at P3 for #190802 (non-standard cells) in one embodiment of the present disclosure.

[Fig. 93] Fig. **93** is a graph showing the results of comparing intracellular pH between #190719 (standard cells) and #190802 (non-standard cells) in one embodiment of the present disclosure.

[Fig. 94] Fig. **94** shows FACS and cell photographs showing the results of investigating the effects of additives in one embodiment of the present disclosure.

[Fig. 95] Fig. **95** is a schematic diagram showing culture conditions for investigating the effects of additives in one embodiment of the present disclosure.

[Fig. 96] Fig. **96** shows FACS results for cells under each culture condition, in one embodiment of the present disclosure.

[Fig. 97] Fig. **97** shows FACS results for cells under each culture condition, in one embodiment of the present disclosure.

[Fig. 98] Fig. **98** shows FACS results for cells under each culture condition, in one embodiment of the present disclosure.

[Fig. 99] Fig. **99** is a graph showing cytokine measurement results in cells under each culture condition, in one embodiment of the present disclosure.

[Fig. 100] Fig. **100** is a graph showing cytokine measurement results in cells under each culture condition.

[Fig. 101] Fig. **101** is a schematic diagram illustrating enhancement of mitochondrial oxidative phosphorylation respiration.

[Fig. 102] Fig. **102** is a graph showing enhancement of mitochondrial oxidative phosphorylation respiration in one embodiment of the present disclosure.

[Fig. 103] Fig. **103** is a schematic diagram showing the relationship between enhancement of mitochondrial oxidative phosphorylation respiration and clinical pharmacological effects.

[Fig. 104] Fig. **104** is a schematic diagram showing the induction of differentiated and matured, human functional corneal endothelial cells through the dedifferentiation pathway from somatic (stem) cells.

[Fig. 105] Fig. **105** is a conceptual diagram explaining that mitochondrial function is influenced by intracellular pH, and the differentiation/dedifferentiation state of cells is defined.

[Fig. 106] Fig. **106** shows FACS results and photographs of #CR04 (standard cells) at P3 in one embodiment of the present disclosure.

[Fig. 107] Fig. **107** shows the results of confirming selective expression of ion channels by immunostaining of cells, in one embodiment of the present disclosure.

[Fig. 108] Fig. **108** shows results showing enhancement of histone acetylation in non-standard cells in one embodiment of the present disclosure.

[Fig. 109] Fig. **109** shows the results of immunoblotting on standard and non-standard cells, in one embodiment of the present disclosure.

[Fig. 110] Fig. **110** shows FACS results and photographs of #191224S (standard cells) at P4 in one embodiment of the present disclosure.

[Fig. 111] Fig. **111** shows FACS results and photographs of #200313 (non-standard cells) at P1 in one embodiment of the present disclosure.

[Fig. 112] Fig. **112** shows FACS results and photographs of #191224S (standard cells) at P4 in one embodiment of the present disclosure.

[Fig. 113] Fig. **113** shows FACS results and photographs of #191224S (standard cells) at P4 in one embodiment of the present disclosure.

[Fig. 114] Fig. **114** is a graph showing measurement results of HAT/HDAC activity in one embodiment of the present disclosure.

[Description of Embodiments]

The embodiment of the present disclosure is disclosed hereinafter. To avoid complicating the disclosure with repeating the same content, explanation is appropriately omitted. Throughout the entire specification, a singular expression should be understood as encompassing the concept thereof in the plural form, unless specifically noted otherwise. Thus, singular articles (e.g., "a", "an", "the" and the like in case of English) should also be understood as encompassing the concept thereof in the plural form unless specifically noted otherwise. Further, the terms used herein should be understood to be used in the meaning that is commonly used in the art, unless specifically noted otherwise. Thus, unless defined otherwise, all terminologies and scientific technical terms that are used herein have the same meaning as the general understanding of those skilled in the art to which the present disclosure pertains. In case of a contradiction, the present specification (including the definitions) takes precedence.

First, the terms and common techniques used in the present disclosure are explained.

As used herein, "about" means ± 10% of the value that follows.

As used herein, "corneal endothelium" and "human corneal endothelium" are used in the meaning that is commonly used in the art. The cornea is one of the lamellar tissues constituting an eye. A cornea is transparent and positioned at a part closest to the external environment. In humans, it is understood that the cornea is comprised of five layers, in order from the outside (body surface), of corneal epithelium, Bowman's membrane (external boundary), Lamina propria, Descemet's membrane (internal boundary), and corneal endothelium. Unless specifically noted otherwise, parts other than epithelium and endothelium may be collectively called "corneal stroma", which is also called as such herein.

As used herein, a cell desired from corneal endothelial tissue is referred to as "corneal endothelial tissue derived cell". Further, a cell that becomes a corneal endothelial cell by differentiation is referred to as a "corneal endothelial progenitor cell".

As used herein, "human functional corneal endothelial cell capable of eliciting a human corneal function when infused into an anterior chamber of a human eye" is a cell that has the ability to elicit the function of the cornea, having the ability to elicit a human corneal function (which is referred to as "human, human corneal function" when referring to humans; although not particularly limited, it is simply referred to as "human corneal function" herein) when infused into an anterior chamber of a human eye. The term, "capable of eliciting a human corneal function", may encompass capability to elicit a corneal endothelial functional property (such as having efficacy leading to improvement on corneal opacity and hydrous edema, resulting in continuous and long-term retention of corneal endothelial tissue cell density and improvement on visual acuity).

As used herein, "human functional corneal endothelial cell capable of eliciting a corneal endothelial functional property when infused into an anterior chamber of a human eye" refers to "cell with functionality of a corneal endothelium, having the ability to express a corneal endothelial functional property (referred to as "human corneal endothelial functional property" when referring to humans, or simply referred to hereinafter although not especially limiting, as "corneal endothelial functional property") when infused into the anterior chamber of a human eye. As a typical function, the cell has efficacy leading to improvement on corneal opacity and hydrous edema, resulting in continuous and long-term retention of corneal endothelial tissue cell density and improvement on visual acuity. This is also referred to as the "corneal endothelial property possessing functional cell of the present disclosure" especially for abbreviation. When used for a human cell, this is referred to as "human functional corneal endothelial cell capable of eliciting a human corneal endothelial functional property when infused into an anterior chamber of a human eye". Since the present disclosure is mainly concerned with human corneal cells, it is understood that a human cell is referred unless specifically noted otherwise. As used herein, the corneal endothelial property possessing functional cells of the present disclosure encompass "functional mature differentiated corneal endothelial cell" having a corneal endothelial functional property as such without further processes and "intermediately differentiated corneal endothelial cell", which lacks some of the functions, but is used similarly or exert the same function as a functional mature differentiated corneal endothelial cell after infusion.

As used herein, "corneal endothelial (cell) functional property" refers to a (cell) functional property useful in retention of visual function that corneal endothelial cells present in corneal endothelial tissue have in a normal state. As used herein, the corneal endothelial cell functional property and the corneal endothelial functional property have the same meaning, and *in vitro* discussions often refer to the former.

As used herein, "functional mature differentiated corneal endothelial cell" refers to a mature differentiated corneal endothelium cell present in healthy human corneal endothelial tissue and any cell having its function (e.g., the above-described corneal endothelial (cell) functional property). This is referred to as a functional mature differentiated human corneal endothelial cell for human cells. In particular, a corneal endothelial (cell) functional property can be confirmed from forming a small hexagonal cobble-stone shape and using an energy metabolism system by mitochondrial function, and from retaining a trait leading to improvement on corneal opacity and hydrous edema, resulting in continuous and long-term retention of corneal endothelial tissue cell density and improvement on visual acuity. It is possible to determine whether the property can have a therapeutic effect when infused (e.g., into the anterior chamber of a human eye). However, this is not limited thereto. A corneal endothelial functional property can be judged or determined by using a surrogate marker as an indicator. Judgment can be made by any one of the 10 types of such surrogate markers or a combination thereof, including (1) retention of endothelial pumping/barrier functions (including Claudin expression), (2) adhesion/attachment to a specific laminin, (3) secreted cytokine profile, (4) produced micro RNA (miRNA) profile, (5) produced metabolite profile, (6) expression of ion channel or monocarboxylic acid transporter leading to the above-mentioned corneal endothelial (cell) functional property leading to improvement on corneal opacity and hydrous edema, resulting in continuous and long-term retention of corneal endothelial tissue cell density and improvement on visual acuity, (7) a property that metabolic enzymes related to the TCA cycle, etc., which leads to the production of phase transition cells, are not present in the cytoplasm or nucleus and are organelle-selectively localized in mitochondria, (8) saturated cell density upon *in vitro* culture, (9) spatial size and distribution of cells obtained in culturing, and (10) cell retention in case of cell infusion after freeze damage cryo treatment by liquid nitrogen on a mouse cornea.

(1) Retention of endothelial pumping/barrier functions can be judged by using a pumping function measuring method or a barrier function measuring method commonly used for corneal endothelia. Examples of such judgment include techniques of applying the methods described in Wigham C, Hodson S.: Current Eye Research, 1, 37-41, 1981, Hodson S, Wigham C.: J Physiol., 342:409-419, 1983, Hatou S., Yamada M., Akune Y., Mochizuki H., Shiraishi A., Joko T., Nishida T., Tsubota K.: Investigative Ophthalmology & Visual Science, 51, 3935-3942, 2010 by using a Ussing chamber utilized in case of a sheet form. Claudin expression can be confirmed by using a known approach in the art such as immunological approach. Any immunological approach known in the art can be used to confirm Claudin expression. However, the cells of the present disclosure are expected to be infused in a suspension. In such a case, it is thus preferable to assess a corneal endothelial function by applying Claudin expression, or any one of (2)-(10) or a combination thereof.

(2) Adhesion/attachment to a specific laminin can be judged using adhesion to laminin 511 (composite of alpha5 chain, beta1 chain, and gamma1 chain), laminin 521 (composite of alpha5 chain, beta2 chain, and gamma1 chain), or a functional fragment thereof (e.g., laminin 511-E8 fragment) and/or increase in integrin (e.g., alpha3beta1, alpha6beta1 or the like) expression with respect thereto as an indicator. Such an approach can be implemented by a cell adhesion assay.

In this regard, laminin alpha chains are discussed. "alpha5 chain" (LAMA5) is a subunit of a protein-laminin of a cell adhesion molecule in an extracellular matrix, and is called LAMA5, KIAA1907, or the like. For human LAMA5, the sequences of the gene and protein are registered as NCBI registration numbers NM_005560 and NP_005551, respectively. OMIM is identified by the accession number 601033. Laminin beta chains are discussed. "beta1 chain" (LAMB1) is a subunit of a protein·laminin of a cell adhesion molecule in an extracellular matrix, and is called LAMB1, CLM, LIS5, or the like. For human LAMB1, the sequences of the gene and protein are registered as NCBI registration numbers NM_002291 and NP_002282, respectively. OMIM is identified by the accession number 150240. "beta2 chain" (LAMB2) (laminin S) is a subunit of a protein·laminin of a cell adhesion molecule in an extracellular matrix, and is called LAMB2, LAMS, NPHS5, or the like. For human LAMB2, the sequences of the gene and protein are registered as NCBI registration numbers NM_002292 and NP_002283, respectively. OMIM is identified by the accession number 150325. Laminin gamma chains are discussed. "gamma1 chain" (LAMC1) is a subunit of a protein·laminin of a cell adhesion molecule in an extracellular matrix, and is called LAMC1, LAMB2, or the like. For human LAMC1, the sequences of the gene and protein are registered as NCBI registration numbers NM_002293 and NP_002284, respectively. OMIM is identified by the accession number 150290.

(3) Secreted cytokine profiles can be judged by measuring the production level of cytokines profiles in "serum" or "anterior aqueous humour" explained elsewhere herein. Such cytokines include, but are not limited to, RANTES, PDGF-BB, IP-10, MIP-1b, VEGF, EOTAXIN, IL-1ra, IL-6, IL-7, IL-8, IL-0, IL-10, IL-12 (p70), IL-13, IL-17, FGFbasic, G-CSF, GM-CSI, IFN-gamma, MCP-1, MIP-1a, TNF-alpha, and the like. Specifically, analysis can be performed using a cytokine measuring kit and analysis system such as Bio-Plex for integrated analysis of cytokines.

(4) The produced microRNA (miRNA) profile can be judged by measurement using the analytical approach for "miRNA profile" explained elsewhere herein. For instance, judgment can be materialized by using a method of analyzing a microRNA expression profile. For example, Toray's "3D-Gene" human miRNA oligochip (miRBase version 17) can be used for the implementation thereof. Total RNAs obtained from samples of both tissue and cells, which is labeled with total miRNA obtained from supernatant and those labeled with a label such as Hy5 by using a kit such as miRCURY LNA(R) microRNA Power Labeling Kits (Exiqon, Vedbaek, Denmark) are prepared. Labeled microRNA is separately hybridized to the surface of a microRNA chip and incubated under a suitable condition (e.g., 32°C for 16 hours) . After this microRNA chip is washed and dried in an ozone-free environment, a scanner such as 3D-Gene scanner 3000 (Toray Industries Inc., Tokyo, JAPAN) can be used for scanning, and 3D-Gene Extraction software (Toray) can be used for analysis.

(5) As for the produced metabolite profile, for example, the metabolic extract of an intracellular metabolite is prepared from a cHCEC culture container having methanol containing an internal standard reagent such as Internal Standard Solution (Human Metabolome Technologies; HMT, Inc., Tsuruoka, Japan). The medium is replaced and cell extract is treated, and CE-MS analysis is preformed to analyze the metabolite. Metabolome analysis can be measured according to the method developed by Soga, et al. (Soga, D. et al., T. Soga, et al., Anal. Chem. 2002; 74: 2233-2239 Anal. Chem. 2000; 72: 1236-1241; T. Soga, et al., J. Proteome Res. 2003; 2: 488-494) and automatic integration software (MasterHands, Keio University, Tsuruoka, Japan (M. Sugimoto, et al., Metabolomics, 2009; 6: 78-95) and MassHunter Quantitative Analysis B.04.00, Agilent Technologies, Santa Clara, CA, USA) is appropriately used for analysis. From the HMT metabolite database, the peak is annotated by a hypothetical metabolite, standardized, and calculated based on the m/z value measured by MT and TOFMS in CE. Hierarchical cluster analysis (HCA) and principal component analysis (PCA) can be performed for metabolome measurement.

(6) Expression of ion channel or monocarboxylic acid transporter leading to the above-mentioned corneal endothelial (cell) functional property leading to improvement on corneal opacity and hydrous edema, resulting in continuous and long-term retention of corneal endothelial tissue cell density and improvement on visual acuity, can be measured using any approaches described herein or any known approaches. For example, as described in Examples, the expression can be measured by immobilizing cells obtained from corneal tissue, immunostaining them with a specific antibody, and observing them with a fluorescence microscope or the like. Besides, the expression of the ion channel or monocarboxylic acid transporter can also be measured by measuring the function thereof.

(7) The property that metabolic enzymes related to the TCA cycle, etc., which leads to the production of phase transition cells, are not present in the cytoplasm or nucleus and are organelle-selectively localized in mitochondria, can also be measured using any approaches described herein or any known approaches. For example, without limitation, it is possible observe the localization of metabolic enzymes organelle-selectively in mitochondria by applying DAVID analysis or the like.

(8) The saturated cell density during *in vitro* culture can be judged by measuring the cell density by using appropriate culture conditions described herein. This may be measured in parallel with the cell size. Photo-taking phase contrast microscope images are taken using an equipment comprising an image capturing system such as a BZ X-700 Microscope system (Keyence, Osaka, Japan) by an inverted microscope system (CKX41, Olympus, Tokyo, Japan). The density can be quantified by using a cell counting software (e.g., BZ-H3C Hybrid cell count software (Keyence)). Preferred saturated cell density in the present disclosure is described elsewhere herein.

(9) The spatial size and distribution of cells obtained in culture can be judged by taking pictures of cells and taking measurements with any software or the like or by measuring the special size and distribution of cells by using appropriate culture conditions described herein. This can be materialized by using raw image processing software such as BZ-H3C Hybrid cell count software (Keyence). The preferred saturated cell density in the present disclosure is described elsewhere herein.

(10) Cell retention in case of cell infusion after freeze damage with cryo treatment by liquid nitrogen on mouse cornea can be judged by making a mouse model. Specifically, the center region (e.g., 2mm) of a cornea of a suitable mouse (e.g., BALB/c) is pretreated by low temperature damage to remove an endothelial cell to make a model. The cell to be judged is injected into the ocular anterior chamber of the model. The characteristics of the corneal clarity are clinically observed. The corneal thickness is assessed by a pachymeter. The adhesion of HCECs is histopathologically tested by human nuclear staining and the cells are examined whether they have a function.

A cell that is not such a cell that is derived from corneal endothelium (including a cell obtained by dedifferentiation of corneal endothelial tissue) (e.g., cell produced by having induced pluripotent stem cell (iPS cell), embryonic stem cell (ES cell) or the like differentiate into corneal endothelia) is within the scope of the functional corneal endothelial cell or functional mature differentiated corneal endothelial cell capable of eliciting a corneal endothelial functional property when infused into the anterior chamber of a human eye of the present disclosure, as long as it has the corneal endothelial functional property described in the present disclosure. In the explanation or experiment set forth herein, the human functional mature differentiated corneal endothelial cell of the present disclosure is also called "human functional mature differentiated corneal endothelial cells", "functional mature differentiated human corneal endothelial cell", "functional cell" "cells of interest", "standard cell" or the like, all of which are used synonymously.

As used herein, "corneal endothelial nonfunctional cell" is a cell other than the corneal endothelial property possessing functional cell of the present disclosure (i.e., "functional mature differentiated corneal endothelial cell"). Such a cell may be called "non-intended cell", "non-qualified cell", "unintended cell", "nonfunctional cell", "non-standard cell" or the like.

As used herein, "cell indicator" refers to any indicator indicating that a certain cell is the corneal endothelial property possessing functional cell of the present disclosure (e.g., functional mature differentiated corneal endothelial cell). Since a cell indicator is a property of mature differentiated human corneal endothelia and any cell with the function thereof, it is also referred to as "functional cell indicator". The specific property is also referred to as the "cell functional property".

As used herein, "culture condition capable of minimizing culture stress" refers to any culture condition capable of minimizing (making least) stress during culture related to cells such as proliferation stress. The "culture stress" can be measured by using, as an index, whether or not a mixture of large and atypical cells having undergone a phase transition is recognized in an amount of 5 to 20% or more by observation with a phase-contrast microscope, and/or by using, as an index, whether or not the cell density from the 30th day to the 40th day of culture is below the 1000•mm$^2$ level. If preliminary experiments on a certain donor show that the index can minimize culture stress, that condition can be applied in the actual cell preparation and administration. Such a culturing condition can be achieved, for example, by culturing with the amount of cell growth factor, e.g., epidermal growth factor, less than the amount at which transformation occurs, but is not limited thereto. Although this amount may vary in accordance with the age of the donor, the cell density at the time of cell seeding, the concentration of the growth factor added, and the like, those skilled in the art can determine such a condition (e.g., amount, etc.) in consideration of various available information, donor information, and the like.

As used herein, "transformation" refers to the traits of a cell to change to an abnormal state, including the meaning of normal cells to divide themselves indefinitely, i.e., canceration, or the meaning of particularly dynamic cells in metaplasia (cells that dedifferentiate to stem cells or change beyond the basic wall of tissue). Examples of transformation include cell state transition (CST) such as EMT, fibrosis, epithelial mesenchymal transition, senescence, dedifferentiation and endothelial-mesenchymal transition. A corneal endothelial cell often undergoes transformation such as epithelial mesenchymal transition such that it is no longer a functional mature differentiated corneal endothelial cell in many cases. Alternatively, the transformation includes endothelial-mesenchymal transition. The manufacturing method of the present disclosure encompasses manufacturing methods that can convert such a cell which has undergone epithelial mesenchymal transition or endothelial-mesenchymal transition into a functional mature differentiated corneal endothelial cell by allowing such a cell to mature and differentiate after dedifferentiation.

As used herein, "less than the amount at which transformation occurs" refers to none, or an amount less than the amount at which transformation (e.g., endothelial-mesenchymal transition) of a corneal endothelial cell of interest occurs, when referring to cell growth factors, etc. When a cell growth factor of an amount less than the amount at which transformation occurs is used, it is a feature of the present disclosure not to elicit transformation including endothelial-mesenchymal transition due to proliferation stress. For example, when EGF is used as a cell growth factor, the amount less than the amount at which transformation occurs is less than about 1 ng/mL, preferably less than about 0.5 ng/mL, and more preferably 0 ng/mL.

As used herein, "endothelial-mesenchymal transition" (EndMT) refers to the transformation of endothelial cells into mesenchymal cells.

As used herein, "epithelial mesenchymal transition" (EMT: also referred to as epithelial mesenchymal transformation) refers to a process in which epithelial cells lose their cell polarity and cell adhesion function with surrounding cells, and become mesenchymal-like cells by gaining migration and infiltration ability.

As used herein, "cell growth factor" is a general term for proteins that promote the growth and proliferation of specific cells in the body of an animal, and is sometimes synonymous with "proliferation factor" and "cell proliferation factor". Examples thereof include an epidermal growth factor (EGF), a fibroblast growth factor (FGF), an insulin-like growth factor (IGF), and a transforming growth factor (TGF and the like) . One of the features of the present disclosure may be that growth factors that give cell stress called proliferation stress are not added to cells during culturing. The intensity of stress varies in accordance with the concentration of addition, the time period of addition, and the timing of addition.

In this regard, starting cells in various samples and manufacturing methods that can be used herein may be functional mature differentiated corneal endothelial cells, cells of interest, or sample considered as comprising a substance derived therefrom that enables gene expression. For example, a cell directly isolated from a corneal endothelium (also referred to as corneal endothelial tissue derived cells) or cell that has acquired a corneal endothelial-like function from differentiation can be used. A corneal endothelial tissue derived cell can be obtained by a known method (Koizumi N, Okumura N, Kinoshita S., Experimental Eye Research. 2012; 95: 60-7). Preferably, a cell and the like obtained from a corneal endothelium donor can be used as a cell sample. Further, cultured cells comprising the corneal endothelial property possessing functional cells of the present disclosure or functional mature differentiated corneal endothelial cells, which were differentiated and induced *in vitro,* can be used as the sample. The cells can be differentiated and induced *in vitro* into the corneal endothelial property possessing functional cells of the present disclosure or functional mature differentiated corneal endothelial cells by processing with a known method such as the AMED method or the like <Ueno M, Matsumura M, Watanabe K, Nakamura T, Osakada F, Takahashi M, Kawasaki H, Kinoshita S, Sasai Y:, Proc Natl Acad Sci USA. 103(25): 9554-9559, 2006.> while using a known cell such as ES cell, iPS cell, or bone marrow stromal cell as the starting material.

For use herein, typically, expressions such as "high expression", "intermediate expression", and "low expression" of miRNA, which are CD44 negative to weakly positive CD24 negative CD26 negative, are used to relatively express the expression intensity of cells. It should be noted that there may be no "intermediate expression", and in such a case, each cell can be identified with "high expression" and "low expression".

As used herein, "cell size" is one of the cell indicators of the corneal endothelial property possessing functional cell of the present disclosure, which is measured by techniques that are commonly used in the art. The cell size is expressed, for example, by cell area. As used herein, "cell area" is one of the cell indicators of the corneal endothelial property possessing functional cell of the present disclosure, and such a cell area can be measured with any software or the like by taking a picture of a cell. Examples of such a measuring approach include a method utilizing image processing software such as BZ-H3C Hybrid cell count software (Keyence). The mean value thereof is referred to as "mean cell area". The arithmetic mean is generally used.

As used herein, "cell density" or "(mean) cell density" is an indicator of a cell expressed by the number of cells present in a certain area. Cell density is measured by any technique that is commonly used in the art. The mean density of a cell population is one of the cell indicators of the corneal endothelial property possessing functional cell of the present disclosure or functional mature differentiated corneal endothelial cell. Arithmetic mean is generally used as the mean. Cell density may be measured in parallel with the cell size and quantified by taking photo-taking phase contrast microscope images using an equipment comprising an image capturing system such as a BZ X-700 Microscope system (Keyence, Osaka, Japan) by an inverted microscope system (CKX41, Olympus, Tokyo, Japan) and using a cell counting software (e.g., BZ-H3C Hybrid cell count software (Keyence)) or the like. The cell density as of saturated cell culture (also referred to as (culture) confluence; saturated cell culture and (culture) confluence as used herein have the same meaning) is used as an indicator. In addition, density as of seeding is also used as a benchmark in the manufacturing method of the present disclosure. Further, cell density may be used as an indicator of a therapeutic result after infusion.

As used herein, "cellular metabolite" refers to any metabolite produced by a cell. "Related biological material of cellular metabolite (the metabolite)" refers to any biological material related to a cellular metabolite (e.g., enzyme that synthesizes the metabolite, metabolizing enzyme, protein associated with a signaling pathway, or the like), which is one of the cell indicators of the corneal endothelial property possessing functional cell of the present disclosure or functional mature differentiated corneal endothelial cell. Examples of metabolites include any product related to products of energy metabolism system in a mitochondrial system, glutathione metabolic system product, methionine metabolic cycle product, lipid metabolite, pentose phosphate pathway product, tricarboxylic acid (TCA) cycle metabolite, glycolytic system metabolite and the like. Tricarboxylic acid (TCA) cycle metabolite and glycolytic system metabolite are especially important. Examples of cellular metabolites and related biological material of the metabolite include succinic acid, Pro, Gly, glycerol 3-phosphate, Glu, lactic acid, arginosuccinic acid, xanthine, N-carbamoyl aspartic acid, isocitric acid, cis-aconitic acid, citric acid Ala, 3-phosphoglyceric acid, hydroxyproline, malic acid, uric acid, betaine, folic acid, Gln, 2-oxoisovaleric acid, pyruvic acid, Ser, hypoxanthine, Asn, Trp, Lys, cholin, Tyr, urea, Phe, Met, carnosine, Asp, ornithine, Arg, creatine, 2-hydroxy glutaminic acid, beta-Ala, citrulline, Thr, Ile, Leu, Val, creatinine, His, and N,N-dimethyl glycine.

Detection, identification, quality control and the like of the cell of the present disclosure can be materialized by using an interactive molecule or substance that binds to a substance used as a marker. In the context of the present disclosure, "interactive molecule" or "substance binding to" a substance used as a marker, is a molecule or substance that at least temporarily binds to a molecule such as a substance to be used as a marker (e.g., CD44) and preferably is capable of indicating that the molecule or substance is bound (e.g., labeled or capable of being labeled). A substance that binds a molecule such as CD44 may be a ligand of a molecule such as CD44. Examples thereof include antibodies, antisense oligonucleotides, siRNA, low molecular weight molecules (LMW), binding peptides, aptamers, ribozymes, peptidomimetics and the like, including binding proteins or binding peptide directed to molecules such as CD44 and nucleic acids directed to a gene of a molecule such as CD44. As used herein, "binding protein" or "binding peptide" for a molecule such as CD44 refers to types of proteins or peptides that bind to a molecule such as CD44, including, but not limited to, polyclonal antibodies or monoclonal antibodies directed to a molecule such as CD44, antibody fragments and protein backbones.

As used herein, "reduction", "decrease" or "suppression" of activity or expression product (e.g., protein, transcript (RNA or the like)) or synonyms thereof refers to: a reduction or a decrease in the amount, quality or effect of a specific activity, transcript or protein; or activity that decreases the same. Among decreases, "elimination" refers to activity, expression product or the like being less than the detection limit and especially referred to as "elimination". As used herein, "elimination" is encompassed by "reduction", "decrease" or "suppression". In addition, "reduction" includes eliciting no expression at all as well as decreasing in the amount or effect of some gene from the state in which it is already expressed.

As used herein, "increase" or "activation" of activity or expression product (e.g., protein, transcript (RNA or the like)) or synonyms thereof refers to: an increase in the amount, quality or effect of a specific activity, transcript or protein; or activity that increases the same. "Increase" also encompasses being elicited (from nothing to existence) in the absence of an amount, quality or effect of a particular activity, transcript or protein, in addition to the presence of an amount, quality or effect of a specific activity, transcript or protein in a state before comparison (a relative increase in the amount already present).

As used herein, "mitochondria dependent oxidative phosphorylation" is also called "mitochondria OXPHOS" and refers to a reaction of oxidative phosphorylation that depends on respiratory activity. As to the reaction associated with "mitochondria OXPHOS", a extracellular flux analyzer, which analyzes the state of intracellular metabolism, can measure the oxygen consumption rate (OCR), which is an index of OXPHOS activity, the extracellular acidification rate (ECAR), which is an index of glycolytic activity.

As used herein, "expression of acetyl-CoA in cytoplasm or nucleus" refers to acetyl-CoA being expressed in the cytoplasm or nucleus. According to the present specification, the expression of acetyl-CoA can be measured by any known approach for analyzing the expression of proteins or by cell immunostaining using an antibody.

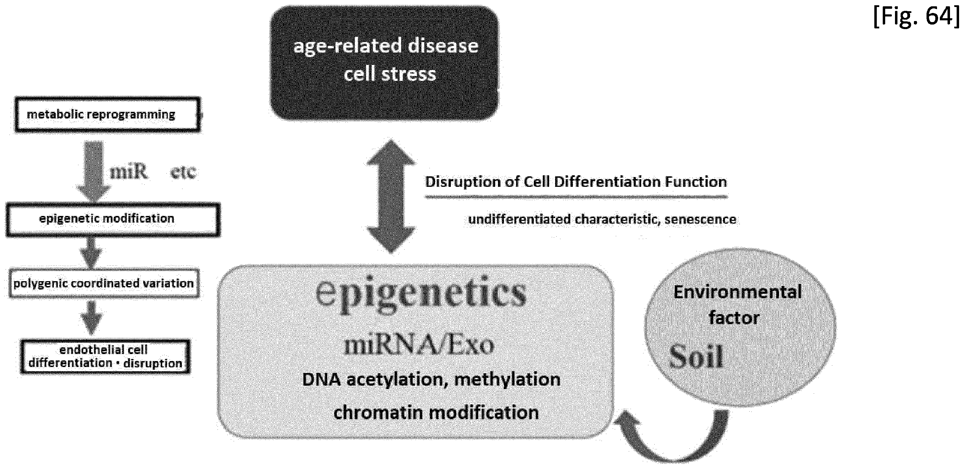

As used herein, "epigenetic multigene expression through histone acetylation by acetyl-CoA" means that the expression of various kinds of genes is regulated by acetylation of histones by acetyl-CoA. Expression of these multigenes causes cell phase transition CST. According to the present specification, epigenetic multigene expression can be measured by approaches as disclosed in, for example, CellMetab. 2015 Mar 3;21(3):349-50., Trendsin Cell Biology, June 2017, Vol.27, No.6 and Sheikhet al. Nature Rev. Genetics, 2019. Furthermore, as to epigenetics regulation by metabolites, cell senescence and disruption of cell differentiation, it is possible to consider them as shown in the schematic diagram of Fig. **64**.

As used herein, "functional protein leading to a corneal endothelial (cell) functional property leading to improvement on corneal opacity and hydrous edema, resulting in continuous and long-term retention of corneal endothelial tissue cell density and improvement on visual acuity" is also referred to as "corneal endothelial (cell) functional property-related functional protein" or "functional protein (of the present disclosure)", and refers to any protein having a function leading to improvement on corneal opacity and hydrous edema, resulting in continuous and long-term retention of corneal endothelial tissue cell density and improvement on visual acuity. It is understood that the above term encompasses any proteins as described herein, such as AQP1, Na-KATPase and NHE1, as well as other proteins. It is understood that the functional protein also encompasses, for example, sodium/hydrogen exchanger 1 (NHE1) and/or aquaporin 1 (AQP-1), and bicarbonic anhydrase 5B (CA5B) (increased expression) as well as citrate synthase (CS), aconitase 2 (ACO2), isocitrate dehydrogenase 2 (IDH2), malate dehydrogenase 2 (MDH2), malic enzyme 3 (ME3), ACSS1, acetyl-CoA acetyltransferase 1 (ACAT1), pyruvate dehydrogenase (PDH), BCAT2, branched-chain ketoacid dehydrogenase 2 (BCKDH2) and the like, in mitochondria.

The "expression" of "functional protein" encompasses: in addition to the expression of the "functional protein", the increase in mitochondria-dependent oxidative phosphorylation in mitochondria; and the absence of elicitation of the expression of acetyl-CoA in the cytoplasm and nucleus and the epigenetic multigene expression through histone acetylation by acetyl-CoA.

In addition, the "expression" of "functional protein" encompasses, in addition to the expression of the "functional protein", the reduction in, or the absence of elicitation of, the expression of protein with a function opposite to that of the "functional protein". Examples thereof encompass no expression, or almost no expression, of ATP citrate lyase (ACLY), aconitase 1 (ACO1), isocitrate dehydrogenase 1 (IDH1), malate dehydrogenase 1 (MDH1), malic enzyme 1 (ME1), ACSS2, acetyl-CoA acetyltransferase 2 (ACAT2) and/or lactate dehydrogenase (LDH).

In this regard, not substantially or hardly "expressed" can be judged by whether or not a human corneal endothelial functional property can be elicited when used in the invention according to the present disclosure.

In view of the foregoing, "expression of a functional protein leading to a corneal endothelial (cell) functional property leading to improvement on corneal opacity and hydrous edema, resulting in continuous and long-term retention of corneal endothelial tissue cell density and improvement on visual acuity is recognized" may also be expressed as "expression of a functional protein leading to a corneal endothelial (cell) functional property leading to improvement on corneal opacity and hydrous edema, resulting in continuous and long-term retention of corneal endothelial tissue cell density and improvement on visual acuity is recognized, or a protein that inhibits the corneal endothelial (cell) functional property is not elicited or reduced".

In the present disclosure, "Rho kinase" or "ROCK" (Rho-associated coiled-coil forming kinase: Rho-bound kinase) refers to serine/threonine kinase which is activated with activation of Rho. Examples thereof include ROKalpha (ROCK-II: Leung, T. et al., J. Biol. Chem., 270, 29051-29054, 1995), p160ROCK (ROKbeta, ROCK-I: Ishizaki, T. et al., The EMBO J., 15(8), 1885-1893, 1996) and other proteins having serine/threonine kinase activity.

Examples of ROCK inhibitors (which are also referred to as Rho kinase inhibitors) include compounds disclosed in the following documents: US Patent No. 4678783, Japanese Patent No. 3421217, International Publication No. WO 95/28387, International Publication No. WO 99/20620, International Publication No. WO 99/61403, International Publication No. WO 02/076976, International Publication No. WO 02/076977, International Publication No. WO 2002/083175, International Publication No. WO 02/100833, International Publication No. WO 03/059913, International Publication No. WO 03/062227, International Publication No. WO 2004/009555, International Publication No. WO 2004/022541, International Publication No. WO 2004/108724, International Publication No. WO 2005/003101, International Publication No. WO 2005/039564, International Publication No. WO 2005/034866, International Publication No. WO 2005/037197, International Publication No. WO 2005/037198, International Publication No. WO 2005/035501, International Publication No. WO 2005/035503, International Publication No. WO 2005/035506, International Publication No. WO 2005/080394, International Publication No. WO 2005/103050, International Publication No. WO 2006/057270, International Publication No. WO 2007/026664 and the like. Such compounds can be manufactured by the methods described in the respective documents where the compounds are disclosed. The specific examples thereof include 1-(5-isoquinolinesulfonyl) homopiperazine or a salt thereof (e.g., fasudil or fasudil hydrochloride), (+)-trans-4-(1- aminoethyl)-1-(4-pyridylcarbamoyl)cyclohexane((R)-(+)-trans-(4-pyridyl)-4-(1-aminoethyl)-cyclohexanecarboxamide) or a salt thereof (e.g., Y-27632 ((R)-(+)-trans-(4-pyridyl)- 4-(1-aminoethyl)-cyclohexanecarboxamide dehydrochloride monohydrate) and the like) and the like. For these compounds, a commercially available product (Wako Pure Chemical Industries, Ltd, Asahi Kasei Pharma Corporation and the like) can also be preferably used. As used herein, the ROCK inhibitor is particularly used in the step of proliferating and/or differentiating or maturing cultured human corneal endothelial cells. In one embodiment, no ROCK inhibitor may be used in the step of dedifferentiation to obtain corneal endothelial progenitor cells. Furthermore, although not wishing to be bound by any theory, the ROCK inhibitor may be used, or may not be used, in the step of dedifferentiation to obtain corneal endothelial progenitor cells in accordance with the passage number of corneal endothelial cells or corneal endothelial progenitor cells to be dedifferentiated, or differentiated or matured, or other cell-specific properties, such as donor age, from which they are derived. For example, if an example is to be given, the ROCK inhibitor may be used only in the step of proliferation and/or differentiation or maturation when the number of passages is small (for example, for low passage numbers (e.g., passage numbers of the order of 1, 2, or 3) and/or for cells from young donors. If the number of passages is large (for example, the number of passages is 4, 5, 6, etc.), it is also possible to use the ROCK inhibitor during cell seeding. As such, it can be one of the outcomes in the present disclosure that the inventors have found that, in certain embodiments, the simple use of the ROCK inhibitor only in the steps of proliferation and/or differentiation or maturation can obtain cells at a level comparable to the case where the ROCK inhibitor is applied for the entire period.

(Preferred embodiments)

Preferred embodiments of the present disclosure are described hereinafter. The embodiments are provided hereinafter for a better understanding of the present disclosure. It is understood that the scope of the present disclosure should not be limited to the following descriptions. Thus, it is apparent that those skilled in the art can appropriately make modifications within the scope of the present disclosure by referring to the descriptions herein. It is understood that the following embodiments of the present disclosure can be used either alone or in combination.

In the present specification, International Publication No. WO 2017/141926 is referred to and incorporated as appropriate in the following various aspects. It is therefore understood that, when appropriate, the contents of WO 2017/141926 are hereby incorporated by reference.

(Human functional corneal endothelial cells capable of eliciting a human corneal endothelial functional property when infused into an anterior chamber of a human eye)

In one aspect of the present disclosure, the present disclosure provides a human functional corneal endothelial cell in which expression of a functional protein leading to a corneal endothelial (cell) functional property leading to improvement on corneal opacity and hydrous edema, resulting in continuous and long-term retention of corneal endothelial tissue cell density and improvement on visual acuity is recognized, or a protein that inhibits the corneal endothelial (cell) functional property is not elicited or is reduced. Such expression of the functional protein, or no elicitation of, or reduction of the protein, can be appropriately performed by those skilled in the art in accordance with the disclosure of the present specification and in consideration of exemplification of Examples. In the present disclosure, the human functional corneal endothelial cell in which expression of a functional protein leading to a corneal endothelial (cell) functional property leading to an improvement on corneal opacity and hydrous edema, resulting in continuous and long-term retention of corneal endothelial tissue cell density and improvement on visual acuity is recognized, or a protein that inhibits the corneal endothelial (cell) functional property is not elicited, or is reduced may also be expressed as "a human functional corneal endothelial cell in which expression of a functional protein leading to a corneal endothelial (cell) functional property leading to improvement on corneal opacity and hydrous edema, resulting in continuous and long-term retention of corneal endothelial tissue cell density and improvement on visual acuity is recognized"; however, note that these terms are synonymous unless otherwise noted.

In one embodiment, the present disclosure provides a human functional corneal endothelial cell capable of eliciting a human corneal endothelial functional property when infused into an anterior chamber of a human eye, where the cell includes at least one selected from the group consisting of: (i)an increase in mitochondria-dependent oxidative phosphorylation in mitochondria; (ii) no increase in expression of acetyl-CoA in the cytoplasm or nucleus; and (iii)a reduction in epigenetic multigene expression through histone acetylation by acetyl-CoA. In that case, the increase in expression of acetyl-CoA also includes expression of those that were not expressed in non-functional corneal endothelial cells. Furthermore, the reduction in epigenetic multigene expression also includes no elicitation at all. In addition, in one embodiment of the present disclosure, such a cell can also include all selected from the group consisting of: (i)an increase in mitochondria-dependent oxidative phosphorylation in mitochondria; (ii)an occurrence of organelle-selective expression of acetyl-CoA in mitochondria, and the absence of expression of acetyl-CoA in the cytoplasm or nucleus; (iii) an expression of ion channel or monocarboxylic acid transporter leading to the above-mentioned corneal endothelial (cell) functional property leading to an improvement on corneal opacity and hydrous edema, resulting in continuous and long-term retention of corneal endothelial tissue cell density and improvement on visual acuity; and (iv) a property that metabolic enzymes related to the TCA cycle, etc., which leads to the production of phase transition cells, are not present in the cytoplasm or nucleus, and are organelle-selectively localized in mitochondria, especially, reduction in, or no occurrence of, epigenetic multigene expression through histone acetylation by acetyl-CoA.

In one embodiment of the present disclosure, the human functional corneal endothelial cell of the present disclosure has not undergone, or substantially has not undergone, endothelial-mesenchymal transformation.

Furthermore, in one embodiment of the present disclosure, in the human functional corneal endothelial cell of the present disclosure, one or more metabolic-related enzymes selected from the group consisting of citrate synthase (CS), aconitase 2 (ACO2), isocitrate dehydrogenase 2 (IDH2), malate dehydrogenase 2 (MDH2), malic enzyme 3 (ME3), ACSS1, acetyl-CoA acetyltransferase 1 (ACAT1), pyruvate dehydrogenase (PDH), BCAT2, and branched-chain ketoacid dehydrogenase 2 (BCKDH2) are expressed in mitochondria; and in other embodiments, and in the cell, at least one of the enzymes selected from the group consisting of ATP citrate lyase (ACLY), aconitase 1 (ACO1), isocitrate dehydrogenase 1 (IDH1), malate dehydrogenase 1 (MDH1), malic enzyme 1 (ME1), ACSS2, acetyl-CoA acetyltransferase 2 (ACAT2), and lactate dehydrogenase (LDH) is not expressed, or is not substantially expressed.

Furthermore, in one embodiment of the present disclosure, in the human functional corneal endothelial cell of the present disclosure, the expression of sodium/hydrogen exchanger 1 (NHE1) and/or aquaporin 1 (AQP-1) is increased; and in other embodiments, in the human functional corneal endothelial cell of the present disclosure, the expression of bicarbonic anhydrase 5B (CA5B) is increased.

Furthermore, in one embodiment of the present disclosure, the human functional corneal endothelial cell of the present disclosure can also be made from a cell, as the origin thereof, selected from the group consisting of a corneal endothelial tissue-derived cell, a pluripotent stem cell, a mesenchymal stem cell, a corneal endothelial progenitor cell collected from a corneal endothelium, a cell collected form a corneal endothelium, and a corneal endothelial precursor cell and a corneal endothelial-like cell made by a direct programming method.

Furthermore, in one embodiment of the present disclosure, provided is a method of quality control or process control of a human functional corneal endothelial cell capable of eliciting a human corneal endothelial functional property when infused into an anterior chamber of a human eye, or a method of detecting a corneal endothelial non-functional cell mixed with a human functional corneal endothelial cell, the method comprising the step of confirming one or more of the following items:

(1) no fibroblast, foreign body, discoloration, or other abnormalities on visual inspection by phase-contrast imaging on the day of transplantation;
(2) number of cell of $1.5 \times 10^6$ cells/450 $\mu$L two weeks prior to and/or on the day of transplantation;
(3) 85% or more cell viability by trypan blue staining;
(4) PDGF-BB: 100 pg/mL or more in a purity test by ELISA of cell supernatant;
(5) in a purity test by FACS of cell supernatant collected two weeks prior to and/or on the day of transplantation,

$CD166^+ > 99\%$
$CD24^+ < 5\%$
$CD26^+ < 5\%$
$CD200^+ < 5\%$
$CD44^{high} < 5\%$
$CD44^{low} > 90\%$
$CD105^{-\sim weak} > 90\%$
$CD90^+ < 5\%$;

(6) effector cell (E-ratio) > 90%;
(7) pump function (Na+/K+ATPase) two days prior to transplantation: positive;
(8) barrier function (ZO-1) two days prior to transplantation: positive;
(9) less than 125 ng/$\mu$L in BSA negative test;
(10) ECD on the day of transplantation to be 1500 cells/mm$^2$ or more;
(11) expression of miR184;
(12) lactic acid production; and
(13) cell size of less than 250 $\mu$m.

In other embodiments, provided is a cell population of a human functional corneal endothelial cell capable of eliciting a human corneal endothelial functional property when infused into an anterior chamber of a human eye, the cell population satisfying one or more of the following items:

(1) no fibroblast, foreign body, discoloration, or other abnormalities on visual inspection by phase-contrast imaging on the day of transplantation;
(2) number of cell of $1.5 \times 10^6$ cells/450 $\mu$L two weeks prior to and/or on the day of transplantation;
(3) 85% or more cell viability by trypan blue staining;

(4) PDGF-BB: 100 pg/mL or more in a purity test by ELISA of cell supernatant;

(5) in a purity test by FACS of cell supernatant collected two weeks prior to and/or on the day of transplantation,

$CD166^+ > 99\%$

$CD24^+ < 5\%$

$CD26^+ < 5\%$

$CD200^+ < 5\%$

$CD44^{high} < 5\%$

$CD44^{low} > 90\%$

$CD105^{-\sim weak} > 90\%$

$CD90^+ < 5\%$;

(6) effector cell (E-ratio) > 90%;

(7) pump function (Na+/K+ATPase) two days prior to transplantation: positive;

(8) barrier function (ZO-1) two days prior to transplantation: positive;

(9) less than 125 ng/$\mu$L in BSA negative test;

(10) ECD on the day of transplantation to be 1500 cells/$mm^2$ or more;

(11) expression of miR184;

(12) lactic acid production; and

(13) cell size of less than 250 $\mu$m, and in this case, it is also possible to provide a cell population that satisfies all the items of (1) to (13).

In actual operation, it is not always necessary to employ all the items, and it is possible to employ only some of (1) to (13) as quality standards.

For example, it is possible to employ (3); $CD44^{high} < 5\%$, $CD44^{low} > 90\%$, $CD105^{-\sim weak} > 90\%$ and $CD90^+ < 5\%$ of (5); (6); (11); (12) and (13) as quality standards.

In addition, an embodiment in which all of (1) to (13) are employed can be employed, for example, in conducting a bioequivalence test when some accident occurs and the identity of cells cannot be guaranteed.

Additionally, it is possible to add one or more of other evaluation items disclosed herein to the test, in addition to or as an alternative to (1) to (13).

As such, the present disclosure can be considered to provide an extremely useful technique in that the present disclosure has produced cultured human corneal endothelial cells that exhibit extremely excellent clinical effects and in that the present disclosure has clarified a technique for identifying the cells.

In other aspects, the present disclosure provides a human functional corneal endothelial cell capable of elicitingeliciting a human corneal endothelial functional property when infused into an anterior chamber of a human eye (also referred to as the corneal endothelial property possessing functional cell of the present disclosure). Since the corneal endothelial property possessing functional cell of the present disclosure has a corneal endothelial functional property of a mature differentiated corneal endothelium and exerts an effect in cell infusion therapy (e.g., capable of eliciting a corneal endothelial functional property when infused into an anterior chamber of a human eye), such a cell can typically be referred to as a human functional corneal endothelial cell capable of eliciting a corneal endothelial functional property when infused into an anterior chamber of a human eye. The corneal endothelial property possessing functional cell of the present disclosure may include functional mature differentiated corneal endothelial cells as well as intermediately differentiated corneal endothelial cells. The functional mature differentiated corneal endothelial cell of the present disclosure is a mature differentiated cell that exerts a corneal endothelial function. An effector cell, which is the optimal subpopulation for infusion, forms a small hexagonal cobble-stone shape, and utilizes an energy metabolism system by a mitochondrial function.

Cells called "cultured corneal endothelial cells" or "cultured human corneal endothelial cells" have been reported. However, it was not known that such cells are comprised of multiple subpopulations, or there is a subpopulation thereamong that is particularly optimal for cell infusion therapy. Thus, the significance of the present disclosure, which has revealed this, is considerable. In particular, prior to the disclosure of the present disclosure, the problem related to heterogeneity of cells associated with regenerative medicine was not clearly recognized for human corneal endothelial cells. The significance of discovering and solving such a problem is considerable. This is because although human corneal endothelial cells (HCEC) cannot undergo cell division in vivo such that the cell cycle stops at the G1 phase, the ability to proliferate is still retained and it was understood that it is very difficult to culture an HCEC for a long period of time in view of recent studies.

An example of application of the present disclosure is noteworthy especially in terms of using an "allo" functional mature differentiated human corneal endothelial cell, which is high quality, free of karyotype abnormality, and does not elicit immunological rejection, as a suspension to enable regeneration of a corneal endothelial function by infusion into the anterior chamber. The medical technique that involves the cell of the present disclosure enables therapy where a corneal endothelial cell from especially young donors is cultured, expanded, and amplified ex vivo, and then a cell suspension is infused into the anterior chamber of a bullous keratopathy patient. The safety and clinical POC (proof of concept) have been demonstrated/established by the present disclosure in human applications in clinical studies based on the guidelines for human stem cell-related clinical studies.

One of the reasons the cells of the present disclosure were able to be provided is the discovery that cells used in infusion therapy are mixtures of heterogeneous cell subpopulations and only some of them are "human functional corneal endothelial cells capable of eliciting a human corneal functional property when infused into an anterior chamber of a human eye" that can be used in therapy.

The present disclosure revealed that karyotype abnormalities occur in a subpopulation selective manner and there are autoantibodies that react to the subpopulation selectively in corneal endothelial cells. It was revealed that the corneal endothelial property possessing functional cell of the present disclosure, especially functional mature differentiated corneal endothelial cell, does not have such an abnormality, and relative to other subpopulations, the expression of HLA class I antigen associated with immunological rejection is relatively low, and expression of CD200 antigen, which had been so far speculated to be a cell marker, was negative. It was also revealed that cytokine (SASP related protein) production associated with cell senescence is high in a non-intended cell.

Prior to the disclosure of the present disclosure, reproducible culturing means were limited. Attempts to grow a cultured human corneal endothelial cell without cell state transition (CST) such as fibrosis, cell senescence, or epithelial mesenchymal transition (EMT) or karyotype abnormality in vitro were notably difficult due to complete lack of knowledge related to the cell properties thereof and knowledge/report related to whether a cell population is comprised of multiple subpopulations or a cell population produced according to culture conditions exhibits stable composition, such that analysis from such viewpoints were not even conducted.

Cultured HCECs tend to undergo CST to have senescent phenotype, EMT, and fibroblastic morphology. The inventors identified a clear cell surface marker identifying these cultured contaminant cells unsuitable as transfusion cells, to allow defining of a HCEC population that can be applied to reconstruction of dysfunctional human corneal endothelial tissue.

Note that, in the present disclosure, it is possible to use cell markers described in WO 2017/141926 as appropriate.

In one embodiment, the corneal endothelial property possessing functional cell of the present disclosure has a corneal expression property of the cell indicator defined herein.

Cell indicators that the corneal endothelial property possessing functional cell of the present disclosure may have include cell surface markers (CD markers and the like), cell product property, cell morphology indicator, genetic property of a cell and the like. Specific examples of cell indicators can include cell surface markers (CD markers and the like); property of proteinaceous product and related biological material of the product; expression property of SASP related protein; expression of miRNA (e.g., intracellular miRNA, secreted miRNA or the like); property of exosome; expression property of cell metabolite and related biological material of the metabolite; cell size; cell density and presence of autoantibody reactive cell. The functional mature differentiated corneal endothelial cell of the present disclosure has such cell indicators that exhibit a cell functional property in a specific range or level or a combination thereof. Thus, it is possible to determine whether a cell is the functional mature differentiated corneal endothelial cell of the present disclosure by defining a specific range or level of cell functional property or a combination thereof for a specific cell indicator. The specific range or level of cell functional property or a combination thereof unique to the functional mature differentiated corneal endothelial cell of the present disclosure was first identified in the present disclosure, whereby various cell subpopulations are identified to allow controlling and testing quality and thus achieving a highly effective therapy. Such cell indicator and the specific range or level of cell functional property or a combination thereof is specifically discussed in more detail below.

In a specific embodiment, the corneal endothelial property possessing functional cell of the present disclosure has a cell functional property comprising CD166 positive and CD133 negative. Additional important cell functional property includes the property of expressing CD44. The expression intensity thereof is not limited to, but is preferably CD44 negative to intermediately positive, more preferably CD44 negative to weakly positive, and still more preferably CD44 negative. The present disclosure has discovered that a corneal endothelial cell or cell differentiated into corneal endothelium-like form can be confirmed to be functional by confirming the cell to be CD166 positive and CD133 negative. In addition, it is possible to find out whether a cell is functional with high precision by confirming, in addition to the above, the expression of CD44 to be low (CD44 negative to intermediately positive, preferably CD44 negative to weakly positive).

Thus, in a preferred embodiment, the corneal endothelial property possessing functional cell of the present disclosure has a cell functional property comprising CD166 positive, CD133 negative and CD44 negative to weakly positive. Although not wishing to be bound by any theory, a corneal endothelial cell or cell differentiated into corneal endothelium-like form was confirmed to be a functional mature differentiated corneal endothelial cell with high functional quality by having three such cell markers. Such functionality is demonstrated in results of clinical researches as accomplishing a high level of therapeutic effect in a short period of time (e.g., about one month) in terms of values in a corneal endothelial cell clarity test (specular), i.e., level exceeding about 1000 (cells/mm$^2$), level exceeding about 2000 (cells/mm$^2$), preferably a level exceeding about 2300 (cells/mm$^2$), more preferably a level exceeding about 2500 (cells/mm$^2$), or in some cases a level exceeding about 3000 (cells/mm$^2$).

More preferably, the corneal endothelial property possessing functional cell of the present disclosure has a cell functional property comprising CD166 positive, CD133 negative and CD44 negative. Although not wishing to be bound by any theory, a high quality cell with highly guaranteed proliferation ability or the like (also referred to as "high quality" functional mature differentiated corneal endothelial cell herein) can be more suitably provided with further limitation to CD44 negative cells. A "high quality" functional mature differentiated corneal endothelial cell has more stability and improved corneal endothelial functional property.

In another embodiment, the corneal endothelial property possessing functional cell of the present disclosure has a cell functional property comprising CD166 positive, CD133 negative, and CD200 negative. For CD200, CD200 positive has been considered to be a property of a corneal endothelial cell. Meanwhile, the present disclosure examined each subpopulation in detail to discover that a CD200 positive cell is a large cell with CST that is not suitable for infusion, and CD200 negative is a property of a functional corneal endothelial cell capable of eliciting a human corneal functional property when infused into an anterior chamber of a human eye. Such a property was unexpected from conventional knowledge and is considered a result of careful analysis of subpopulations in the present disclosure.

In another embodiment, the corneal endothelial property possessing functional cell of the present disclosure has a cell functional property comprising CD166 positive, CD133 negative, CD44 negative to CD44 weakly positive and CD90 negative to week positive. This can further guarantee the homogeneity of cells. Alternatively, cell surface antigens include CD166-positive, CD133-negative, CD44-negative to intermediate-positive, and CD90-negative phenotypes. In another embodiment, cell surface antigens include CD166-positive, CD133-negative, and CD44-negative to CD44-weak phenotypes; alternatively, cells express cell surface antigens, including CD44 negative to CD44 weakly positive phenotypes.

The corneal endothelial property possessing functional cell of the present disclosure may further have an additional cell functional property. Such a cell functional property may include, but not limited to, one or more expression properties among the following expression properties: CD90 negative (CD90 negative to weakly positive), CD105 negative to weakly positive, CD24 negative, CD26 negative, LGR5 negative, SSEA3 negative, MHC1 weakly positive (especially weakly positive relative to a cell with state transition), MHC2 negative, PDL1 positive, ZO-1 positive, Na$^+$/K$^+$ ATPase positive, and Claudin 10 positive. Alternatively, the group may be a group consisting of CD105 negative to weak positive, CD24 negative, CD26 negative, LGR5 negative, SSEA3 negative, MHC1 weak positive, MHC2 negative, ZO-1 positive, and Na+/K+ ATPase positive.

As used herein, the expression intensity of a cell indicator marker such as a CD marker is indicated as negative (may be indicated as -; when - and +/- are distinguished, both are encompassed) for substantially no expression. As used herein, negative encompassed dull positive. Not negative, i.e., expression significantly observed is indicated as positive (i.e., may be indicated as + when indicated in two categories of + and -). When expression levels are especially distinguished, the intensity thereof is classified into three levels and identified by weakly positive, intermediately positive, and strongly positive. In context of the displayed graph of results from FACS measurement or the like, they may be indicated by the number of +s. Weakly positive, intermediately positive, and strongly positive may be indicated as +, ++, and +++, respectively, which are synonymous. In such a case distinctions can be made as "weakly positive", "intermediately positive", and "strongly positive". This may be referred simply as positive when distinction is not made. Intensity that do not reach weakly positive is generally referred to as negative. Such levels of intensity are used in a manner commonly used in the art. These levels are relative and are defined below. For instance, "-" refers to expression that is substantially not observed. Expression that is observed is classified into three levels, weakly positive, intermediately positive, and strongly positive. Signals can be classified into negative, weakly positive, intermediately positive, and strongly positive for FACS separation.

Specific levels indicated as negative, dull positive, weakly positive, intermediately positive, and strongly positive to indicate signal intensity in FACS can be identified by using mean fluorescence signal intensity (MFI). Cell distribution can be displayed as a histogram and shown as negative, dull positive, weakly positive, intermediately positive, and strongly positive after relative comparison. The baseline of judgment for a more specific measurement value is explained. Specifically, the following levels are examples thereof.

The intensity of expression of a cell indicator marker such as a CD marker is typically different in terms of fluorescence intensity due to the type of fluorescence of a label or equipment setting. Herein, the range of weak fluorescence intensity is about less than 3800, range of intermediate fluorescence intensity is about 3800 or greater and less than 27500, and range of strong fluorescent intensity is about 27500 or greater under the following condition: PE-Cy7-labeled anti-human CD44 antibody (BD Biosciences) is used and Area Scaling Factor of Blue laser of FACS Canto II is set to 0.75 and the voltage of PE-Cy7 is set to 495. In the Examples of the present specification, the mean fluorescence intensity of negative control (isotype control) at this setting was about 50 (range of 55 +/- 25; while there may be a small deviation even under the same setting depending on the cell lot, those skilled in the art can carry out the test while understanding such deviations). Thus, in view of "range of weak fluorescence intensity is about less than 3800, range of intermediate fluorescence intensity is about 3800 or greater and less than 27500, and range of strong fluorescent intensities is about 27500 or greater", mean fluorescence intensity of negative control (isotype control) PE-Cy7: about 50 [approximately 33-80]. Thus, weak: < 76-fold, intermediate: 76 to 550-fold, and strong > 550-fold. A staining intensity pattern that is the same as the negative control (isotype control) is determined to be negative, and positive when the pattern is shifted even slightly.

When used in the present specification, examples of other settings include the following.

*Area Scaling Factor: FSC=0.5, Blue laser= 0.75, Red laser=0.8
*voltage: FSC=270, SSC=400, FITC=290, PE=290, PerCP-Cy5.5=410, PE-Cy7=495, APC=430
*Examples of mean fluorescence intensity of negative control (isotype control) include the following.
FITC: about 130 [approximately 65-225]
PE: about 120 [approximately 73-204]
PerCP-Cy5.5: about 120 [approximately 74-191]
PE-Cy7: about 50 [approximately 33-80]
APC: about 110 [approximately 67-196]

The intensity of a cell marker can be readily assessed by techniques such as fluorescence activating cell sorting, immunohistochemical technique or the like (not limited thereto). For the above-described markers and expression levels thereof, "negative" refers to a lack of expression of the markers or notably low levels thereof and "positive" refers to notable expression. Transition of a cell marker from "negative" to "positive" indicates a change from a lack of expression or low level of expression to a high level or notable level of expression. The term "weakly positive" refers to weak expression, i.e., low level of expression and is also denoted as "low expression". Since "intermediately positive" refers to readily detectable intermediate level of expression, it is also denoted as "intermediate expression". "Strongly positive" refers to notable expression which is very readily detectable strong expression, i.e., high level of expression, and is also denoted as "high expression". In this regard, transition from "weakly positive" to "intermediately positive", "intermediately positive" to "strongly positive" or "strongly positive" to "intermediately positive", or "intermediately positive" to "weakly positive" expression can be readily confirmed. For example, a non-intended cell exhibit CD44 strongly positive, progenitor cell exhibits CD44 intermediately positive, and the mature differentiated functional corneal endothelial cell of the present disclosure exhibits CD44 negative or CD44 weakly positive. As shown for instance in the Examples, two or more cell surface markers or the like can be used to classify a cell into a subpopulation or the like.

Additional cell indicators used in the present disclosure include expression intensities of MHC-1 and MHC-2, which are both associated with lack of immunological rejection. Since the present disclosure is used clinically in cell infusion therapy, no or low immunological rejection is preferred.

Additional cell indicators used in the present disclosure include ZO-1 and Na$^+$/K$^+$ ATPase. They are properties that are closely related to the expression of functionality of a human corneal endothelial cell. Thus, it is preferred that they are both clearly expressed (+) in a regular manner.

While the corneal endothelial property possessing functional cell provided by the present disclosure enables a revolutionary therapeutic method in clinical applications, quality control is necessary. Thus, a highly reliable method therefor is required. The present disclosure may provide identification and quality control of a functional mature differentiated corneal endothelial cell that does not undergo cell state transition (CST) or karyotype abnormality (aneuploidity).

In one embodiment, the corneal endothelial property possessing functional cell of the present disclosure can have a property specific to the functionality of a specific cytokine or a related substance thereof. Examples of such a property include, but are not limited to, high PDGF-BB production, low IL-8 production, low MCP-1 production, high TNF-alpha production, high IFNgamma production, high IL-IR antagonist production, low VEGF production and the like. Cytokine levels reflecting a state of attaching others such as an inflammatory cell are not preferable as an indicator. Cytokine levels reflecting a normal state is preferred.

The cells of the present disclosure preferably satisfy the standards below in a quality test prior to use.

The visual test at this stage includes confirming the presence of a hexagonal cobble-stone shape and lack of fibrosis.

In the present disclosure, an example of the quality standard is to satisfy the standards shown in the table below.

[Table 1]

|  | Sample | Methods | Specification |
|---|---|---|---|
| Visual inspection | T-25 flask on the day of transplantation | Phase Contrast Image | No fibroblastic cells , foreign body, discoloration or other abnormality |

(continued)

| | Sample | Methods | Specification |
|---|---|---|---|
| Recoverd cell number /Cell viability | Harvested cell suspension (2 wks before / The day of Transplantation) | hemocytometer-based trypan blue dye exclusion | $1.5 \times 10^6$ cells/450 uL Viability: >85% |
| PDGFbb | Culture Supernatant | ELISA | >100 pg/mL |
| Cell surface markers | Harvested cell suspension (2 wks before / The day of Transplantation) | FACS | CD166+: >99%, CD24+: <5%, CD26+: <5%, CD200+: <5%, CD44high: <5%, CD44low>90%, CD105-/weak: >90%, CD90+<5% E-ratio: >90% |
| Na+/K+ ATPase, ZO-1 expression | 24 well plate [2 days before] | ICC | Positive |
| Negative test for BSA from the medium | Washed solution of the final product | ELISA | <125 ng/mL |
| ECD | T-25 flask on the day of transplantation | hemocytometer Software KSS-400EB (Konan) | > 1500 cells/mm$^2$) |
| miRNA* | Culture Supernatant | Realtime PCR | miR184 |
| Metabolic Products* | Culture Supernatant | | **lactic acid** |
| Cell Size* | PBS(-) treated cells | Hybrid Cel Count Software | < 250 $\mu$m) |

The corneal endothelial property possessing functional cell of the present disclosure preferably has a small cell area, i.e., is small cell. In the present disclosure, a cell area is generally assessed with PBS-treated cells under image captured conditions. That is, a measurement value of hybrid cell count is measured with area while having spaces between cells because an image of PBS-treated cells is taken. That is, the cell area is measured at a lower value than in a matured and differentiated state with tight junction formation at saturated cell culture (confluent) in culture. The present disclosure has revealed that a functional cell has high quality by having a small area per cell to have the highest cell density in culture. This is recognized as the same or higher level of cell area and cell density as endothelial cells of normal corneal endothelial tissue. Examples of preferred cell area of PBS-treated cells upon saturated cell culture (confluence) include about $250\mu$m$^2$ or less for the mean of the cell population or individual cells, and more preferably about $245\mu$m$^2$ or less, about $240\mu$m$^2$ or less, about $235\mu$m$^2$ or less, about $230\mu$m$^2$ or less, about $225\mu$m$^2$ or less, about $220\mu$m$^2$ or less, about $215\mu$m$^2$ or less, about $210\mu$m$^2$ or less, about $205\mu$m$^2$ or less, about $200\mu$m$^2$ or less and the like. Meanwhile, examples of values achieved as a preferred cell area of the corneal endothelial property possessing functional cell of the present disclosure include, but are not limited to, about $150\mu$m$^2$ or greater, about $155\mu$m$^2$ or greater, about $160\mu$m$^2$ or greater, about $165\mu$m$^2$ or greater, about $170\mu$m$^2$ or greater, about $175\mu$m$^2$ or greater, about $180\mu$m$^2$ or greater, and the like. The cell area can be measured by any approach known in the art. A typical example is a measurement method using phase contrast microscope images. Images can be taken herein using a commercially available system such as an inverted microscope system (CKX41, Olympus, Tokyo, Japan). For measuring area distribution, a target cell can be pretreated to facilitate measurement by washing with PBS(-) three times or the like and a phase contrast microscope image can be obtained by using a commercially available system such as BZ X-700 Microscope system, for example (Keyence, Osaka, Japan). Further, area distribution can be quantified using commercially available software such as BZ-H3C Hybrid cell count software (Keyence).

Thus, the corneal endothelial property possessing functional cell of the present disclosure advantageously has the above-described preferred value in at least one of the cell indicators selected from the group consisting of cell size, cell density, and presence of an autoantibody reactive cell.

The corneal endothelial property possessing functional cell of the present disclosure preferably has a cell functional property homologous to the corneal endothelial property possessing functional cell of the present disclosure (i.e., including functional mature differentiated corneal endothelial cell and intermediately differentiated corneal endothelial cell), preferably a cell functional property corresponding to the functional mature differentiate corneal endothelial cell, for at least one cell indicator selected from the group consisting of: a cell surface marker; a proteinaceous product and a related biological material of the product; a SASP related protein; miRNA; an exosome; a cellular metabolite comprising an amino acid and a related biological material of the metabolite; cell size; cell density and the presence of an autoantibody reactive cell explained herein. For example, any specific numerical value, range, or level described in the explanation regarding each indicator of the present specification is used or a combination thereof may be used as a preferred cell indicator in the present disclosure. When a certain candidate cell has a value of such cell indicators that the corneal endothelial property possessing functional cell of the present disclosure defined herein, preferably functional mature differentiated corneal endothelial cell should have, the candidate cell is determined to be a functional mature differentiated corneal endothelial cell or intermediately differentiated corneal endothelial cell that expresses a human corneal endothelial functional property when infused into the anterior chamber of a human eye. The following indicators can also be referred in addition to, or in parallel with, the determination with the aforementioned cell indicators. In particular, a function of the corneal endothelial property possessing functional cell of the present disclosure can be confirmed by formation of a small hexagonal cobble-stone shape and use of an energy metabolism system by mitochondrial function, and determined by whether it can be therapeutically effective upon infusion (e.g., into the anterior chamber of the eye). The indicators are not limited thereto, such that surrogate marker-like indicators are also effective. As such an indicator, any one of the following ten types of surrogate markers or a combination thereof can be used: (1) retention of endothelial pumping/barrier functions (including Claudin expression), (2) high adhesion/attachment to laminin 511 or a fragment E8 thereof, (3) secreted cytokine profile; production of PDGFbb, TNFalpha, IFNgamma, or IL-1 receptor antagonist is at or above refrence value, (4) stipulation by produced micro RNA (miRNA) profile, (5) stipulation by produced metabolite profile, (6) expression of ion channel or monocarboxylic acid transporter leading to the above-mentioned corneal endothelial (cell) functional property leading to improvement on corneal opacity and hydrous edema, resulting in continuous and long-term retention of corneal endothelial tissue cell density and improvement on visual acuity, (7) a property that metabolic enzymes related to the TCA cycle, , etc., which leads to the production of phase transition cells, are not present in the cytoplasm or nucleus and are organelle-selectively localized in mitochondria, (8) saturated cell density during *in vitro* culture, (9) spatial size or distribution of cells obtained in culture; and (10) adhesion to corneal endothelial surface in case of cell infusion after freeze damage by cryo treatment by liquid nitrogen on a mouse cornea. In particular, although not wishing to be bound by any theory, this is because a proteinaceous product or a related biological material of the product can mostly determine whether cells are CST cells, and miRNA can remove part or all of the unintended cells, and cell metabolite or a related biological material of the metabolite can distinguish an intermediately differentiated corneal endothelial cell from a functional mature differentiated corneal endothelial cell, such that a higher quality functional corneal endothelial cell can be selectively propagated in cultures.

In a preferred embodiment, the corneal endothelial property possessing functional cell of the present disclosure, especially mature differentiated corneal endothelial cell, does not have a karyotype abnormality. The biggest obstacle in applying cHCECs to cell injection regenerative medicine is that cHCECs in many cases exhibit aneuploidy during culture with several passages, as demonstrated by Miyai et al (Miyai T, et al., Mol Vis. 2008; 14:942-50). Aneuploidy observed in cHCECs is induced in culture due to cell division. In this regard, the inventors provide a new finding, i.e., the presence or absence of aneuploidy in cHCECs is closely associated with a specific cell subpopulation that is dominant among cHCECs. The inventors have discovered that a specific cell subpopulation without aneuploidy appears along with a specific pattern of surface phenotype with a functional mature differentiated corneal endothelial cell present in corneal tissue. Refined culture conditions for selectively proliferating a cell subpopulation consisted mostly of functional mature differentiated corneal endothelial cells without karyotype abnormality was successfully established such that a safe and stable regenerative medicament can be provided by infusing a functional mature differentiated corneal endothelial cell into the anterior chamber in the form of cell suspension for treating a corneal endothelial disorder such as bullous keratopathy. In this manner, the present disclosure discovered that a karyotype abnormality occurs subpopulation selectively, which was not known up to this point. In addition, use of the technique in the present disclosure enables selection of a subpopulation that is substantially free of karyotype abnormalities.

In another aspect, the present disclosure provides a cell population comprising corneal endothelial property possessing functional cells of the present disclosure, especially functional mature differentiated corneal endothelial cells.

The cell population of the present disclosure preferably has a mean cell density at saturated cell culture (confluence) of at least about 1500 cells/mm$^2$ or higher, at least about 1600 cells/mm$^2$ or higher, at least about 1700 cells/mm$^2$ or higher, at least about 1800 cells/mm$^2$ or higher, at least about 1900 cells/mm$^2$ or higher, or at least about 2000 cells/mm$^2$ or higher. Since a cell population comprising the corneal endothelial property possessing functional cells of the present disclosure has a small cell size, it is understood that the cells are provided at a correspondingly high density. The cell density is a characteristic found with high quality corneal endothelial properties, or conversely, measurement of such cell density can be used as one indicator for selecting high quality mature differentiated functional corneal endothelial cells. Since cell density is a numerical value that is directly related to cell area, cell density can be similarly computed by measuring cell area by any approach known in the art. As discussed above, a typical example thereof includes a measurement method using a phase contrast microscope image, wherein the image can be taken with a commercially available system such as an inverted microscope system (CKX41, Olympus, Tokyo, Japan). For measuring area distribution, target cells can be pretreated to facilitate measurement by washing with PBS(-) three times or the like and a phase contrast microscope image can be obtained for example by using a commercially available system such as BZ X-700 Microscope system (Keyence, Osaka, Japan). Further, area distribution can be quantified using commercially available software such as BZ-H3C Hybrid cell count software (Keyence).

In a preferred embodiment, the mean cell density of the cell population of the present disclosure is at least about 2100 cells/mm$^2$ or higher, at least about 2200 cells/mm$^2$ or higher, at least about 2300 cells/mm$^2$ or higher, at least about 2400 cells/mm$^2$ or higher, or at least about 2500 cells/mm$^2$ or higher, but the mean cell density is not limited thereto. The upper limit can be any materializable value. Examples of materializable upper limit include about 3000 cells/mm$^2$ or higher, about 3100 cells/mm$^2$ or higher, about 3200 cells/mm$^2$ or higher, about 3300 cells/mm$^2$ or higher, about 3400 cells/mm$^2$ or higher, about 3500 cells/mm$^2$ or higher, about 3600 cells/mm$^2$ or higher, about 3700 cells/mm$^2$ or higher, about 3800 cells/mm$^2$ or higher, about 3900 cells/mm$^2$ or higher, about 4000 cells/mm$^2$ or higher, and the like. It is understood that any combination of such upper limit and lower limit is used as the preferred range of cell density of the cell population of the present disclosure.

The characteristic of such a cell density or cell area can be applied to clinical application suitability assessment of a cultured final cell product with the phase contrast quantification technique of cultured cells by hybrid cell counting. The functional mature differentiated corneal endothelial cells of the present disclosure are revealed as having a small area per cell and the highest cell density in culture. Cultured human corneal endothelial cells made by the manufacturing method of the present disclosure exhibit the same level of cell area and cell density as endothelial cells of normal corneal endothelial tissue, i.e., cell area of 216$\mu$m$^2$ and cell density of 2582 cells/mm$^2$, as exemplified in the Examples.

In one embodiment, the cell population of the present disclosure is characterized by the presence of the corneal endothelial property possessing functional cells of the present disclosure at a ratio that is higher than a naturally-occurring ratio. This is because therapy that is more effective using a naturally available corneal endothelial cell population can be provided by providing a cell population with a ratio of cells capable of eliciting a corneal endothelial functional property which is higher than the naturally-occurring ratio. The ratio of such cells capable of eliciting a corneal endothelial functional property can be increased because a technique that can identify and select out numerous subpopulations of the corneal endothelial property possessing functional cells of the present disclosure (e.g., functional mature differentiated corneal endothelial cells or intermediately differentiated corneal endothelial cells) is provided.

In a preferred embodiment, it is advantageous that at least 5 % or greater, about 10 % or greater, about 15 % or greater, about 20 % or greater, about 25 % or greater, about 30 % or greater, about 35 % or greater, about 40 % or greater, about 45 % or greater, about 50 % or greater, about 55 % or greater, about 60 % or greater, about 65 % or greater, about 70 % or greater, about 75 % or greater, about 80 % or greater, about 85 % or greater, about 90 % or greater, about 95 % or greater, about 98 % or greater, or about 99 % or greater of cells in the subpopulation of the present disclosure are the corneal endothelial property possessing functional cells of the present disclosure. In this regard, such cells comprised in a cell population can have a corneal endothelial cell functional property described herein. For instance, as cells contained in a cell population, cells comprising a cell functional property including CD166 positive and CD133 negative and, as needed, CD44 negative to intermediately positive are selected out. Although not wishing to be bound by any theory, the reason the cell population of the present disclosure achieves an effect is because an excellent therapeutic effect or prophylactic effect is exhibited upon infusion of the cell population into a subject by comprising a certain level of the corneal endothelial property possessing functional cells of the present disclosure. In a preferred embodiment, it is advantageous that about 70% or more of cells in the cell population of the present disclosure are the corneal endothelial property possessing functional cells of the present disclosure. This because at this level, a cell density (e.g., about 2300 cells/mm$^2$) which is considered a benchmark for successful corneal cell infusion therapy can be achieved by the presence of the corneal endothelial property possessing functional cells of the present disclosure. In a more preferred embodiment, it is advantageous that about 90% or more of cells in the cell population of the present disclosure are the corneal endothelial property possessing functional cells of the present disclosure. This is because the ratio of the presence of the corneal endothelial property possessing functional cells of the present disclosure at this level cannot be accidentally achieved such that it is necessary to establish a technique and information that can precisely and reliably identify and separate a cell population while this was more or less impossible with conventional techniques. The cell density which is considered a benchmark of successful corneal cell infusion therapy can be calculated by measuring the mean cell density of cells integrated into the human corneal endothelial surface after infusion of a cell population. Such a cell density may be at least about 1000 cells/mm$^2$ or greater, preferably at least about 1100 cells/mm$^2$ or greater, preferably at least about 1200 cells/mm$^2$ or greater, preferably at least about 1300 cells/mm$^2$ or greater, preferably at least about 1400 cells/mm$^2$ or greater, preferably at least about 1500 cells/mm$^2$ or greater, preferably at least about 1600 cells/mm$^2$ or greater, preferably at least about 1700 cells/mm$^2$ or greater, preferably at least about 1800 cells/mm$^2$ or greater, preferably at least about 1900 cells/mm$^2$ or greater, preferably at least about 2000 cells/mm$^2$ or greater, preferably at least about 2200 cells/mm$^2$ or greater, preferably at least about 2300 cells/mm$^2$ or greater, preferably at least about 2400 cells/mm$^2$ or greater, preferably at least about 2500 cells/mm$^2$ or greater, preferably at least about 2600 cells/mm$^2$ or greater, preferably at least about 2700 cells/mm$^2$ or greater, preferably at least about 2800 cells/mm$^2$ or greater, preferably at least about 2900 cells/mm$^2$ or greater, and preferably at least about 3000 cells/mm$^2$ or greater.

In a more preferred embodiment, the cell population of the present disclosure is characterized by the ratio of functional mature differentiated corneal endothelial cells which is present at a higher ratio than the naturally-occurring ratio. Functional mature differentiated corneal endothelial cells express a human corneal endothelial functional property upon direct infusion into the anterior chamber of the eye. This is because therapy that is more effective than such therapy that uses a naturally available corneal endothelial cell population can be provided by providing a cell population with a higher percentage of high quality cells than the naturally occurring percentage. The ratio of such cells given high quality functionality can be increased, because a technique that can identify and select out numerous subpopulations of functional mature differentiated corneal endothelial cells is provided.

In a preferred embodiment, it is advantageous that at least 5 % or more, about 10 % or more, about 15 % or more, about 20 % or more, about 25 % or more, about 30 % or more, about 35 % or more, about 40 % or more, about 45 % or more, about 50 % or more, about 55 % or more, about 60 % or more, about 65 % or more, about 70 % or more, about 75 % or more, about 80 % or more, about 85 % or more, about 90 % or more, about 95 % or more, about 98 % or more, or about 99 % or more of cells in the cell population of the present disclosure are functional mature differentiated corneal endothelial cells. The ratio of such functional mature differentiated corneal endothelial cells may be denoted herein as the "E-ratio". The calculation method of E-ratio is described elsewhere herein. In this regard, such cells comprised in a cell population can have a corneal endothelial cell functional property described herein. For instance, as cells comprised in a cell population, cells with CD166 positive, CD133 negative, and CD44 negative to weakly positive (preferably CD44 negative) are selected out. Alternatively, cells with CD166 positive, CD133 negative, and CD200 negative can be selected out. Although not wishing to be bound by any theory, the reason the cell population of the present disclosure with enhanced quality achieves an effect is because an excellent therapeutic effect or prophylactic effect is further exhibited upon infusion of the cell population into a subject by comprising a certain level of functional mature differentiated corneal endothelial cells. In a preferred embodiment, it is advantageous that about 40% or more of cells in the cell population of the present disclosure are mature differentiated functional corneal endothelial cells. This is because a high quality cell density (e.g., about 1000 cells/mm$^2$ or higher, preferably about 2000 cells/mm$^2$, and generally about 2300 cells/mm$^2$ for cells integrated into the corneal endothelial surface) which is considered a benchmark for a successful corneal cell infusion therapy can be more reliably achieved by the presence of the functional cells at this level. In a more preferred embodiment, it is advantageous that at least about 70% or more, more preferably at least 80% or more, still more preferably at least about 90% or more of cells in the cell population of the present disclosure are functional mature differentiated corneal endothelial cells. This is because the ratio of the presence of functional cells at this level cannot be accidentally achieved such that it is necessary to establish a technique and information that can precisely and reliably identify and separate a cell population; this was more or less impossible with conventional techniques. The ratio of functional mature differentiated corneal endothelial cells can be further enhanced by using the technique of the present disclosure. For instance, it is possible to provide a cell population in which at least about 95% or more, at least about 96% or more, at least bout 97% or more, at least about 98% or more or at least about 99% or more of cells are functional mature differentiated corneal endothelial cells. In addition, it is demonstrated that a therapeutic result which exceeds about 2300 cells/mm$^2$ (e.g., about 3000 cells/mm$^2$) can be achieved in not even one month after infusion by providing such a cell population comprising functional mature differentiated corneal endothelial cells. Thus, a fast and high quality therapeutic technique that was conventionally available is provided.

In one embodiment, the corneal endothelial property possessing functional cells (including functional mature differentiated corneal endothelial cells) or cell population of the present disclosure is characterized by lower expression of cell degeneration associated antigens or HLA class I antigens associated with immunological rejection relative to other subpopulations. The corneal endothelial property possessing functional cell of the present disclosure, especially functional mature differentiated corneal endothelial cell, does not have autoantibodies seen in other subpopulations. Thus, said cell is recognized as an immunologically stable cell.

In another aspect, the present disclosure provides a product comprising the corneal endothelial property possessing functional cells of the present disclosure or cell population. Such a product may be in any form such as cellular processed products and the like prepared for administration to humans and the like, but the product is not limited thereto. It is desirable that such a cell product preferably has not undergone unintended transformation, has no or little effect from physiologically active substances produced by cell/tissue, has no or little effect on a normal cell or tissue, has no or little possibility of forming a heterotopic tissue, have no or little possibility of inducing an undesired immune reaction, has no or little possibility of tumorigenesis or oncogenesis, has been subjected to safety assessment as defined in gene therapy product guidelines in case gene transfer has been performed, and has cleared general toxicity test or the like.

In another aspect, the present disclosure provides a method of preserving the corneal endothelial property possessing functional cells of the present disclosure, functional mature differentiated corneal endothelial cells, or cell population, the method comprising passaging the cells or the cell population by exchanging a medium. In this regard, the present disclosure discovered that a cell functional property is maintained and preserved by such exchanging of a medium. Any medium can be used for the medium used herein, while it is advantageous to use, preferably, an ingredient or medium used in the cell manufacturing method explained herein.

In another embodiment, the present disclosure provides a method of delivering the corneal endothelial property possessing functional cells of the present disclosure, functional mature differentiated corneal endothelial cells, or cell population, the method comprising implementing the method of preserving the corneal endothelial property possessing functional cells of the present disclosure, functional mature differentiated corneal endothelial cells, or cell population.

Sorting is a typical procedure for selecting out the corneal endothelial property possessing functional cell of the present disclosure or functional mature differentiated corneal endothelial cell. Examples of other methods that can be used include methods involving selective apoptosis induction (miRNA switching) or necrosis induction (glucose starvation or the like) to a non-intended cell by utilizing the difference in cell properties of a cell of interest and non-intended cell. However, the present disclosure generally enhances the purity of the corneal endothelial property possessing functional cells of the present disclosure or functional mature differentiated corneal endothelial cells.

In one embodiment, the present disclosure provides a cell bank comprising the corneal endothelial property possessing functional cells or cell population of the present disclosure. A cell bank refers to an organization or system for holding "cells" (generally cultured cells) that are created or collected through research or the like and providing the cells to other researchers or businesses.

In another aspect, the present disclosure provides a product comprising the corneal endothelial property possessing functional cells of the present disclosure or cell population. Such a product may be in any form such as cellular processed products and the like prepared for administration to humans and the like, but the product is not limited thereto. It is desirable that such a cell product preferably has not undergone unintended transformation, has no or little effect from physiologically active substances produced by cell/tissue, has no or little effect on a normal cell or tissue, has no or little possibility of forming a heterotopic tissue, have no or little possibility of inducing an undesired immune reaction, has no or little possibility of tumorigenesis or oncogenesis, has been subjected to safety assessment as defined in gene therapy product guidelines in case gene transfer has been performed, and has cleared general toxicity test or the like.

In another aspect, the present disclosure provides a medicament comprising a functional corneal endothelial cell capable of eliciting a human corneal tissue function (in particular, a human corneal endothelial functional property) when infused into an anterior chamber of a human eye (the corneal endothelial property possessing functional cell of the present disclosure) or a functional mature differentiated corneal endothelial cell. The cell used in the medicament of the present disclosure can include any cell also described elsewhere herein. The present disclosure also notably improves other therapeutic assessment items such as corneal thickness, vision, and the like. For instance, when assessment of corneal thickness is studied, a therapeutic effect is achieved earlier compared to conventional methods, where the corneal thickness is sufficiently reduced by raising the E-ratio, such that a notable improvement is observed. The medicament of the present disclosure also notably improves other items, such as vision, stromal edema and total score thereof. Further, a severe adverse event was not observed and a non-severe adverse event was hardly observed, such that the medicament of the present disclosure is understood to provide excellent therapeutic result. The medicament of the present disclosure can perform cultured corneal endothelial cell infusion on a patient with a corneal disorder, such as bullous keratopathy.

In one specific embodiment, the medicament of the present disclosure is used for treating a corneal endothelial dysfunction, disorder, or disease. Such a corneal endothelial dysfunction, disorder, or disease comprises, but is not limited to, at least one selected from the group consisting of corneal endothelial disorder Grade 3 and corneal endothelial disorder Grade 4 (typically bullous keratopathy) (e.g., Fuchs endothelial corneal dystrophy, PEX-BK (pseudoexfoliation bullous keratopathy; bullous keratopathy involving pseudoexfoliation syndrome), post-laser iridotomy bullous keratopathy, post-cataract surgery bullous keratopathy (including pseudophakic or aphakic bullous keratopathy), post-glaucoma surgery bullous keratopathy, and post-trauma bullous keratopathy, bullous keratopathy of an unknown cause after multiple surgeries, post-corneal transplantation graft failure, congenital corneal endothelial dystrophy, and congenital anterior chamber angle hypoplasia syndrome. The grade system used herein is based upon the severity classification of corneal endothelial disorders, which is based on Japanese Journal of Ophthalmology 118: 81-83, 2014. For instance, an example of bullous keratopathy includes post-laser iridotomy bullous keratopathy, which involves a surgery that opens a hole with laser on the iris of a patient with ocular pressure which is difficult to control only with a glaucoma therapeutic agent to improve the flow of aqueous humour. Meanwhile, it is understood that corneal endothelium is hit by flowing water thereof to damage the endothelium. The medicament of the present disclosure is considered as exhibiting a notable effect. Fuchs corneal dystrophy is a congenital genetic disease considered to affect 4-5% of 40-50 year olds or older individuals in Europe and the US. The endothelium in the center of the cornea falls off to exhibit opacity. Fuchs corneal dystrophy is the leading cause of corneal transplantation in Europe and the US. The medicament of the present disclosure is also considered to exhibit a notable effect on Fuchs corneal dystrophy. Further, the medicament is also effective for bullous keratopathy after multiple operations with an unknown cause called Multiple OP-BK. Typical example of such a multiple operation includes an operation with concurrent vitreoretinal operation and cataract+intraocular lens insertion generally called "triple operation" and the like.

The medicament of the present disclosure can be administered to a subject in any manner, but it is desirable that a cell contained in the medicament of the present disclosure is administered into the anterior chamber in a preferred embodiment. A technique of infusing a cultured corneal endothelial cell into the anterior chamber is established. Although not wishing to be bound by any theory, this is because the concept of regenerating corneal endothelia by intra-anterior chamber infusion is (1) minimally invasive, (2) involves no artificial material, and (3) allows use of a highly functional corneal endothelial cell from a young donor with little senescence as a master cell. Further, this is because a corneal endothelial function is most efficiently regenerated by infusion of the cell into the anterior chamber. This is also because the process of the present disclosure has revealed that the safety and clinical POC has been established in human applications by studies based on the guidelines for clinical studies using a human stem cell (exploratory clinical studies) for ex vivo culture expansion and then infusion of cell suspension into the anterior chamber of a patient (with bullous keratopathy or the like).

In addition to a cell, the medicament of the present disclosure may be administered in conjunction with an additional agent. As such an additional agent, agents that are generally used in ophthalmic therapy (e.g., steroid agent, antimicrobial, antibacterial or NSAID) may be used. Such an addition agent may be comprised in the cell medicament of the present disclosure as a medicament, or provided in a separately administered form. In a separately provided or administered form, the additional agent is provided as a kit or combined agent. When used as a kit or combined agent, a package insert or the like that describes the usage method thereof may also be combined.

With regard to the "additional agent", it is also possible to infuse a product of the cells detailed herein, alone or with the cells, instead of the infusion of the present cells. The present disclosure also includes cells and products thereof suitable for such administration.

The medicament, pharmaceutical composition or agent (therapeutic agent, prophylactic agent or the like) of the present disclosure can be provided as a kit. In a specific embodiment, the present disclosure provides an agent pack or kit comprising one or more containers filled with one or more ingredients of the composition or medicament of the present disclosure. In some cases, information showing approval for manufacture, use or sale for human administration by a government agency can be shown on such a container in a form defined by the government agency restricting the manufacture, use or sale of a medicament or biological product.

The medicament of the present disclosure may further comprise a cell infusion vehicle. Such a cell infusion vehicle may be provided after mixing with the cell of the present disclosure, or separately. In a separately provided or administered form, such a solution is provided as a kit or combined agent. When used as a kit or combined drug, a package insert or the like that describes the usage method thereof may also be combined.

As used herein, "kit" refers to a unit generally providing portions to be provided (e.g., inspection drug, diagnostic drug, therapeutic drug, antibody, label, manual and the like) into two or more separate sections. This form of a kit is preferred when a composition that should not be provided in a mixed state and is preferably mixed immediately before use for safety or the like is intended to be provided. Such a kit advantageously comprises an instruction or manual describing how the provided portions (e.g., inspection drug, diagnostic drug, or therapeutic drug) are used or how a reagent should be handled. When the kit is used herein as a reagent kit, the kit generally comprises an instruction describing how to use an inspection drug, diagnostic drug, therapeutic drug, antibody and the like.

As used herein, "instruction" is a document with an explanation of the method of use of the present disclosure for a physician or other users. The instruction has an instructive description of the detection method of the present disclosure, method of use of a diagnostic agent, or administration of a medicament or the like. Further, an instruction may have a description instructing oral administration or administration to the esophagus (e.g., by injection or the like) as a site of administration. The instruction is prepared in accordance with a format defined by the regulatory agency of the country in which the present disclosure is practiced (e.g., the Ministry of Health, Labour and Welfare in Japan, Food and Drug Administration (FDA) in the U.S. or the like), with an explicit description showing approval by the regulatory agency. The instruction is a socalled package insert (label) and is typically provided in, but not limited to, paper media. The instructions may also be provided in a form such as electronic media (e.g., web sites provided on the Internet or emails).

For "cell infusion vehicle" used herein, any solution can be used as long as a cell can be maintained. Cell infusion vehicles include those which can be used as an intraocular irrigating solution or the like. Examples of solutions used as a cell infusion vehicle include Opti-MEM, additive added form thereof, Opeguard-MA, Opeguard-F and the like. The cell infusion vehicle used in the present disclosure may further comprise at least one of albumin, ascorbic acid, and lactic acid. Based on the knowledge obtained herein, patients can be classified with a metabolite or the like used as an indicator, the cell of the present disclosure can be appropriately prepared in accordance with the pathological condition of the classified patients, and suitable therapy can be performed thereon.

(Method of manufacturing a human functional corneal endothelial cell capable of eliciting a human corneal endothelial cell functional property when infused into an anterior chamber of a human eye)

In one aspect, the present disclosure provides a method of manufacturing a human functional corneal endothelial cell capable of eliciting a human corneal function when infused into an anterior chamber of a human eye, the method comprising the step of: (b) proliferating and/or differentiating or maturing a corneal endothelial progenitor cell under a culture condition capable of minimizing culture stress, such as proliferation stress.

In another aspect, the present disclosure provides a method of manufacturing a human functional corneal endothelial cell capable of eliciting a human corneal function when infused into an anterior chamber of a human eye, the method comprising the steps of: (a) dedifferentiating a human corneal endothelial tissue-derived cell to obtain a corneal endothelial progenitor cell; and (b) proliferating and/or differentiating or maturing the corneal endothelial progenitor cell under a culture condition capable of minimizing culture stress, such as proliferation stress.

In still another aspect, the present disclosure provides a method of manufacturing a human functional corneal endothelial cell capable of eliciting a human corneal function when infused into an anterior chamber of a human eye, the method comprising the steps of: (a) dedifferentiating a human corneal endothelial tissue-derived cell to obtain a corneal endothelial progenitor cell; and (b) proliferating and/or differentiating or maturing the corneal endothelial progenitor cell in the presence of a cell growth factor with an amount less than the amount at which transformation occurs.

In still yet another aspect, the present disclosure provides a method of manufacturing a human functional corneal endothelial cell capable of eliciting a human corneal function when infused into an anterior chamber of a human eye, the method comprising the step of: proliferating and/or differentiating or maturing a corneal endothelial progenitor cell in the presence of a cell growth factor with an amount less than the amount at which transformation occurs.

In a preferred embodiment, the human corneal function includes a corneal endothelial cell functional property, and still preferably, the human corneal function is a corneal endothelial cell functional property.

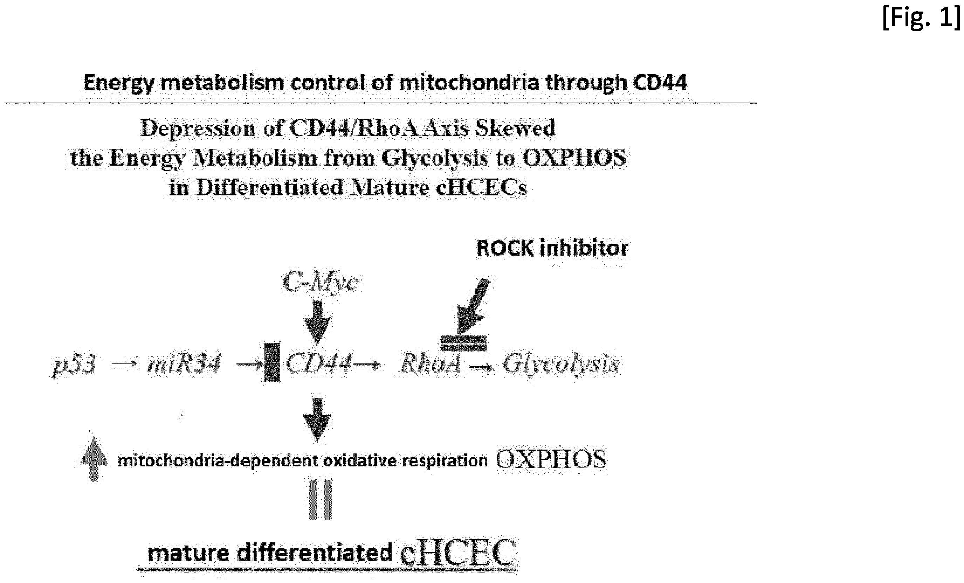

In one embodiment of the present disclosure, the expression intensity of the CD44 antigen influences the function of the human functional corneal endothelial cell of the present disclosure. The mitochondrial energy metabolism control action mediated by CD44 can be considered as shown in Fig. **1.**

For example, cultured human corneal endothelial cells with confirmed early clinical efficacy manifestation, clinical efficacy, long-term stable clinical effects, as defined by the mitochondrial localization of the metabolic-related enzymes below, are an example of the cells of the present disclosure:

[Table 2]

| dedifferentiated progenitor cell | | differentiated mature cell | |
|---|---|---|---|
| **ACLY** | VS | **CS** | **ACLY, ATP**-**citrate lyase**, CS, citrate synthase |
| **ACO1** | | **ACO2** | Aconitase 1/2 |
| **IDH1** | | **IDH2** | Isocitrate dehydrogenase 1/2 |
| **MDH1** | | **MDH2** | Malate dehydrogenase |
| **ME1** | | **ME3** | Malic acid enzyme1/3 |
| **ACSS2** | | **ACSS1** | |
| **ACAT2** | | **ACAT1** | Acetyl-CoA acetyltransferase 1/2 |
| **LDH** | | **PDH** | Lactate dehydrogenase, Pyruvate dehydrogenase |
| | | **BCAT2** | |
| | | **BCKDH2** | Branched-chain ketoacid dehydrogenase 2 |
| cytoplasm · nucleus | | mitochondria | |

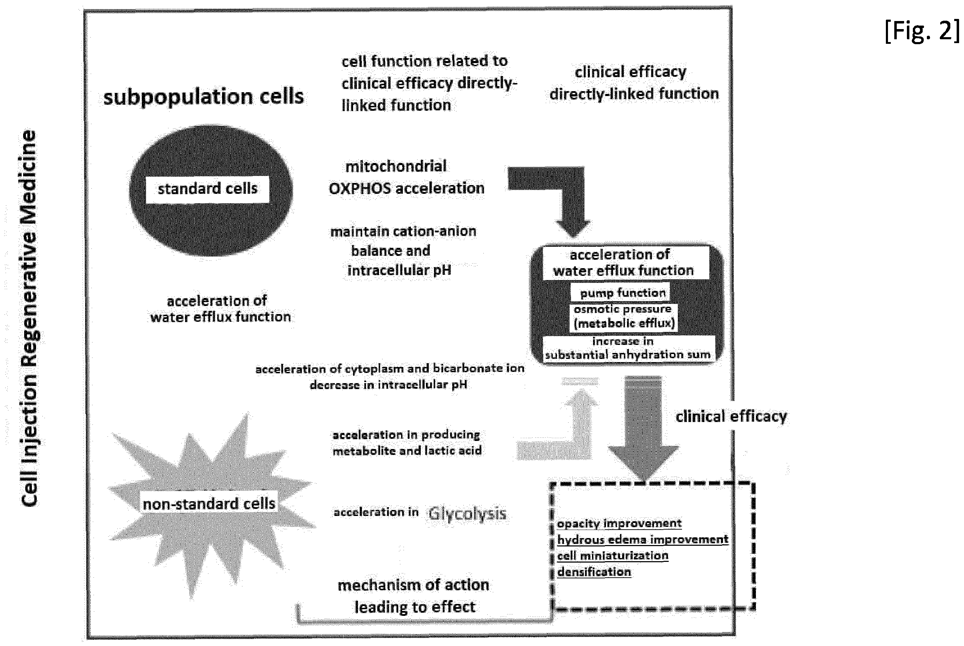

In one embodiment of the present disclosure, with regard to the functional properties of cultured human corneal endothelial cells that lead to clinical efficacy, as shown in Fig. **2,** as cell functions that lead to clinical efficacy in a standard cell, mitochondrial OXPHOS is activated to maintain cation-anion balance and intracellular pH, which enhances a water efflux function, leading to improvement on corneal opacity, improvement on hydrous edema, miniaturization of corneal endothelial cells, and cell densification of endothelial tissue.

In one preferred embodiment of the present disclosure, in making the human functional corneal endothelial cell of the present disclosure, it is possible to use a culture method in which a ROCK inhibitor (e.g., Y-27632) is continuously added to a culture medium; alternatively, no ROCK inhibitor is added during dedifferentiation or at the early stage of culture, and the ROCK inhibitor is allowed to be present only during the step of proliferation and/or differentiation or maturation, so that high quality standard cells or a highly-pure cell population of high quality standard cells can be produced. Furthermore, in other embodiments, it is also possible to use a culture method in which no TGF-β inhibitor (e.g., SB-431542) is added to the culture medium. In one embodiment, it has been found that the cytoplasm is better without suppression of TGF-β action, based on the analysis results at the subpopulation level. Furthermore, in other embodiments, it is possible to use a culture method in which no p38 MAP kinase inhibitor (e.g., SB203580) is added to the culture medium, or in which the inhibitor is added only at the final maturation step of the culture. In one embodiment, it has been found that the cytoplasm is better without suppression of the p38 MAP kinase under conditions where cell stress is not applied, based on the analysis results at the subpopulation level.

One of the useful points of the present disclosure is that the inventors have found that the minimal use of a reagent, such as a ROCK inhibitor, a TGF-β inhibitor, a p38 MAP kinase inhibitor, and/or an EGF may provide cells of quality, at an economically low cost, comparable to regular use of the reagent.

In addition, it may also be a useful point that, with an *in vitro* manufacturing method, it has become possible to obtain cells at the level obtained in living tissue, and therefore, it has become possible to estimate the quality in the cells in living tissue.

In a non-limiting preferred embodiment, although not wishing to be bound by any theory, as for the TGF-β inhibitor (e.g., SB-431542), it is preferred that no such inhibitor is added throughout the culture period.

In a non-limiting preferred embodiment, although not wishing to be bound by any theory, as for the p38 MAP kinase inhibitor (e.g., SB203580), no such inhibitor may be added throughout the culture period, or the inhibitor may take the form of being added only at the final 28-35 day stage of culture.

Furthermore, in one embodiment of the present disclosure, in making the human functional corneal endothelial cell of the present disclosure, it is also possible not to use a human mesenchymal stem cell conditioned medium. This is because, in the case of medical deployment for tens of thousands of people, the donor difference/lot difference in human MSCs becomes a barrier, the contained SASP destabilizes the quality, and miR paracrine-suppresses the phase transition paracrine, but the lot difference is large. In a non-limiting preferred embodiment, although not wishing to be bound by any theory, it may be understood that no addition of the human mesenchymal stem cell conditioned medium (MSC-CM) is sufficient throughout the entire culture period.

Furthermore, in one embodiment of the present disclosure, in making the human functional corneal endothelial cell of the present disclosure, it is also possible not to add the EGF, which is a type of cell growth factor, thereby enabling stable production of cells of interest. It is also possible to obtain cells of interest at a high yield even during long-term passaging such as P5. In one embodiment, it is preferable not to use EGF throughout the culture period, in the culture method (manufacturing method) of the present disclosure. In another embodiment, it is possible to use a culture method in which the addition is performed only at the initial stage of culture (e.g., within 7 days, etc.). In one alternative embodiment, it is understood that EGF may be used at concentrations that do not cause culture stress.

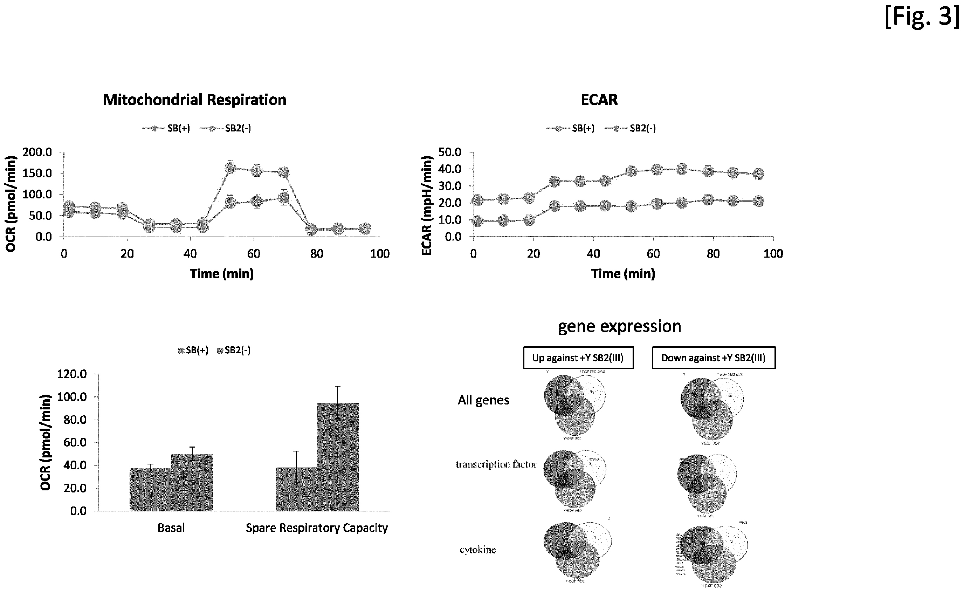

In one embodiment of the present disclosure, even though the manufacturing method was amended as described above, no changes affecting cultured human corneal endothelial cell function were observed as a result of exhaustive search of gene expression changes and assay of mitochondrial respiratory function using flux analyzer (Fig. 3).

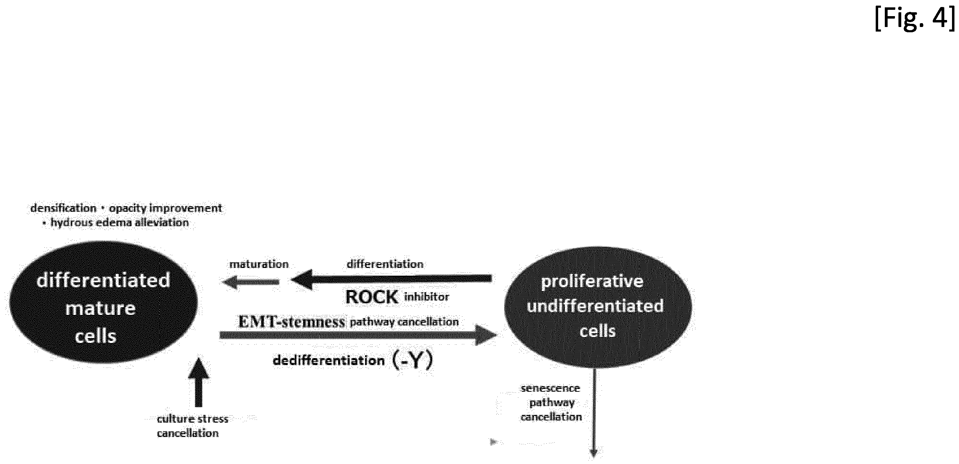

In one embodiment of the present disclosure, induction of differentiated or matured, human functional corneal endothelial cells through the dedifferentiation pathway from somatic (stem) cells can be performed as shown in Fig. **4.** According to this method, the proliferative/passageable number is P6: 83,000-fold level in young donors, cells for about 10,000 eyes can be obtained from one eye, the donor age range expands, and cells for about 600 eyes can be obtained from one eye at P4 even from middle-aged donors. In addition, it is possible to achieve maintenance of good cell morphology/shape, maintenance of high density, cell area of about 200 mm, and small distribution.

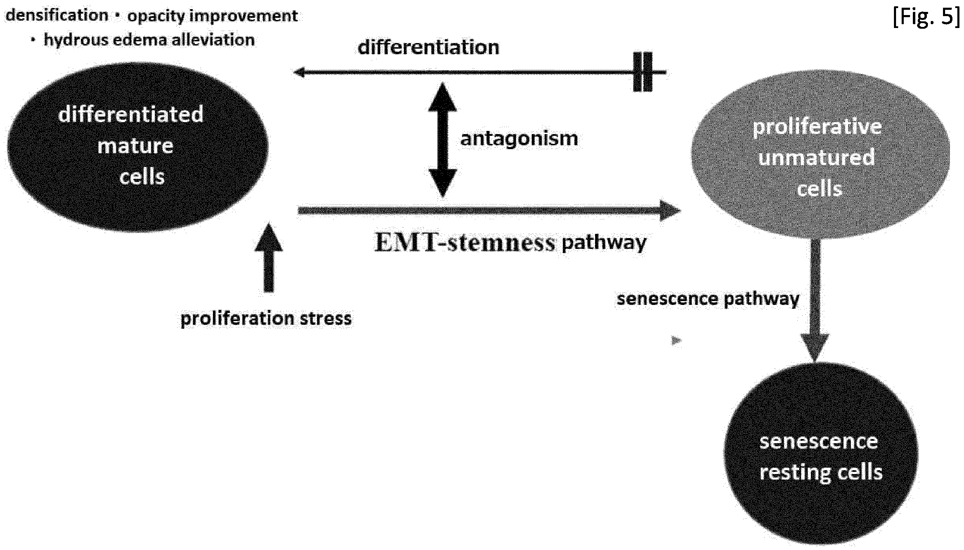

In one embodiment of the present disclosure, although not wishing to be bound by any theory, the manufacturing method as shown in Fig. **5** causes phase transition including differentiation and EMT in parallel, and thus, disadvantages due to antagonistic action may occur. However, as in the present disclosure, side effects due to antagonistic action to differentiation action due to transformation can be resolved by the step of proliferating and/or differentiating or maturating a corneal endothelial progenitor cell under a culture condition capable of minimizing culture stress, such as proliferation stress, and/or, by the performance of the step of proliferating and/or differentiating or maturating in the presence of a regulated minute amount of cell growth factors.

In one embodiment of the present disclosure, the transformation includes endothelial-mesenchymal transition, and the step of proliferating and/or differentiating or maturating can also be performed in the presence of the ROCK inhibitor.

Furthermore, in one embodiment of the present disclosure, as to candidate cells obtained after the step of proliferating and/or differentiating or maturating, at least one of the features selected from the group consisting of: (i) having a property that a metabolic enzyme related to the TCA cycle, etc., and a metabolite, such as AcetylCoA, are not present in the cytoplasm or nucleus so as not to lead to the production of contaminant phase transition cells, but are organelle-selectively localized in mitochondria; (ii) increase in mitochondria-dependent oxidative phosphorylation in mitochondria; (iii) reduction in epigenetic multigene expression through histone acetylation by acetyl-CoA (including no elicitation); (iv) increase in expression of sodium/hydrogen exchanger 1 (NHE1) and/or aquaporin 1 (AQP-1); and (v) increase in expression of bicarbonic anhydrase 5B (CA5B), is confirmed; and when the candidate cell includes at least one of the features, it is possible to further include a step of identifying the candidate cell to be the human functional corneal endothelial cell.

In the present disclosure, one of the features may be provision of cells defined by traits such as AQP1 upregulation related to water efflux directly linked to the expression of intracellular transporters for ion channels Carbonic Anhydrase (CA), NHE-1 and monocarboxylic acids [metabolites such as pyruvic acid and lactic acid] expressed by cells involved in the control of intracellular pH, reduction in corneal opacity and hydrous edema, and other clinical efficacy. As a result of having these features, the present disclosure provides cells with precisely defined functions related to intracellular pH and, as a result, regulation of cell size and regulation of mitochondrial function.

Accordingly, in one embodiment of the present disclosure, as to the mitochondria of candidate cells obtained after the step of proliferating and/or differentiating or maturing, the mitochondria is confirmed as to whether one or more metabolic-related enzymes selected from the group consisting of citrate synthase (CS), aconitase 2 (ACO2), isocitrate dehydrogenase 2 (IDH2), malate dehydrogenase 2 (MDH2), malic enzyme 3 (ME3), ACSS1, acetyl-CoA acetyltransferase 1 (ACAT1), pyruvate dehydrogenase (PDH), BCAT2, and branched-chain ketoacid dehydrogenase 2 (BCKDH2) are expressed therein; and when the expression is confirmed, it is possible to further include a step of identifying the candidate cell to be a human functional corneal endothelial cell.

Furthermore, in one other embodiment of the present disclosure, as to the candidate cell obtained after the step of proliferating and/or differentiating or maturing, the candidate cell is confirmed as to whether at least one enzyme selected from the group consisting of ATP citrate lyase (ACLY), aconitase 1 (ACO1), isocitrate dehydrogenase 1 (IDH1), malate dehydrogenase 1 (MDH1), malic enzyme 1 (ME1), ACSS2, acetyl-CoA acetyltransferase 2 (ACAT2), and lactate dehydrogenase (LDH) is expressed therein; and when the enzyme is not expressed or is not substantially expressed, it is possible to further include a step of identifying the candidate cell to be a human functional corneal endothelial cell.

Furthermore, in other embodiments, as to the candidate cell obtained after the step of proliferating and/or differentiating or maturing, the candidate cell is confirmed as to whether the expression of sodium/hydrogen exchanger 1 (NHE1) and/or aquaporin 1 (AQP-1), which is a water channel, is increased; and when the progress is confirmed, it is possible to further include a step of identifying the candidate cell to be a human functional corneal endothelial cell. In another embodiment, as to the candidate cell obtained after the step of proliferating and/or differentiating or maturing, the candidate cell is confirmed as to whether the expression of bicarbonic anhydrase 5B (CA5B) is increased; and when the progress is confirmed, it is possible to further include a step of identifying the candidate cell to be a human functional corneal endothelial cell.

Furthermore, in one embodiment of the present disclosure, it is also possible to have a step of confirming whether the human functional corneal endothelial cell has been made from a cell, as the origin thereof, selected from the group consisting of a corneal endothelial tissue-derived cell, a pluripotent stem cell, a mesenchymal stem cell, a corneal endothelial progenitor cell collected from a corneal endothelium, a cell collected form a corneal endothelium, and a corneal endothelial precursor cell and a corneal endothelial-like cell made by a direct programming method. In other embodiments, it is possible to encompass a step of making a corneal endothelial progenitor cell from a cell, as the origin thereof, selected from the group consisting of a corneal endothelial tissue-derived cell, a pluripotent stem cell, a mesenchymal stem cell, a corneal endothelial progenitor cell collected from a corneal endothelium, a cell collected form a corneal endothelium, and a corneal endothelial precursor cell and a corneal endothelial-like cell made by a direct programming method.

As used herein, a "corneal endothelial tissue derived cell or corneal endothelial progenitor cell", as defined elsewhere herein, refers to a cell that becomes the corneal endothelial property possessing functional cell of the present disclosure or functional mature differentiated corneal endothelial cell from a cell derived from corneal endothelial tissue and differentiation via a dedifferentiating step, respectively. Such a cell encompasses any cell such as cells differentiated into corneal endothelial cell-like cells, from iPS cells, ES cells, or the like and progenitor cells before differentiation into corneal endothelial cells, in addition to cells obtained from a donor corneal endothelium, as well as intermediately differentiated corneal endothelial cells defined herein.

In the manufacturing method in the present disclosure, a corneal endothelial tissue-derived cell that can be used as a starting material is collected from a living body. Alternatively, starting materials that can be used, may be corneal endothelial progenitor cells, such as cells differentiated from stem cells or progenitor cells. Examples of such differentiated cells may include, but are not limited to, cells differentiated from various stem cells (e.g., induced pluripotent stem cells (iPS cells), embryonic stem cells (ES cells), fertilized eggs, and somatic stem cells). Thus in a specific embodiment, examples of the corneal endothelial tissue-derived cells or corneal endothelial progenitor cells used (as a starting material) in the present disclosure include, but are not limited to, pluripotent stem cells, mesenchymal stem cells, corneal endothelial progenitor cells collected from a corneal endothelium, corneal endothelial cells collected from a corneal endothelium, corneal endothelial progenitor cells and corneal endothelium-like cells produced by direct programming method and the like. In this regard, examples of pluripotent stem cells include, but are not limited to, induced pluripotent stem cells (iPS cells), embryonic stem cells (ES cells) and the like. Thus, it is understood that the corneal endothelial cells or progenitor cells thereof used (as a starting material) in the present disclosure include cells prepared by differentiating induced pluripotent stem cells (iPS cells), embryonic stem cells (ES cells) or the like into corneal endothelium-like cells. Techniques of differentiating induced pluripotent stem cells (iPS cells), embryonic stem cells (ES cells) or the like into corneal endothelium-like cells are known in the art, such as the AMED method (Ueno et al supra), WO 2013/051722 (KEIO UNIVERSITY), and the like, but the techniques are not limited thereto.

When a cell that is not differentiated into a corneal endothelium-like cell is used, it is preferable to comprise a step of differentiating or maturing and differentiating into a corneal endothelium-like cell.

(Quality Control)

In one embodiment of the present disclosure, as to a method of quality control or process control of a human functional corneal endothelial cell capable of eliciting a human corneal endothelial functional property when infused into an anterior chamber of a human eye, it is possible to include a step of confirming whether one or more metabolic-related enzymes selected from the group consisting of citrate synthase (CS), aconitase 2 (ACO2), isocitrate dehydrogenase 2 (IDH2), malate dehydrogenase 2 (MDH2), malic enzyme 3 (ME3), ACSS1, acetyl-CoA acetyltransferase 1 (ACAT1), pyruvate dehydrogenase (PDH), BCAT2, and branched-chain ketoacid dehydrogenase 2 (BCKDH2) are expressed in a mitochondria of the cell. In this case, it is also possible to further include a step of confirming that expression of acetyl-CoA in the cytoplasm and nucleus, and epigenetic multigene expression through histone acetylation by acetyl-CoA, are not elicited in the human functional corneal endothelial cell.

Furthermore, in one other embodiment of the present disclosure, in conducting quality control, it is possible to further include a step of confirming that ATP citrate lyase (ACLY), aconitase 1 (ACO1), isocitrate dehydrogenase 1 (IDH1), malate dehydrogenase 1 (MDH1), malic enzyme 1 (ME1), ACSS2, acetyl-CoA acetyltransferase 2 (ACAT2), and/or lactate dehydrogenase (LDH) is not expressed, or is not substantially expressed, in the human functional corneal endothelial cell. In other embodiments, it is possible to further include a step of confirming that the expression of sodium/hydrogen exchanger 1 (NHE1) and/or aquaporin 1 (AQP-1), or bicarbonic anhydrase 5B (CA5B), is increased in the human functional corneal endothelial cell. Furthermore, in other embodiments, in conducting quality control, it is possible to include a step of measuring intracellular pH in the human functional corneal endothelial cell.

(Other Embodiments)

The judging method and analyzing method according to one or more embodiments of the present disclosure have been described based on the embodiments, but the present disclosure is not limited to such embodiments. Various modifications applied to the present embodiments and embodiments constructed by combining constituent elements in different embodiments that are conceivable to those skilled in the art are also encompassed within the scope of one or more embodiments of the present disclosure as long as such embodiments do not deviate from the intent of the present disclosure.

As used herein, "or" is used when "at least one or more" of the matters listed in the sentence can be employed. When explicitly described herein as "within the range of two values", the range also includes the two values themselves.

As described above, the present disclosure has been described while showing preferred embodiments to facilitate understanding. The present disclosure is described hereinafter based on Examples. The aforementioned description and the following Examples are not provided to limit the present disclosure, but for the sole purpose of exemplification. Thus, the scope of the present disclosure is not limited to the embodiments and Examples specifically described herein and is limited only by the scope of claims.

[Examples]

While the present disclosure is further explained hereinafter with Examples, the present disclosure is not limited thereto.

The experimental procedures and materials used in the present disclosure are described hereinafter. Although the experimental procedures below are used in the embodiments, similar results can be obtained by using other experimental procedures.

The Examples below were carried out under the approval of the ethics committee associated with the organization to which the inventors belong, while complying with medical ethical regulations such as the Declaration of Helsinki, regulations such as the GCH, and regulations stipulated by Kyoto Prefectural University of Medicine and others. After the necessary informed consent was obtained, the experiments below were conducted.

(Example 1: I. Examination Experiment of Added EGF Concentration)

In this Example, the concentration of epidermal growth factor (EGF) used, in the manufacture of human functional corneal endothelial cells, was examined. Details will be provided below.

(Materials and Methods)

Donor information was as follows: #202, ABG-404OSCN/ODCN (right eye left eye), age 64, endothelial cell density (ECD) = 3104/3070, cause of death (COD): Metastatic Neuroendocrine Carcinoma of the Uterus, D-P (time from corneal donor death to placement of cornea in preservation solution) = 12:15, D-C (time from corneal donor death to initiation of cell culture) = 8D.

The concentration of added EGF was examined under the culture conditions below. Details are as follows.

Culture with 0+Y, SB2, EGF, Asc (ascorbic acid)
↓
P1 Culture with +Y, SB2, EGF, Asc
↓
P2 Culture in 4 ways with EGF(-), 0.5 ng/mL, 1 ng/mL, 5 ng/mL (+Y, SB2, Asc)
↓ Passage from each condition in P2
P3 Culture in 4 ways with EGF(-), 0.5 ng/mL, 1 ng/mL, 5 ng/mL (+Y, SB2, Asc)
↓ Passage from each condition in P3
P4 Culture in 4 ways with EGF(-), 0.5 ng/mL, 1 ng/mL, 5 ng/mL (+Y, SB2, Asc)
*Trypsin (TrypLE, ThermoFisher) concentration ×10 at the time of detachment, seeding cell number ECD800

(In the above, P indicates the number of passages.)

Culture conditions are as follows.

SB2, EGF, and Asc mean SB203580, epithelial cell growth factor, and ascorbic acid, respectively. The epidermal growth factor was purchased from Wako Pure Chemical Industries, Ltd. (Osaka, Japan), and SB203580 (SB2) was obtained from Cayman Chemical (Ann Arbor, Mich.). Dulbecco's Modified Eagle Medium-High Glucose (DMEMHG) and fetal bovine serum were obtained from Gibco Industries (Langley, OK), and plastic culture plates were obtained from Corning. Unless otherwise indicated, all other chemical substances were purchased from Sigma-Aldrich, Inc. (St. Louis, Missouri).

EGF(-), 0.5 ng/mL, 1 ng/mL and 5 ng/mL are the above SB2, EGF and Asc with or without the addition of the respective concentrations of EGF.

(Results)

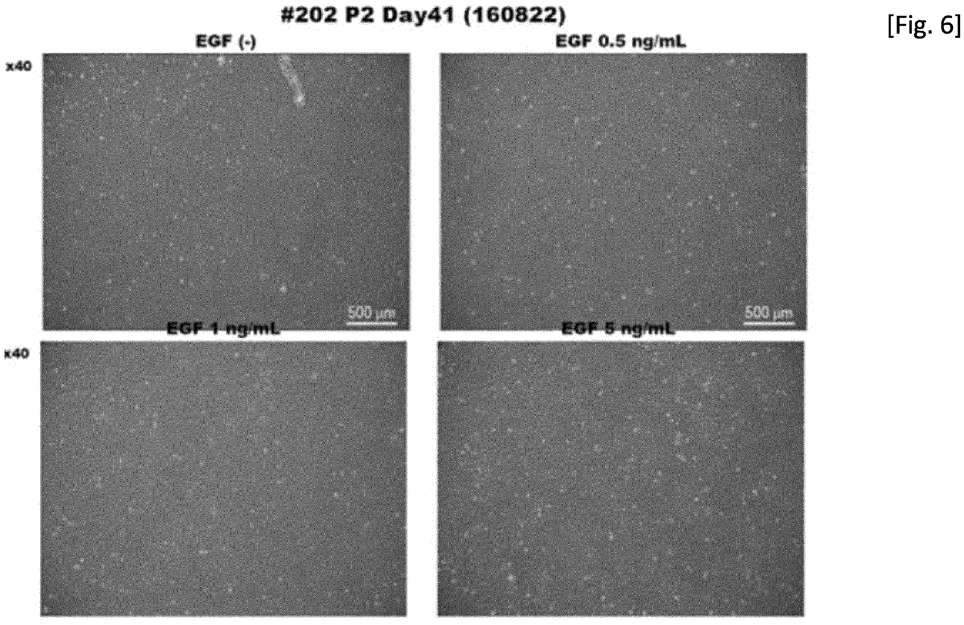

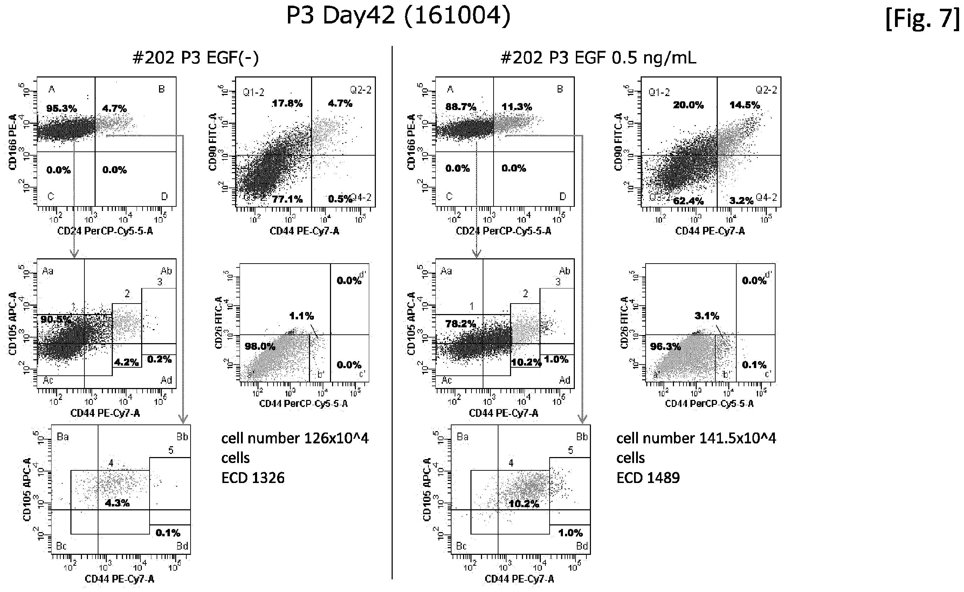

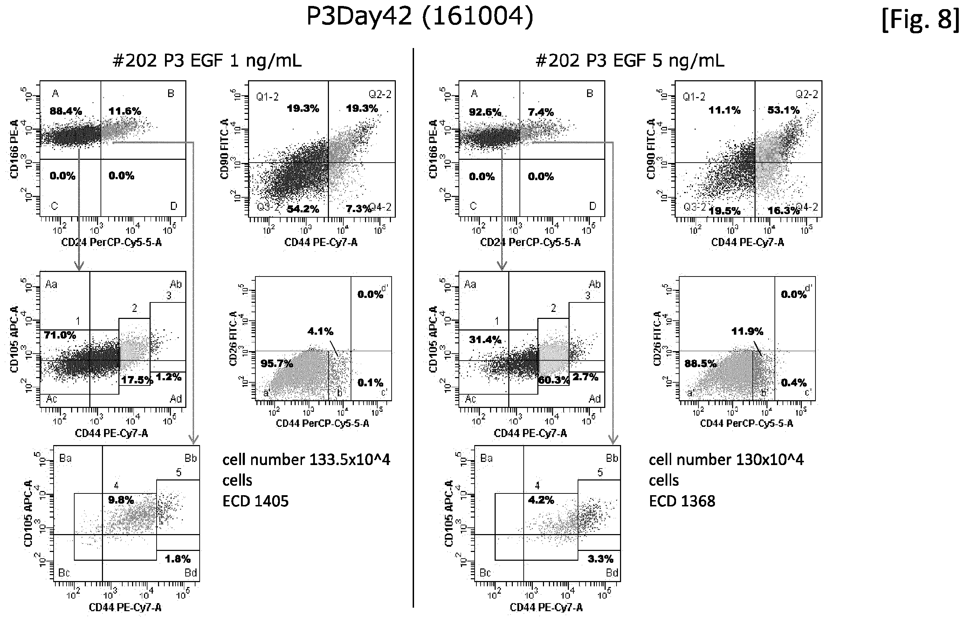

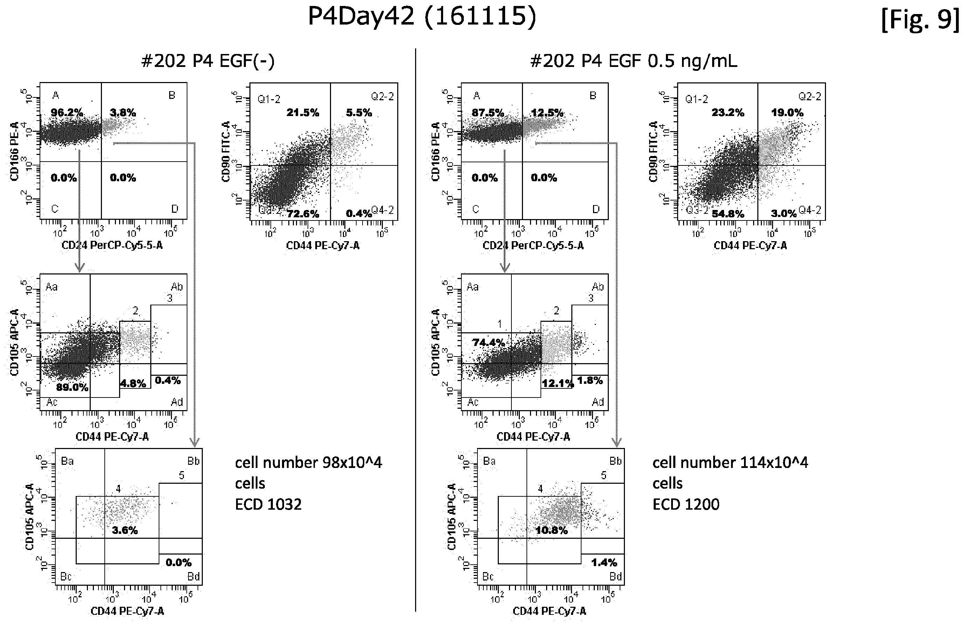

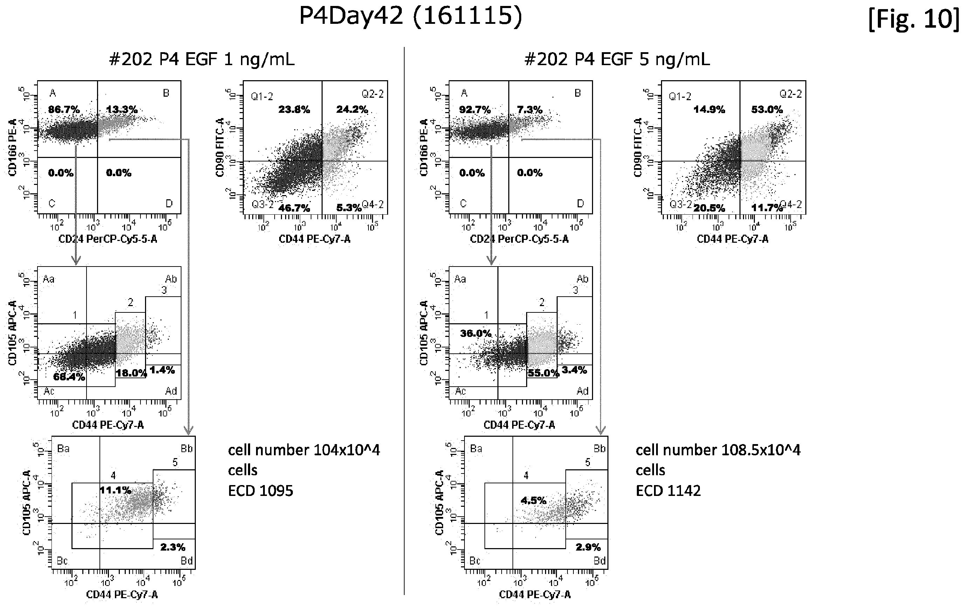

Four photographs taken on D41 of P2 are shown in Fig. 6. Furthermore, The results of FACS at P3 to P4 are shown in Figs. 7 to 10.

From these results, although not wishing to be bound by any theory, judging from both the cell shape under microscopy and the cell surface CD antigen trait, it was found that no addition of EGF was desirable at any passage from P2 to P4. In addition, as a significance of growth stress release with no addition of growth factor EGF, it is conceivable that, when no EGF is added during the making of the human functional corneal endothelial cell of the present disclosure, the cell phase transition CST including EMT is released, the cellular senescence cycle is circumvented, and mature differentiated cells are efficiently manufactured.

(Example 2: II. EGF Addition Effect from the Time of Primary Culture)

Next, the effect of adding EGF during primary culture was investigated. Details are shown below.

(Materials and Methods)

Next, the effect of adding EGF from the time of P0 primary culture was investigated.

Donor information was as follows: #214, ABS-355OSCN/ODCN, Nancy Y(+), age 18, endothelial cell density (ECD) = 3571 / 3401, cause of death (COD): Multitrauma 2' MVA, D-P = 12:32, D-C = 7D.

In addition, culture conditions and the like are as follows.

P0 Culture two eyes collectively in 2 ways with EGF(-), 0.5 ng/mL (+Y, SB2, Asc)
↓ Passage from each condition in P0
P1 Culture in two ways with EGF(-), 0.5 ng/mL (+Y, SB2, Asc)
↓ Passage from each condition in P1

P2 Culture in two ways with EGF(-), 0.5 ng/mL (+Y, SB2, Asc) TrypLE concentration ×10 at the time of detachment, at seeding ECD800

Y, SB2 and Asc mean Y27632, SB203580 and ascorbic acid, respectively. The Rho-associated protein kinase (ROCK) inhibitor Y-27632(Y) and epidermal growth factor were purchased from Wako Pure Chemical Industries, Ltd. (Osaka, Japan), and SB203580 (SB2) was obtained from Cayman Chemical (Ann Arbor, Mich.). Dulbecco's Modified Eagle Medium-High Glucose (DMEMHG) and fetal bovine serum were obtained from Gibco Industries (Langley, OK), and plastic culture plates were obtained from Corning. Unless otherwise indicated, all other chemical substances were purchased from Sigma-Aldrich, Inc. (St. Louis, Missouri).

EGF(-) and 0.5 ng/mL are the above Y, SB2 and Asc with or without the addition of the respective concentrations. Example 1 is followed except for those specifically described.

(Results)

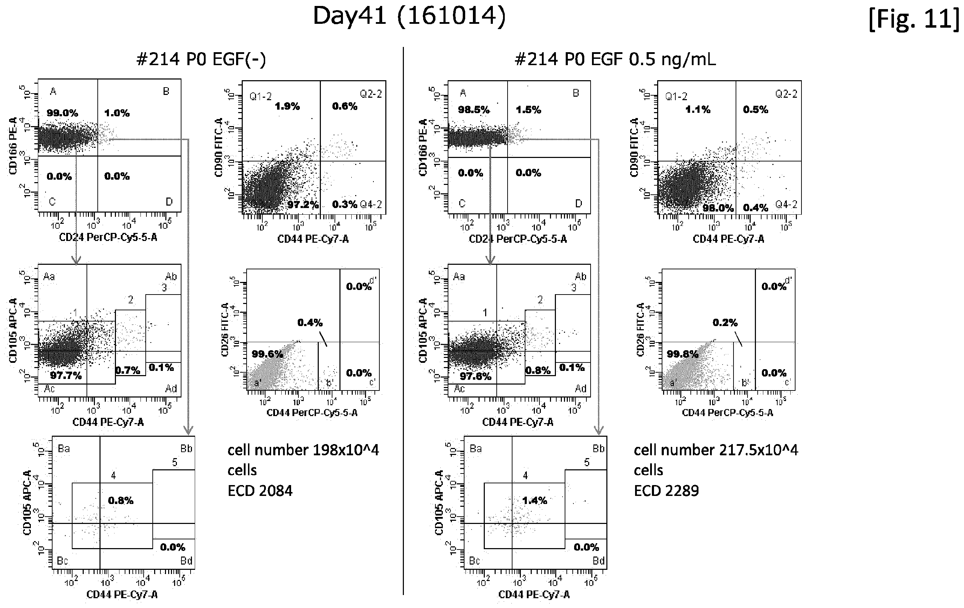

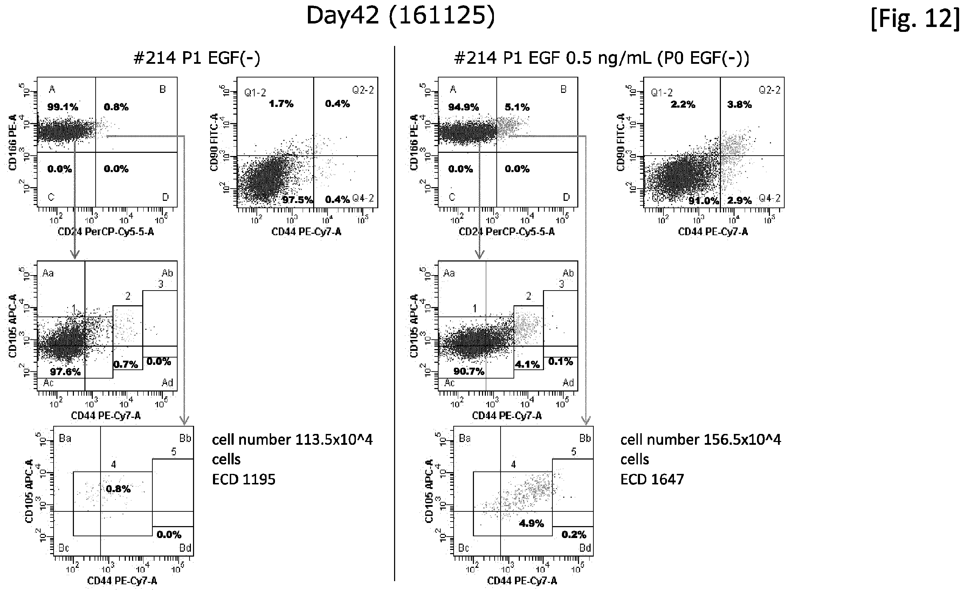

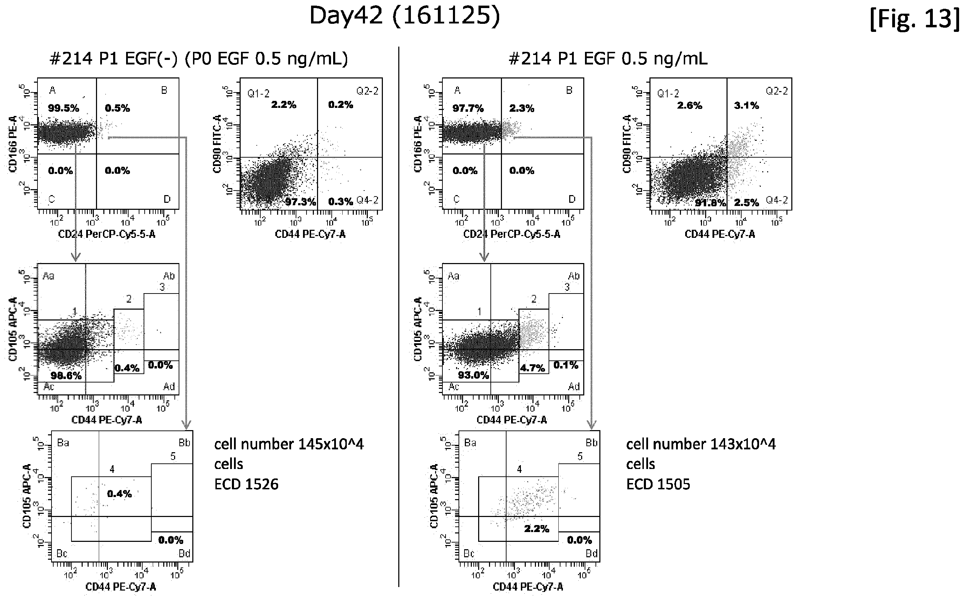

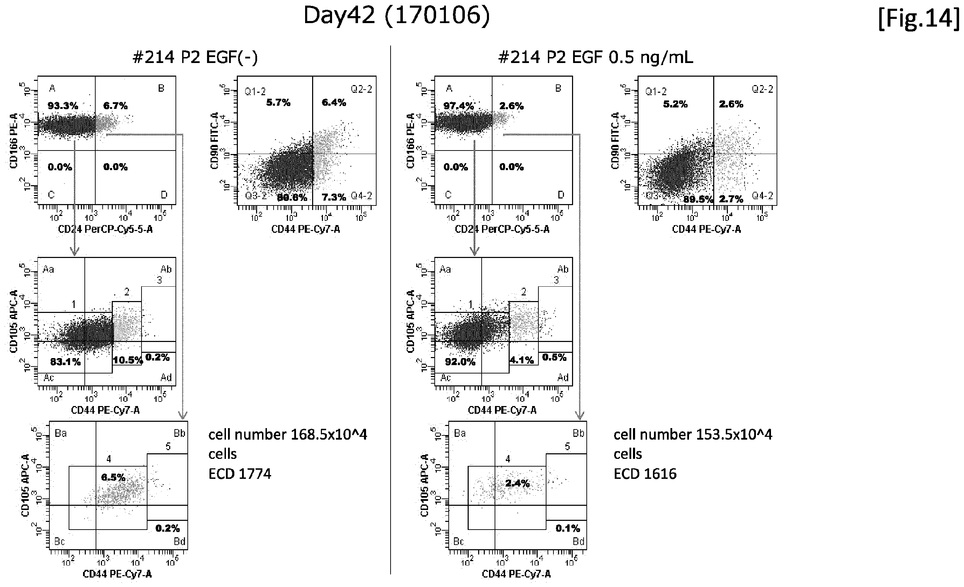

The results of FACS at P0 to P2 are shown in Figs. **11** to **14.** Although not wishing to be bound by any theory, the ratio of corneal endothelial cells of the present disclosure increases when EGF is not added; thus it is understood that culture stress is reduced when EGF is not added (or when the concentration is low), and more preferable cells are manufactured. Accordingly, this is understood to be preferable for the manufacture of a human functional corneal endothelial cell capable of eliciting a human corneal function when infused into an anterior chamber of a human eye.

Accordingly, on the basis of the above matters, in the present disclosure, it is understood to be important to proliferate and/or differentiate or maturate a corneal endothelial progenitor cell under a culture condition capable of minimizing culture stress, such as proliferation stress, for culturing with a high standard cell ratio.

(Example 3: III. Assay with mIR Expression without EGF Addition, Addition Effect of ROCK Inhibitor)

In this example, it was tested whether a cell standard without EGF addition could be assayed for mIR expression, and the effect of adding a ROCK inhibitor was also verified.

(Materials and Methods)

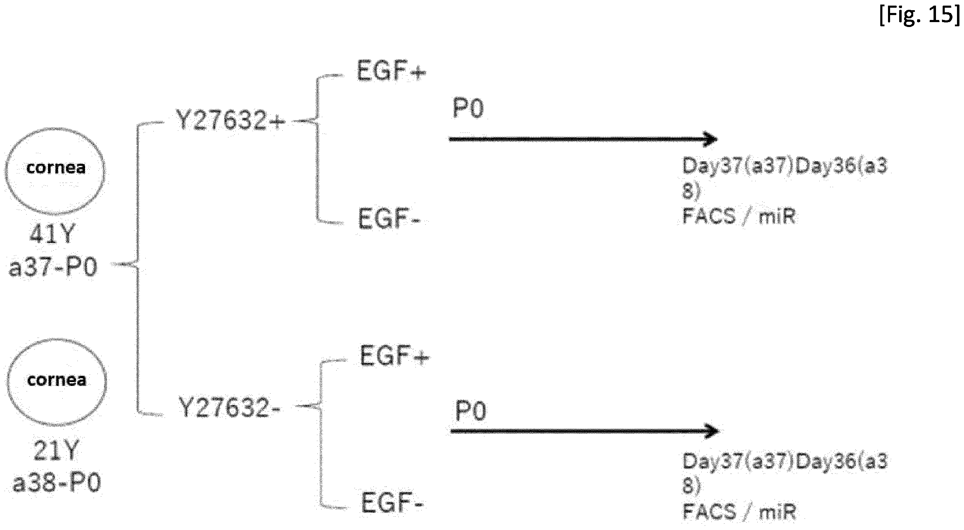

Cases were considered as shown in Fig. **15,** and intracellular gene variations of miR378, miR146, miR34, and miR184 were measured.
Y27632 was used as a ROCK inhibitor.
Culture conditions conform to Examples 1 and 2.

(Results)

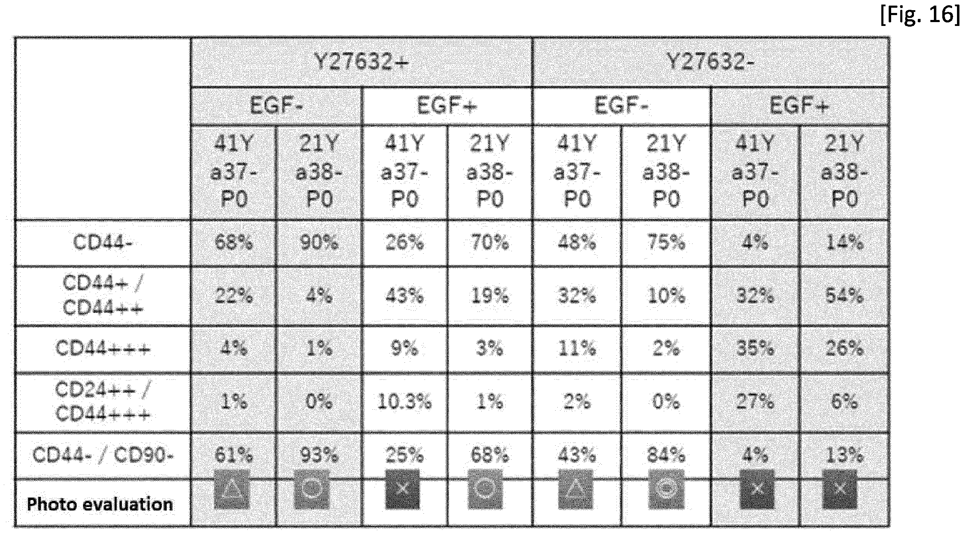

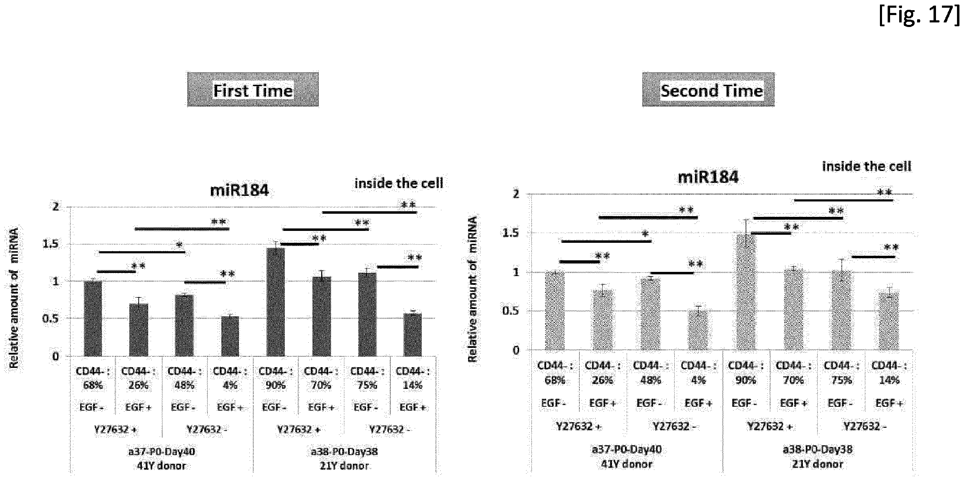

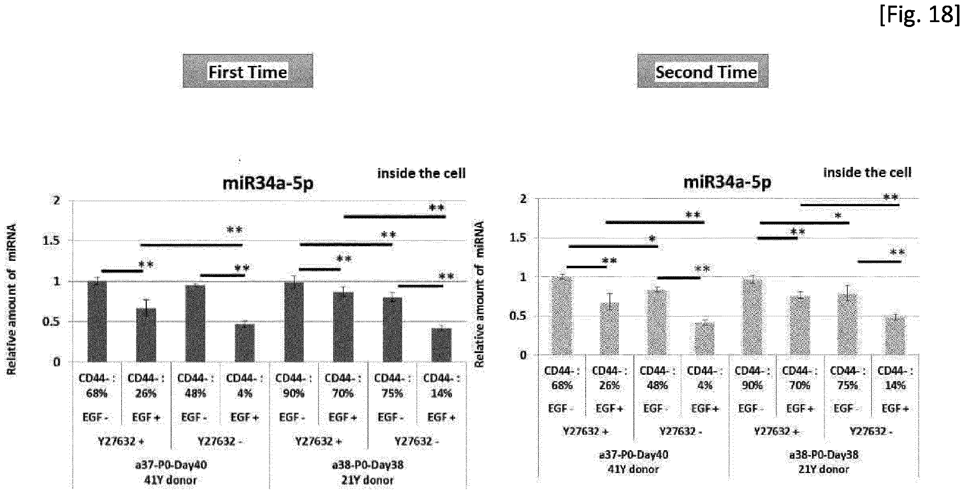

The results of FACS measurement and photographic evaluation are shown in Fig. **16.** As a result, it was found that the influence of age difference was overlaid on Y+/-.
The results of miR184, miR34, miR378, and miR146 gene expression variations in the presence or absence of EGF and in the presence or absence of Y by qRT-PCR are shown in Figs. **17** and **18.**
Based on this result, although not wishing to be bound by any theory, it was found that: (1) miR184 is inversely proportional to intracellular CD44 gene expression level; (2) the expression level thereof increases in ages younger than middle age; (3) the expression level thereof increases with the addition of the ROCK inhibitor than without the addition of the ROCK inhibitor; and (4) the expression level thereof increases with no EGF addition than with EGF addition.
Furthermore, although not wishing to be bound by any theory, it was found that: (1) miR34a-5p is inversely proportional to the intracellular CD44 gene expression level within the same donor, but cross-donor comparison suggests that the contrast with CD44 may be reversed; (2) the expression level thereof increases in ages younger than middle age; (3) the expression level thereof increases with the addition of the ROCK inhibitor than without the addition of the ROCK inhibitor; and (4) the expression level thereof increases with no EGF addition than with EGF addition.
Based on this result, although not wishing to be bound by any theory, it was confirmed that no EGF addition is desirable, even on the basis of miR184, 34a expression intensity of cell property index. It was also confirmed that the addition of the ROCK inhibitor increases the expression level of miR184 and 34a.
In view of the foregoing results as well, it is understood to be important, in the present disclosure, to proliferate and/or differentiate or maturate a corneal endothelial progenitor cell under a culture condition capable of minimizing culture stress, such as proliferation stress.

(Example 4: IV. Assay by Cell Functional Property Metabolites when no EGF is added, Addition Effect of ROCK Inhibitor)

Subsequently, cells of the present disclosure without EGF addition were assayed for cell functional property metabolites. In addition, the effect of adding a ROCK inhibitor was also judged.

(Materials and Methods)

In this Example, differences between the metabolite properties of human functional corneal endothelial cells and the metabolite properties of non-intended cells were confirmed.

(Metabolite Assay)

Measurement of metabolites in culture media

For measurement of metabolites in culture supernatant (CS), an 80 μL Milli-Q (Merck1 KGaA, Darmstadt, Germany) containing 20 μL of CS and internal standard solution 1 (H3304-1002; Human Metabolome Technologies, Inc., Yamagata, Japan), was thoroughly mixed. Cationic compounds were measured by CE time-of-flight mass spectrometry (CE-TOFMS) in positive mode, and anionic compounds were measured by CE tandem MS (CE-MS/MS) in positive and negative modes. Hierarchical cluster analysis (HCA) was performed using the proprietary software "Peak-Stat". Differences in values were analyzed statistically using Student's t-test or one-way analysis of variance (ANOVA) with Bonferroni's post hoc test (GraphPadPrism 6.0, GraphPad software). A p-value of $<0.05$ was considered statistically significant.
The effect of adding a ROCK inhibitor was determined according to Example 3.

(Results)

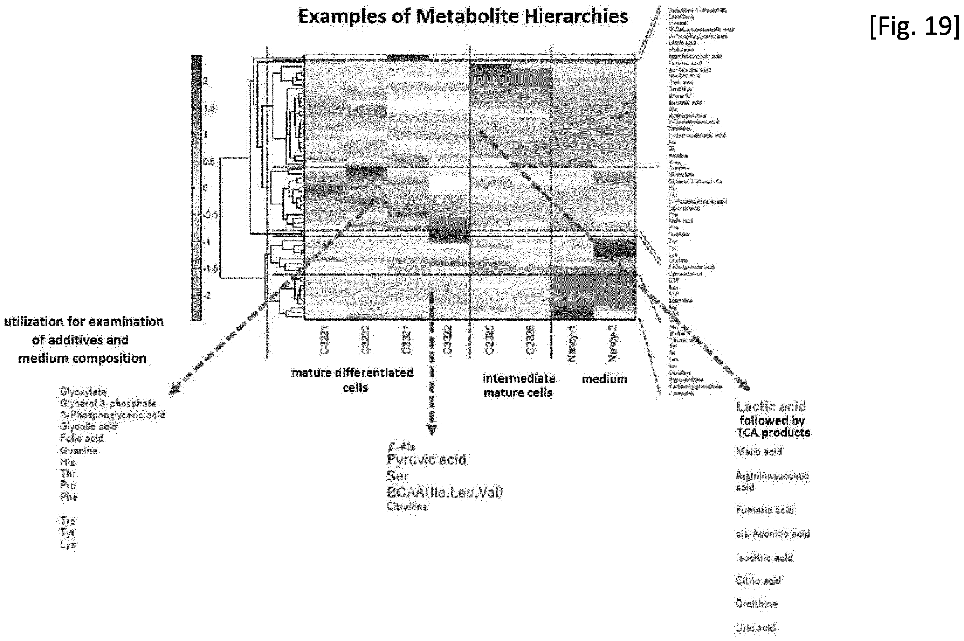

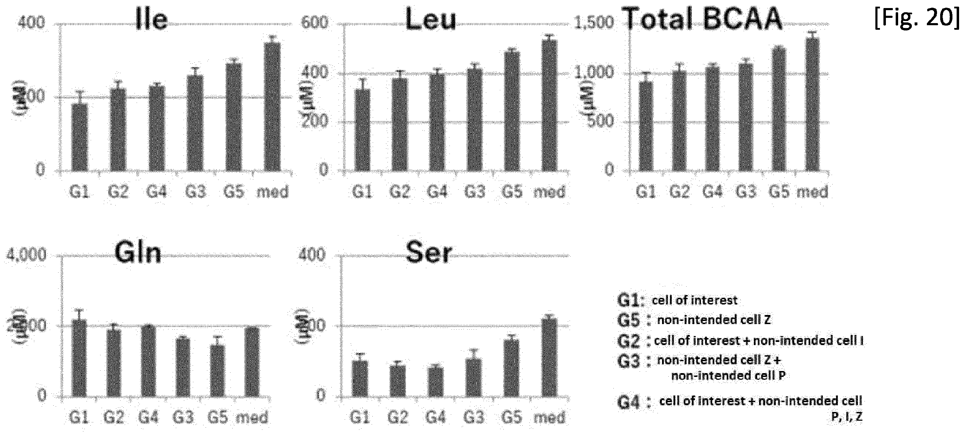

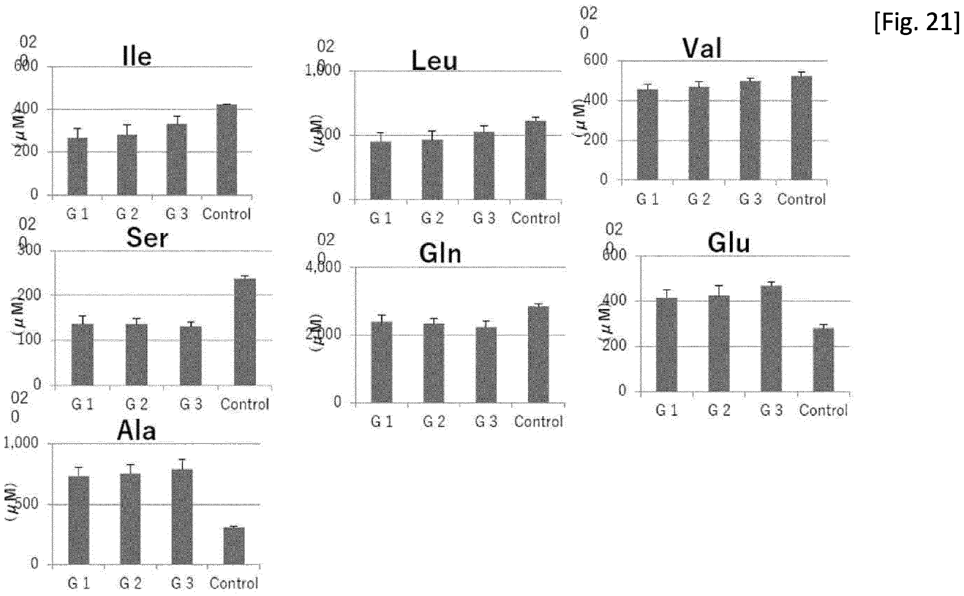

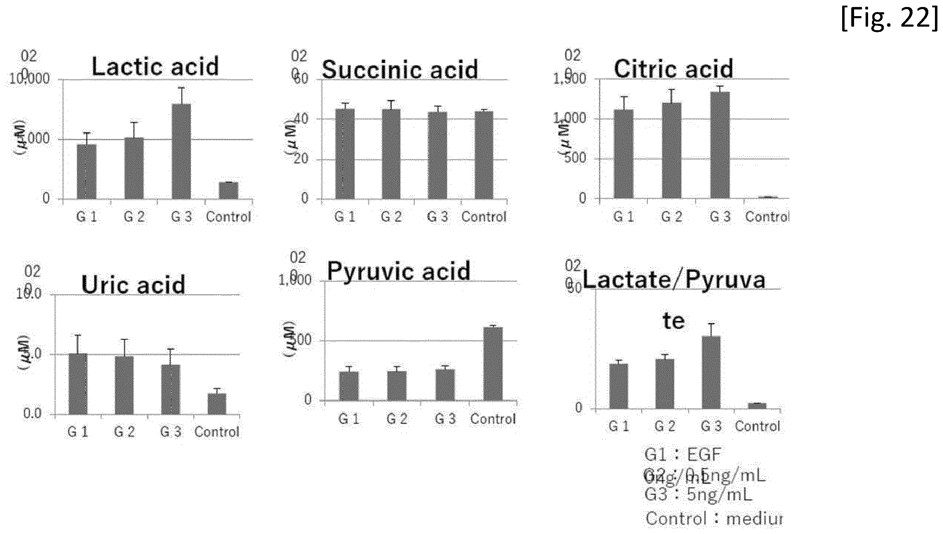

An example of metabolite hierarchy is shown in Fig. **19.**
In addition, results of confirming the metabolite properties between human functional corneal endothelial cells capable of eliciting a human corneal function when infused into an anterior chamber of a human eye (cells of interest), for which the present disclosure is intended, and cells for which the present disclosure is not intended (non-intended cells) are shown in Figs. **20** to **22.**
As a result, it was found that the cells of interest showed poor increase in Lactic Acid and L/P (lactic acid/pyruvic acid) ratio, which indicates low anaerobic glycolytic activity. It was also found that Gln increased in the cells of interest, while Glu did not increase so much, which indicates that Gln synthesis pathway is progressed by Glutaminolysis.
In addition, it was found that the cells of interest showed a remarkably low level of BCAA, which indicates that the metabolism is highly dependent on BCAA.
Furthermore, it was found that the amount of branched chain amino acids (Ile, Leu) produced, which is one of the characteristics of the cells of interest, decreased when the amount of EGF added was reduced, and this increased the target cell content.
Although not wishing to be bound by any theory, from the above results, it was found that the metabolites of cultured endothelial cells showed a non-intended cell profile by adding EGF.
In addition, it was found that: since the increase in Lactic Acid and L/P ratio is poor when the amount of EGF added is small, the content of cells of interest with low anaerobic glycolytic activity increases, that is, cultured endothelial cells exhibit a non-intended cell profile with high anaerobic glycolytic activity due to the addition of EGF.
In view of the foregoing results as well, it is understood to be important, in the present disclosure, to proliferate and/or differentiate or maturate a corneal endothelial progenitor cell under a culture condition capable of minimizing culture stress, such as proliferation stress.

(Example 5: V. Effects of Various Additives)

In this Example, the effects of various additives were tested. Details are given below.
V-1. Product evaluation in new culture method without p38 MAPK inhibitor (SB203580)
In this Example, a procedure (also referred to as SB2-) without the addition of SB203580, a p38 MAPK inhibitor, was tested.

(Materials and Methods)

Lot CT09 P5 was used to assay the effects of additives to evaluate the product in a SB2- new culture method.

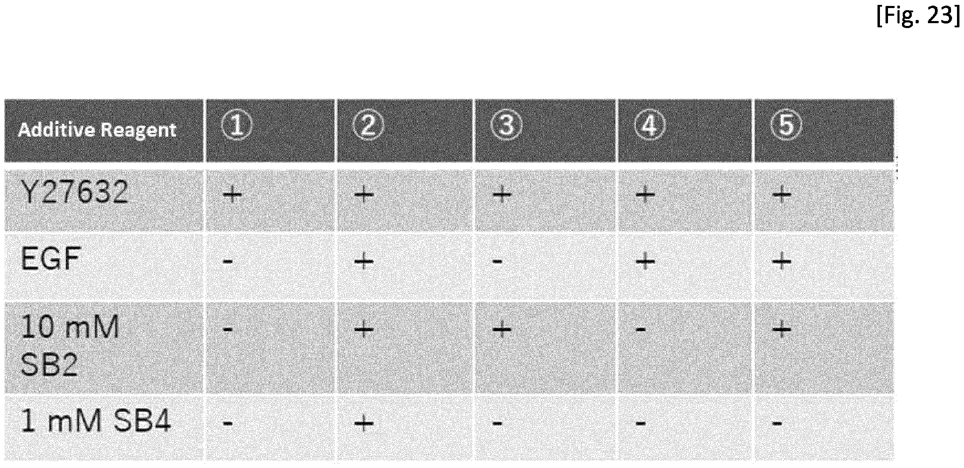

First, using CT09 P4 (remaining cells for FBS lot assay), the culture supernatant was collected the day before the passage (Day 42), and then the CT09 P5 was passaged with ECD400 (181219), followed by culturing in Nancy medium (FBS#1652794, containing ascorbic acid) under conditions 1 to 5 shown in Fig. **23** (2 wells for each 6-well plate). From the 1st week to the 5th week, the culture supernatant was collected, one well was subjected to FACS measurement on Day 34 (190122), and the culture supernatant was measured for IL-8 and PDGF-bb by ELISA.

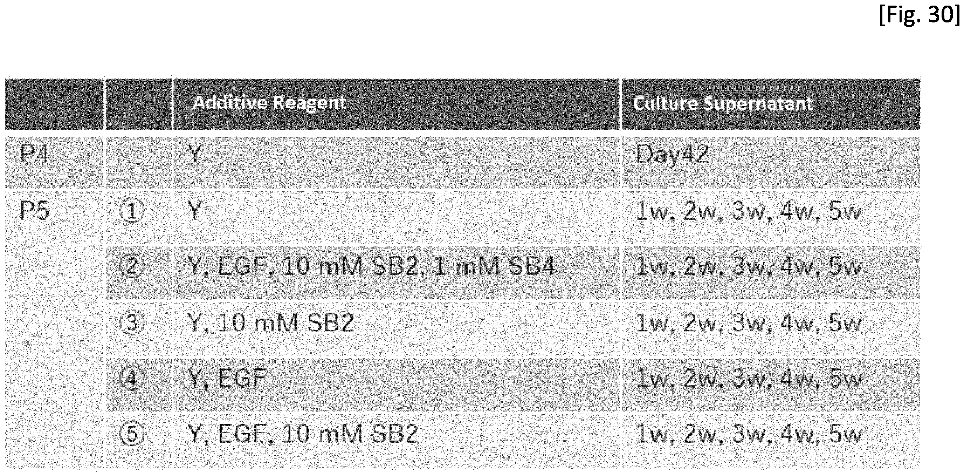

For the experimental conditions, Fig. 30 shows a culture supernatant and sample list of ELISA PDGF-bb and IL-8, CT09, P4 and P5.

(Results)

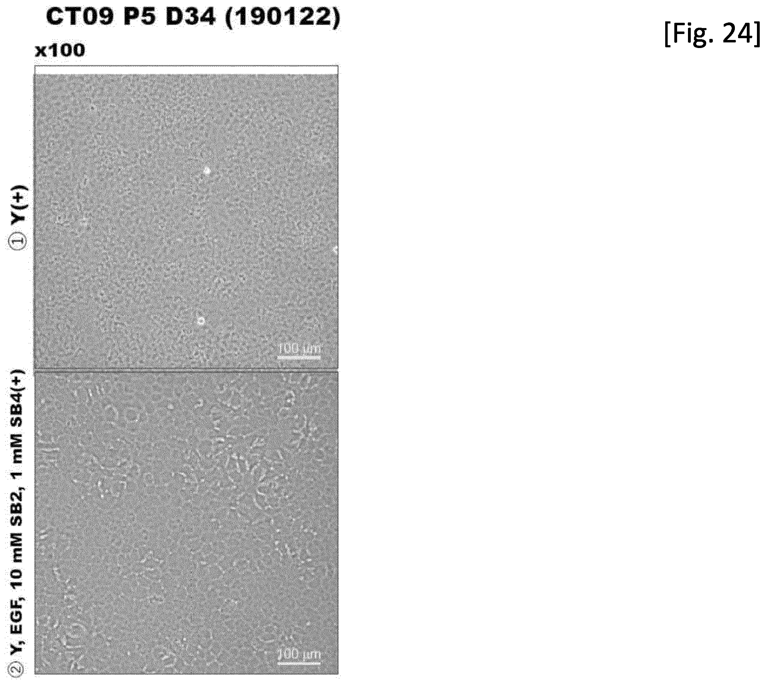

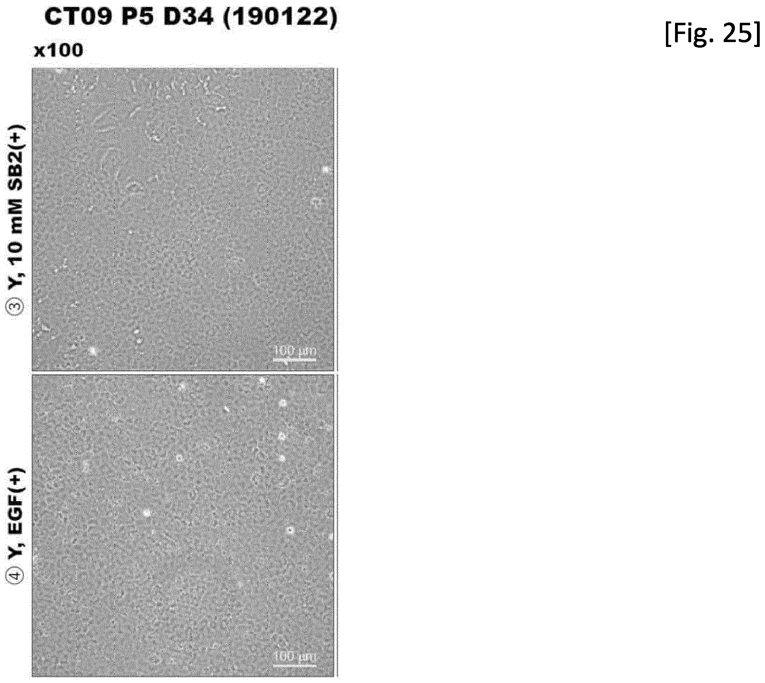

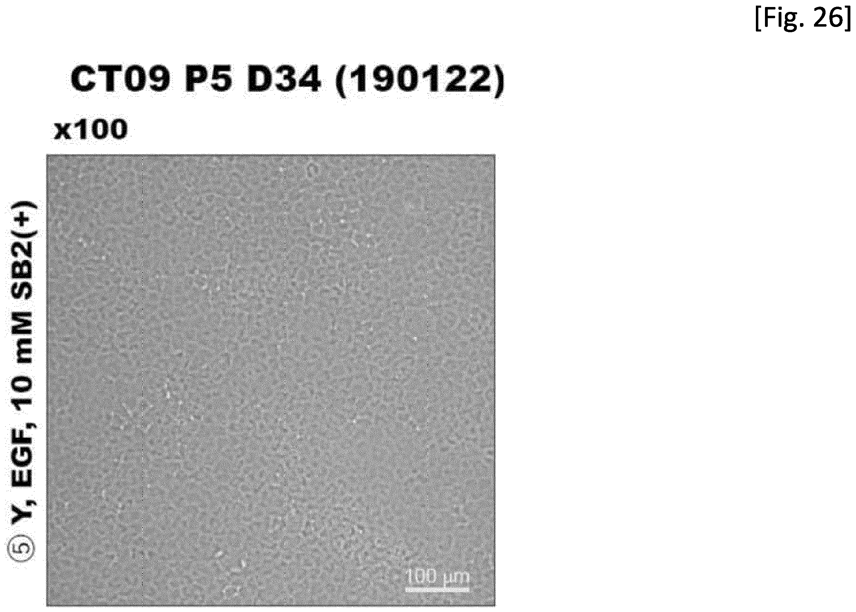

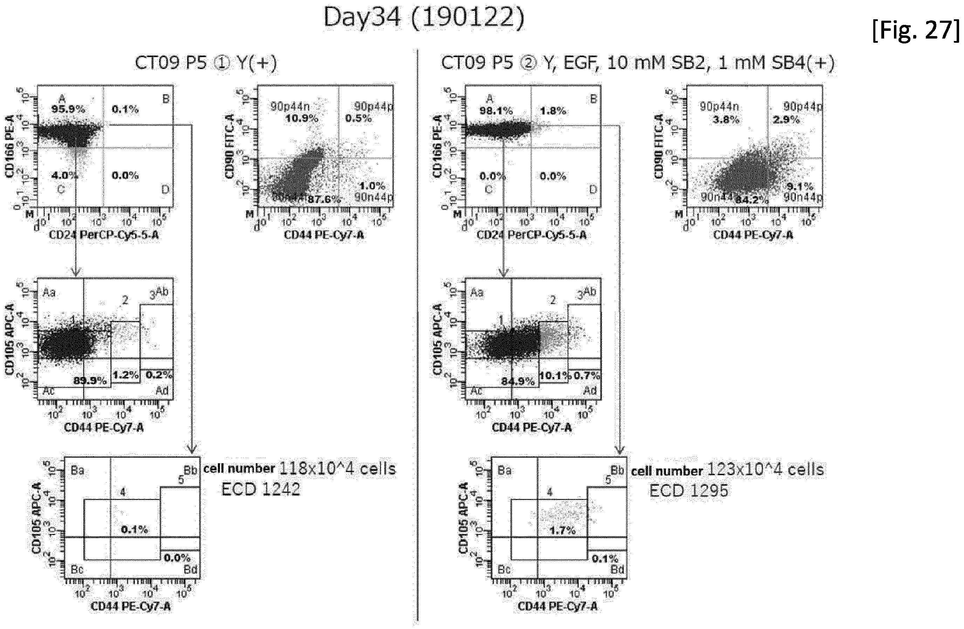

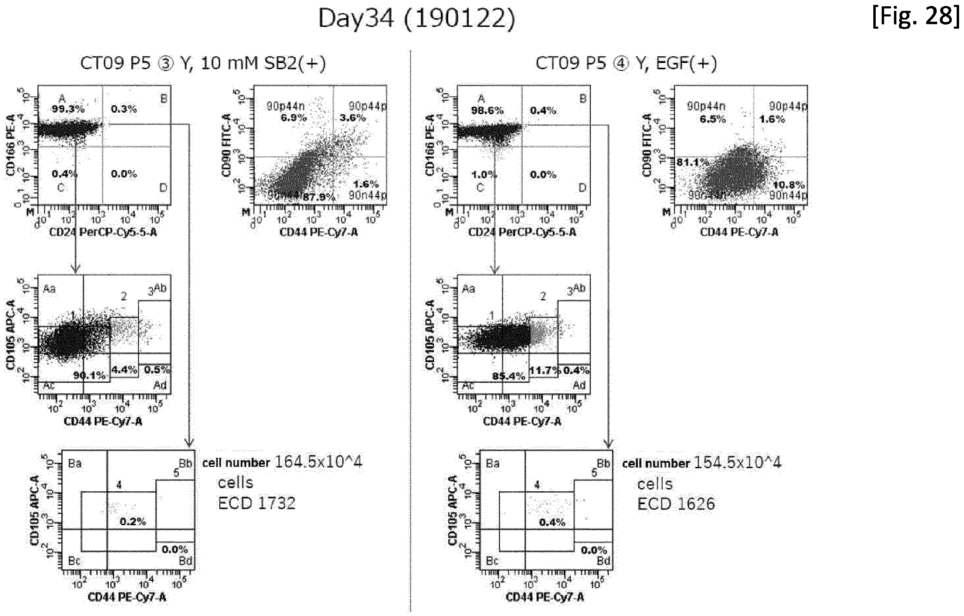

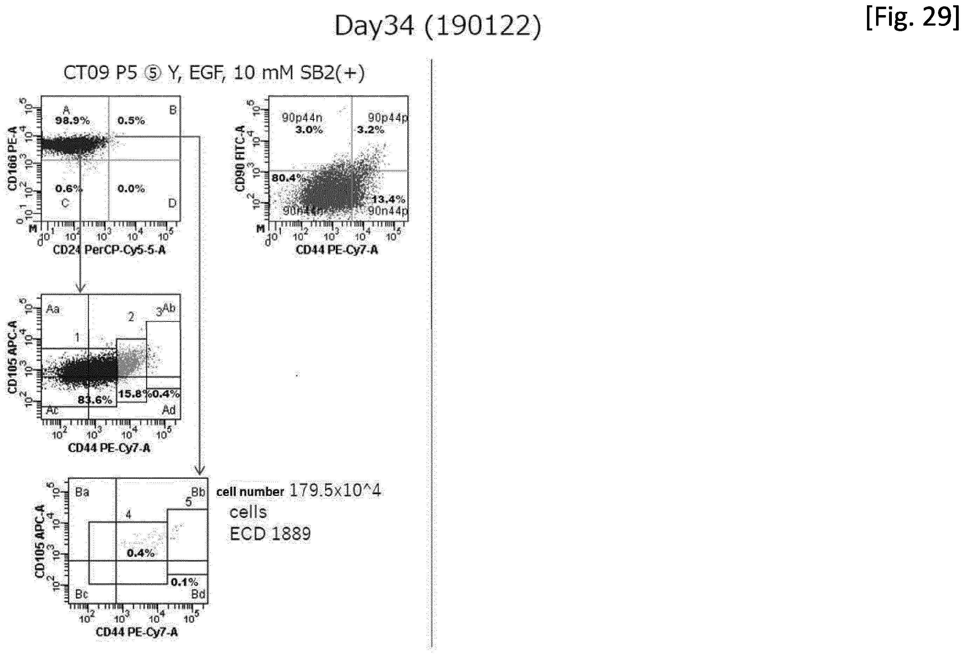

Photographs of CT09 P5 under each condition are shown in Figs. **24** to **26,** and FACS results on Day 34 are shown in Figs. **27** to **29,** respectively.

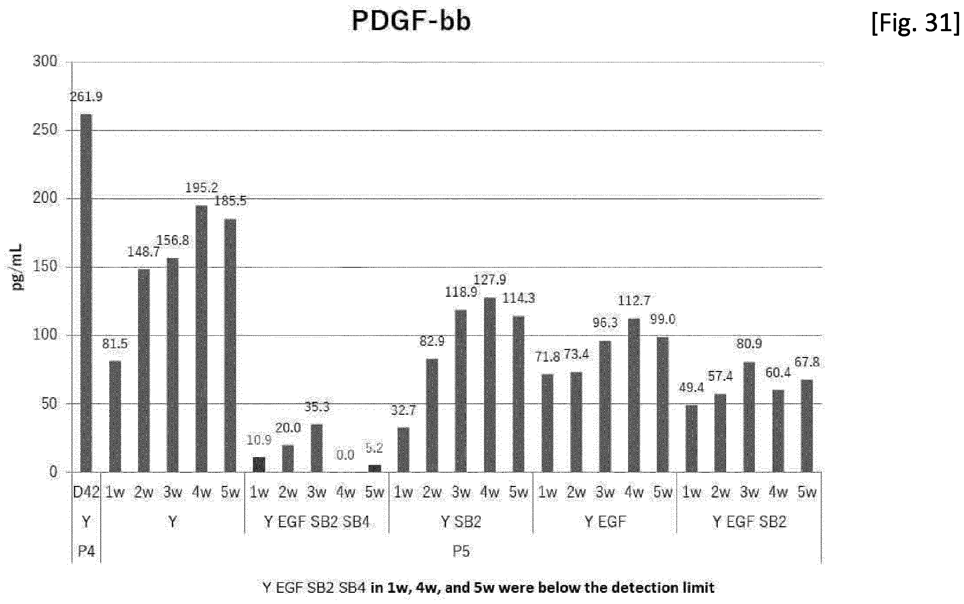

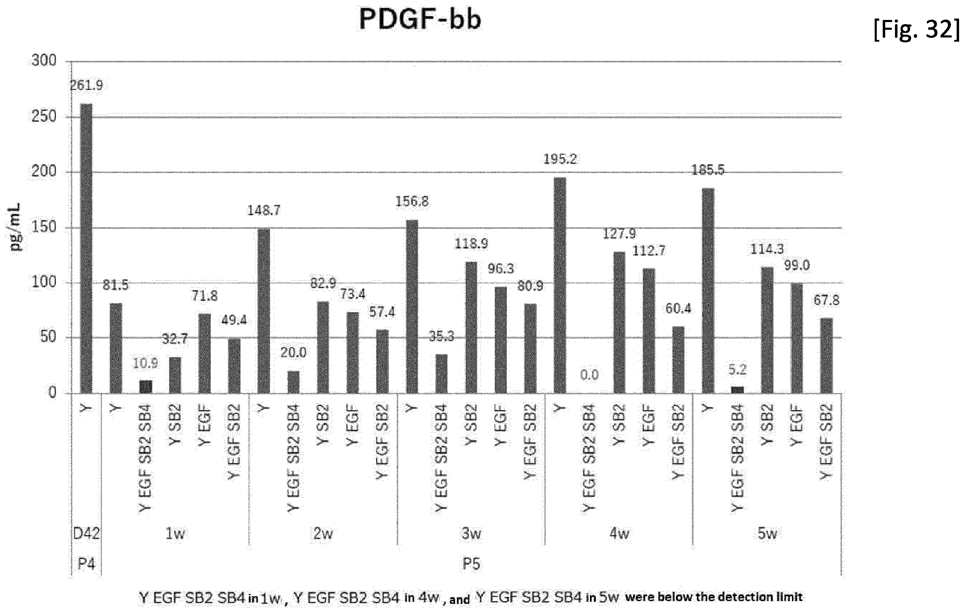

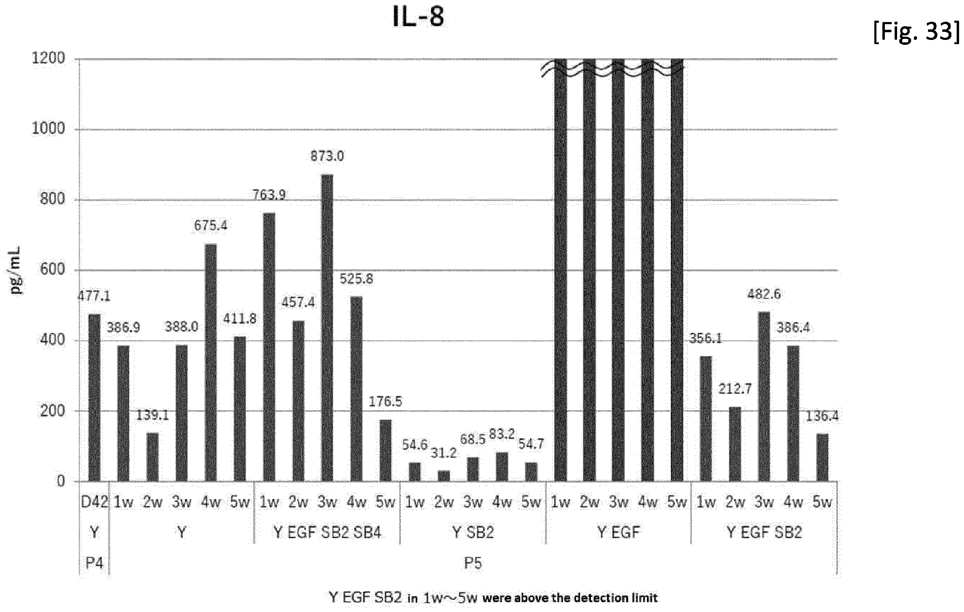

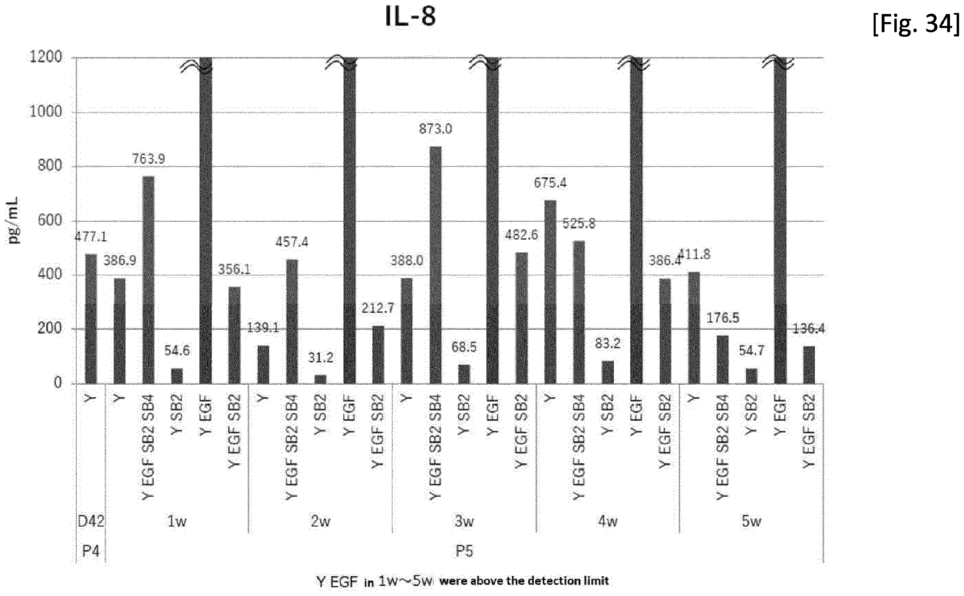

In addition, the culture supernatant and sample list of ELISA PDGF-bb and IL-8 CT09 P4 and P5 are shown in Fig. **30**. The results of classifying PDGF-bb by additive are shown in Fig. **31,** and the results of classifying it by week are shown in Fig. **32,** respectively. In addition, the results of classifying IL-8 by additive are shown in Fig. **33,** and the results of classifying it by week are shown in Fig. **34,** respectively.

From the above results, although not wishing to be bound by any theory, regarding the effects of the additives, it was found overall that addition of SB4, no addition of EGF, addition of Y from Day 10, no addition of SB2 or the addition thereof only at the final stage of culture, are desirable.

V-2. Mitochondrial Respiratory Capacity

In this Example, mitochondrial respiratory capacity was examined.

(Materials and Methods)

Corneal endothelial cells (HCEC) were obtained from donor corneas provided by SightLife (Seattle, WA, USA), and the HCEC were cultured according to a published protocol (Toda M, Ueno M, et al. Invest Ophthalmol Vis Sci. 2017;58:2011) with some modifications. HCEC of passages 2-5 were used for all experiments. SPs containing CST were passaged at low cell density. The SPs of cHCEC were identified by cell surface markers (CD24, 44, 105, 166) using phase-contrast microscopy images and flow cytometry.

(Results)

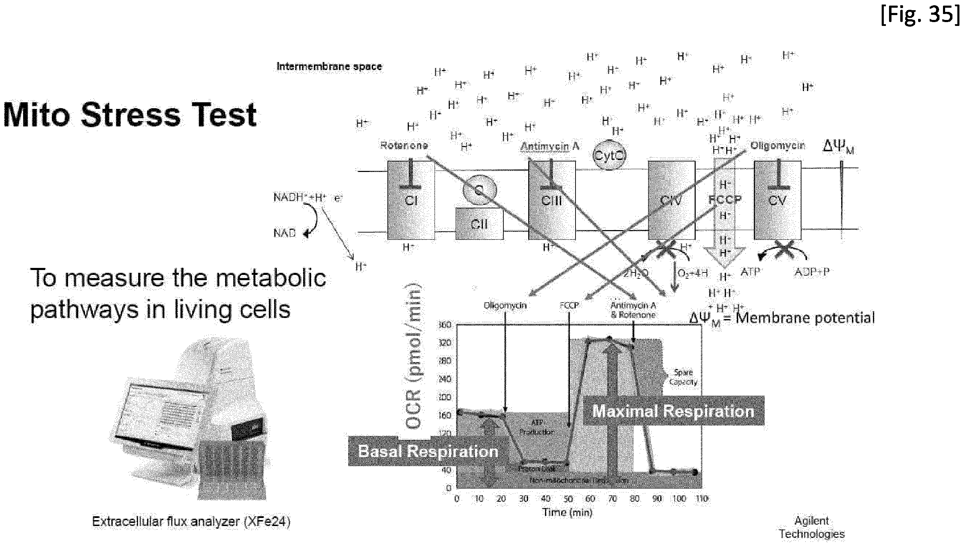

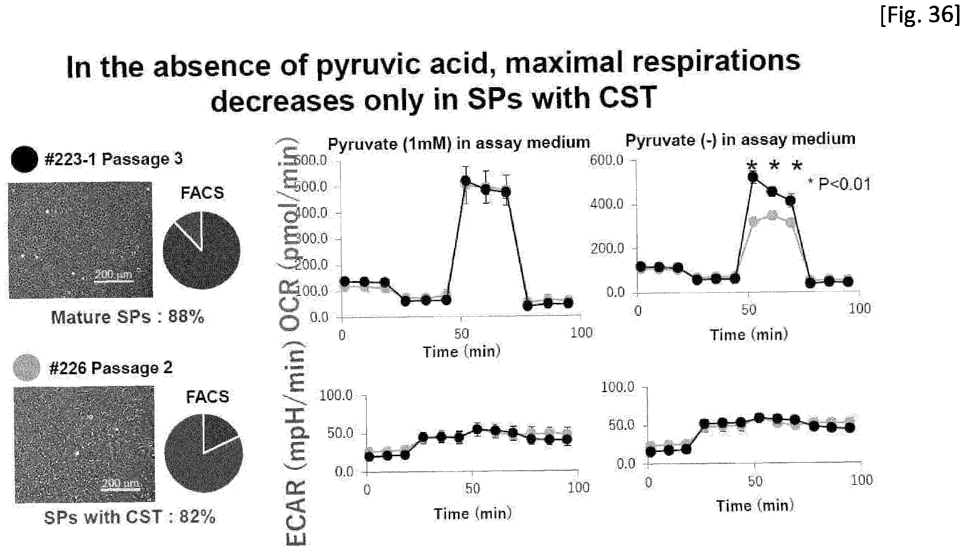

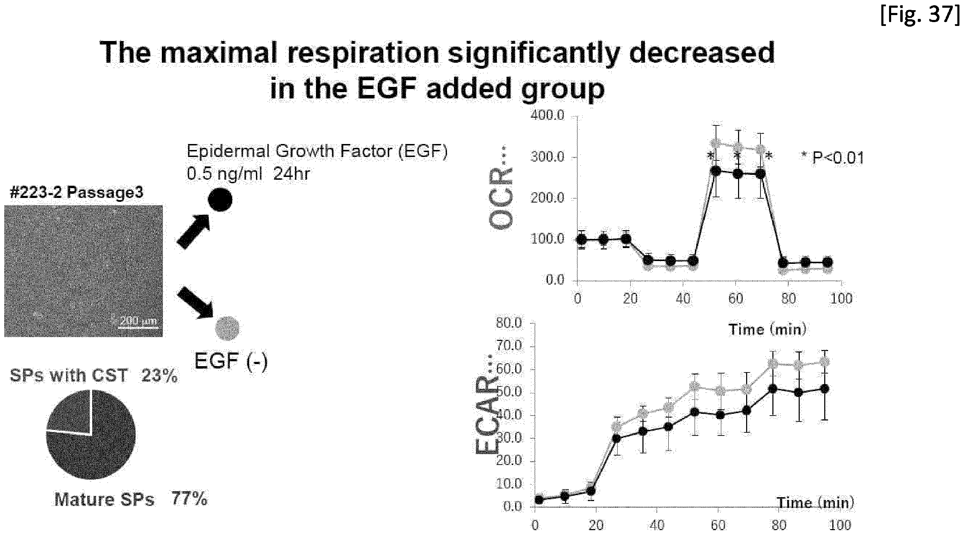

The results are shown in Figs. **35** to **37.**

From the above results, it was found that standard cells have high mitochondrial respiration capacity. In addition, it was found that EGF addition lowers the standard cell ratio, but correspondingly reduces mitochondrial respiratory capacity and oxidative phosphorylation (OXPHOS).

(Example 6: VI. Addition of ROCK inhibitor)

In this Example, the effect of adding a ROCK inhibitor (e.g., Y27632) on production of a human functional corneal endothelial cell capable of eliciting a human corneal function when infused into an anterior chamber of a human eye (the cell of interest) was investigated.

(Materials and Methods)

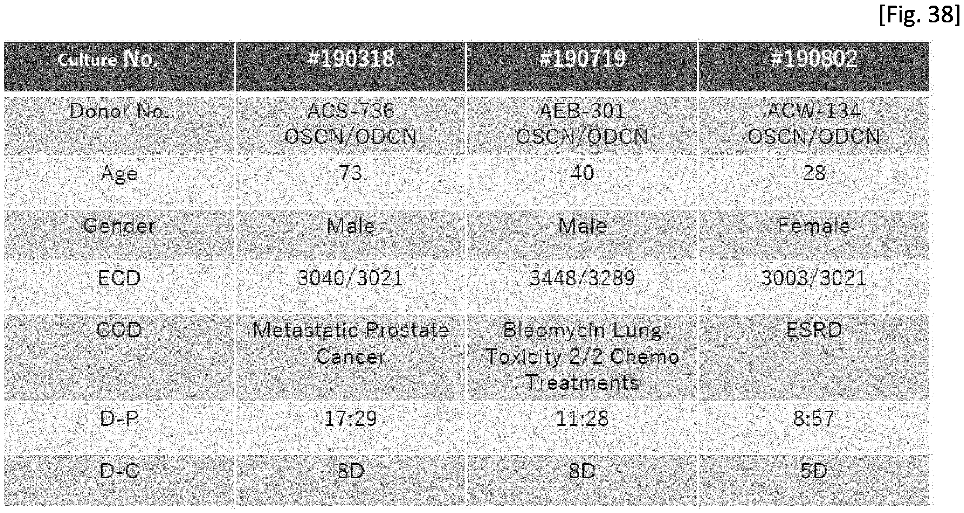

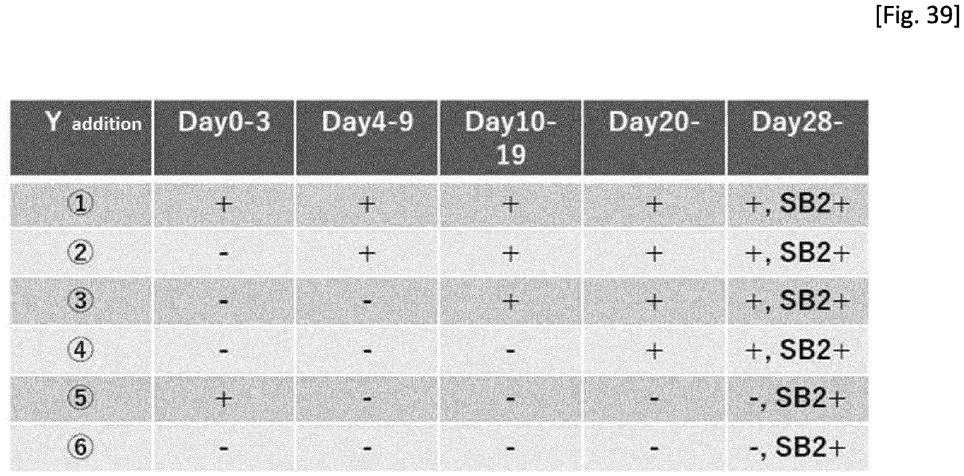

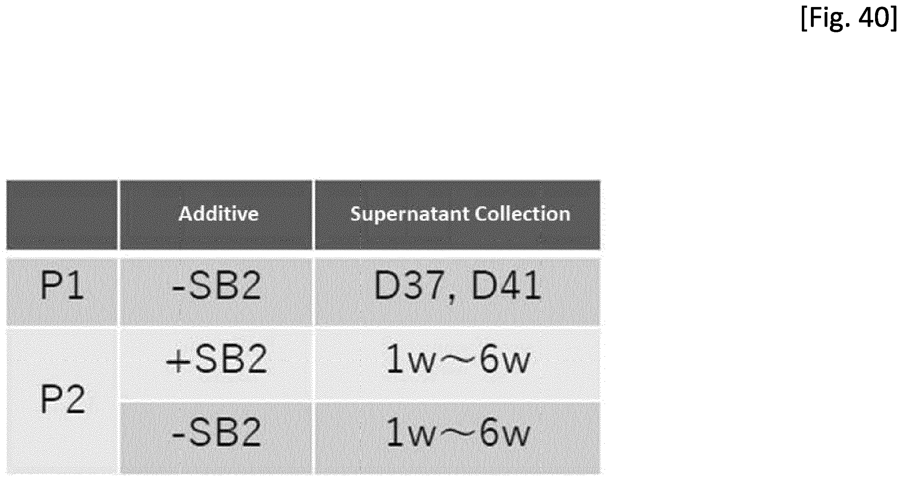

Donor information is shown in Fig. **38**. As to culture conditions, the Y addition timing is examined at P1 and P2 in #190719, and the Y addition timing is examined at P1 in #190802. Therefore, culture supernatants of each of 1w to 5w were collected under the conditions shown in Fig. **39**. In addition, in #190318, culture supernatants were collected at P1 and P2 under the conditions shown in Fig. **40.**

(Results)

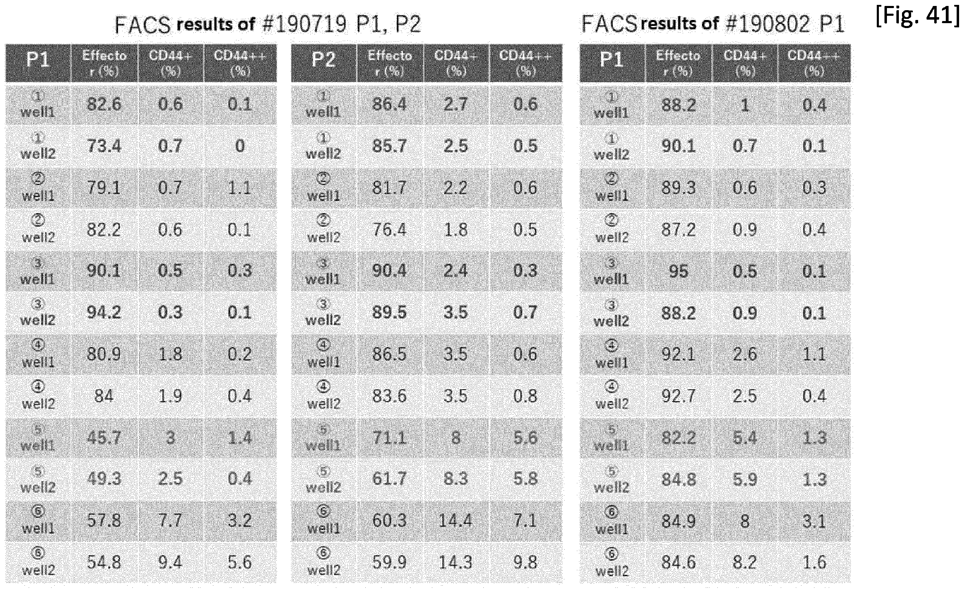

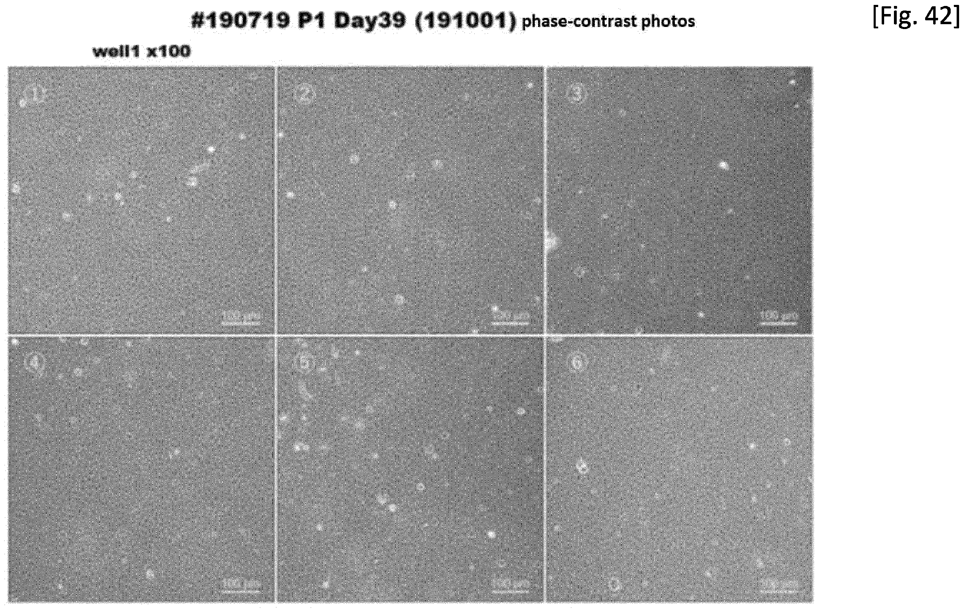

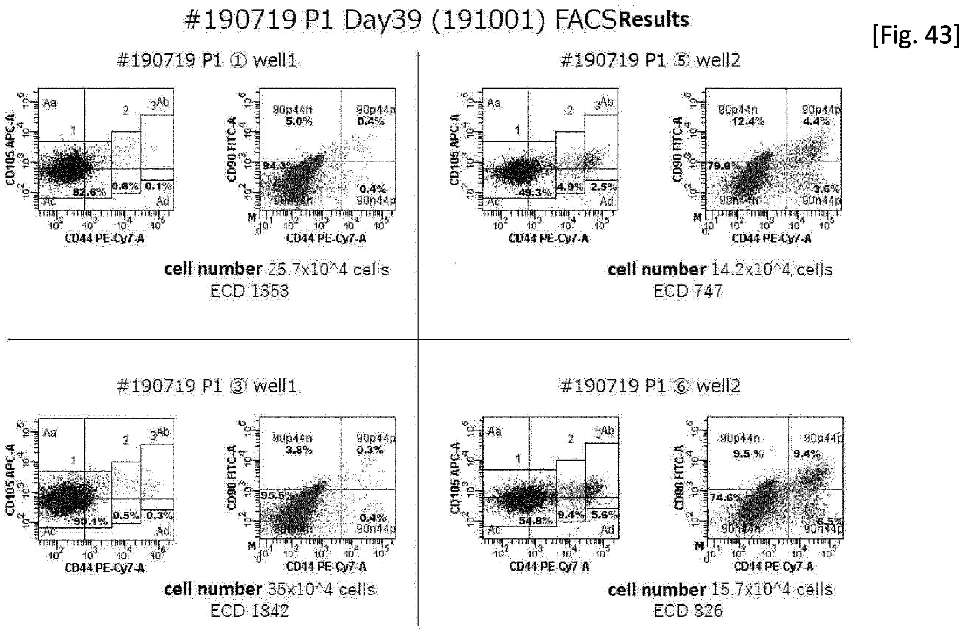

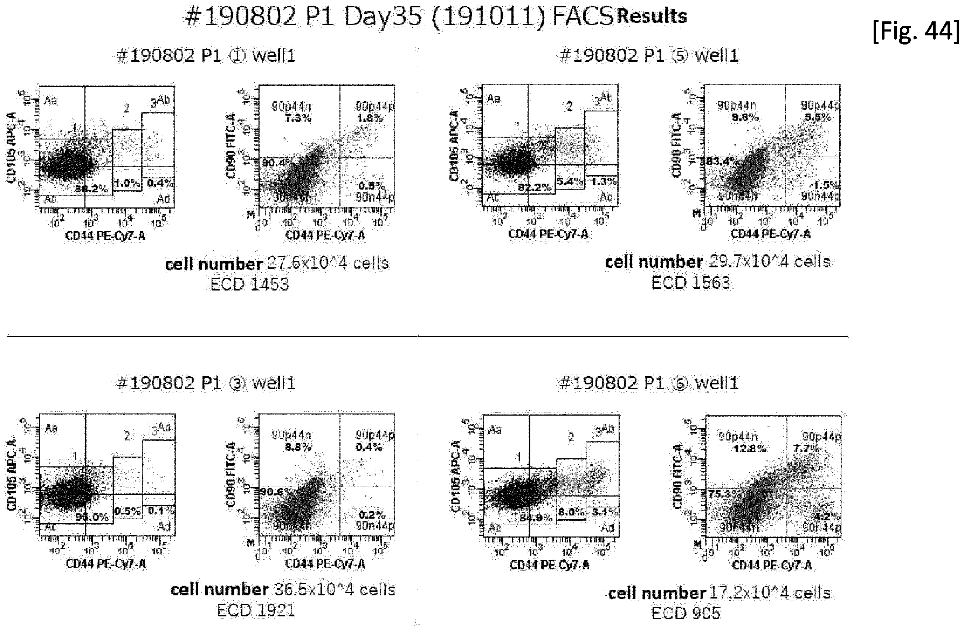

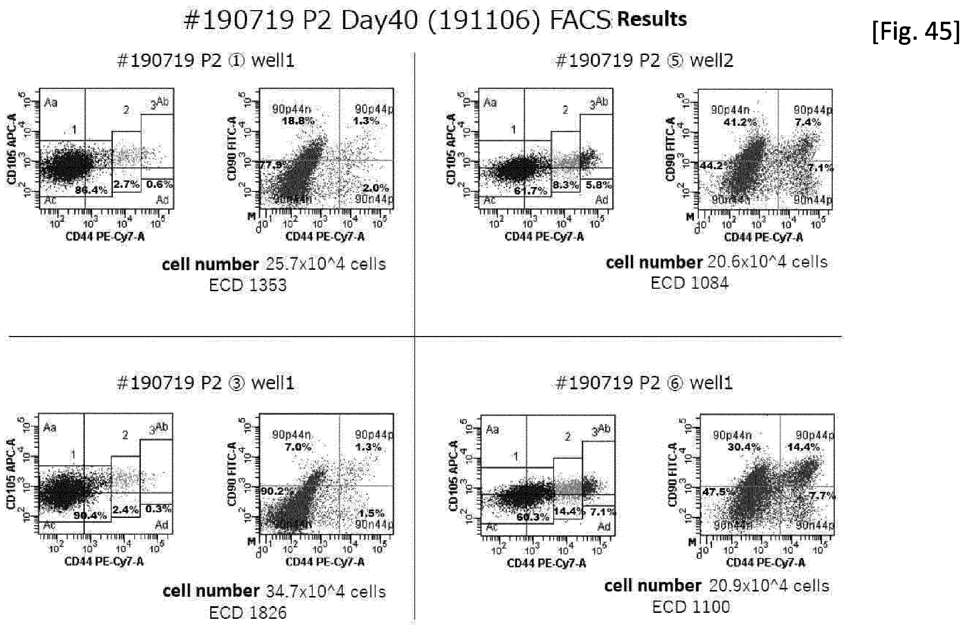

FACS results are shown in Figs. **41** and **43** to **45,** and cultured cell photographs are shown in Fig. **42.** From these results, the addition of the ROCK inhibitor on the 10th day after the start of culture resulted in a higher ratio of standard cells in all of the three experiments. Although not wishing to be bound by any theory, from these results, it is understood that it is preferable to culture in the presence of a ROCK inhibitor in the step of proliferation and/or differentiation or maturation. From these, it was found that efficient differentiation essential for obtaining corneal endothelial progenitor cells is induced in the present disclosure.

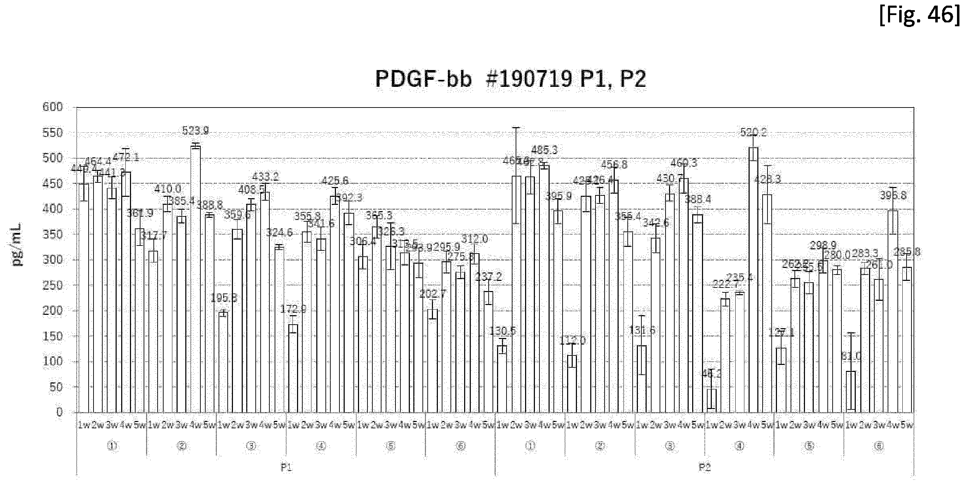

ELISA PDGF-bb measurement results (for each item) in the culture supernatant for #190719 are shown in Fig. **46.** As a result, in both experiments P1 and P2, even when a ROCK inhibitor was added 10 days after the start of culture, PDGF, one of the standards of mature standard cells, increased from differentiation to maturation; and it was shown to be meaningless to add expensive Y for adhesion purposes from the first day of culture under these culture conditions. This is probably because the addition at the stage of differentiation does not inhibit dedifferentiation. It was also found that P1 peaked at 4w and slightly decreased at 5w.

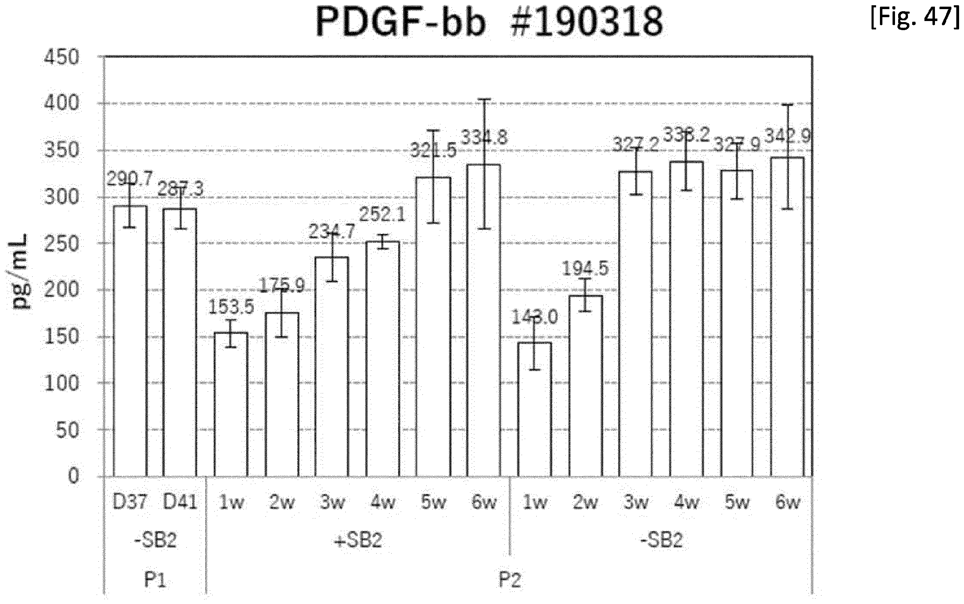

ELISA PDGF-bb measurement results (for each item) in the culture supernatant for #190318 are shown in Fig. **47.** As a result, it was found that the method in which the p38 MAPK inhibitor SB2 was not added resulted in high PDGF levels at 3 w, indicating early induction of differentiation.

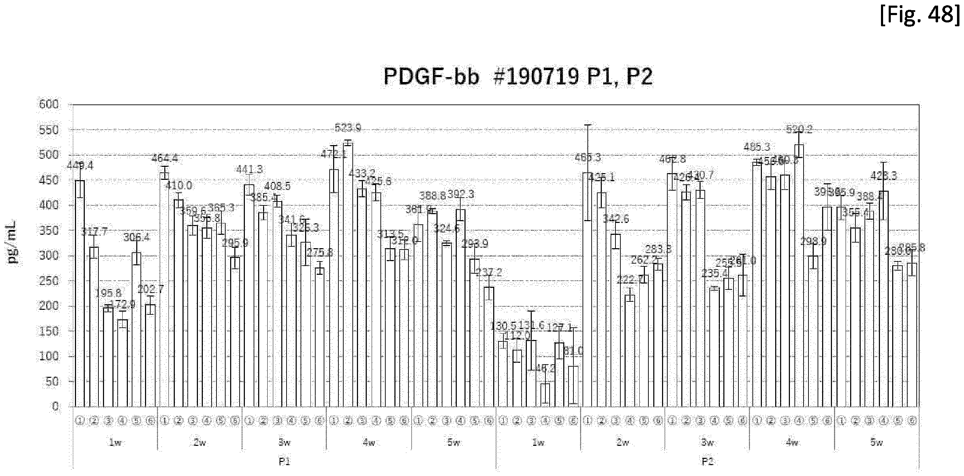

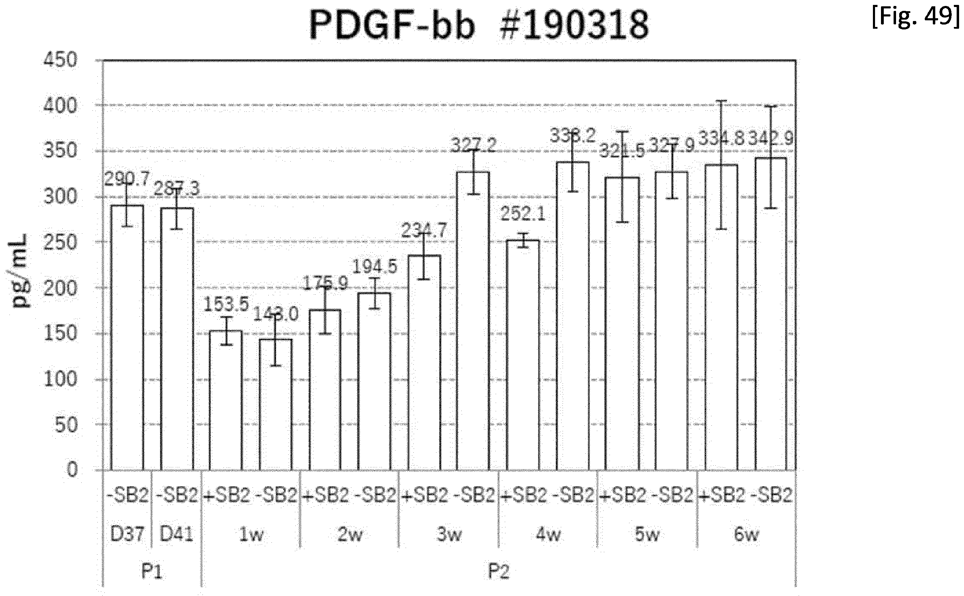

ELISA PDGF-bb measurement results (weekly) in the culture supernatant for #190719 are shown in Fig. **48,** and the ELISA PDGF-bb measurement results (weekly) in the culture supernatant for #190318 are shown in Fig. **49,** respectively. From these results, although not wishing to be bound by any theory, it was found that, in P1 and P2 for #190719, addition of Y27632 resulted in high PDGF levels and slightly low levels in the P1 growth phase. Although not wishing to be bound by any theory, it was also found that PDGF is an independent factor with little relation to the presence or absence of SB203580.

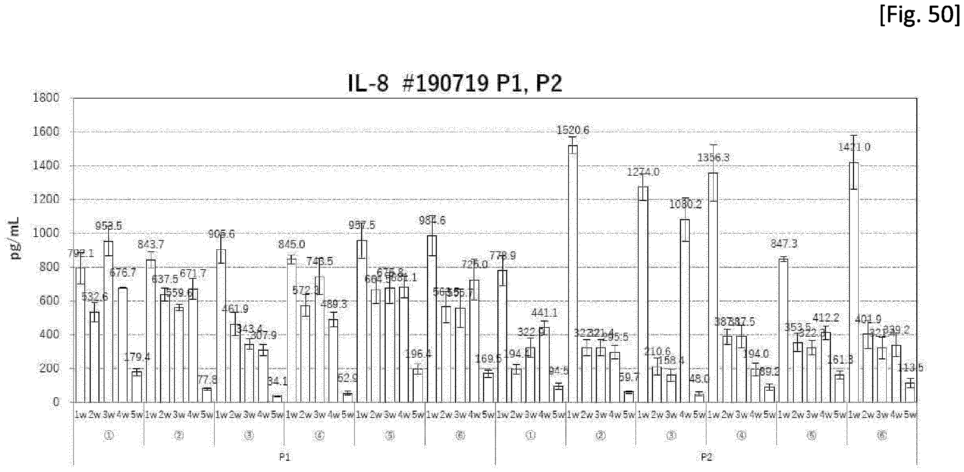

ELISA IL-8 measurement results (for each item) in the culture supernatant for #190719 are shown in Fig. **50.** As a result, in both experiments P1 and P2, even when a ROCK inhibitor was added 10 days after the start of culture, IL-8, one of the standards of mature standard cells, was lowest from differentiation to the maturation stage. It was also found that the P1 level was high during the proliferation phase and decreased as the differentiation progressed, and that the P2 level also decreased as the differentiation progressed.

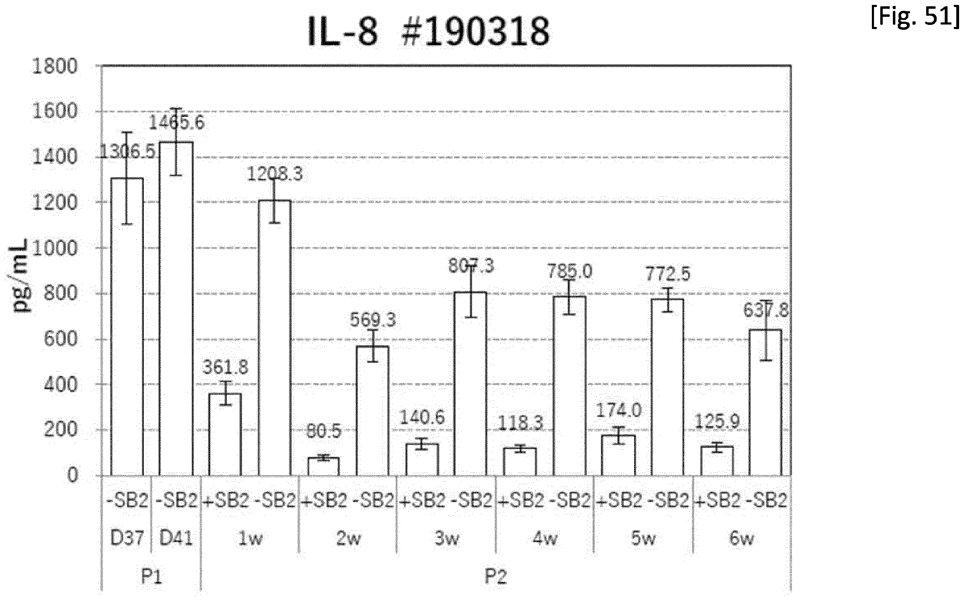

ELISA IL-8 measurement results (weekly) in the culture supernatant for #190318 are shown in Fig. **51.** As a result, it was found that IL-8 varies almost depending on the presence or absence of SB2.

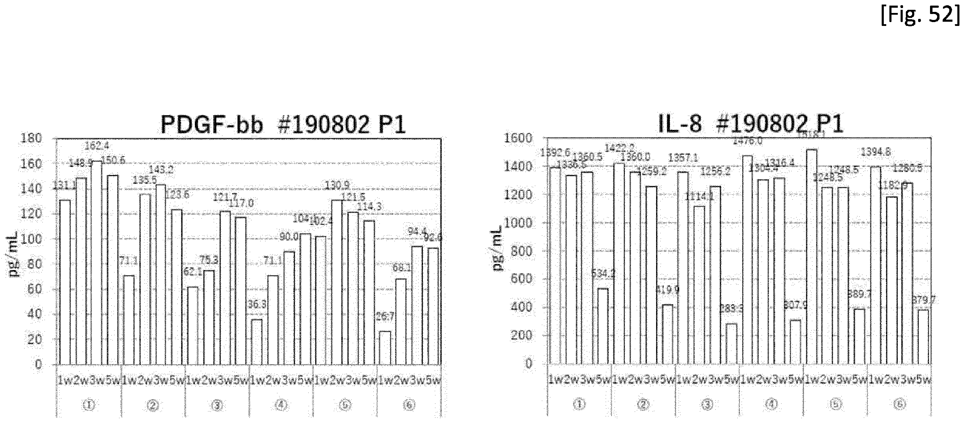

ELISA PDGF-bb and IL-8 measurement results (for each item) in the culture supernatant for #190802 are shown in Fig. **52.** From these results, it was found that PDGF increased dependently on differentiation and induction by a ROCK inhibitor (Y27632), and IL-8 decreased dependently on a p38 MAPK inhibitor (SB203580).

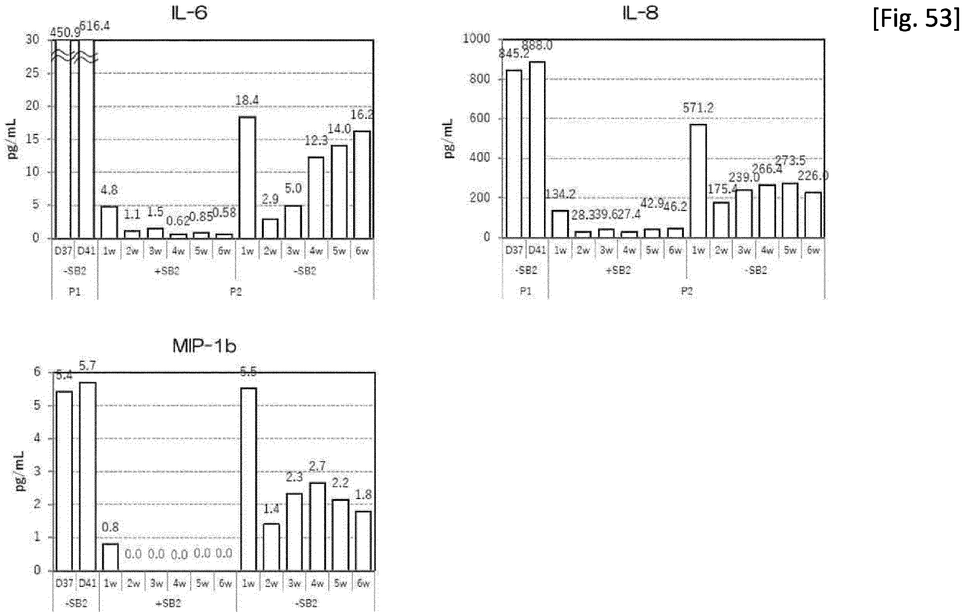

Results of cytokine measurement (BioPlex) in the culture supernatant for #190318 (for each item) are shown in Fig. **53.** As a result, it was found that all three of IL-6, IL-8 and MIP-1b are dependent on the p38 MAPK inhibitor (SB203580) .

In view of the foregoing results as well, in the present disclosure, it was ascertained that efficient differentiation essential for obtaining corneal endothelial progenitor cells was induced.

(Example 7: VII. Addition of ROCK inhibitor)

In this Example as well, following on from Example 6, conditions regarding the ROCK inhibitor were further investigated. Details are given below.

(Materials and Methods)

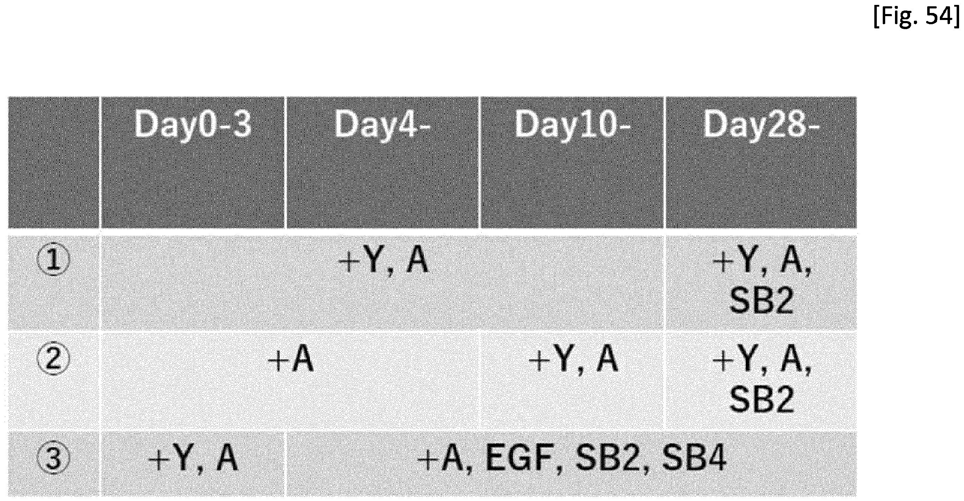

Using P4 of #190719, the culture supernatant was collected at 1 to 5 w to examine the difference in accordance with the presence or absence of the ROCK inhibitor. The additive conditions are shown in Fig. **54.** Donor information was as follows: #190719S/D, AEB-301, OSCN/ODCN, Age= 40 / Gender = M, ECD = 3448 / 3289, COD: Bleomycin Lung Toxicity 2/2 ChemoTreatments, D-P = 11:28, D-C = 8D.

(Results)

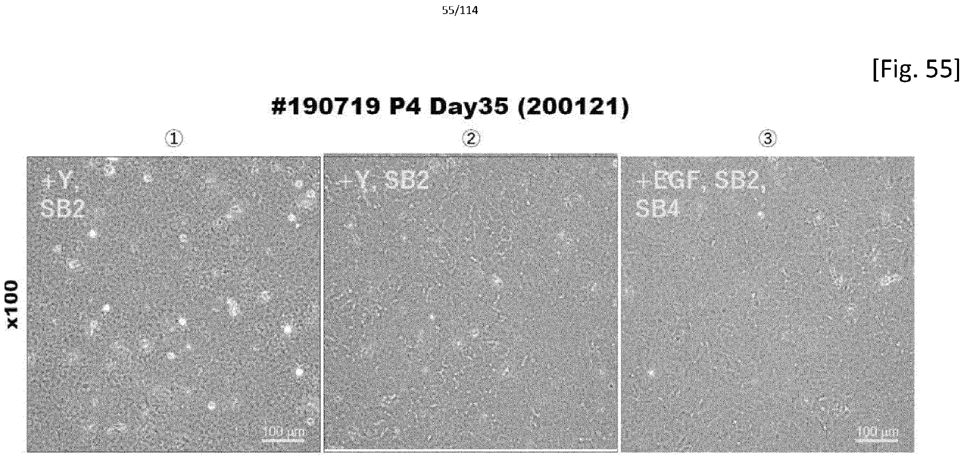

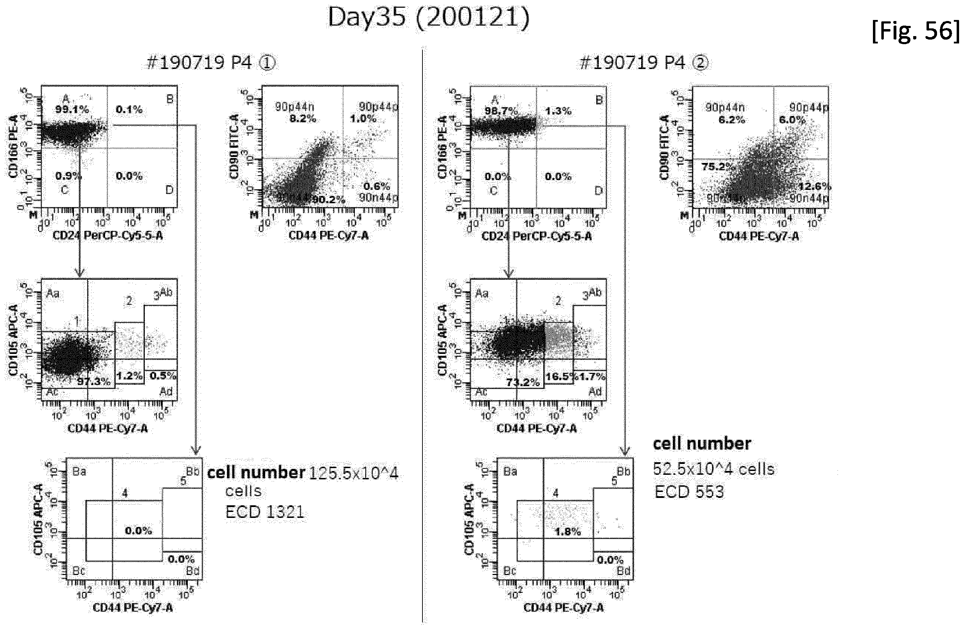

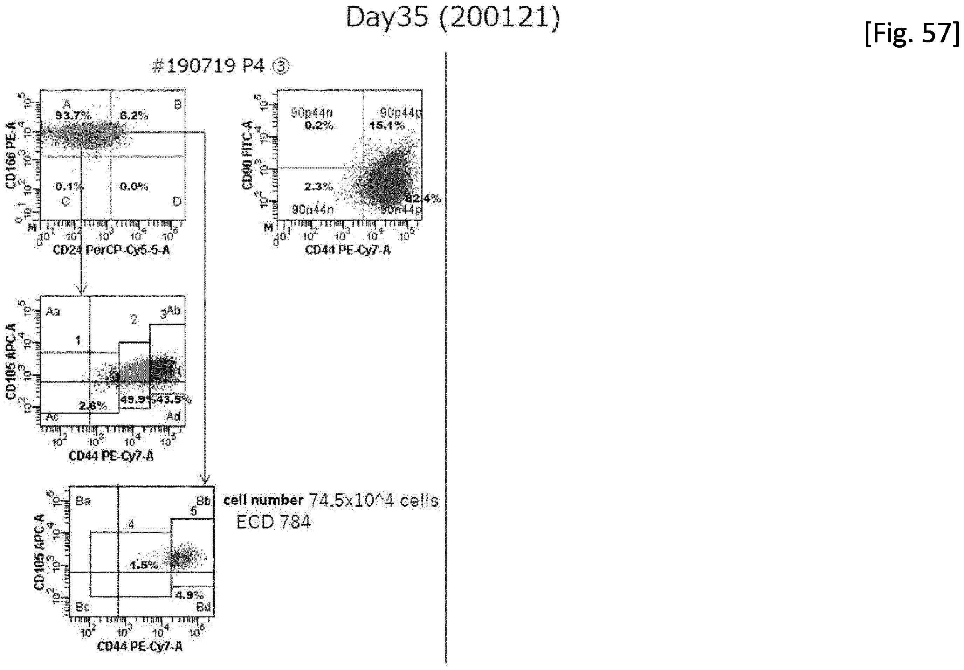

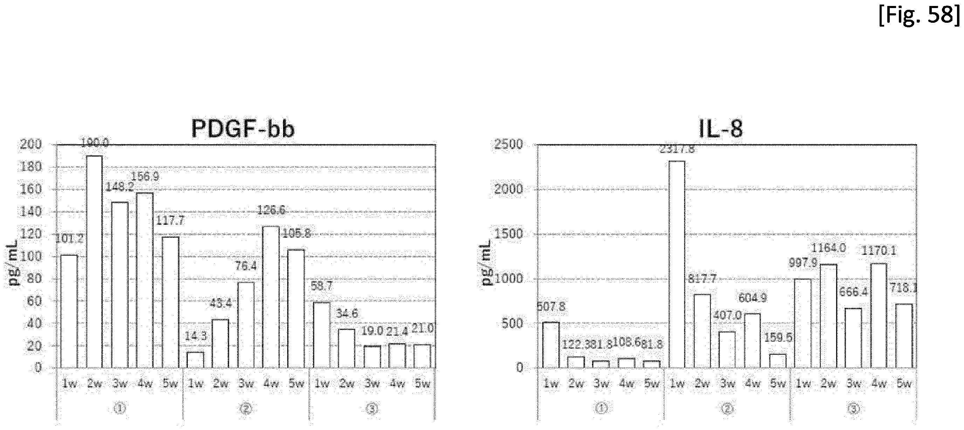

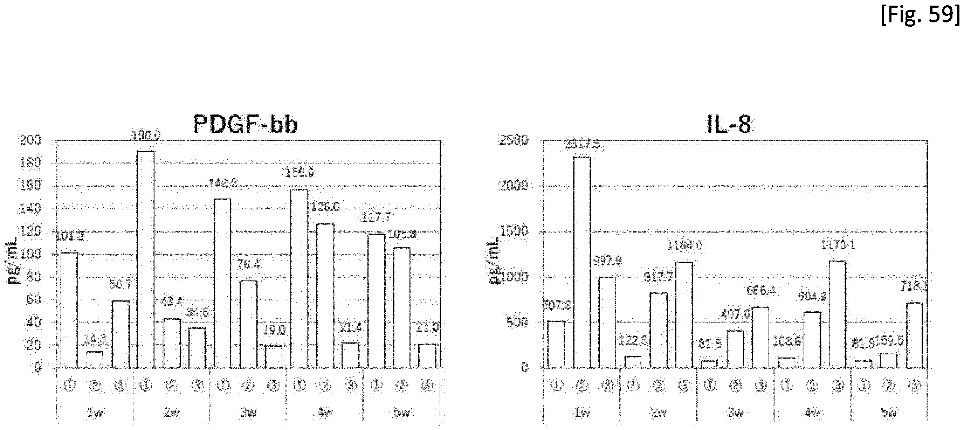

The photographs of DAY35 are shown in Fig. **55,** and the FACS results are shown in Figs. **56** and **57,** respectively. Furthermore, the results for each item of PDGF-bb and IL-8 by ELISA are shown in Fig. **58,** and the weekly results thereof are shown in Fig. **59,** respectively.

In view of the above results, even in the three-group comparison experiment, no mature standard cells were detected by FACS even when the ROCK inhibitor was added at the start of the culture for 3 days. It was also found that PDGF, which is one of the standards, was at a low level and does not satisfy the standard values. IL-8 levels were also higher than the standard, indicating the need for addition at the stage of differentiation.

From the above, in one embodiment, it is understood that it is preferable to perform the culturing in the presence of a ROCK inhibitor in the step of proliferation and/or differentiation or maturation.

(Example 8: VIII. Adhesion Enhancement by ROCK inhibitor)

Next, ROCK inhibitors were tested for adhesion enhancement. In order to investigate whether the enhancement of adhesion by the ROCK inhibitor is related to standard cell manufacture, cells cultured under the following conditions were compared and verified at P0 with or without Y addition.

(Materials and Methods)

AEM-510 (62Y, Male), ECD ODCN: 3058, and OSCN: 3058 were used, which were stored at 4°C on January 30th, corneal treatment was performed on January 31st at 10:00, OD collagenase treatment was started at 10:45, and OS collagenase treatment was started at 11:05. Thereafter, seeding was started at 14:45 and cultured with Y-, A (ascorbic acid) + for OD and Y +, A + for OS. At 10:00 on February 4, the medium was replaced, and both eyes: Y+, A+ were photographed (phase contrast x4, x10).

(Results)

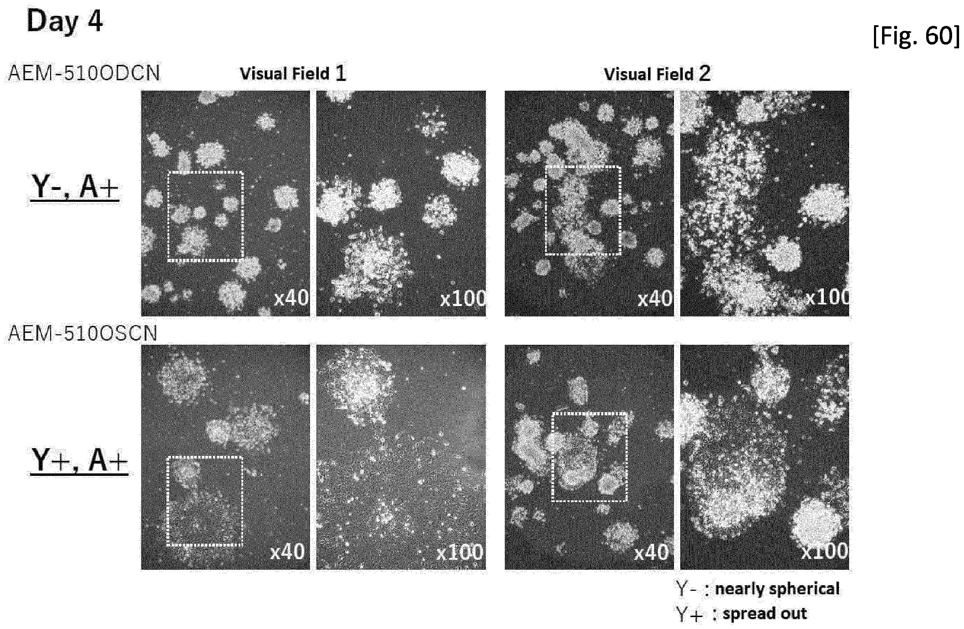

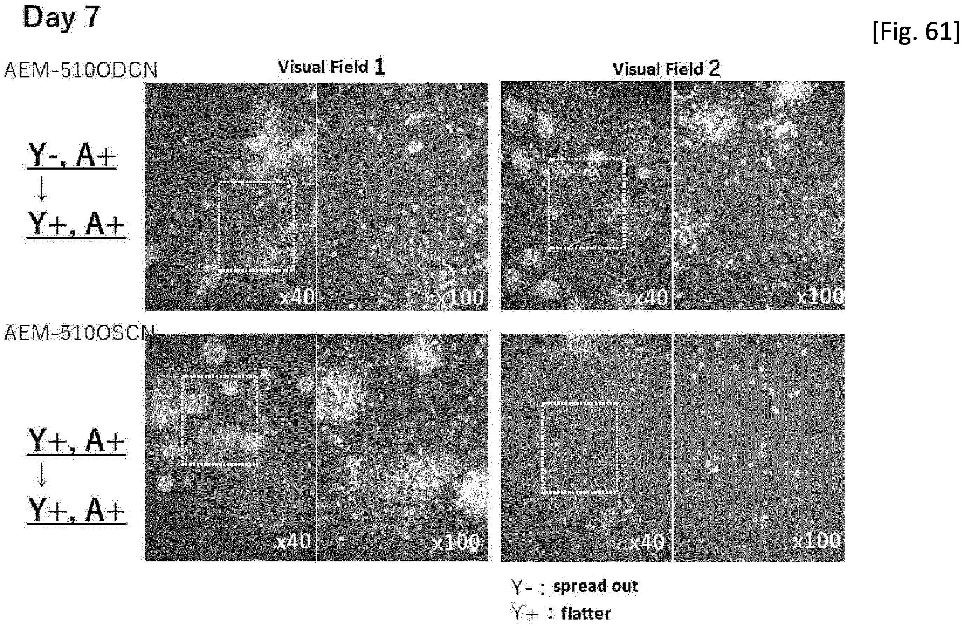

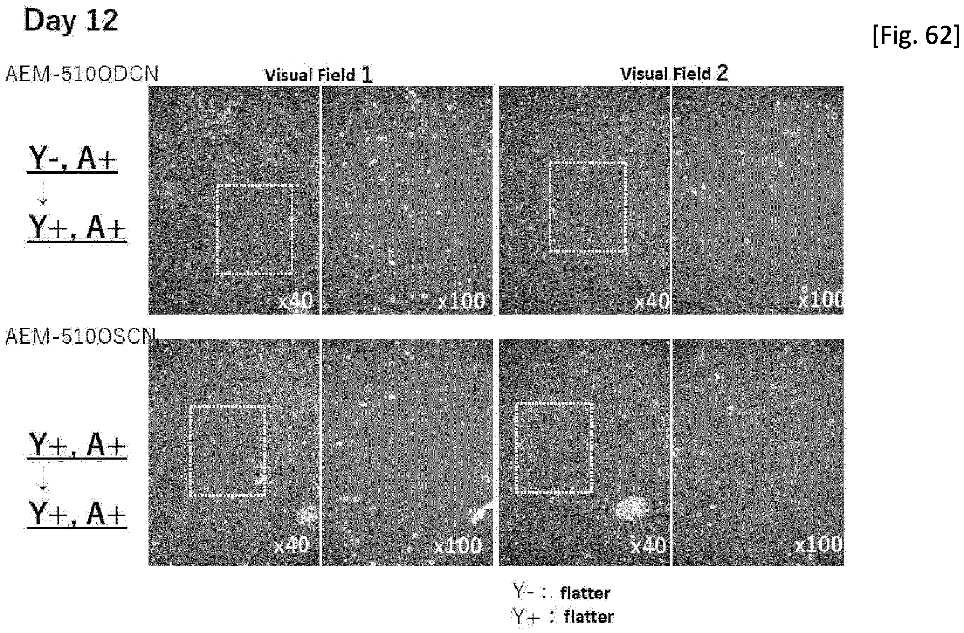

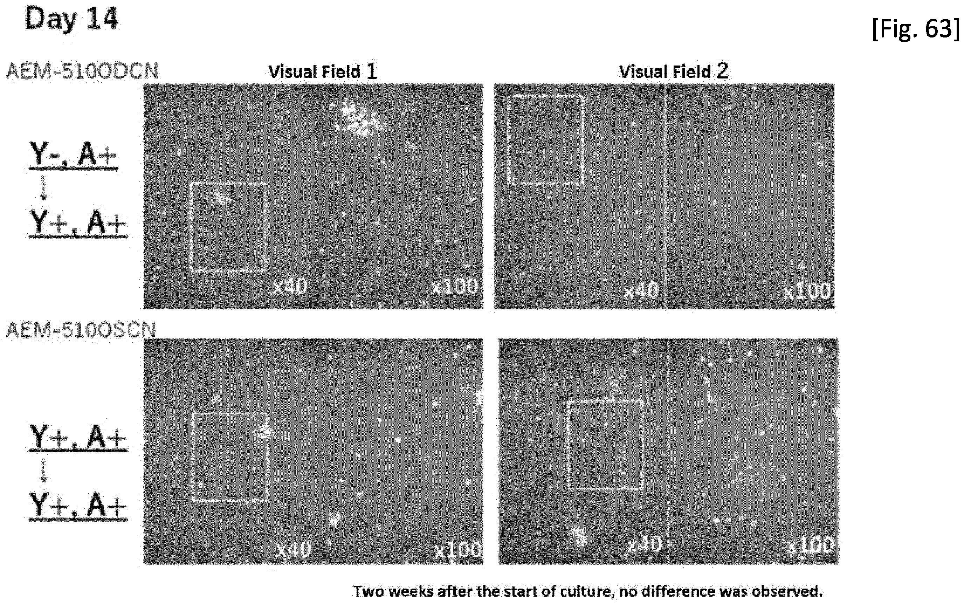

The results are shown in Figs. **60** to **63.** In the embodiment shown in this Example, although the ROCK inhibitor Y27632 certainly promoted the adhesion to the incubator after seeding the cells, no difference was observed on the 14th day of culture even though Y27632 was added for the first time after the 4th day. It is thus understood that culturing in the presence of a ROCK inhibitor can also provide standard cells, in the step of proliferation and/or differentiation or maturation.

(Example 9: IX. Epigenome Regulation by Signal Transduction from Mitochondria to Nucleus)

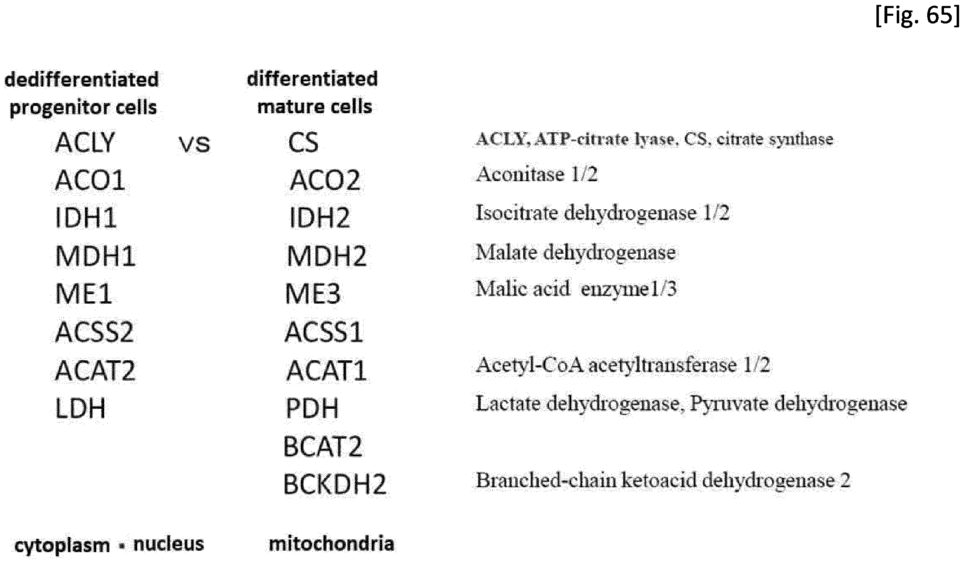

In this Example, epigenome regulation by signal transduction from mitochondria to the nucleus was investigated.
Cell Metab. 2015 Mar 3;21(3):349-50., Trends inCell Biology, June 2017, Vol.27, No.6, and Sheikh et al. Nature Rev. Genetics, 2019 provide detailed information on epigenetic regulation by signal transduction from mitochondria to the nucleus. Epigenetics regulation by metabolites, cell senescence and disruption of cell differentiation can be considered as shown in the schematic diagram of Fig. **64.** As to organelle-localized enzyme, the ones shown in Fig. **65** are conceivable, and the isozymes expressed in the dedifferentiated progenitor cells described above are present in the cytoplasm and nucleus, thereby acetylating histones via nuclear translocation AcCoA.
In this Example, it was verified that the expression of proteins such as metabolic enzymes is significantly different between standard cells and non-standard cells that have undergone phase transition, thereby demonstrating clear differences in organelle-selective localization of isoenzymes.

(Materials and Methods)

A so-called DAVID analysis was performed. The procedure is shown below.

(HCEC)

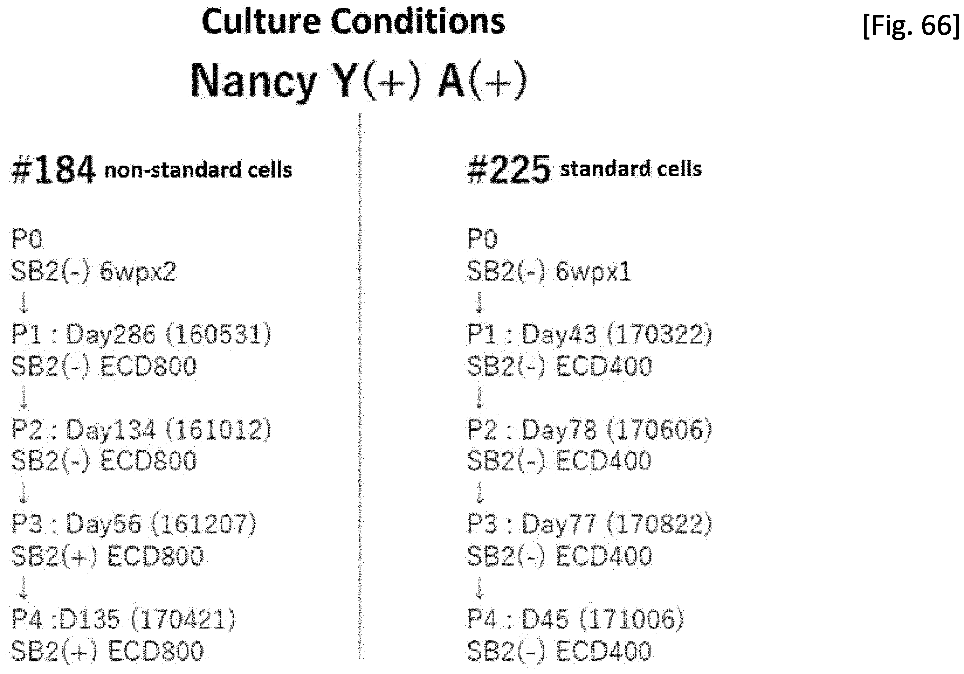

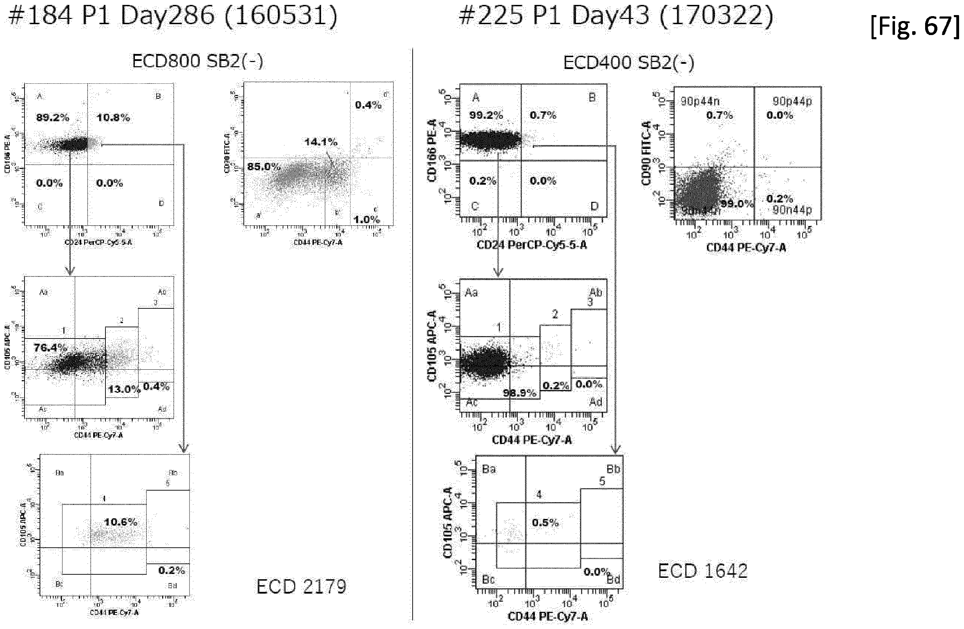

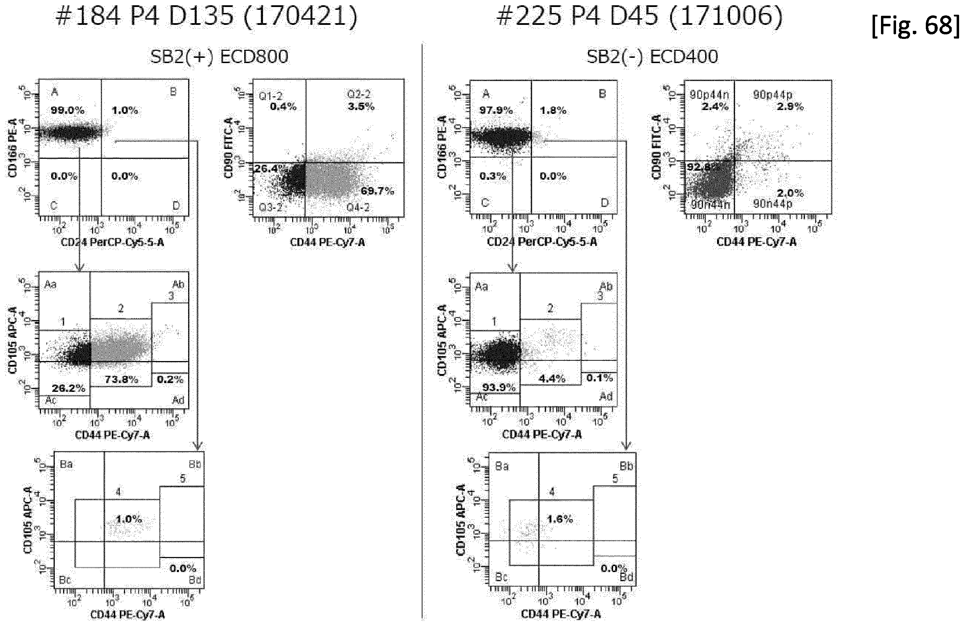

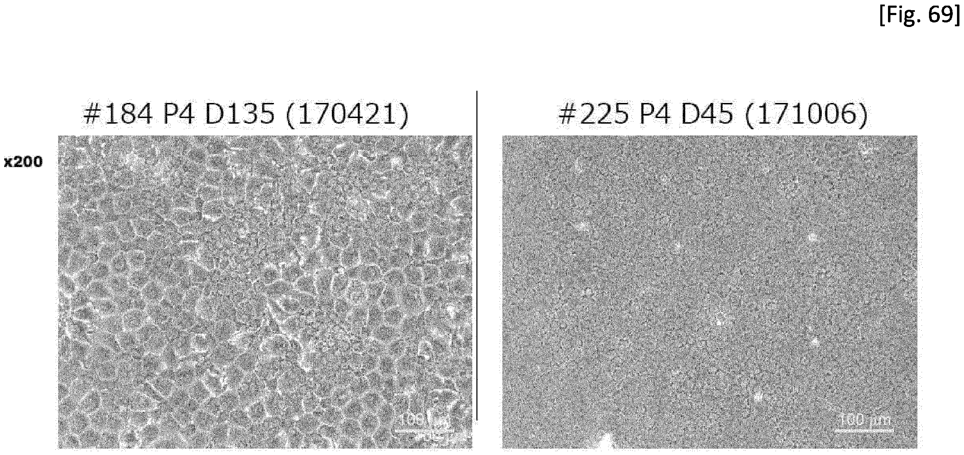

First, HCEC were cultured according to a published protocol with some modifications. Descemet's membrane containing CECs was detached from the donor cornea and digested with 1 mg/mL collagenase A (RocheApplied Science, Penzberg, Germany) for 2 hours at 37°C. HCECs obtained from a single donor cornea were seeded into one well of type I collagen-coated 6-well plates (Corning, Inc., Corning, NY). Media were prepared according to published protocols. When HCEC reached confluence, they were harvested with 10× TrypL Select (ThermoFisher Scientific, Inc., Waltham, MA) treatment for 12 minutes at 37°C and then passaged. HCEC of passage 2-3 were used for all experiments. Donor information was as follows: #184, ABF-9560SCN/ODCN, Age = 11, Gender = Female, ECD = 3207/3588; and #225, ABZ-612 OSCN/ODCN, Age= 27, Gender = Male, ECD = 3195 / 3425. In addition, the culture conditions are as shown in Fig. **66.** The FACS results at P1 and P4 are shown in Figs. **67** and **68,** and the respective cell photographs are shown in Fig. **69,** respectively.

(Cultured Human Corneal Endothelial Cells (cHCECs) Passage and Preparation of Cell Suspension)

All work other than microscopic photography was performed in a safety cabinet, and when entering the culture room, hands were washed thoroughly and a mask and gloves were worn.
PBS(-) and medium (Nancy medium) were warmed to 37°C in advance, and the added reagent (gray box) was returned to room temperature. The cells in culture were taken out, the lot number was confirmed, and the cells were observed under a phase-contrast microscope. Photographs were taken at 40x magnification (24-well: 1 location, 12-well: 1 location, 6-well: 2 locations, T-25: 3 locations) and 100x magnification (24-well: 2 locations, 12-well: 2 locations, 6-well: 3 locations, T-25: 3 locations) with a phase-contrast microscope camera. The cells were placed in a safety cabinet and the culture supernatant was collected into tubes (1.5-mL or 15-mL) using a Pipetman or disposable pipette. PBS(-) (24-well: 500 mL, 12-well: 1 mL, 6-well: 2.5 mL, T-25: 7 mL) was injected (first time), and the culture vessel was gently shaken to wash the inside of the well. PBS (-) in the well was removed with a disposable pipette, and PBS (-) (24-well: 500 mL, 12-well: 1 mL, 6-well: 2.5 mL, T-25: 7 mL) was injected (second time), and the culture vessel was gently shaken to wash the inside of the well. PBS (-) in the well was removed with a disposable pipette, and PBS (-) (24-well: 500 mL, 12-well: 1 mL, 6-well: 2.5 mL, T-25: 7 mL) was injected (3rd time). A 10-minute timer was started, and photographs were taken at 40x magnification (24-well: 1 location, 12-well: 1 location, 6-well: 2 locations, T-25: 3 locations) and 100x magnification (24-well: 2 locations, 12-well: 2 locations, 6-well: 3 locations, T-25: 3 locations) with a phase-contrast microscope camera. The cells were allowed to stand in a $CO_2$ incubator for 10 minutes (until the timer sounded). TrypLE Select (10x) was warmed to 37°C in a constant temperature bath and placed in a safety cabinet before the 10 minute timer had expired. TrypLE Select (10x) was diluted to (5x) with PBS (-) (1:1 dilution), and PBS(-) was removed with a disposable pipette and completely removed with P-1000. TrypLE Select (5x) was injected (24-well: 200 mL, 12-well: 400 mL, 6-well: 1 mL, T-25: 2.5 mL) and allowed to stand in a $CO_2$ incubator for 15 minutes. It was confirmed under a phase-contrast microscope that more than half of the cells were rounded, and the cells were detached from the bottom surface by tapping. When the round cells were less than half of the whole, the cells were again allowed to stand in the $CO_2$ incubator for 2 to 5 minutes (the maximum enzymatic treatment time with TrypLE was set to 20 minutes in total, and even when the round cells were less than half of the cells, after a total of 20 minutes, the cells were used in the next step).

Subsequently, the cells were placed in a safety cabinet, and repeatedly aspirated and discharged to the bottom with a P-1000 chip to detach the cells and collect them in a 1.5-mL Proteosave tube or a 15-mL tube. Here, the light of the safety cabinet may be turned on. Using P-1000, the culture supernatant collected per well (24-well: 200 mL, 12-well: 400 mL, 6-well: 1 mL, T-25: 2.5 mL) was added, followed by washing up wells and collecting in 21 tubes. After centrifugation with a centrifuge (300 × g; Eppendorf #5452000034 desktop centrifuge at 2,100 rpm for 3 minutes, TOMY #LC-220 tube centrifuge at 1,200 rpm for 5 minutes), the chip was replaced with a P-1000 chip (long), and the supernatant was removed. The chip was replaced with a new one, and the collected medium was added per well (24-well: 100 mL, 12-well: 200 mL, 6-well: 500 mL, T-25: 1 mL) to suspend the cells. After centrifugation with a centrifuge (300 × g; Eppendorf #5452000034 desktop centrifuge at 2,100 rpm for 3 minutes, TOMY #LC-220 tube centrifuge at 1,200 rpm for 3 minutes), the chip was replaced with a P-1000 chip (long), and the supernatant was removed. The chip was replaced with a new one, and the collected medium was added per well (24-well: 200 mL, 12-well: 400 mL, 6-well: 1 mL, T-25: 2.5 mL) to suspend the cells. A chip was attached to the P-20, and 10 mL was quickly extracted from the cell suspension and placed in a 96-well plate for measurement (FALCON: 35591, 96-well assay plate U non-sterile polystyrene without bottom lid). Ten mL of trypan blue was added to 10 mL of the cell suspension, mixed by pipetting, 10 mL was taken, and the number of viable cells was counted using a hemocytometer.

Cell Number Calculation

$$\text{Number of viable cells (count value)} / \underline{\hspace{1cm}} \text{(number of compartments)} \times 2 \times 10^4 = \text{number of viable cells (cells/mL)}$$

$$\text{Number of viable cells (cells/mL)} \times \text{suspension volume (mL)} = \text{total number of viable cells (cells)}$$

For FACS analysis, $16 \times 10^4$ cells (one type of antibody + negative control) were dispensed into Proteosave tubes and the rest were passaged. FACS required $8 \times 10^4$ cells per antibody type. Passages were seeded at an Endothelial Cell Density (ECD: cells/mm$^2$) of 400 (191001 time point) (24-well: $7.6 \times 10^4$ cells, 12-well: $15.2 \times 10^4$ cells, 6-well: $38 \times 10^4$ cells, T-25: $100 \times 10^4$ cells).

(Adjustment of Medium)

The amount of medium plus something extra to be (24-well: 500 mL, 12-well: 1 mL, 6-well: 2.5 mL, T-25: 7 mL) used was taken into a suitable tube and an additive reagent (each 1/1000 volume for the medium volume) was added. Since the added reagent was sensitive to light, the light in the safety cabinet was turned off during preparation and medium replacement. The plate was shaken lengthwise and crosswise to evenly seed the cells, and cultured in a $CO_2$ incubator.

(FACS Measurement)

<Necessary Reagents>

□ cHCEC suspension ($16 \times 10^4$ cells required for one type of FACS antibody + Negative Control (NC))
□ FACS Buffer (+NaN$_3$)

BSA (globulin-free) 0.5%/NaN$_3$ 0.05%/1×PBS 100 mL
FACS Buffer (-NaN$_3$)
BSA (globulin-free) 0.5%/1×PBS 100mL
Antibodies for FACS (ex. CD90-FITC/CD166-PE/CD24-PerCP-Cy5.5/CD44-PE-Cy7/CD105-APC)
Cell strainer
Ice box

<FACS Measurement>

The FACS was started up, and an antibody solution (hereinafter light-shielded, on ice) was prepared as follows.

| antigen | fluorescent dye | Volume (μL) × sample type plus something extra (μL) |
|---|---|---|
| CD90 | FITC | 4 |
| CD166 | PE | 4 |
| CD24 | PerCP-Cy5.5 | 1 |
| CD44 | PE-Cy7 | 0.25 |
| CD105 | APC | 1 |
| FACS | Buffer(+NaN$_3$) | 9.75 (total vol. 20μL) |

When there were multiple samples, the extra solution was adjusted.
The cell suspension was centrifuged at 250×g (1,800 rpm) for 2 minutes at 4°C, the supernatant was removed using P-200, the concentration was adjusted to $4 \times 10^6$ cells/mL and suspended in FACS Buffer (+NaN3). When the suspension was less than 30 μL, it was suspended in 30 μL and 20 μL was used for the reaction and 10 μL for NC.
Twenty μL of the antibody solution and 20 μL of the suspension were mixed well by pipetting, and the tube was wrapped in aluminum to shield from light and rotated at 4°C for 2 hours (up to 4 hours maximum) on a rotator. Centrifugation was performed at 250×g (1,800 rpm) for 2 minutes at 4°C, the supernatant was discarded, followed by washing with 100 μL of FACS Buffer (+NaN3). Centrifugation was performed at 250×g (1,800 rpm) for 2 minutes at 4°C, the supernatant was discarded, followed by suspending in 350 μL of FACS buffer (-NaN3), and transferring through a cell strainer to a 5-mL tube. NCs were suspended in FACS Buffer (-NaN3) to a total of 350 μL of the rest of the initial suspension, and transferred to a 5-mL tube through a cell strainer. During the FACS measurement, the sample was kept light-shielded and on ice, the FACS laser value and target cell range were determined, and a file was created (to follow: Open Book → Open Syringe → Green → Cytometer tab: Laser → Area scaling for HCEC label: FSC 0.5/Blue 0.75/Red 0.80). The NC was set to SIT and allowed to run briefly (Flow rate: set to medium, Acquire data 5 seconds → Remove sample from SIT and put on ice) to check the fluorescence parameter graph and target cell range. It was confirmed whether a peak appeared between 102 and 103 for the parameter, and if there was a deviation, the numerical value of the parameter was increased or decreased during the run, followed by confirming at Restart to perform the adjustment. If the peak goes outside the graph when the sample is allowed to run, all the peaks outside the graph will be converted to the maximum value of 105, so adjustment is necessary so as not to exceed it. Adjustments were made so that dead cells and cell clumps were not included in the measurement range as target cells. NC and samples were allowed to run in this order (Acquire data → Record data → 10,000 events or 5 min → (after 5 min) Stop Recording → Stop acquire) . After the sample was finished for running, a FACS shutdown procedure was performed.

Combined Proteomics by Liquid Chromatography/Mass Spectrometry (LC/MS)

Cell lysates of passage 4 cHCEC were used for proteome analysis. High quality (HQ) cHCECs including CD44-/+ mature differentiated cHCEC SPs were analyzed at a rate of 93.9% (effector ratio = E ratio, n=3), and low quality (LQ) cHCEC including CD44++/+++ immature cHCEC SPs were analyzed at a rate of 73.8% (n=3). Cell lysates from 3 aliquots of each HQ cHCEC or LQ cHCEC were dried, followed by reconstituting in 20 mmol/L HEPES-NaOH (pH 58.0), 12 mmol/L sodium deoxycholate and 12 mmol/L sodium N-lauroyl sarcosinate. After reduction with 20 mmol/L dithiothreitol at 100°C for 10 minutes and alkylation with 50 mmol/L iodoacetamide at ambient temperature for 45 minutes, proteins were digested with immobilized trypsin (Thermo Fisher Scientific) while shaking at 1000 rpm at 37°C for 6 hours. After removal of sodium deoxycholate, the resulting peptides were desalted with Oasis HLB m-elution plates (Waters) and subjected to mass spectrometry. Peptides were analyzed by an LTQ-Orbitrap-Velos mass spectrometer (Thermo Fisher Scientific) coupled with an Ulti-Mate 3000 RSLC nanoflow HPLC system (Thermo Fisher Scientific). Protein identification and quantitative analysis were performed with MaxQuant software. MS/MS spectra were searched with regard to the Homo sapiens protein database in Swiss-Prot, while setting a false discovery rate of 1% for both the peptide identification filter and the protein identification filter. Only "Razor unique peptides" were used for the calculation of relative protein concentrations. For protein composite analysis, all detected peaks were normalized by correcting the median value to 1.0-104.

(LC/MS Dataset Analysis)

The LC/MS dataset consisted of a total of 4641 types of proteins and was obtained by using Proteome Discoverer 2.2 software. After removing data for which abundance ratios could not be calculated, the inventors analyzed the remaining data with the web-based program DAVID v6.8 (The Database for Annotation, Visualization and Integrated Discovery; https://david.ncifcrf.gov). It ended up being 4315 genes, each given a unique DAVID gene ID for subsequent analysis. As for gene expression analysis, the inventors calculated statistical P-values and fold changes between the two groups, drew volcano plots, and extracted genes that were differentially expressed in HQ and LQ cHCEC. Further investigation of the genes of interest and associated genes/pathways suggested to be associated with cHCEC metabolism was performed by using DAVID and its options "BIOCARTA" and "KEGG_PATHWAY". The reference genes/pathways in the figures were changed slightly with reference to the original databases of BioCarta (https://cgap.nci.nih.gov/Pathways/BioCarta_Pathways) or KEGG (KyotoEncyclopediaof Genes and Genomes; https://www.genome.jp/kegg/). As for gene ontology (GO) analysis, data were divided into three groups based on the range of abundance ratios (LQ/HQ) and analyzed with DAVID using the "GOTERM_DIRECT" option for each group. GO results were sorted by P-value and the top 10 GO terms in each group were displayed. For LC/MS data analysis, the significance of differences between HQ and LQ cHCECs was assessed by Student's or Welch's t-test after confirmation by F-test.

(DAVID Analysis of Proteomics)

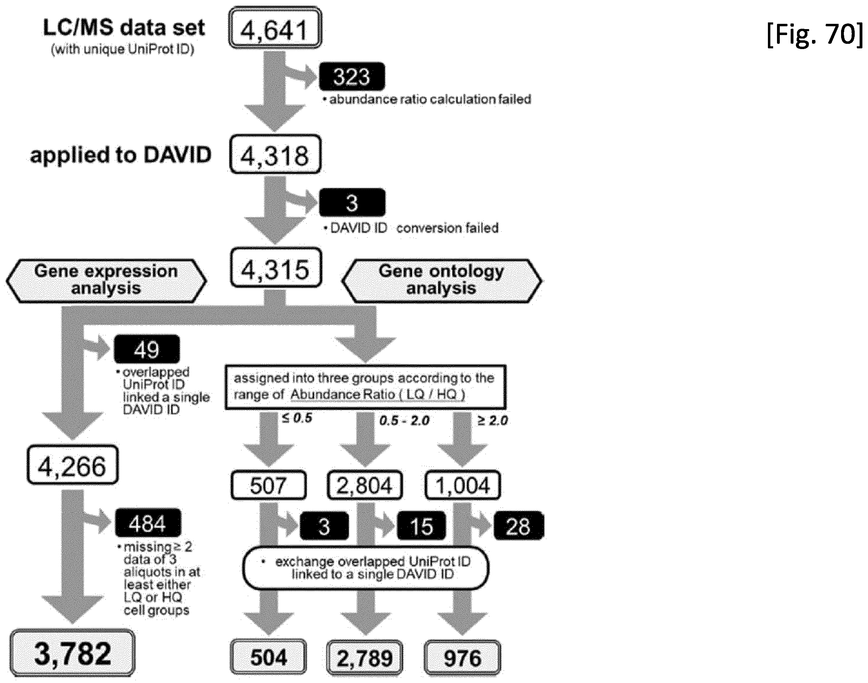

Subsequently, DAVID analysis of proteomics was performed in the following order (Fig. **70**):

(1) Three-group analysis
(2) Analysis of with a ratio exceeding 27
(3) Analysis with GOTERM alone
(4) Comparison on mitochondria after DAVID analysis.

(1) Three-group analysis

For 4641 Proteins, the 323 proteins without Abundance Ratio: (225) phase transition non-standard cell LQ/(184) standard cell HQ were excluded (323). The remaining 4318 Proteins were divided into groups based on Abundance Ratio: LQ/HQ (if there is even one data, the protein is subject to analysis).

The proteins were divided into three groups as shown in the table below.

[Table 3]

| 3 groups | Abundance Ratio: (225) / (184) | Number of Proteins | Number converted to DAVID ID | Number of DAVID ID |
|---|---|---|---|---|
| 1 | **Group with significantly higher 225 $\geqq$ 2** | 1006 | 1004 | 976 DAVID IDs |

(continued)

| 3 groups | Abundance Ratio: (225) / (184) | Number of Proteins | Number converted to DAVID ID | Number of DAVID ID |
|---|---|---|---|---|
| 2 | **Group with ambiguous differences** 2~0.5 | 2805 | 2804 | 2789 DAVID IDs |
| 3 | **Group with significantly higher 184** $\leqq$ 0.5 | 507 | 507 | 504 DAVID IDs |

(2) Analysis of those with a ratio exceeding 27

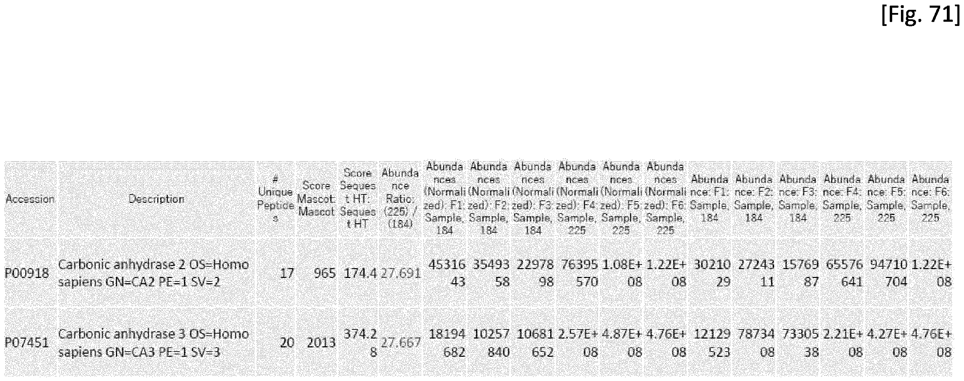

Next, those with a ratio exceeding 27 were analyzed. In Fig. **71,** DAVID analysis was performed on two of which Abundance Ratio: LQ/HQ exceeds 27, and the result was cluster 0. When examination was performed using the KEGG PATHWAY, Nitrogen metabolism increased. The inventors searched for commonalities between the genes (17) included in the KEGG PATHWAY Nitrogen metabolism and the proteins (4641) analyzed this time, and picked up seven (CA12, CA2, CA3, CA5B, GLUD1, GLUD2, GLUL). These were further subjected to DAVID, and KEGG PATHWAY (Nitrogen metabolism) was searched and confirmed.

(3) Analysis with GOTERM alone

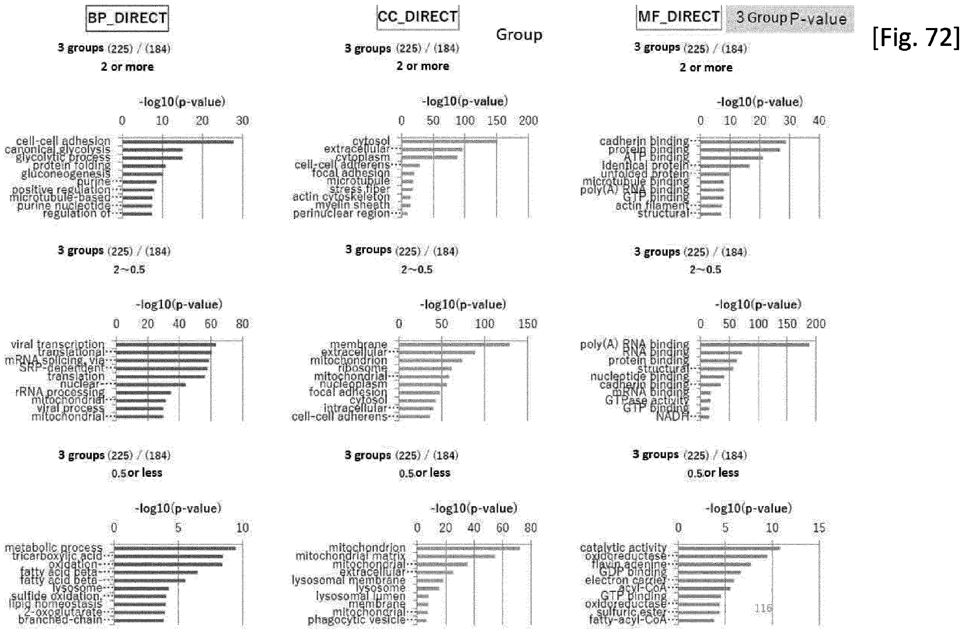

Next, analysis with GOTERM alone was performed. In the DAVID analysis, analysis can be performed using about 50 databases, and in the DAVID recommended (Defaults) analysis, about 10 of them (e.g., UP_KEYWORD, KEGG_PATHWAY, INTERPRO, etc.) are supposed to be used (including GOTERM); this time, the analysis was performed using only three of them (GOTERM_BP_DIRECT, GOTERM_CC_DIRECT, GOTERM_MF_DIRECT). Biological Process (BP): intracellular function. Cellular Component (CC): component of cell. Molecular Function (MF): function of a molecule. The results are shown in Fig. 72.

(4) Comparison on mitochondria after DAVID analysis

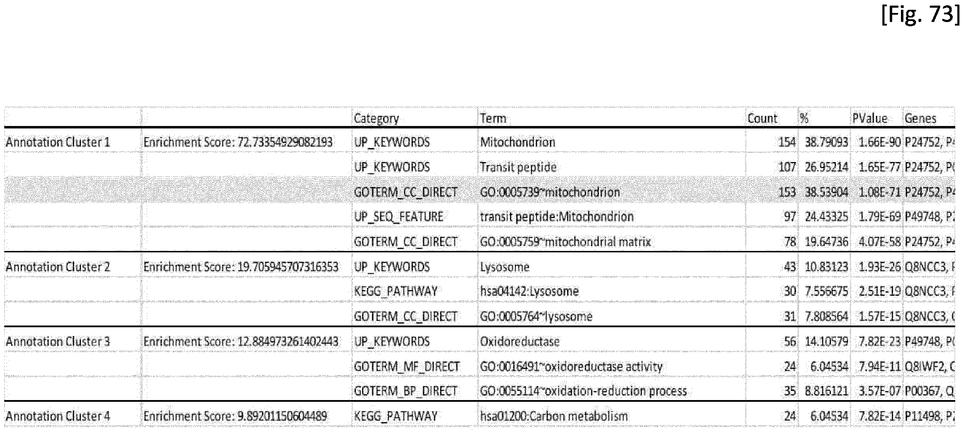

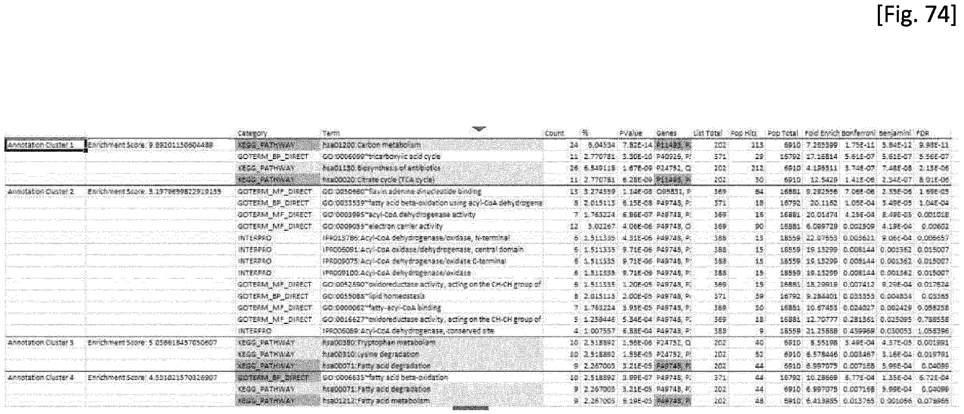

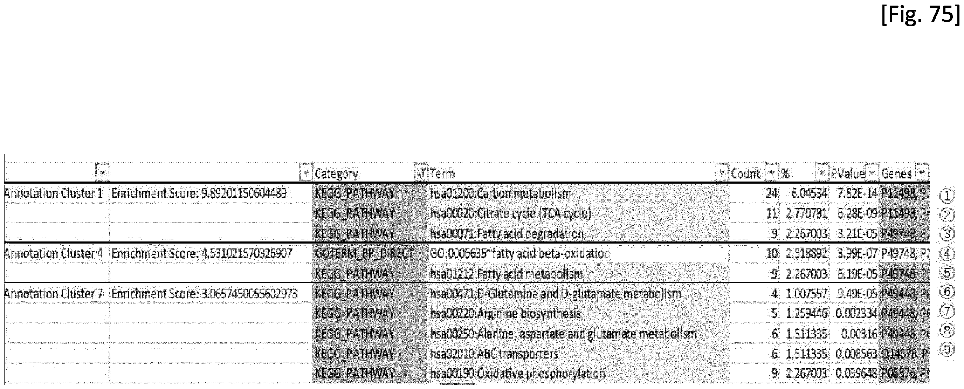

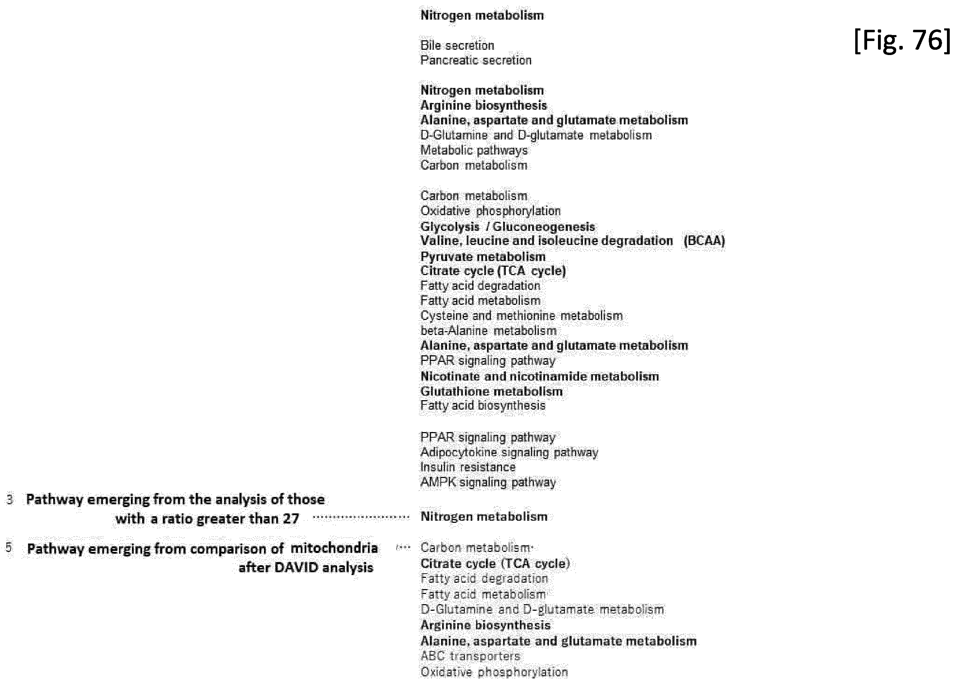

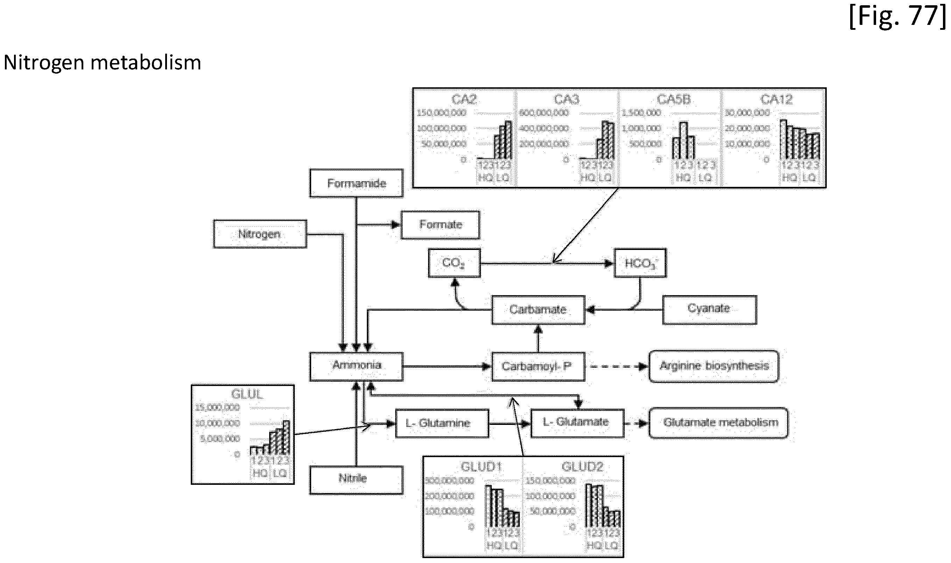

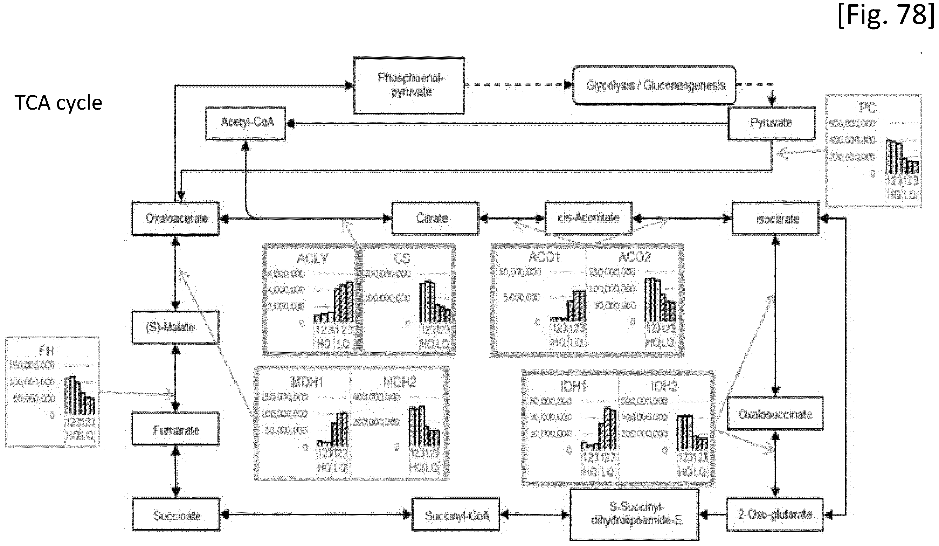

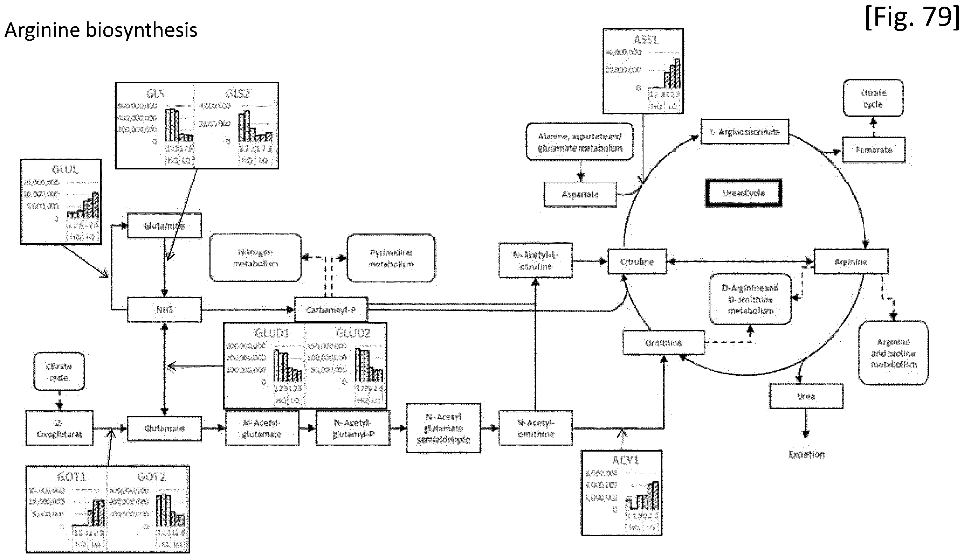

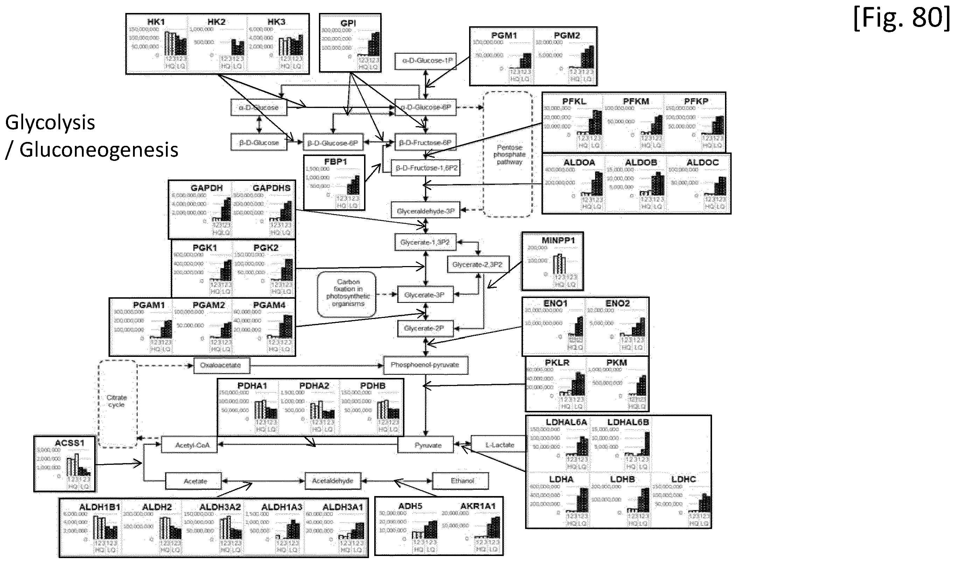

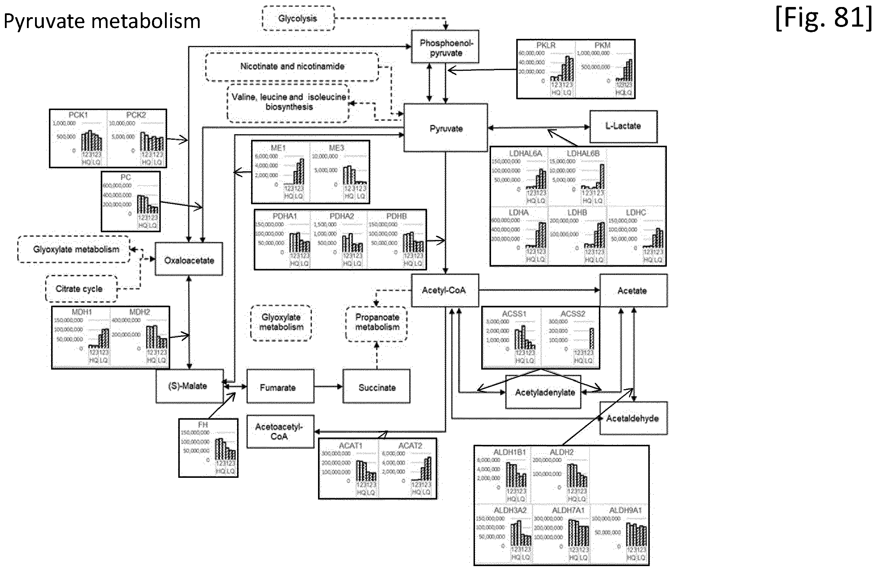

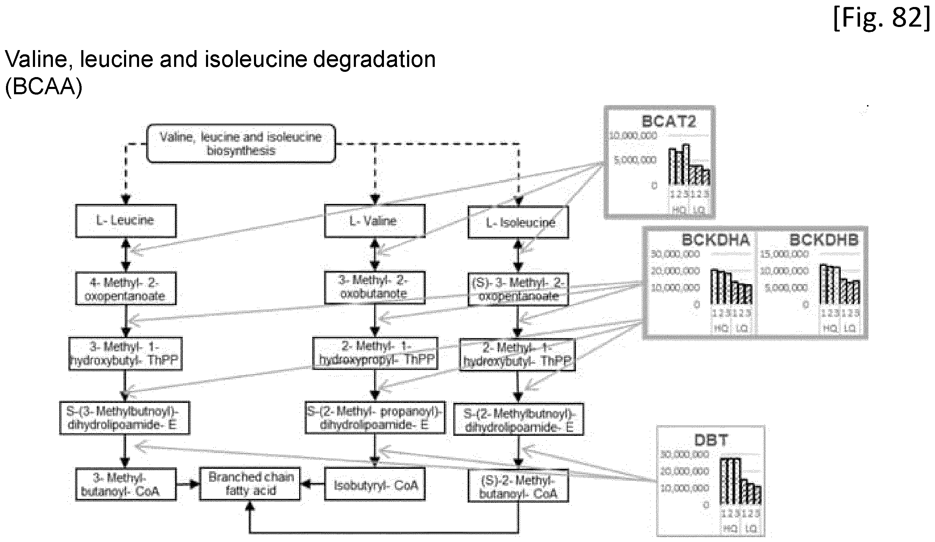

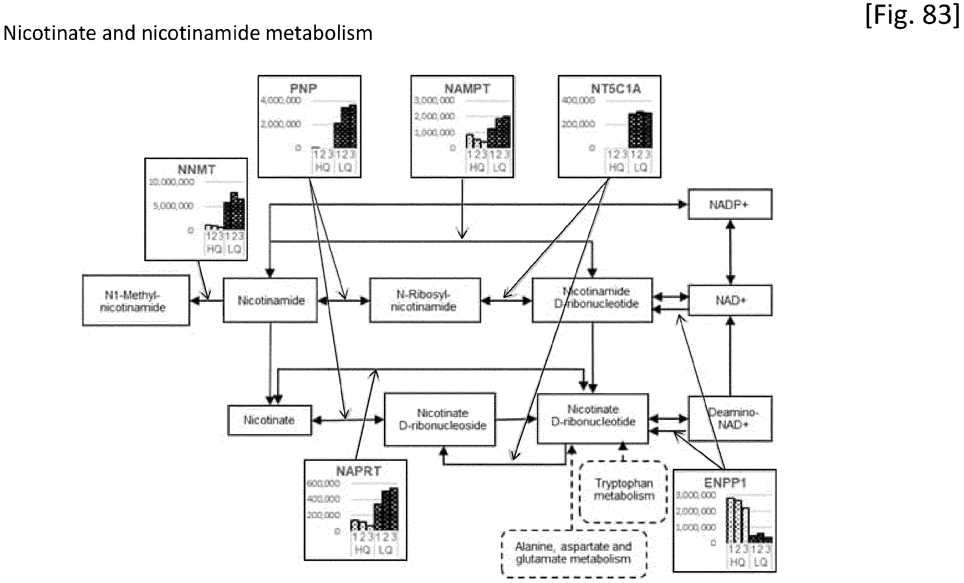

Subsequently, a comparison was made for mitochondria after DAVID analysis. In Annotation Cluster 1 (EnrichmentScore:72.73354929082193) of the AbundanceRatio: LQ/ HQ 0.5-0.2 group (398), many of them were related to mitochondria (Fig. **73**). Accordingly, in order to confirm the PATHWAY a little more, 398 were analyzed with DAVID again, and clustered with the items selected in DAVID's Defaults among the items of GOTERM, PASTHWAY, and PROTEINDOMAINS (Fig. **74**). From the clustering result, the pathway to be investigated was selected (Fig. **75**). The pathway was examined and included in Genes' graph. Furthermore, pathways of interest among the metabolic pathways were searched and illustrated using KEGGPathway [bold], and the protein expression intensities of enzymes and substrates involved in the metabolic pathway were compared between the phase-transition non-standard cells and standard cells (Figs. **76** to **83**).

(Example 10: X. Ion Channel and/or Monocarboxylic Acid Transport System Cell Immunostaining)

In this Example, the ion channel and/or monocarboxylic acid transport system was investigated.

(Materials and Methods)

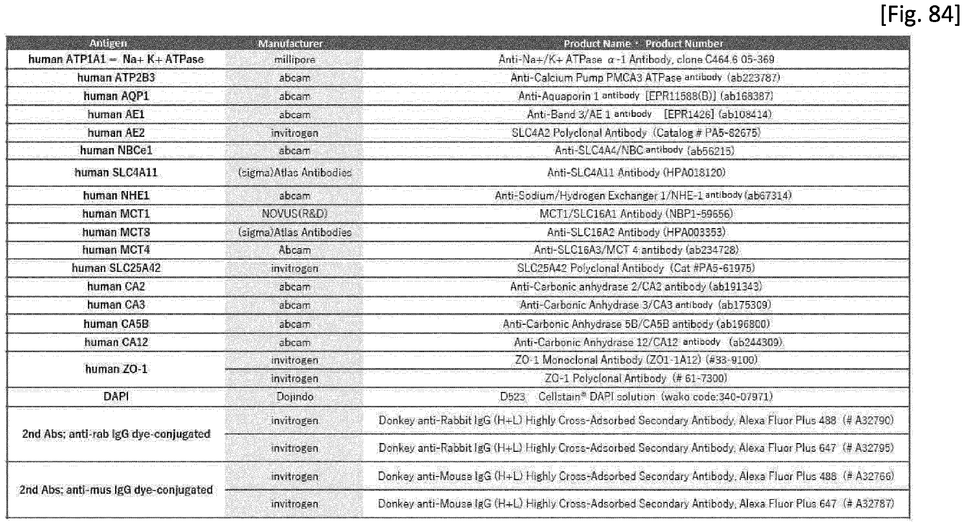

Cells obtained from corneal tissues of the following donor were immunostained. Cells obtained from corneal tissues of the following donors were immunostained. The antibody data used are shown in Fig. **84.**
#190802S/D, ACW-134OSCN/ODCN, Age 28 / Gender: female, ECD = 3003 / 3021, Cause of death: ESRDD-P = 08:57, D-C=5D

The results of immunostaining were as shown in the table below.

[Table 4]

| Culture Conditions | Day 0 ~ 4 | Day 5 ~ 41, 42 or 43 |
|---|---|---|
| ① | Nancy + 10 $\mu$M Y-27632 | |

(continued)

| Culture Conditions | Day 0 ~ 4 | Day 5 ~ 41, 42 or 43 |
|---|---|---|
| ② | Nancy + 10 $\mu$M Y-27632 +10 $\mu$M SB203580 + 5 ng/mL EGF | Nancy + 10 $\mu$M SB203580 + 5 ng/mL EGF |

Where the reference numeral 1 refers to standard cells, while the reference numeral 2 refers to phase transition non-standard cells and an EGF-added culture. Both were used for experiments after culturing until Day 0-41, 42 or 43. Each cell was imaged at 200× and stained in duplicate.

The following items were prepared for Immunofluorescent Staining of cHCECs (24-well plate).

☐ fixing solution (-30°C ice-cold special grade methanol (nacalai #21915-35) or 4% PFA/phosphate buffer (wako #163-20145) reconstituted to RT)
☐ PBS (-)
☐ PBS (-)/0.2% TritonX-100 (0.5 mL × wells × 1.1 = mL)
☐ 1%BSA/PBS(-) (BSA: EIA/RIA grade, nacalai #01281084) (BSA g / PBS(-) mL)
☐ Primary antibody (including isotype control)
☐ Fluorescently labeled secondary antibody
☐ DAPI (Doujin Kagaku, wako #340-07971)

The procedure is as follows. All volumes are for 1-well per 24-well plate.

First, the culture supernatant of the cells was removed, and the cells were washed with 0.5 mL of PBS(-) (once). A 0.5 mL fixing solution was added and allowed to stand at room temperature for 15 minutes (-30°C for MeOH). 0.5 mL of PBS(-)/0.2% TritonX-100 was added and incubated at room temperature for 15 minutes. The solution was removed, 0.5 mL of 1% BSA/PBS(-) was added, and blocking was performed at room temperature for 60 minutes. The solution was removed, the primary antibody was diluted with 1% BSA/PBS(-), 0.3 mL was added, and the mixture was incubated O/N at 4°C. The mixture was washed with PBS(-) 0.5 mL for 5 minutes (three times). The solution was removed, and the fluorescence-labeled secondary antibody was diluted with 1% BSA/PBS(-), added 0.3 mL, and incubated at room temperature for 60 minutes or more. The mixture was washed with PBS(-) 0.5 mL for 5 minutes (once). The solution was removed, and DAPI was diluted 200-fold with PBS(-), 0.3 mL was added, and incubated at room temperature for 5 to 15 minutes. The mixture was washed with PBS(-) 0.5 mL for 5 minutes (twice). 0.5 mL of PBS(-) was added, followed by observing under a fluorescence microscope.

(Results)

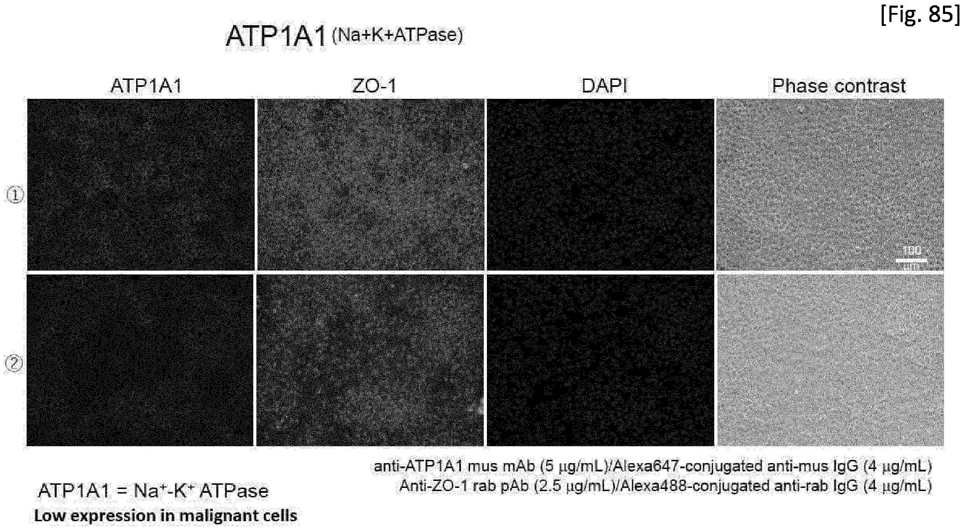

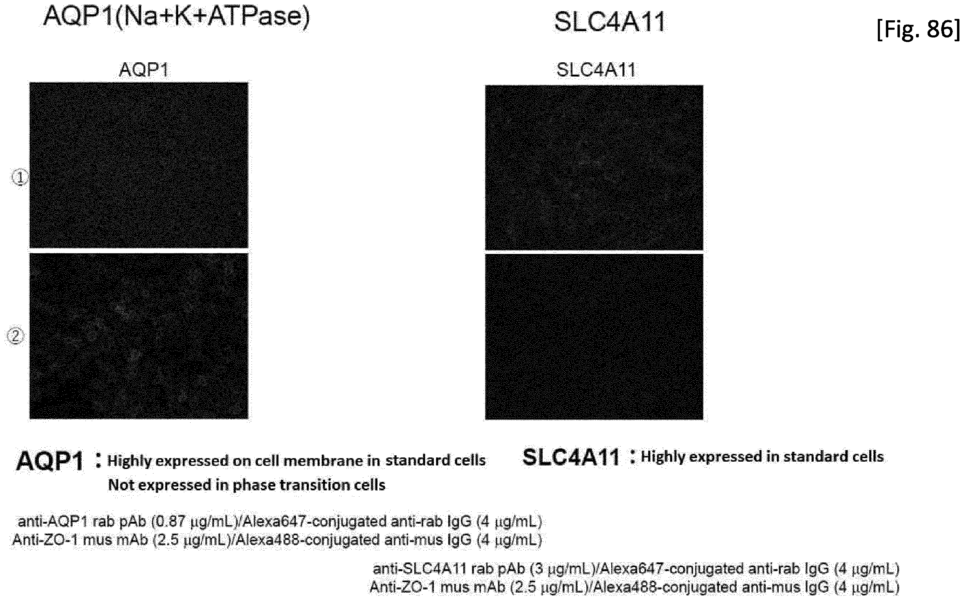

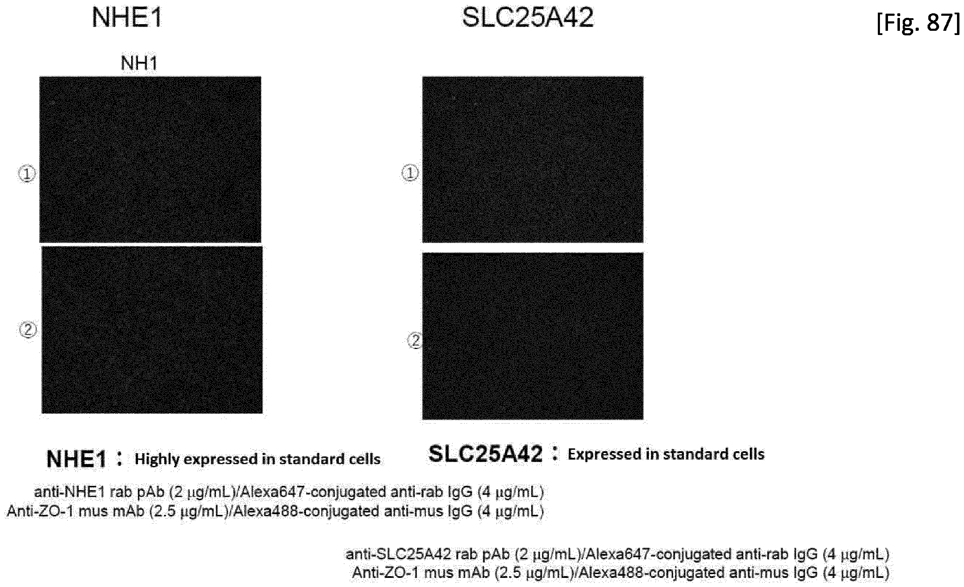

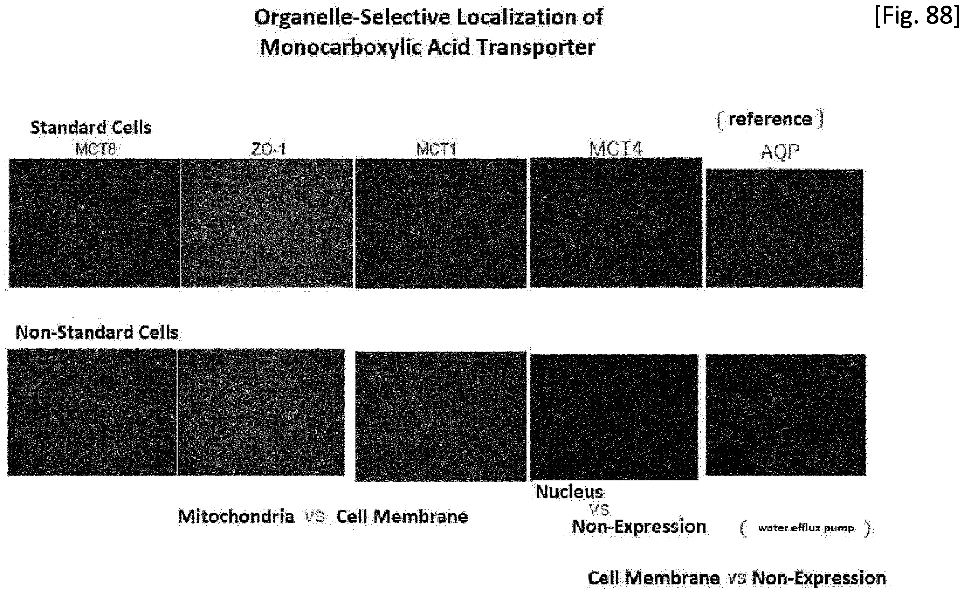

The results are shown in Figs. **85** to **88.** As a result, ATP1A1 (Na + K+ATPase) and AQP1 were found to be highly expressed in the cell membrane of standard cells, but not expressed in non-standard cells. The same was true for SLC4A11, NHE1 and SLC25A42. From the above, although not wishing to be bound by any theory, organelle-selective localization of monocarboxylic acid transporters was revealed. From the above, it is understood that the expression of a functional protein leading to a corneal endothelial (cell) functional property leading to improvement on corneal opacity and hydrous edema, resulting in continuous and long-term retention of corneal endothelial tissue cell density and improvement on visual acuity, is observed in standard cells.

(Example 11: XI. Transporter SLC Family Protein)

Next, in this Example, it was observed what kinds of transporter SLC family protein would be expressed in standard cells. Details are given below.

(Materials and Methods)

In this Example, experiments with regard to expression were conducted on transporter SLC family proteins.

Integrated proteomic analysis on cell lysates by liquid chromatography-tandem mass spectrometry (LC-MS/MS) was performed on triplicate specimens for either CD44-/+ (mature) predominantly constitutive cHCEC or CD44++/+++ cell state transition (CST) predominantly constitutive cHCEC. The cell culture conditions and the like conform to those described in the above Examples.

Expression patterns of cation/anion transporters (ion transporters), monocarboxylic acid transporters and solute carrier (SLC) family proteins, and carbonic anhydrase (CA) were assayed.

MS/MS spectra were analyzed with regard to the human protein sequence database (SwissProt) using the Mascot or SEQUEST search engines and using Proteome Discoverer 2.2 software (Thermo Fisher Scientific).

(Results)

Abundance ratio of transporter SLC family proteins between standard cells and non-standard cells

[Table 5]

| SLC | CD44+/- Mature | CD44++/+++ Immature | Abundance Ratio: LQ / HQ | SLC | CD44+/- Mature | CD44++/+++ Immature | Abundance Ratio: LQ / HQ |
|---|---|---|---|---|---|---|---|
| SLC38A9 | 279,670 | 1,038,741 | 3.79 | SLC25A17 | 236,234 | 209,620 | 0.89 |
| SLC29A1 | 3,455,069 | 7,308,576 | 2.34 | SLC44A2 | 310,542 | 1,548,507 | 0.89 |
| SLC4A4 | 105,500,458 | 229,542,708 | 2.30 | SLC37A4 | 1,095,906 | 1,400,143 | 0.88 |
| SLC4A8 | 3,796,133 | 7,656,416 | 2.01 | SLC25A32 | 363,164 | 384,589 | 0.87 |
| SLC4A10 | 3,796,133 | 7,656,416 | 2.01 | SLC35B2 | 1,872,269 | 1,729,347 | 0.87 |
| SLC4A5 | 2,312,220 | 4,794,261 | 1.91 | SLC4A7 | 294,720 | 260,213 | 0.85 |
| SLC4A11 | 50,546,128 | 89,063,520 | 1.78 | SLC30A10 | 171,704 | 141,781 | 0.81 |
| SLC22A5 | 322,411 | 714,180 | 1.71 | SLC12A3 | 11,433,655 | 9,585,361 | 0.80 |
| SLC12A5 | 1,856,121 | 2,838,114 | 1.52 | SLC30A9 | 1,744,423 | 1,187,762 | 0.80 |
| SLC35E1 | 1,625,432 | 2,253,161 | 1.42 | SLC31A1 | 660,141 | 503,064 | 0.79 |
| SLC12A4 | 10,378,434 | 14,666,246 | 1.42 | SLC25A29 | 722,315 | 497,456 | 0.78 |
| SLC12A6 | 2,210,806 | 3,065,058 | 1.41 | SLC11A2 | 392,014 | 300,096 | 0.75 |
| SLC26A2 | 163,889 | 231,090 | 1.39 | SLC20A2 | 3,042,435 | 2,304,231 | 0.75 |
| SLC30A6 | 407,091 | 533,951 | 1.38 | SLC2A10 | 126,042 | 89,617 | 0.73 |
| SLC2A1 | 230,606,501 | 303,430,009 | 1.29 | SLC33A1 | 1,242,970 | 902,993 | 0.73 |
| SLC9A3R2 | 4,177,277 | 6,038,517 | 1.26 | SLC25A4 | 25,441,755 | 17,282,960 | 0.66 |
| SLC16A1 | 3,507,482 | 4,645,505 | 1.20 | SLC12A2 | 61,663,638 | 36,793,474 | 0.66 |
| SLC30A5 | 558,454 | 1,195,746 | 1.16 | SLC25A46 | 2,216,003 | 1,578,340 | 0.65 |
| SLC38A10 | 628,475 | 2,653,239 | 1.14 | SLC27A4 | 1,680,554 | 755,727 | 0.57 |
| SLC5A3 | 305,731 | 309,788 | 1.11 | SLC15A4 | 1,555,735 | 802,875 | 0.57 |
| SLC39A14 | 756,331 | 795,508 | 1.09 | SLC25A12 | 31,430,385 | 17,953,087 | 0.55 |
| SLC25A23 | 452,908 | 482,822 | 1.06 | SLC27A3 | 304,044 | 158,972 | 0.54 |
| SLC9A7 | 1,472,075 | 1,452,862 | 1.03 | SLC35A4 | 1,678,013 | 883,511 | 0.54 |
| SLC39A7 | 4,079,632 | 3,791,026 | 0.97 | SLC25A24 | 31,179,877 | 16,051,185 | 0.54 |
| SLC25A25 | 2,580,637 | 2,100,732 | 0.97 | SLC41A3 | 786,005 | 383,158 | 0.52 |
| SLC46A1 | 1,275,839 | 1,142,464 | 0.90 | SLC35F6 | 5,682,952 | 3,266,977 | 0.52 |
| SLC16A3 | 1,442,144 | 1,290,811 | 0.89 | | | | |

| SLC | CD44+/-Mature | CD44++/+++ Immature | Abundance Ratio: LQ / HQ |
|---|---|---|---|
| SLC25A11 | 71,153,370 | 36,197,055 | 0.51 |
| SLC25A16 | 359,043 | 185,525 | 0.51 |
| SLC25A1 | 81,593,104 | 39,600,698 | 0.50 |
| SLC25A20 | 15,657,043 | 6,639,090 | 0.50 |
| SLC25A31 | 166,168,896 | 84,494,219 | 0.50 |
| SLC25A5 | 125,582,119 | 59,082,701 | 0.47 |
| SLC25A6 | 637,430,466 | 298,085,421 | 0.46 |
| SLC26A11 | 402,059 | 173,655 | 0.46 |
| SLC27A1 | 915,164 | 239,109 | 0.44 |
| SLC25A44 | 594,141 | 252,302 | 0.44 |
| SLC12A9 | 3,961,037 | 1,599,000 | 0.42 |
| SLC25A3 | 345,073,331 | 140,545,606 | 0.41 |
| SLC38A7 | 538,630 | 200,046 | 0.38 |
| SLC3A2 | 6,627,101 | 1,940,976 | 0.37 |
| SLC17A5 | 6,130,425 | 2,566,155 | 0.36 |
| SLC25A13 | 183,959,205 | 57,648,687 | 0.33 |
| SLC25A10 | 23,584,455 | 8,528,975 | 0.32 |
| SLC7A14 | 2,651,013 | 244,597 | 0.23 |
| SLC9A1 | 560,043 | < 1,000 | 0.01 |
| SLC44A1 | 252,736 | < 1,000 | 0.01 |
| SLC2A13 | 358,625 | < 1,000 | 0.01 |
| SLC12A1 | 3,460,743 | < 1,000 | 0.01 |
| SLC25A42 | 122,509 | < 1,000 | 0.01 |
| SLC25A18 | 78,877 | < 1,000 | 0.01 |
| SLC4A1AP | 4,246,087 | < 1,000 | 0.01 |
| SLC16A2 | 202,342 | < 1,000 | 0.01 |

From the above results, enzymes related to nicotinic acid and nicotinamide metabolites were most upregulated in CST cHCEC, whereas only ectonucleotide pyrophosphatase was upregulated in mature cHCEC. Interestingly, glutamate-ammonia ligase (GLUL) expression was elevated in CST cHCEC, whereas the expression of glutamate dehydrogenase 1, 2 (GLUD1, 2) and glutaminase 2 (GLS2) was decreased. By integrated proteomics, the expression patterns of cation/anion transporters (ion transporters), monocarboxylic acid transporters (MCT) and solute carrier (SLC) family proteins as well as the carbonic anhydrase (CA) were observed showing organelle-selective localized expression in mature cHCECs (cells of interest) and CSTcHCECs (non-intended cells).

(Example 12: XII. Intracellular pH Measurement)

In this Example, the relationship with intracellular pH was investigated for cells of interest. Details are given below.

(Materials and Methods)

Subsequently, intracellular pH was measured. Donor information is as follows: Lot#190802P3: #190802S/D, ACW-134 OSCN/ODCN, Age 28 / Gender: female, Endothelial cell density ECD = 3003 / 3021, Cause of death: ESRD, D-P =08:57, D-C = 5D.

(Intracellular pH Measurement)

Subpopulation analysis and pH measurements were performed using phase-contrast micrographs showing cell morphology and cell surface CD antigen expression profiles.

The intracellular pH of cultured human corneal endothelial cells is considered to regulate cell size and mitochondrial function. Below, standard cells and phase transition non-standard cells were prepared under culture conditions, followed by assaying the pH, to verify that the intracellular pH of CD44- standard cells was lower than that of CD44++/+++ phase transition non-standard cells.

[Table 6]

| Culture Conditions (24-well plate) | Day 0 ~ 4 | Day 5 ~ 42 |
|---|---|---|
| ①: 9 wells | Nancy + 10 μM Y-27632 | |
| ②: 12 wells | Nancy + 10 μM Y-27632 +10 μM SB203580 + 5 ng/mL EGF | Nancy + 10 μM SB203580 + 5 ng/mL EGF |

(cHCECs intracellular pH Measurement Method)

(Adjustment Reagent)

HEPES buffer (153mM NaCl, 5 mM KCl, 5 mM glucose, 20 mM HEPES, pH7.4)

Calibration buffer(130 mM KCl, 10 mM NaCl, 1 mM MgSO4, 10 mM Na-MOPS) ... pH6.6/7.0/7.2/7.4/7.8/8.2
2 mg/mL Nigercin/EtOH stock solution ... aliquot and store at -30°C
1 mM BCECF-AM/DMSO solution (Doujin Kagaku B221: add and mix 72.612 mL DMSO to 50 mg/tube, followed by dispensing and storing at -30°C)

(Items to be Prepared)

Black flat bottom 96-well plate (Thermo Fisher: 237105 individually wrapped with lid, untreated)
Consider the number of cells that can be collected ($1 \times 10^5$ cells/sample; 6 samples to be used for calibration, so at least 7 to $8 \times 10^5$ cells were prepared)
pHi measuring reagent (HEPES, 1 mM BCECF-AM/DMSO, 2 mg/mL Nigercin/EtOH)
cHCEC detachment-related reagent (PBS, TrypLE/PBS)

(Methods)

(Preparation of Cell Suspension (Cells to be Measured))

Cells were photographed with a phase-contrast microscope camera, and human cultured corneal endothelial cells were detached with PBS(-) (photographing cells with a phase-contrast microscope camera) and $5 \times$TrypLE/PBS(-).
The number of cells was counted (actual value: cells/mL, total cells/mL), and the cells were suspended in HEPES Buffer at $2\text{-}10 \times 10^5$ cells/1 mL HEPES in one 1.5-mL Proteosave tube. Five mL of 1 mM BCECF-AM/DMSO was added per mL (finalconc. 5 mM) (actual value: cells/mL $\times$ the number of tubes), followed by bringing into reaction for 30 minutes in a 37°C and 5% $CO_2$ incubator in a culture room. On a laboratory testing bench, the tubes were centrifuged at $300 \times$g (1,867 rpm: eppendorf centrifuge 5418) for 3 minutes, the supernatant was removed, and the cell pellets were suspended in 200 mL of HEPES buffer (first wash). The tubes were centrifuged at $300 \times$g (1,867 rpm) for 3 minutes, followed by removing the supernatant, suspending in HEPES Buffer to $2 \times 10^5$ cells/mL HEPES, and putting together into a 15-mL tube (ex. to be $8 \times 10^5$ cells/4 mL HEPES). The cells were aliquoted from this cell suspension into 500 mL/1.5-mL Proteosave tubes. (ex. two tubes for a total volume of $8 \times 10^5$ cells/4 mL HEPES) . Before the aliquoting, the tubes were mixed so that the cell suspension was homogenous (after the aliquoting, the inventors proceeded to prepare the cell suspension for calibration). The tubes were centrifuged at $300 \times$g (1,867 rpm) for 3 minutes (second wash), the supernatant was removed, and the cell pellets were suspended in 500 mL of HEPES buffer.

(Preparation of Cell Suspension for Calibration)

A buffer solution for calibration was prepared, and cells were aliquoted from the cell suspension into six 500 mL/1.5-mL Proteosave tubes. The tubes were centrifuged at $300 \times$g (1,867 rpm) for 3 minutes (second wash), the supernatant was removed, and the cell pellets were suspended in 500 mL of each pH solution. Two mg/mL Nigercin/EtOH was added at 2.5 mL per 500 mL (final conc. 10 mg/mL), followed by incubating for 10 minutes at room temperature. This cell suspension for calibration and cell suspension were added at 150 mL/well (96-well plate) into 3 wells.

(Measurement)

Using a fluorescence plate reader (GloMax Explorer, Promega), measurements were made at an excitation of 500 nm and an emission wavelength of 530 nm (for GloMax, set excitation Blue (475 nm) and emission filter 500-550 nm).

(Results)

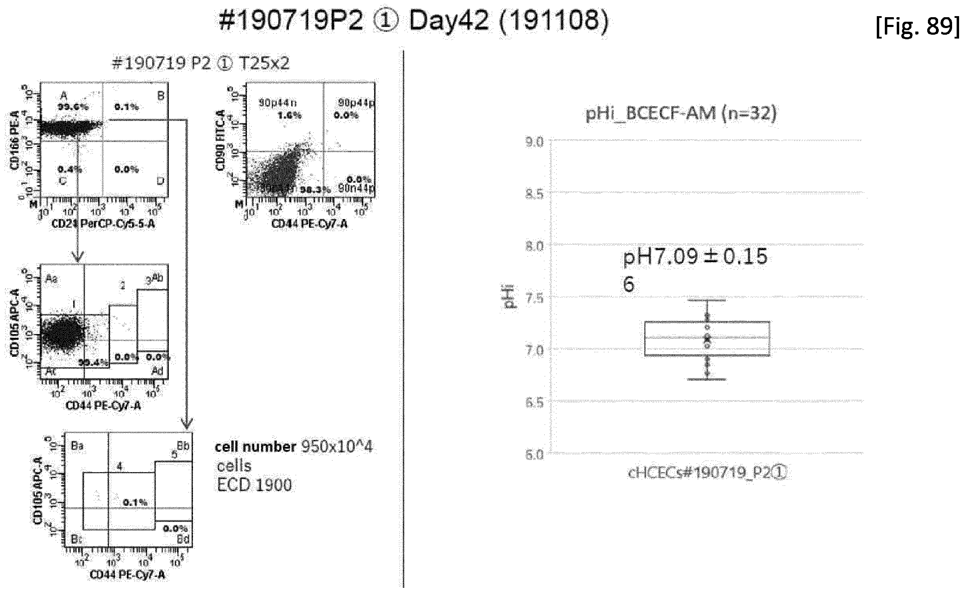

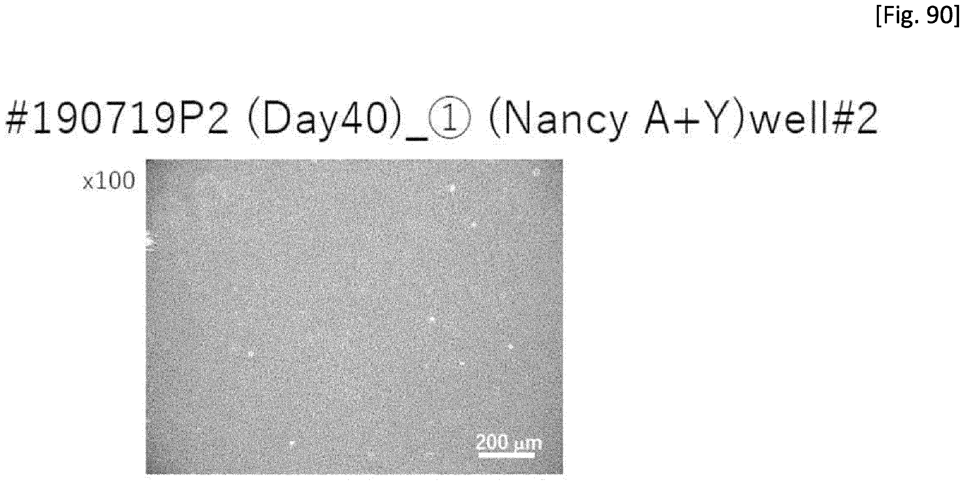

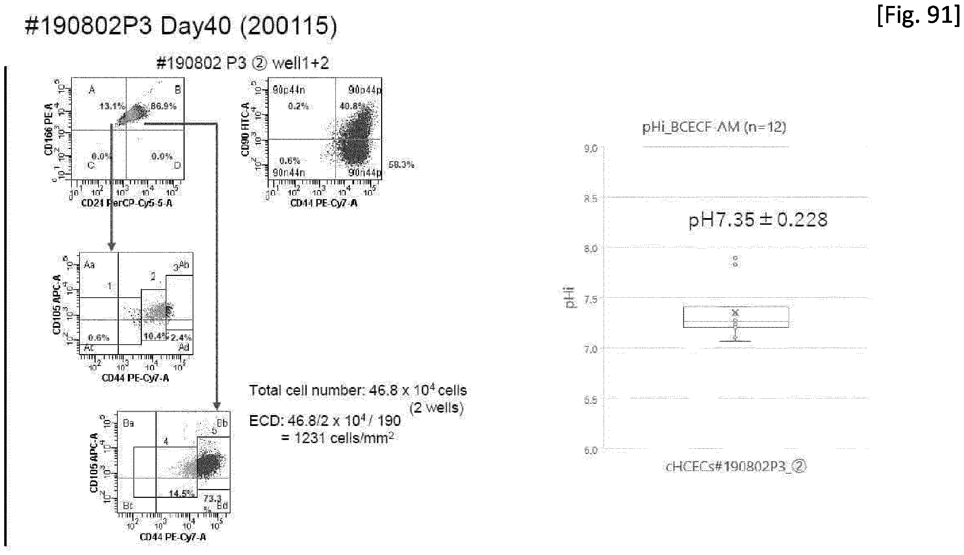

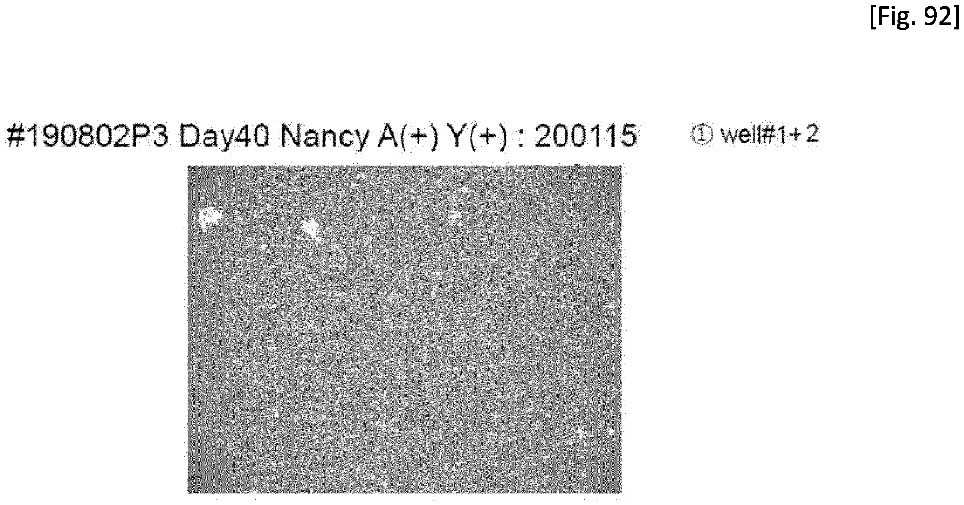

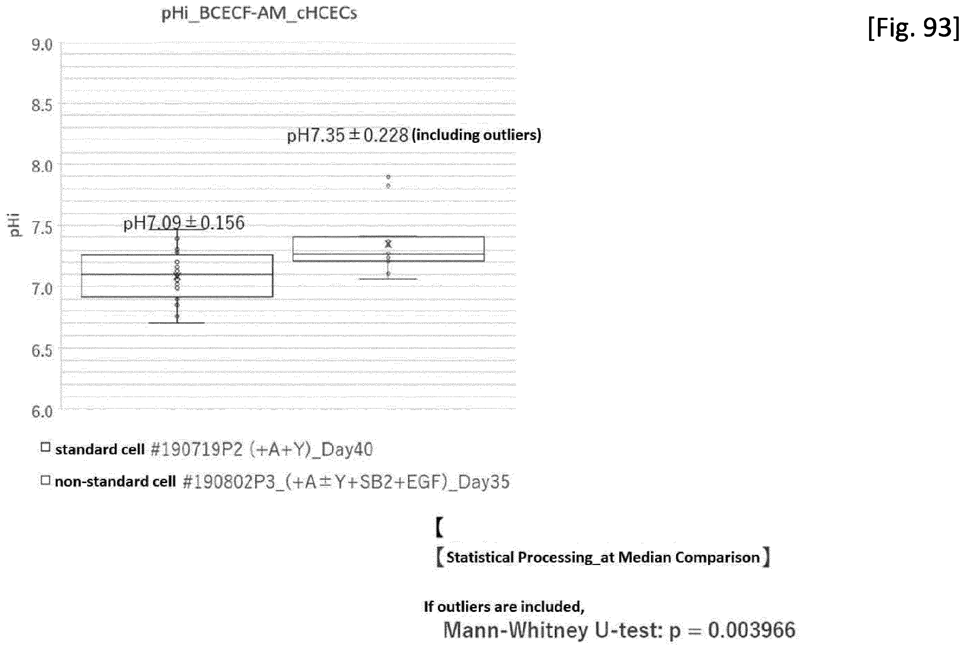

The results are shown in Figs. **89** to **93.**

(Example 13: XIII. Additive Effects)

In this example, the effects of additives were further investigated. Details are given below.

(Materials and Methods)

P3 T-251 was passaged to 24wp and used (=P4). C37, ABH-096 OSCN/ODCN, Age = 26Y / ECD = 3255 / 3137, COD: Trauma, D-P = 17:57 / D-C = 8D

[Table 7]

| | seeding/passage date | culture days | seeding ECD | passage time ECD | software analysis ECD | Effector | semi-pass |
|---|---|---|---|---|---|---|---|
| PO | **August 4** | 45 | [264] | 2284 | 2887 | ND | ND |
| P1 | **September 18** | 42 | 859 | 2040 | 2388 | 958% | 0.7% |
| P2 | **October 30** | 49 | 808 | 1848 | 1746 | 95.9% | 1.0% |
| P3 | **December 18** | 39 | 891 | 1789 | 1747 | 75,0% | 20.2% |
| P4 | **January 26** | | 800 | | | | |
| *ND: Not Determined | | | | | | | |

(Results)

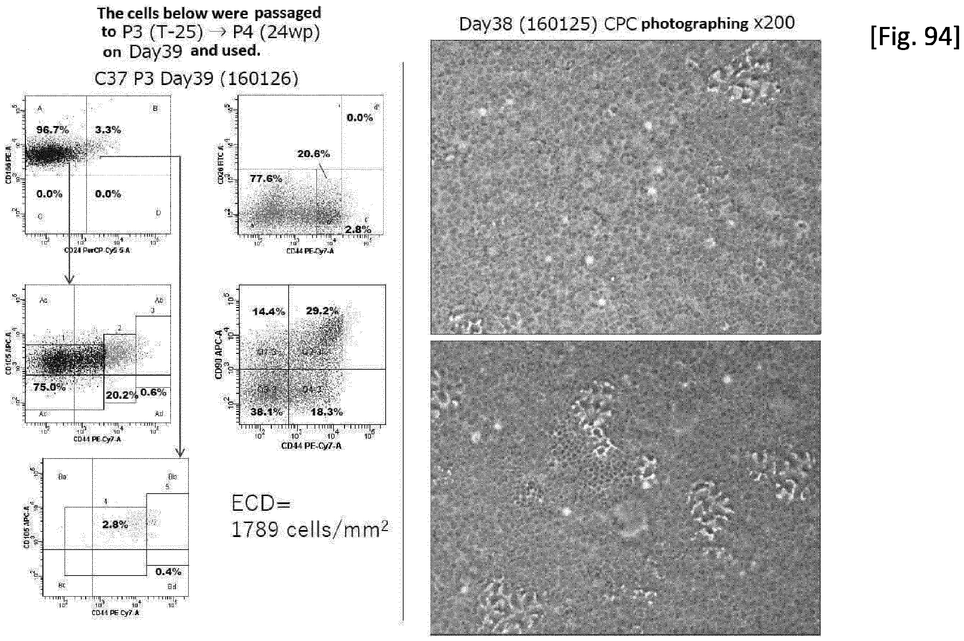

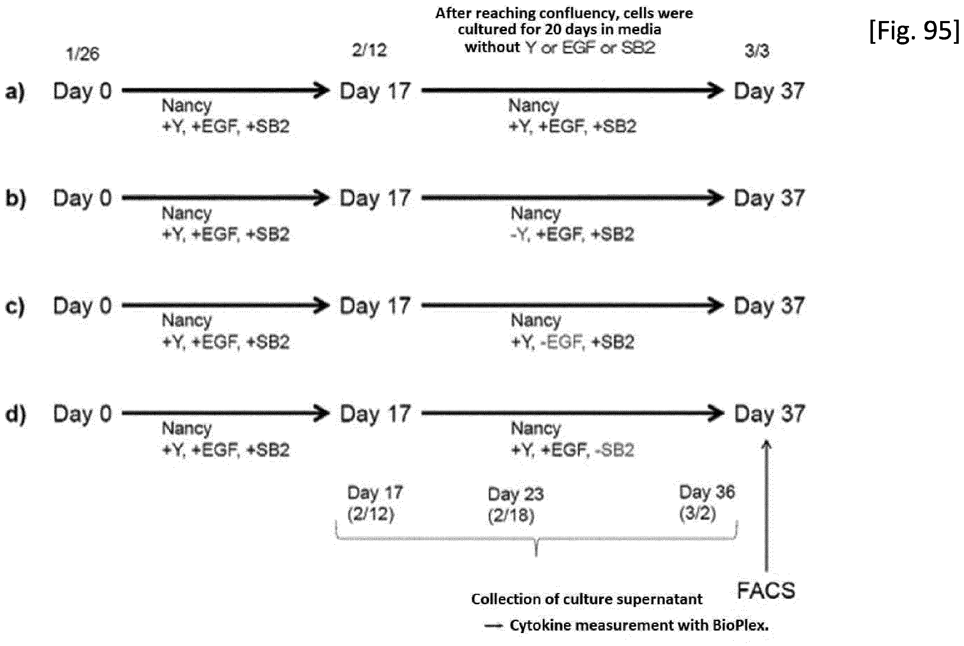

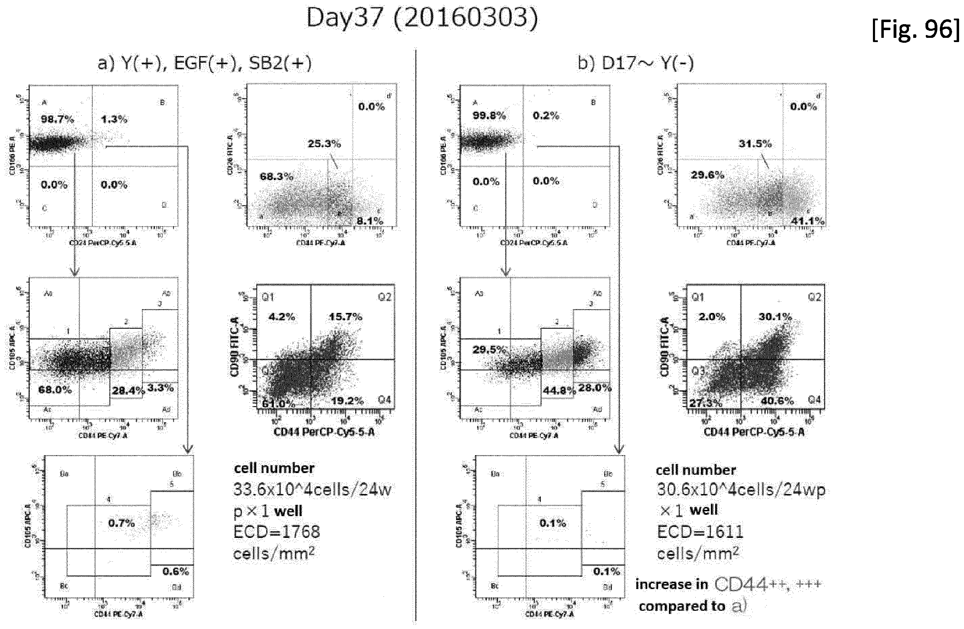

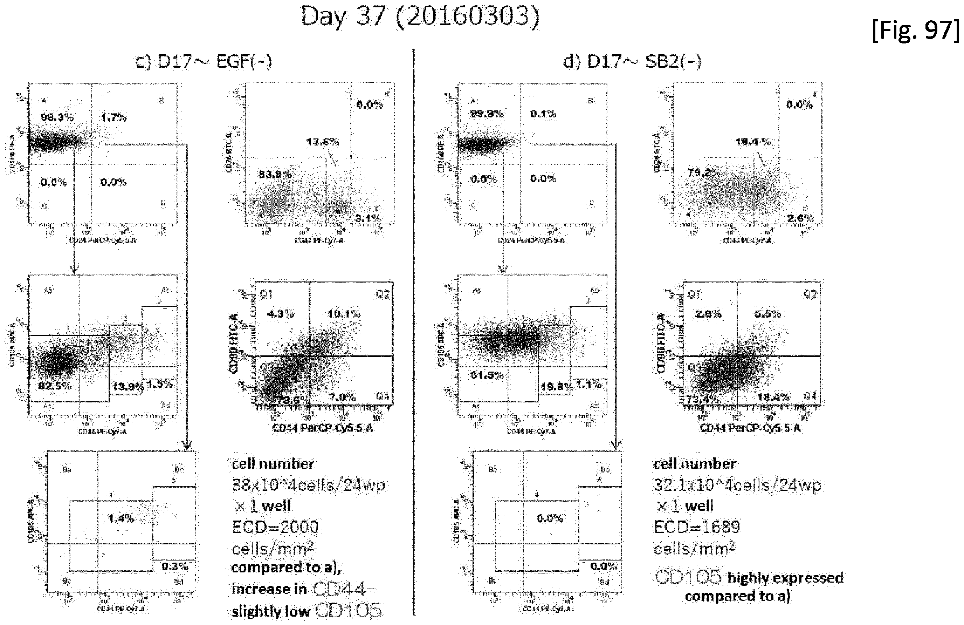

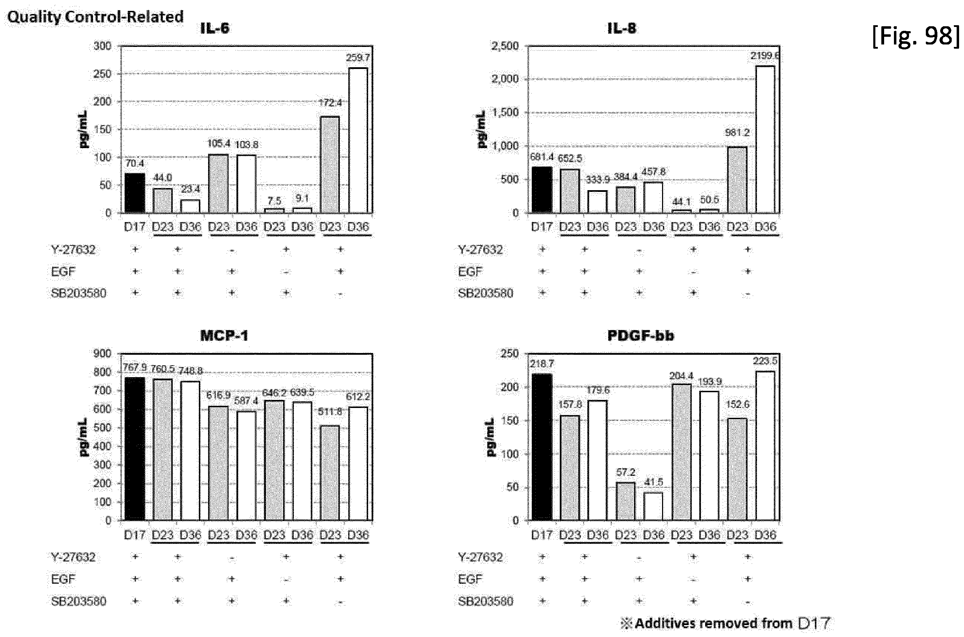

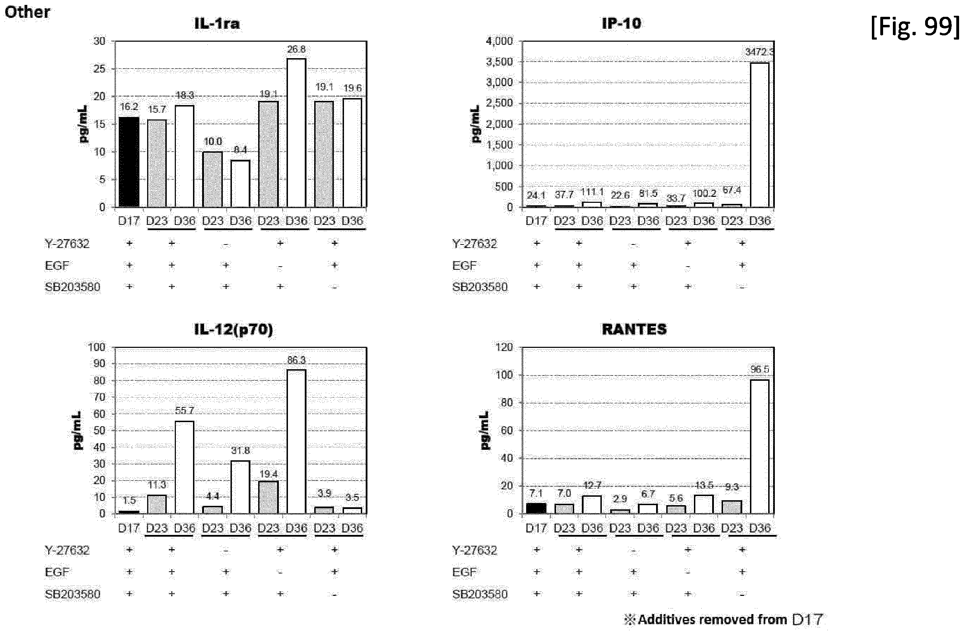

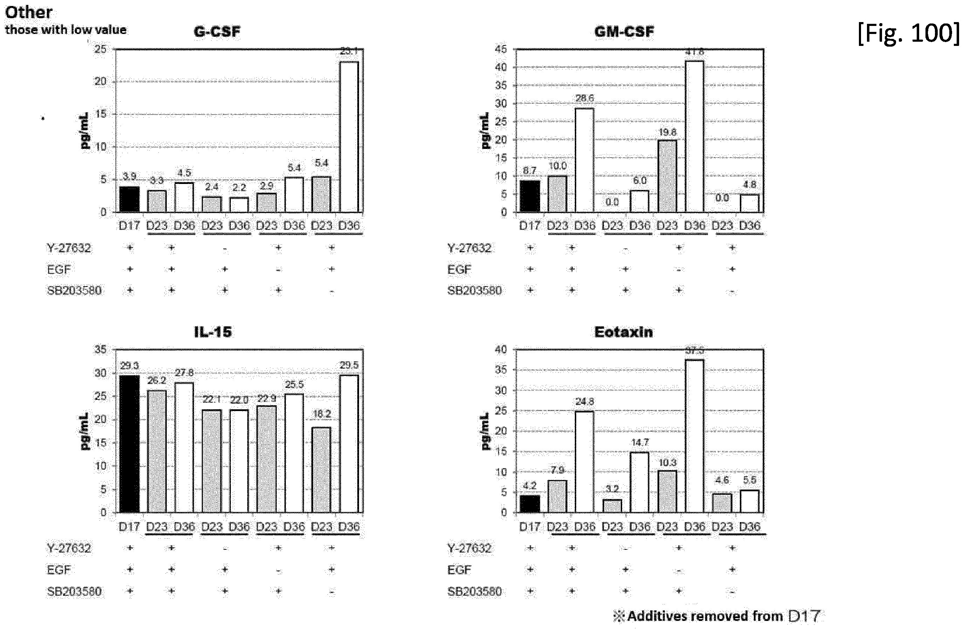

The results are shown in Figs. **94** to **100.** Among the measurement items, IL-1b, IL-2, IL-4, IL-5, IL-7, IL-9, IL-10, IL-13, IL-15, IL-17, basic FGF, IFN-g, MIP-1a, MIP-1b, TNF-a, and VEGF were below the detection limit.
According to the above Examples 1 to 13, it is understood to be important to proliferate and/or differentiate or maturate a corneal endothelial progenitor cell under a culture condition capable of minimizing culture stress, such as proliferation stress. Furthermore, it is understood that the expression of a functional protein leading to a corneal endothelial (cell) functional property leading to improvement on corneal opacity and hydrous edema, resulting in continuous and long-term retention of corneal endothelial tissue cell density and improvement on visual acuity, is observed in standard cells. In addition, in a preferred embodiment, it is understood that it is preferable to perform the culturing in the presence of a ROCK inhibitor in the step of proliferation and/or differentiation or maturation.

(Example 14: Increase in Oxidative Phosphorylation Respiration)

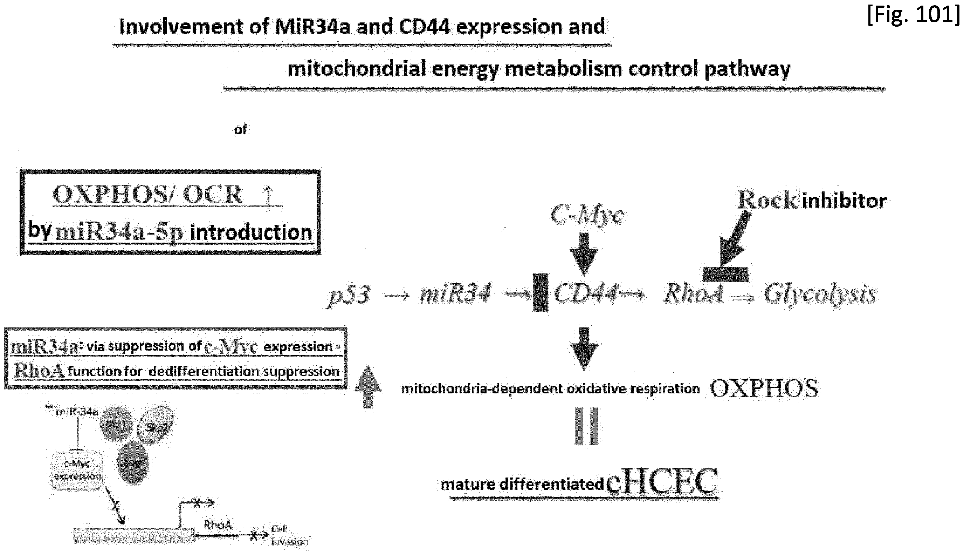

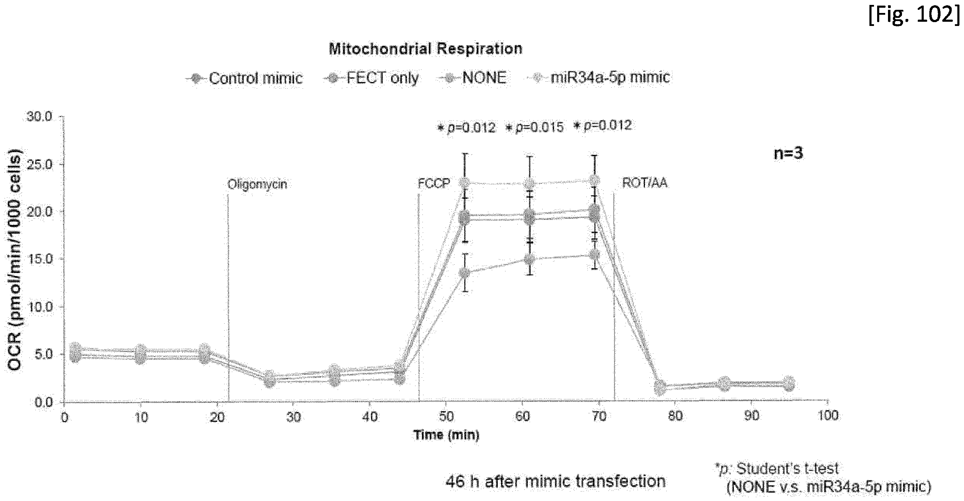

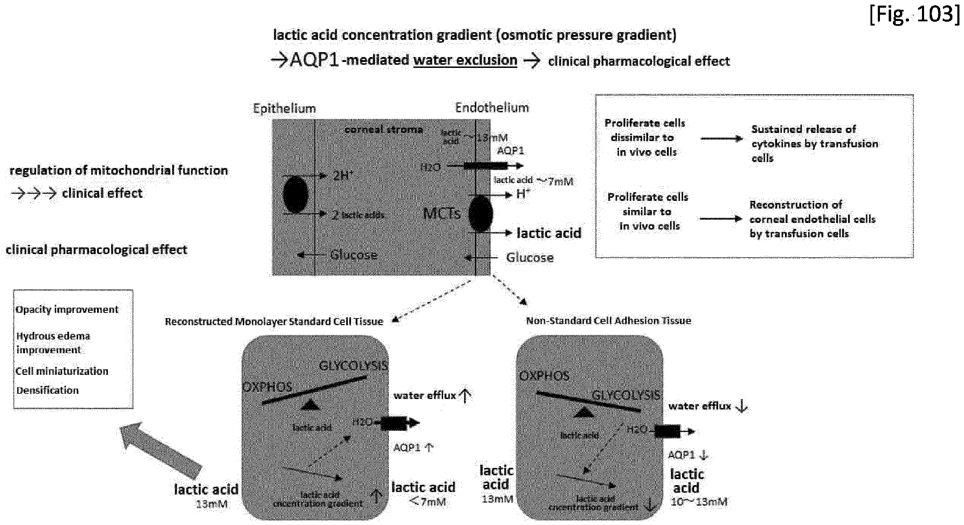

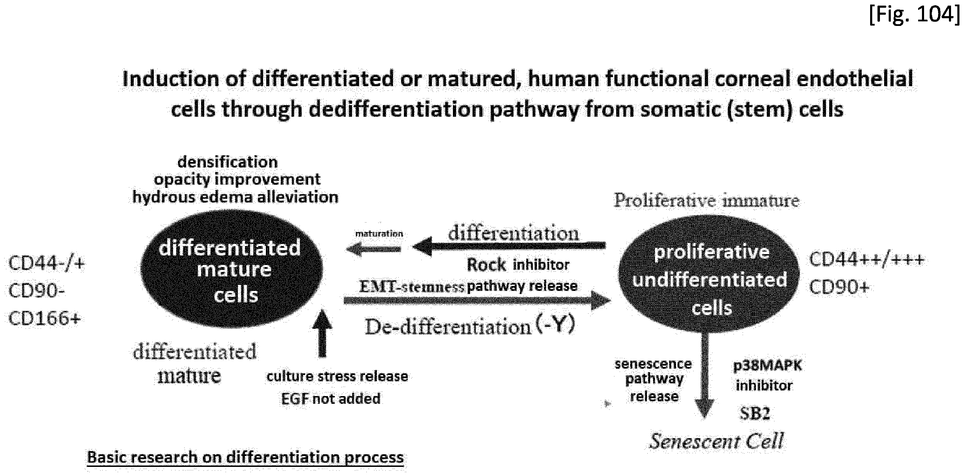

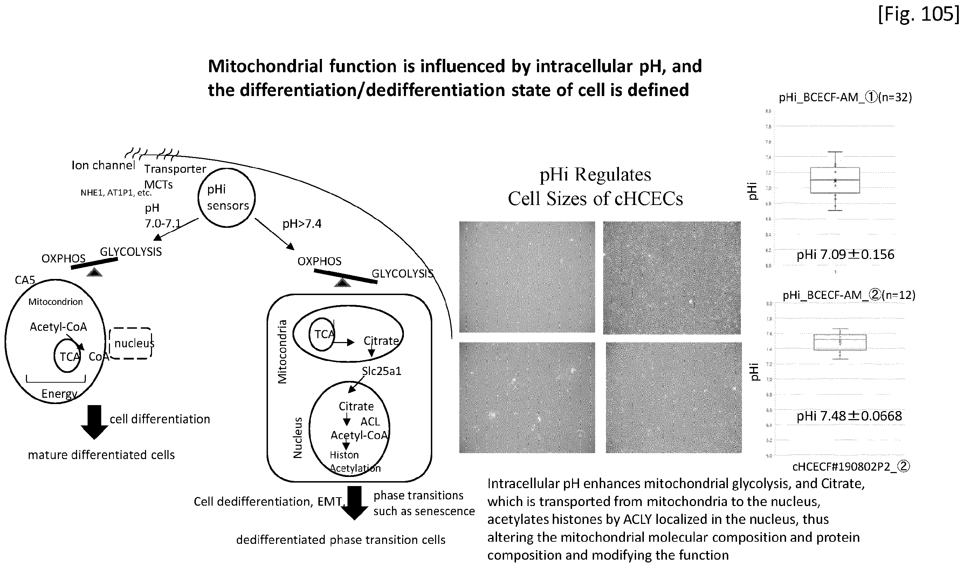

As described with reference to Fig. **1,** Mir34a expression is reduced in non-standard cells, resulting in increased mitochondrial glycolysis. Thus, MiR43a mimics were forcibly introduced into cells to enhance the expression of miR43a, where enhancement of oxidative phosphorylation respiration was confirmed (Figs. **101** and **102**).
Enhanced mitochondrial oxidative phosphorylation respiration OXPHOS effluxes water in the corneal stroma, leading to clinical pharmacological effects (Fig. **103**). Furthermore, the increase in proliferative undifferentiated cells and subsequent differentiation can produce standard cells, or high-quality cells, with excellent pharmacological effects (Fig. **104**).
Whether the cells of the present disclosure become mature differentiated cells or dedifferentiated cells is believed to be due to the influence of intracellular pH on mitochondrial function (Fig. **105**).
The corneal endothelial cells of the present disclosure function to excrete excess water to the anterior chamber side of the corneal stroma to keep the corneal stroma transparent. For that reason, reduction or clearing of corneal opacity, non-thinning of corneal thickening, and the like, can be mentioned, which are evaluated as clinical effects. In cell-injected medically qualified cells, mitochondrial OXPHOS is progressed, and a concentration gradient of lactic acid is formed in endothelial cells from the parenchymal side to the anterior chamber side, and as a result, osmotic pressure promotes water efflux through AQP1 channels. Therefore, as an evaluation of cell functional properties, it is possible to mention mitochondrial oxidative phosphorylation function, AQP1 channel expression, and the like. Further, since intracellular pH suppresses the decrease of dedifferentiation-suppressing miR34a, suppresses the expression of CD44, and maintains mitochondrial OXPHOS as described above, it is possible to mention decreased expression of intracellular mir34a, CD44, and the like, as an evaluation of cell functional properties. The intracellular cation-anion balance maintains the intracellular pH in the neutral range of 7.0-7.2, thereby maintaining high levels of mitochondrial OXPHOS. As for an evaluation of cell functional properties, it is possible to mention Na + K + ATPase (AT1P1), NBCe1 (bicarbonate ion channel), NHE1 (Na+, H+ exchange ion channel), MCT4 (lactate transporter: released to the anterior aqueous humor side), SLC4A11, and the like. Further, as for a functional evaluation method for mixture of non-qualified cells with final product-qualified cells, conceivable is an approach to confirm that the intracellular cation/anion balance deviates the intracellular pH to the alkaline range; and as for an evaluation of cell functional properties, it is possible to confirm the presence or absence of histone acetylation, mitochondrial dysfunction, and the like.

(Example 15: Immunostaining of Cells)

In Example 10, ion channels and/or monocarboxylic acid transport systems were confirmed based on proteomics results; in this Example, selective expression of ion channels was confirmed by immunostaining of cells.

Results of FACS + Ion Channel Molecular Immunostaining (Lot # CR04_P3: donor information)

#CR04

ORL2002-314 LCN/RCN

- Age = 12 / Gender = male
- ECD=3546/3460
- Cause of death: Blunt head trauma
- D-P = 6:42
- D-C = --D

FACS Measurement Results

(Lot#CR04_Day41)

Seeding was performed at ECD 800-900 by CPC, followed by moving the cells to Mikuruma, Kyoto on Day 2, then culturing until Day 41, and detaching with 5xTrypLE select, and the cells were used in FACS. Culture conditions are shown in the table below.

| culture conditions (24-wp) | Day 0 ~ 41 |
|---|---|
| standard cells | Nancy + 10 $\mu$M Y-27632 + 20 $\mu$g/mL ascorbic acid |

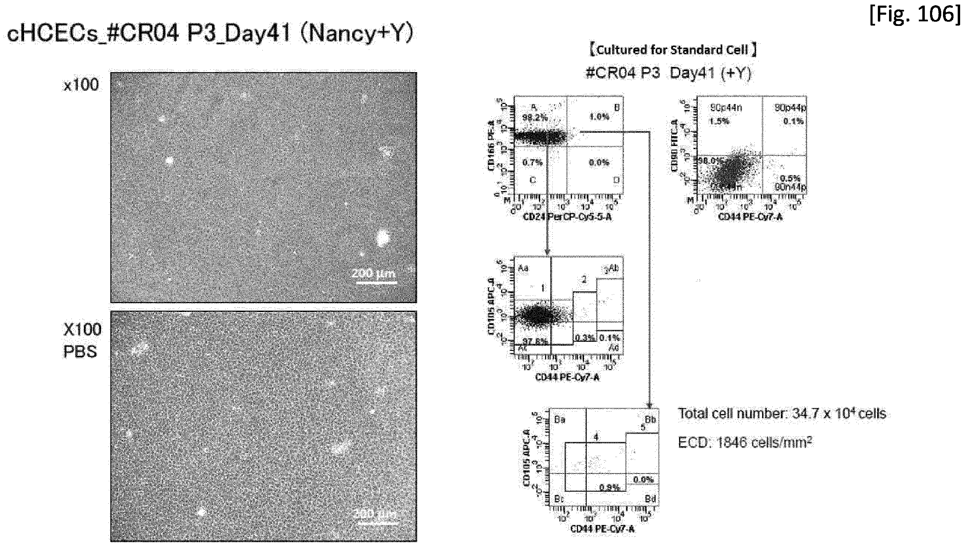

In addition, photographs taken on Day 41 are shown in Fig. **106.**
Furthermore, with regard to immunostaining, the cells were cultured until Day 41 as described above, and the cells were then used for the experiment, where images were acquired at 200x magnification. The antibodies used are shown in the table below.

| antigen | manufacturer | product name and number | concentration used (µg/mL) |
|---|---|---|---|
| human ATP1A1 = Na+ K+ ATPase | millipore | Anti-Na+/K+ ATPase α-1 Antibody, clone C464.6 05-369 | 5 |
| human AQP1 | abcam | Anti-Aquaporin 1 Antibody (EPR11588(B)) (ab168387) | 0.87 |
| human AE2 | invitrogen | SLC4A2 Polyclonal Antibody (Catalog # PA5-82675) | 6.67 |
| human NBCe1 | Santa Cruz | Anti-SLC4A4 (G-9) Antibody (sc-515543) | 0.4 |
| human NHE1 | abcam | Anti-Sodium/Hydrogen Exchanger 1/NHE-1 Antibody (ab67314) | 2 |
| human ZO-1 | invitrogen | ZO-1 Monoclonal Antibody (ZO1-1A12) (#33-9100) | 1 |
| | invitrogen | ZO-1 Polyclonal Antibody (# 61-7300) | 1 |
| Acetyl-Histone H3 | millipore | Anti-acetyl-Histone H3 (#06-599) | 1 |
| DAPI | Dojindo | D523　Cellstain® DAPI solution (wako code:340-07971) | |
| | invitrogen | Donkey anti-Rabbit IgG (H+L) Highly Cross-Adsorbed Secondary Antibody, Alexa Fluor Plus 488 (# A32790) | 1 |
| 2nd Abs; anti-rab igG dye-conjugated | invitrogen | Donkey anti-Rabbit IgG (H+L) Highly Cross-Adsorbed Secondary Antibody, Alexa Fluor Plus 647 (# A32795) | 1 |
| | invitrogen | Donkey anti-Mouse IgG (H+L) Highly Cross-Adsorbed Secondary Antibody, Alexa Fluor Plus 488 (# A32766) | 1 |
| 2nd Abs; anti-mus igG dye-conjugated | invitrogen | Donkey anti-Mouse IgG (H+L) Highly Cross-Adsorbed Secondary Antibody, Alexa Fluor Plus 647 (# A32787) | 1 |

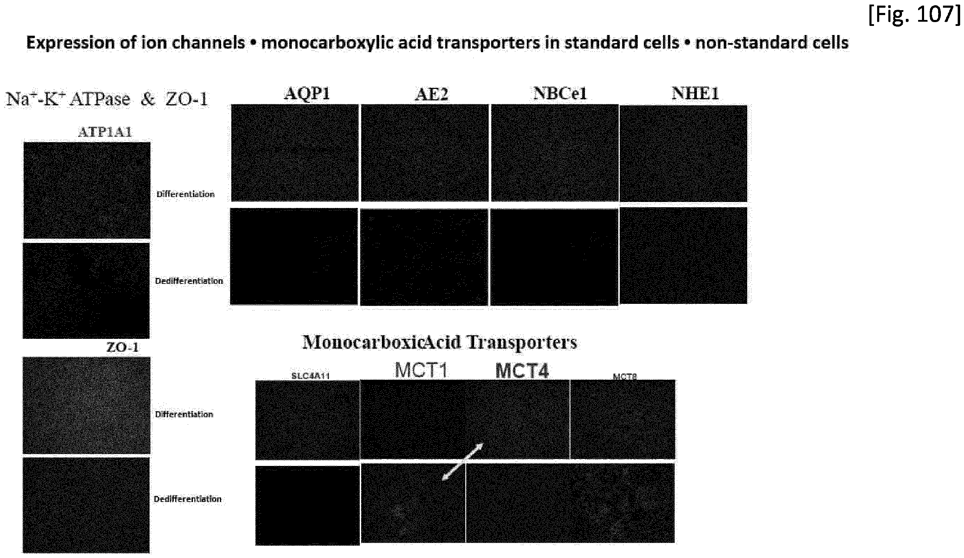

As a result, ATP1A1 was localized to the cell membrane; NHE1 was localized intracellularly, and partially also localized to the cell membrane; AQP1 was localized to the cell membrane; NBCe1 was localized to the cell membrane; AE2 was localized intracellularly and to the cell membrane; and Acetyl-Histone H3 was under-expressed. A summary of the results is shown in Fig. **107.**

(Example 16: Enhancement in Histone Acetylation in Non-Standard Cells)

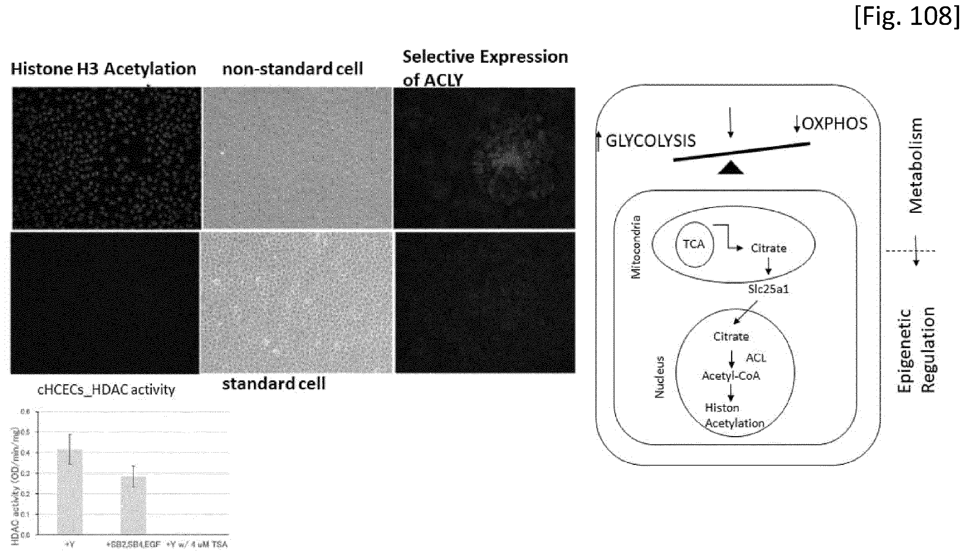

In this Example, the reason why non-standard cells are generated was confirmed. As shown in Fig. **108,** it was suggested that intracellular pH enhances mitochondrial glycolysis, and Citrate, which is transported from mitochondria to the nucleus, acetylates histones by ACLY and ACSS2 localized in the nucleus, thus altering the mitochondrial Matrix constituent protein composition.

(Example 17: Comparison between Standard Cells and Non-Standard Cells)

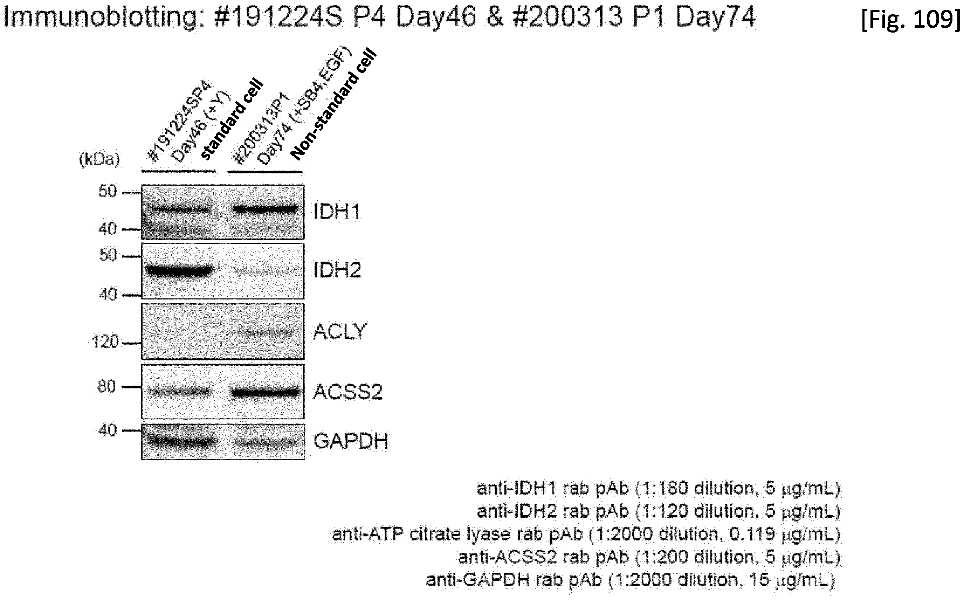

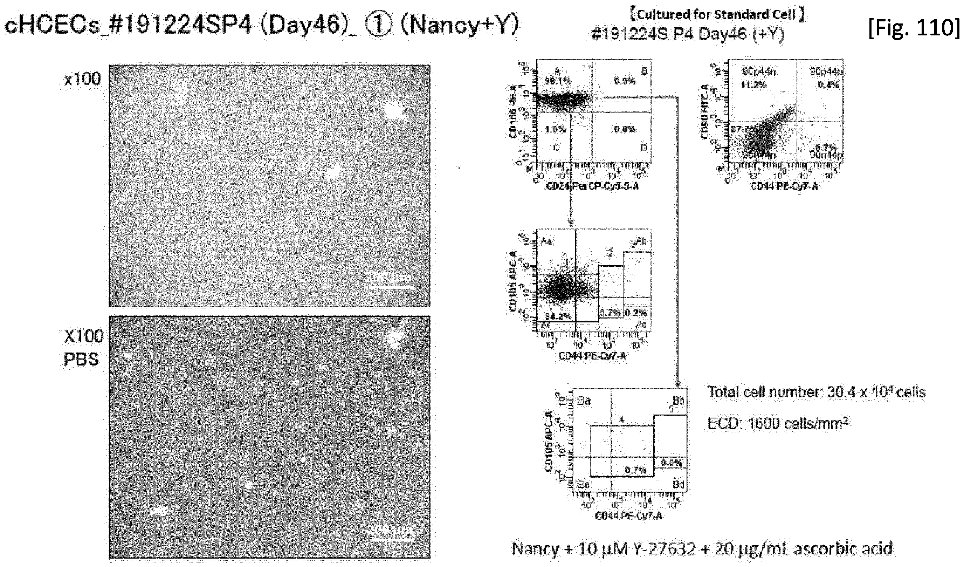

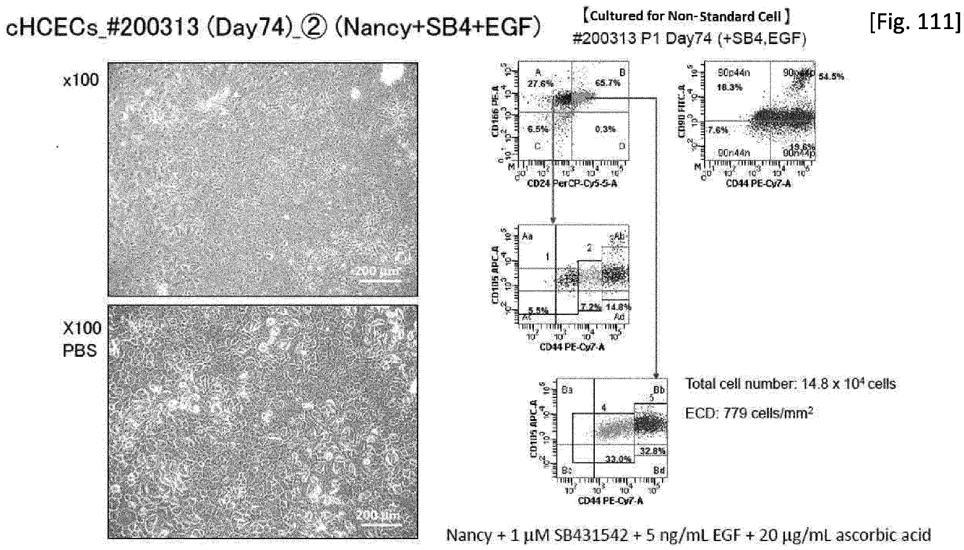

In this Example, the expression of specific protein in standard cells and non-standard cells (CST cells) was compared. #191224S P4 Day 46 (+Y) was used as standard cells, and #200313 P1 Day 74 (+SB4, EGF) was used as non-standard cells. The results are shown in Figs. **109** to **111.**

In addition, in order to confirm that histone deacetylase activity is reduced in standard cells as one of the reasons why histone acetylation is enhanced in non-standard cells, HAT/HDAC activity was measured in standard cells and non-standard cells. The measurement of HAT/HDAC activity was performed as follows.

The following Lot #191224S_P4 was used as a donor.

#191224S
AEN-024 OSCN
Age = 29 / Gender = male
ECD = 2941
Cause of death: Acute Respiratory Distress Syndrome
D-P = 07:32
D-C = 7D

The culture conditions were set as shown in the table below.

| culture conditions (6-wp) | Day 0 ~ 42 |
|---|---|
| **D42: 2 well each** | ① Nancy + 10 μM Y-27632 |
| | ② Nancy+ 1 μM SB431542 + 5 ng/mL EGF |

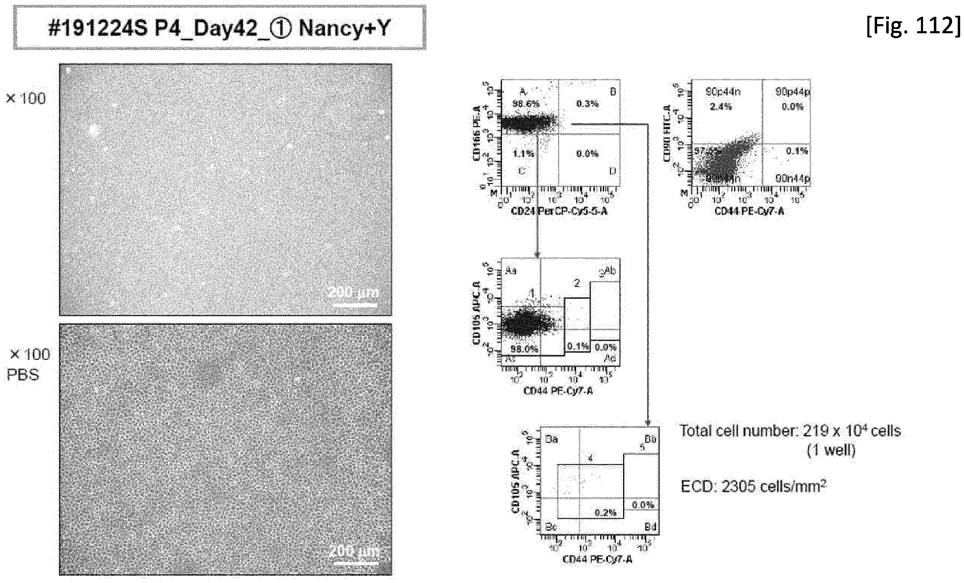

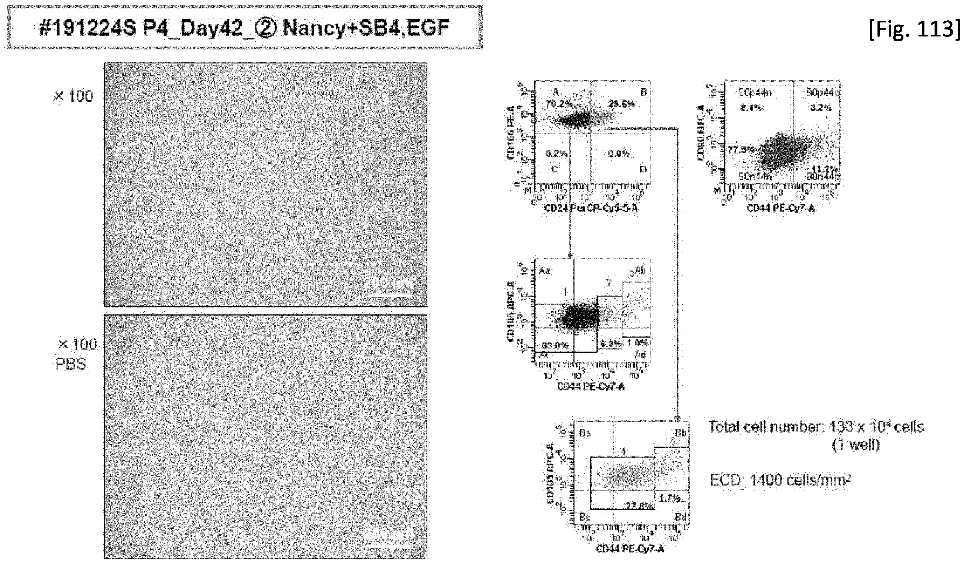

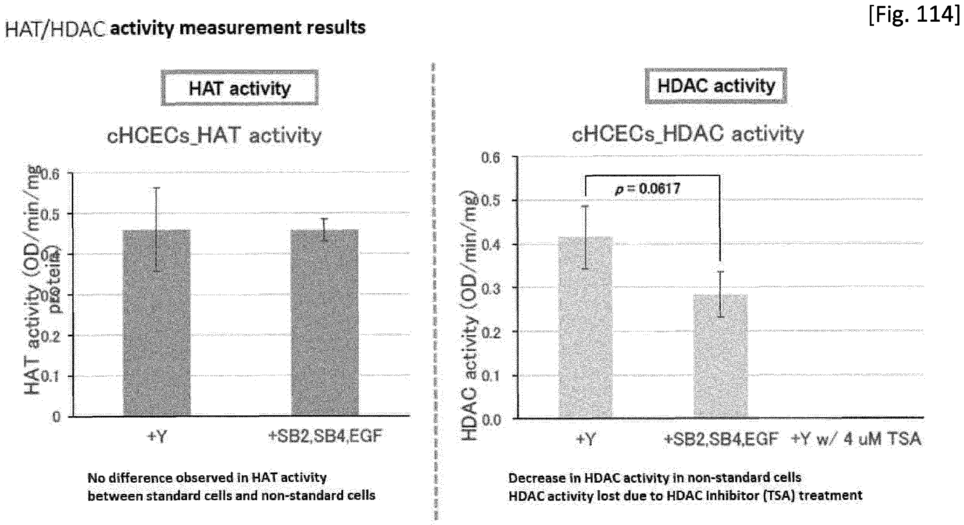

After seeding with ECD400 for the reference numeral 1 and ECD800 for the reference numeral 2 (passage from non-standard cells), the cells were cultured until Day 42. One well of each was detached by FACS: 5xTrypLE select, and used for experiments (same sample as for measuring miRNA expression). In addition, 1 well was subjected to nuclear protein extraction, and the cells were directly recovered using a Nuclear Extraction Kit. The results are shown in Figs. **112** to **114.**

As for the substrate requirement of the mitochondrial respiratory system, it was confirmed that anaplerosis was enhanced in CST cells compared to standard cells, and that BCAT2 and BCKDH involved in branched-chain amino acid metabolism in mitochondria were enhanced in standard cells. Interestingly, mitochondrial ubiquitous metabolic enzymes CS, ACO2, IDH2, MDH2 and ME3 involved in the TCA pathway as well as ACSS1 and ACAT1 involved in AcetylCoA production were all enhanced in normal cells, whereas the expression of ACLY, ACO1, IDH1, MDH1 and ME1, which are isozymes ubiquitously present in the cytoplasm, and ACSS2 and ACAT2 involved in AcetylCoA production in the cytoplasm and nucleus were enhanced in CST cells, which are non-qualified cells. This suggests the possibility that under the influence of environmental factors such as culture stress, acetylCoA in the cytoplasm and nucleus is epigenetically involved in histone acetylation, thereby regulating the expression of CST cells. It is considered that the uneven distribution of nuclear-localized AcetylCoA plays an important role in the disruption of differentiation of dedifferentiated cells.

(Note)

As described above, the present disclosure is exemplified by the use of its preferred Embodiments. However, it is understood that the scope of the present disclosure should be interpreted solely based on the claims. It is also understood that any patent, any patent application, and any references cited herein should be incorporated herein by reference in the same manner as the contents are specifically described herein. The present application claims priority to Japanese Patent Application No. 2020-32139 filed on February 27, 2020 with the Japan Patent Office, the entire content of which is incorporated by reference as if it constitutes the content of the present application.

[Industrial Applicability]

The present disclosure finds applicability in the medical industry and related industries related to corneal endothelial regenerative medicine.

**Claims**

1. A method of manufacturing a functional human corneal endothelial cell capable of eliciting a human corneal function when injected into an anterior chamber of a human eye, the method comprising the step of:
(b) proliferating and/or differentiating or maturing a corneal endothelial progenitor cell under a culture condition capable of minimizing culture stress, such as proliferation stress.

2. The method according to claim 1, wherein the human corneal function comprises a human corneal endothelial cell functional property.

3. The method according to claim 1 or 2, further comprising the step of: (a) dedifferentiating a human corneal endothelial tissue-derived cell to obtain the corneal endothelial progenitor cell.

4. The method of manufacturing a functional human corneal endothelial cell capable of eliciting a human cornea function, and especially a human corneal endothelium functional property, when injected into an anterior chamber of a human eye, according to any one of claims 1-3, the method proliferating and/or differentiating or maturing a corneal endothelial progenitor cell in the presence of a cell growth factor with an amount less than the amount at which transformation occurs.

5. The method according to any one of claims 1-4, wherein the cell growth factor comprises an epidermal growth factor (EGF) .

6. The method according to any one of claims 1-5, wherein the transformation comprises endothelial-mesenchymal transformation.

7. The method according to any one of claims 1-6, wherein the step of proliferating and/or differentiating or maturing is performed in the presence of a ROCK inhibitor.

8. The method according to any one of claims 1-6, comprising the step of confirming that the cell obtained in the step (b) is a cell in which mitochondria-dependent oxidative phosphorylation is increased in mitochondria and acetyl-CoA expression in the cytoplasm or nucleus is not increased, and in which epigenetic multigene expression through histone acetylation by acetyl-CoA is not induced.

9. The method according to any one of claims 1-8, comprising the step of confirming that the cell obtained in the step (b) is a cell in which one or more metabolism-related enzymes selected from the group consisting of citrate synthase (CS), aconitase 2 (ACO2), isocitrate dehydrogenase 2 (IDH2), malate dehydrogenase 2 (MDH2), malic enzyme 3 (ME3), ACSS1, acetyl-CoA acetyltransferase 1 (ACAT1), pyruvate dehydrogenase (PDH), branched chain amino acid transaminase 2(BCAT2), and branched-chain ketoacid dehydrogenase 2 (BCKDH2) are expressed in mitochondria.

10. The method according to any one of claims 1-9, comprising the step of confirming that the cell obtained in the step (b) is a cell in which ATP citrate lyase (ACLY), aconitase 1 (ACO1), isocitrate dehydrogenase 1 (IDH1), malate dehydrogenase 1 (MDH1), malic enzyme 1 (ME1), acetyl-CoA synthetase 2 (ACSS2), acetyl-CoA acetyltransferase 2 (ACAT2), and/or lactate dehydrogenase (LDH) is not expressed, or is hardly expressed.

11. The method according to any one of claims 1-10, comprising the step of confirming that expression of ion channel and/or monocarboxylic acid transporter leading to a corneal endothelium (cell) functional property leading to improvement on corneal opacity and hydrous edema, resulting in sustained long-term retention of corneal endothelial tissue cell density and improvement on visual acuity is recognized in the cell obtained in the step (b).

**12.** The method according to any one of claims 1-11, comprising the step of confirming that expression of sodium/hydrogen exchanger 1 (NHE1) and/or aquaporin 1 (AQP-1) is increased in the cell obtained in the step (b).

**13.** The method according to claim 9 or 10, comprising the step of confirming that expression of bicarbonic anhydrase 5B (CA5B) is increased in the cell obtained in the step (b).

**14.** The method according to any one of claims 1-12, comprising the step of confirming that the cell obtained in the step (b) has a property that a metabolic enzyme related to a TCA cycle, etc., and a metabolite, such as AcetylCoA, are not present in the cytoplasm or nucleus so as not to lead to the production of contaminant cell state transition cells and are organelle-selectively localized in mitochondria.

**15.** The method according to any one of claims 1-14, wherein the functional human corneal endothelial cell is made from a cell, as the origin thereof, selected from the group consisting of: a corneal endothelial tissue-derived cell; a pluripotent stem cell; a mesenchymal stem cell; a corneal endothelial progenitor cell collected from a corneal endothelium; a cell collected form a corneal endothelium; and a corneal endothelial precursor cell and a corneal endothelial-like cell made by a direct programming method.

**16.** A functional human corneal endothelial cell in which expression of a functional protein leading to a corneal endothelium (cell) functional property leading to improvement on corneal opacity and hydrous edema, resulting in sustained long-term retention of corneal endothelium tissue cell density and improvement on visual acuity is recognized or in which a protein that inhibits the corneal endothelium (cell) functional property is not elicited or is reduced.

**17.** The cell according to claim 16, wherein the cell is a functional human corneal endothelium cell capable of eliciting a human corneal endothelium functional property when injected into an anterior chamber of a human eye, in which one or more metabolism-related enzymes selected from the group consisting of citrate synthase (CS), aconitase 2 (ACO2), isocitrate dehydrogenase 2 (IDH2), malate dehydrogenase 2 (MDH2), malic enzyme 3 (ME3), acetyl-CoA synthetase 1 (ACSS1), acetyl-CoA acetyltransferase 1 (ACAT1), pyruvate dehydrogenase (PDH), branched chain amino acid transaminase 2 (BCAT2), and branched-chain ketoacid dehydrogenase 2 (BCKDH2) are expressed in mitochondria.

**18.** The cell according to claim 16 or 17, wherein the cell is a functional human corneal endothelial cell capable of eliciting a human corneal endothelium functional property when injected into an anterior chamber of a human eye, including at least one selected from the group consisting of: a cell in which mitochondria-dependent oxidative phosphorylation is increased in mitochondria or acetyl-CoA expression in the cytoplasm or nucleus is not increased; and a cell in which epigenetic multigene expression through histone acetylation by acetyl-CoA is not induced.

**19.** The cell according to any one of claims 16-18, wherein the cell is a human corneal endothelial cell, in which ATP citrate lyase (ACLY), aconitase 1 (ACO1), isocitrate dehydrogenase 1 (IDH1), malate dehydrogenase 1 (MDH1), malic enzyme 1 (ME1), acetyl-CoA synthetase 2 (ACSS2), acetyl-CoA acetyltransferase 2 (ACAT2), and/or lactate dehydrogenase (LDH) is not expressed or is not substantially expressed.

**20.** The cell according to any one of claims 16-19, wherein expression of sodium/hydrogen exchanger 1 (NHE1) and/or aquaporin 1 (AQP-1) is increased in the functional human corneal endothelial cell.

**21.** The cell according to any one of claims 16-20, wherein expression of bicarbonic anhydrase 5B (CA5B) is increased in the functional human corneal endothelial cell.

**22.** The cell according to any one of claims 16-21, wherein the functional human corneal endothelial cell comprises all selected from the group consisting of: (i) a property that a metabolic enzyme related to the TCA cycle, etc., and a metabolite, such as AcetylCoA, are not present in the cytoplasm or nucleus so as not to lead to the production of contaminant cell state transition cells and are organelle-selectively localized in mitochondria; (ii) increase in mitochondria-dependent oxidative phosphorylation in mitochondria; (iii) reduction in epigenetic multigene expression through histone acetylation by acetyl-CoA (including no elicitation); (iv) increase in expression of sodium/hydrogen exchanger 1 (NHE1) and/or aquaporin 1 (AQP-1); and (v) increase in expression of bicarbonic anhydrase 5B (CA5B).

**23.** The cell according to any one of claims 16-22, wherein endothelial-mesenchymal transition has not occurred or has not substantially occurred.

24. A functional human corneal endothelial cell capable of eliciting a human corneal endothelium functional property when injected into an anterior chamber of a human eye, wherein endothelial-mesenchymal transition has not occurred or has not substantially occurred.

25. The cell according to any one of claims 16-24, wherein the functional human corneal endothelial cell is made from a cell, as the origin thereof, selected from the group consisting of: a corneal endothelial tissue-derived cell; a pluripotent stem cell; a mesenchymal stem cell; a corneal endothelial progenitor cell collected from a corneal endothelium; a cell collected form a corneal endothelium; and a corneal endothelial precursor cell and a corneal endothelial-like cell made by a direct programming method.

26. A cell population comprising a cell manufactured by the method according to any one of claims 1-15 and/or a cell according to any one of claims 16-25.

27. A method of quality control or process control of a functional human corneal endothelium cell capable of eliciting a human corneal endothelium functional property when injected into an anterior chamber of a human eye, the method comprising the step of confirming that one or more metabolism-related enzymes selected from the group consisting of citrate synthase (CS), aconitase 2 (ACO2), isocitrate dehydrogenase 2 (IDH2), malate dehydrogenase 2 (MDH2), malic enzyme 3 (ME3), acetyl-CoA synthetase 1 (ACSS1), acetyl-CoA acetyltransferase 1 (ACAT1), pyruvate dehydrogenase (PDH), branched chain amino acid transaminase 2 (BCAT2), and branched-chain ketoacid dehydrogenase 2 (BCKDH2) are expressed in mitochondria of the cell.

28. The method according to claim 27, further comprising the step of confirming that expression of acetyl-CoA in the cytoplasm and nucleus, and epigenetic multigene expression through histone acetylation by acetyl-CoA, are not elicited in the cell.

29. The method according to claim 27 or 28, further comprising the step of confirming that expression of sodium/hydrogen exchanger 1 (NHE1) and/or aquaporin 1 (AQP-1) is increased in the cell.

30. The method according to any one of claims 27-29, further comprising the step of confirming that expression of bicarbonic anhydrase 5B (CA5B) is increased in the cell.

31. A method of quality control or process control of a functional human corneal endothelium cell capable of eliciting a human corneal endothelium functional property when injected into an anterior chamber of a human eye, or a method of detecting a non-functional corneal endothelial cell present as a mixture with a functional human corneal endothelial cell, the method comprising the step of confirming one or more of the following items:

(1) no fibroblast, foreign body, discoloration, or other abnormalities on visual inspection by phase-contrast imaging microscopy on the day of transplantation;
(2) number of cell of $1.5 \times 10^6$ cells/450 $\mu$L two weeks prior to and/or on the day of transplantation;
(3) 85% or more cell viability by trypan blue staining;
(4) PDGF-BB: 100 pg/mL or more in a purity test by ELISA of cell supernatant;
(5) in a purity test by FACS of cell supernatant collected two weeks prior to and/or on the day of cell injection,

$CD166^+ > 99\%$
$CD24^+ < 5\%$
$CD26^+ < 5\%$
$CD200^+ < 5\%$
$CD44^{high} < 5\%$
$CD44^{low} > 90\%$
$CD105^{-\sim weak} > 90\%$
$CD90^+ < 5\%$;

(6) effector cell (E-ratio) > 90%;
(7) pump function (Na+/K+ ATPase) two days prior to transplantation: positive;
(8) barrier function (ZO-1) two days prior to transplantation: positive;
(9) less than 125 ng/$\mu$L in BSA negative test;
(10) endothelial cell density (ECD) on the day of transplantation to be 1500 cells/mm$^2$ or more;
(11) expression of miR184;

(12) lactic acid production; and
(13) cell size of less than 250 $\mu$m.

32. A cell population of a functional human corneal endothelial cell capable of eliciting a human corneal endothelial functional property when injected into an anterior chamber of a human eye, the cell population satisfying one or more of the following items:

    (1) no fibroblast, foreign body, discoloration, or other abnormalities on visual inspection by phase-contrast imaging on the day of transplantation;
    (2) number of cell of $1.5 \times 10^6$ cells/450 $\mu$L two weeks prior to and/or on the day of transplantation;
    (3) 85% or more cell viability by trypan blue staining;
    (4) PDGF-BB: 100 pg/mL or more in a purity test by ELISA of cell supernatant;
    (5) in a purity test by FACS of cell supernatant collected two weeks prior to and/or on the day of transplantation,

        $CD166^+ > 99\%$
        $CD24^+ < 5\%$
        $CD26^+ < 5\%$
        $CD200^+ < 5\%$
        $CD44^{high} < 5\%$
        $CD44^{low} > 90\%$
        $CD105^{-\sim weak} > 90\%$
        $CD90^+ < 5\%$;

    (6) effector cell (E-ratio) > 90%;
    (7) pump function (Na+/K+A TPase) two days prior to transplantation: positive;
    (8) barrier function (ZO-1) two days prior to transplantation: positive;
    (9) less than 125 ng/$\mu$L in BSA negative test;
    (10) ECD on the day of transplantation to be 1500 cells/mm$^2$ or more;
    (11) expression of miR184;
    (12) lactic acid production; and
    (13) cell size of less than 250 $\mu$m.

[Fig. 1]

Energy metabolism control of mitochondria through CD44

Depression of CD44/RhoA Axis Skewed
the Energy Metabolism from Glycolysis to OXPHOS
in Differentiated Mature cHCECs

ROCK inhibitor

C-Myc

p53 → miR34 → ▮ CD44→ RhoA → Glycolysis

mitochondria-dependent oxidative respiration OXPHOS

mature differentiated cHCEC

EP 4 116 411 A1

[Fig. 2]

**subpopulation cells**

cell function related to clinical efficacy directly-linked function

clinical efficacy directly-linked function

standard cells

acceleration of water efflux function

mitochondrial OXPHOS acceleration

maintain cation-anion balance and intracellular pH

acceleration of water efflux function

pump function
osmotic pressure (metabolic efflux)
increase in substantial anhydration sum

clinical efficacy

opacity improvement
hydrous edema improvement
cell miniaturization
densification

acceleration of cytoplasm and bicarbonate ion decrease in intracellular pH

acceleration in producing metabolite and lactic acid

acceleration in Glycolysis

mechanism of action leading to effect

**non-standard cells**

Cell Injection Regenerative Medicine

[Fig. 3]

[Fig. 4]

EP 4 116 411 A1

densification · opacity improvement
· hydrous edema alleviation

differentiated mature cells

maturation ← differentiation

ROCK inhibitor

EMT-stemness pathway cancellation

dedifferentiation (-Y)

proliferative undifferentiated cells

culture stress cancellation

senescence pathway cancellation

[Fig. 5]

densification・opacity improvement
・hydrous edema alleviation

differentiation

differentiated mature cells

antagonism

proliferative unmatured cells

EMT-stemness pathway

proliferation stress

senescence pathway

senescence resting cells

[Fig. 6]

#202 P2 Day41 (160822)

[Fig. 7]

P3 Day42 (161004)

#202 P3 EGF 0.5 ng/mL

cell number 141.5x10^4 cells
ECD 1489

#202 P3 EGF(-)

cell number 126x10^4 cells
ECD 1326

# P3Day42 (161004)

[Fig. 8]

#202 P3 EGF 1 ng/mL

cell number 133.5x10^4 cells
ECD 1405

#202 P3 EGF 5 ng/mL

cell number 130x10^4 cells
ECD 1368

EP 4 116 411 A1

[Fig. 9]

# P4Day42 (161115)

[Fig. 10]

#202 P4 EGF 1 ng/mL

cell number 104x10^4 cells
ECD 1095

#202 P4 EGF 5 ng/mL

cell number 108.5x10^4 cells
ECD 1142

EP 4 116 411 A1

# Day41 (161014)

[Fig. 11]

## #214 P0 EGF(-)

cell number 198x10^4 cells
ECD 2084

## #214 P0 EGF 0.5 ng/mL

cell number 217.5x10^4 cells
ECD 2289

EP 4 116 411 A1

# Day42 (161125)

[Fig. 12]

## #214 P1 EGF(-)

cell number 113.5x10^4 cells
ECD 1195

## #214 P1 EGF 0.5 ng/mL (P0 EGF(-))

cell number 156.5x10^4 cells
ECD 1647

# Day42 (161125)

[Fig. 13]

## #214 P1 EGF(-) (P0 EGF 0.5 ng/mL)

cell number 145x10^4 cells
ECD 1526

## #214 P1 EGF 0.5 ng/mL

cell number 143x10^4 cells
ECD 1505

EP 4 116 411 A1

[Fig.14]

## Day42 (170106)

EP 4 116 411 A1

#214 P2 EGF(-)

#214 P2 EGF 0.5 ng/mL

cell number 168.5x10^4 cells
ECD 1774

cell number 153.5x10^4 cells
ECD 1616

[Fig. 15]

[Fig. 16]

|  | Y27632+ | | | | Y27632- | | | |
|---|---|---|---|---|---|---|---|---|
|  | EGF- | | EGF+ | | EGF- | | EGF+ | |
|  | 41Y a37- P0 | 21Y a38- P0 | 41Y a37- P0 | 21Y a38- P0 | 41Y a37- P0 | 21Y a38- P0 | 41Y a37- P0 | 21Y a38- P0 |
| CD44- | 68% | 90% | 26% | 70% | 48% | 75% | 4% | 14% |
| CD44+ / CD44++ | 22% | 4% | 43% | 19% | 32% | 10% | 32% | 54% |
| CD44+++ | 4% | 1% | 9% | 3% | 11% | 2% | 35% | 26% |
| CD24++ / CD44+++ | 1% | 0% | 10.3% | 1% | 2% | 0% | 27% | 6% |
| CD44- / CD90- | 61% | 93% | 25% | 68% | 43% | 84% | 4% | 13% |
| Photo evaluation | △ | ○ | × | ○ | △ | ◎ | × | × |

[Fig. 17]

[Fig. 18]

First Time

Second Time

inside the cell

miR34a-5p

**Examples of Metabolite Hierarchies**

[Fig. 19]

utilization for examination
of additives and
medium composition

Glyoxylate
Glycerol 3-phosphate
2-Phosphoglyceric acid
Glycolic acid
Folic acid
Guanine
His
Thr
Pro
Phe

Trp
Tyr
Lys

β-Ala
Pyruvic acid
Ser
BCAA(Ile,Leu,Val)
Citrulline

Lactic acid
**followed by
TCA products**

Malic acid

Argininosuccinic
acid

Fumaric acid

cis-Aconitic acid

Isocitric acid

Citric acid

Ornithine

Uric acid

mature differentiated
cells

intermediate
mature cells

medium

C3221  C3222  C3321  C3322  C2325  C2326  Nancy-1  Nancy-2

[Fig. 20]

G1: cell of interest
G5: non-intended cell Z
G2: cell of interest + non-intended cell I
G3: non-intended cell Z + non-intended cell P
G4: cell of interest + non-intended cell P, I, Z

[Fig. 21]

[Fig. 22]

G1：EGF 0.4g/mbng/mL
G2：0.5ng/mL
G3：5ng/mL
Control：mediur

[Fig. 23]

| Additive Reagent | ① | ② | ③ | ④ | ⑤ |
|---|---|---|---|---|---|
| Y27632 | + | + | + | + | + |
| EGF | - | + | - | + | + |
| 10 mM SB2 | - | + | + | - | + |
| 1 mM SB4 | - | + | - | - | - |

EP 4 116 411 A1

[Fig. 24]

CT09 P5 D34 (190122)

x100

① Y(+)

② Y, EGF, 10 mM SB2, 1 mM SB4(+)

100 μm

100 μm

CT09 P5 D34 (190122)

x100

③ Y, 10 mM SB2(+)

④ Y, EGF(+)

[Fig. 25]

EP 4 116 411 A1

[Fig. 26]

CT09 P5 D34 (190122)
x100

⑤ Y, EGF, 10 mM SB2(+)

Day34 (190122)

[Fig. 27]

[Fig. 28]

[Fig. 29]

## Day34 (190122)

CT09 P5 ⑤ Y, EGF, 10 mM SB2(+)

cell number 179.5x10^4
cells
ECD 1889

EP 4 116 411 A1

[Fig. 30]

EP 4 116 411 A1

| | | Additive Reagent | Culture Supernatant |
|---|---|---|---|
| P4 | | Y | Day42 |
| P5 | ① | Y | 1w, 2w, 3w, 4w, 5w |
| | ② | Y, EGF, 10 mM SB2, 1 mM SB4 | 1w, 2w, 3w, 4w, 5w |
| | ③ | Y, 10 mM SB2 | 1w, 2w, 3w, 4w, 5w |
| | ④ | Y, EGF | 1w, 2w, 3w, 4w, 5w |
| | ⑤ | Y, EGF, 10 mM SB2 | 1w, 2w, 3w, 4w, 5w |

# PDGF-bb

[Fig. 31]

Y EGF SB2 SB4 **in 1w, 4w, and 5w were below the detection limit**

EP 4 116 411 A1

PDGF-bb

[Fig. 32]

Y EGF SB2 SB4 in 1w, Y EGF SB2 SB4 in 4w, and Y EGF SB2 SB4 in 5w were below the detection limit

EP 4 116 411 A1

[Fig. 33]

## IL-8

Y EGF SB2 in 1w〜5w were above the detection limit

EP 4 116 411 A1

IL-8

[Fig. 34]

Y EGF in 1w〜5w were above the detection limit

[Fig. 35]

**Mito Stress Test**

To measure the metabolic pathways in living cells

Extracellular flux analyzer (XFe24)

$\Delta\Psi_M$ = Membrane potential

Agilent Technologies

[Fig. 36]

In the absence of pyruvic acid, maximal respirations decreases only in SPs with CST

[Fig. 37]

[Fig. 38]

| Culture No. | #190318 | #190719 | #190802 |
|---|---|---|---|
| Donor No. | ACS-736 OSCN/ODCN | AEB-301 OSCN/ODCN | ACW-134 OSCN/ODCN |
| Age | 73 | 40 | 28 |
| Gender | Male | Male | Female |
| ECD | 3040/3021 | 3448/3289 | 3003/3021 |
| COD | Metastatic Prostate Cancer | Bleomycin Lung Toxicity 2/2 Chemo Treatments | ESRD |
| D-P | 17:29 | 11:28 | 8:57 |
| D-C | 8D | 8D | 5D |

EP 4 116 411 A1

[Fig. 39]

| Y addition | Day0-3 | Day4-9 | Day10-19 | Day20- | Day28- |
|:---:|:---:|:---:|:---:|:---:|:---:|
| ① | + | + | + | + | +, SB2+ |
| ② | - | + | + | + | +, SB2+ |
| ③ | - | - | + | + | +, SB2+ |
| ④ | - | - | - | + | +, SB2+ |
| ⑤ | + | - | - | - | -, SB2+ |
| ⑥ | - | - | - | - | -, SB2+ |

EP 4 116 411 A1

[Fig. 40]

| | Additive | Supernatant Collection |
|---|---|---|
| P1 | -SB2 | D37, D41 |
| P2 | +SB2 | 1w~6w |
| | -SB2 | 1w~6w |

EP 4 116 411 A1

## FACS results of #190719 P1, P2

| P1 | Effector (%) | CD44+ (%) | CD44++ (%) |
|---|---|---|---|
| ① well1 | 82.6 | 0.6 | 0.1 |
| ① well2 | 73.4 | 0.7 | 0 |
| ② well1 | 79.1 | 0.7 | 1.1 |
| ② well2 | 82.2 | 0.6 | 0.1 |
| ③ well1 | 90.1 | 0.5 | 0.3 |
| ③ well2 | 94.2 | 0.3 | 0.1 |
| ④ well1 | 80.9 | 1.8 | 0.2 |
| ④ well2 | 84 | 1.9 | 0.4 |
| ⑤ well1 | 45.7 | 3 | 1.4 |
| ⑤ well2 | 49.3 | 2.5 | 0.4 |
| ⑥ well1 | 57.8 | 7.7 | 3.2 |
| ⑥ well2 | 54.8 | 9.4 | 5.6 |

| P2 | Effector (%) | CD44+ (%) | CD44++ (%) |
|---|---|---|---|
| ① well1 | 86.4 | 2.7 | 0.6 |
| ① well2 | 85.7 | 2.5 | 0.5 |
| ② well1 | 81.7 | 2.2 | 0.6 |
| ② well2 | 76.4 | 1.8 | 0.5 |
| ③ well1 | 90.4 | 2.4 | 0.3 |
| ③ well2 | 89.5 | 3.5 | 0.7 |
| ④ well1 | 86.5 | 3.5 | 0.6 |
| ④ well2 | 83.6 | 3.5 | 0.8 |
| ⑤ well1 | 71.1 | 8 | 5.6 |
| ⑤ well2 | 61.7 | 8.3 | 5.8 |
| ⑥ well1 | 60.3 | 14.4 | 7.1 |
| ⑥ well2 | 59.9 | 14.3 | 9.8 |

## FACS results of #190802 P1

| P1 | Effector (%) | CD44+ (%) | CD44++ (%) |
|---|---|---|---|
| ① well1 | 88.2 | 1 | 0.4 |
| ① well2 | 90.1 | 0.7 | 0.1 |
| ② well1 | 89.3 | 0.6 | 0.3 |
| ② well2 | 87.2 | 0.9 | 0.4 |
| ③ well1 | 95 | 0.5 | 0.1 |
| ③ well2 | 88.2 | 0.9 | 0.1 |
| ④ well1 | 92.1 | 2.6 | 1.1 |
| ④ well2 | 92.7 | 2.5 | 0.4 |
| ⑤ well1 | 82.2 | 5.4 | 1.3 |
| ⑤ well2 | 84.8 | 5.9 | 1.3 |
| ⑥ well1 | 84.9 | 8 | 3.1 |
| ⑥ well2 | 84.6 | 8.2 | 1.6 |

[Fig. 41]

EP 4 116 411 A1

#190719 P1 Day39 (191001) phase-contrast photos

well1 x100

EP 4 116 411 A1

# #190719 P1 Day39 (191001) FACS Results

[Fig. 44]

#190802 P1 Day35 (191011) FACS Results

# #190719 P2 Day40 (191106) FACS Results

[Fig. 45]

### #190719 P2 ① well1

**cell number** 25.7x10^4 cells
ECD 1353

### #190719 P2 ⑤ well2

**cell number** 20.6x10^4 cells
ECD 1084

### #190719 P2 ③ well1

**cell number** 34.7x10^4 cells
ECD 1826

### #190719 P2 ⑥ well1

**cell number** 20.9x10^4 cells
ECD 1100

[Fig. 46]

## PDGF-bb #190719 P1, P2

EP 4 116 411 A1

PDGF-bb #190318

[Fig. 47]

[Fig. 48]

PDGF-bb #190719 P1, P2

EP 4 116 411 A1

[Fig. 49]

EP 4 116 411 A1

PDGF-bb #190318

[Fig. 50]

IL-8 #190719 P1, P2

EP 4 116 411 A1

[Fig. 51]

IL-8 #190318

[Fig. 52]

[Fig. 53]

[Fig. 54]

EP 4 116 411 A1

|  | Day0-3 | Day4- | Day10- | Day28- |
|---|---|---|---|---|
| ① | +Y, A | | | +Y, A, SB2 |
| ② | +A | | +Y, A | +Y, A, SB2 |
| ③ | +Y, A | +A, EGF, SB2, SB4 | | |

[Fig. 55]

#190719 P4 Day35 (200121)

Day35 (200121)

[Fig. 56]

#190719 P4 ①

#190719 P4 ②

cell number 125.5x10^4 cells
ECD 1321

cell number
52.5x10^4 cells
ECD 553

EP 4 116 411 A1

113

[Fig. 57]

[Fig. 58]

EP 4 116 411 A1

[Fig. 59]

[Fig. 60]

[Fig. 61]

# Day 12

[Fig. 62]

AEM-510ODCN

Visual Field 1 　　　　Visual Field 2

Y-, A+
↓
Y+, A+

x40　x100　x40　x100

AEM-510OSCN

Y+, A+
↓
Y+, A+

x40　x100　x40　x100

Y- : flatter
Y+ : flatter

EP 4 116 411 A1

# Day 14

[Fig. 63]

Two weeks after the start of culture, no difference was observed.

EP 4 116 411 A1

[Fig. 64]

[Fig. 65]

| dedifferentiated progenitor cells | | differentiated mature cells | |
|---|---|---|---|
| ACLY | vs | CS | ACLY, ATP-citrate lyase, CS, citrate synthase |
| ACO1 | | ACO2 | Aconitase 1/2 |
| IDH1 | | IDH2 | Isocitrate dehydrogenase 1/2 |
| MDH1 | | MDH2 | Malate dehydrogenase |
| ME1 | | ME3 | Malic acid enzyme1/3 |
| ACSS2 | | ACSS1 | |
| ACAT2 | | ACAT1 | Acetyl-CoA acetyltransferase 1/2 |
| LDH | | PDH | Lactate dehydrogenase, Pyruvate dehydrogenase |
| | | BCAT2 | |
| | | BCKDH2 | Branched-chain ketoacid dehydrogenase 2 |

cytoplasm · nucleus          mitochondria

EP 4 116 411 A1

**Culture Conditions**

[Fig. 66]

## Nancy Y(+) A(+)

**#184** non-standard cells

P0
SB2(-) 6wpx2
↓
P1 : Day286 (160531)
SB2(-) ECD800
↓
P2 : Day134 (161012)
SB2(-) ECD800
↓
P3 : Day56 (161207)
SB2(+) ECD800
↓
P4 :D135 (170421)
SB2(+) ECD800

**#225** standard cells

P0
SB2(-) 6wpx1
↓
P1 : Day43 (170322)
SB2(-) ECD400
↓
P2 : Day78 (170606)
SB2(-) ECD400
↓
P3 : Day77 (170822)
SB2(-) ECD400
↓
P4 : D45 (171006)
SB2(-) ECD400

[Fig. 67]

#225 P1 Day43 (170322)

ECD400 SB2(-)

ECD 1642

#184 P1 Day286 (160531)

ECD800 SB2(-)

ECD 2179

#184 P4 D135 (170421)

#225 P4 D45 (171006)

[Fig. 68]

SB2(+) ECD800

SB2(-) ECD400

[Fig. 69]

[Fig. 70]

EP 4 116 411 A1

[Fig. 71]

| Accession | Description | # Unique Peptides | Score Mascot: Mascot | Score Sequest HT: Sequest HT | Abundance Ratio: (225) / (184) | Abundances (Normalized): F1: Sample, 184 | Abundances (Normalized): F2: Sample, 184 | Abundances (Normalized): F3: Sample, 184 | Abundances (Normalized): F4: Sample, 225 | Abundances (Normalized): F5: Sample, 225 | Abundances (Normalized): F6: Sample, 225 | Abundance: F1: Sample, 184 | Abundance: F2: Sample, 184 | Abundance: F3: Sample, 184 | Abundance: F4: Sample, 225 | Abundance: F5: Sample, 225 | Abundance: F6: Sample, 225 |
|---|---|---|---|---|---|---|---|---|---|---|---|---|---|---|---|---|---|
| P00918 | Carbonic anhydrase 2 OS=Homo sapiens GN=CA2 PE=1 SV=2 | 17 | 965 | 174.4 | 27.691 | 4531643 | 3549358 | 2297898 | 76395570 | 1.08E+08 | 1.22E+08 | 3021029 | 2724311 | 1576987 | 65576641 | 94710704 | 1.22E+08 |
| P07451 | Carbonic anhydrase 3 OS=Homo sapiens GN=CA3 PE=1 SV=3 | 20 | 2013 | 374.28 | 27.667 | 18194682 | 10257840 | 10681652 | 2.57E+08 | 4.87E+08 | 4.76E+08 | 12129523 | 78734508 | 7330538 | 2.21E+08 | 4.27E+08 | 4.76E+08 |

EP 4 116 411 A1

[Fig. 72]

[Fig. 73]

EP 4 116 411 A1

| | | Category | Term | Count | % | PValue | Genes |
|---|---|---|---|---|---|---|---|
| Annotation Cluster 1 | Enrichment Score: 72.73354929082193 | UP_KEYWORDS | Mitochondrion | 154 | 38.79093 | 1.66E-90 | P24752, P∠ |
| | | UP_KEYWORDS | Transit peptide | 107 | 26.95214 | 1.65E-77 | P24752, P( |
| | | GOTERM_CC_DIRECT | GO:0005739~mitochondrion | 153 | 38.53904 | 1.08E-71 | P24752, P∠ |
| | | UP_SEQ_FEATURE | transit peptide:Mitochondrion | 97 | 24.43325 | 1.79E-69 | P49748, P. |
| | | GOTERM_CC_DIRECT | GO:0005759~mitochondrial matrix | 78 | 19.64736 | 4.07E-58 | P24752, P∠ |
| Annotation Cluster 2 | Enrichment Score: 19.705945707316353 | UP_KEYWORDS | Lysosome | 43 | 10.83123 | 1.93E-26 | Q8NCC3, F |
| | | KEGG_PATHWAY | hsa04142:Lysosome | 30 | 7.556675 | 2.51E-19 | Q8NCC3, F |
| | | GOTERM_CC_DIRECT | GO:0005764~lysosome | 31 | 7.808564 | 1.57E-15 | Q8NCC3, C |
| Annotation Cluster 3 | Enrichment Score: 12.884973261402443 | UP_KEYWORDS | Oxidoreductase | 56 | 14.10579 | 7.82E-23 | P49748, P( |
| | | GOTERM_MF_DIRECT | GO:0016491~oxidoreductase activity | 24 | 6.04534 | 7.94E-11 | Q8IWF2, C |
| | | GOTERM_BP_DIRECT | GO:0055114~oxidation-reduction process | 35 | 8.816121 | 3.57E-07 | P00367, Q |
| Annotation Cluster 4 | Enrichment Score: 9.89201150604489 | KEGG_PATHWAY | hsa01200:Carbon metabolism | 24 | 6.04534 | 7.82E-14 | P11498, P. |

[Fig. 74]

EP 4 116 411 A1

| | | Category | Term | Count | % | PValue | Genes | List Total | Pop Hits | Pop Total | Fold Enrich | Bonferroni | Benjamini | FDR |
|---|---|---|---|---|---|---|---|---|---|---|---|---|---|---|
| Annotation Cluster 1 | Enrichment Score: 9.89201150604489 | KEGG_PATHWAY | hsa01200:Carbon metabolism | 24 | 6.04534 | 7.82E-14 | P11498, P; | 202 | 113 | 6910 | 7.265399 | 1.75E-11 | 5.84E-12 | 9.98E-11 |
| | | GOTERM_BP_DIRECT | GO:0006099~tricarboxylic acid cycle | 11 | 2.770781 | 3.30E-10 | P40926, P; | 371 | 29 | 16792 | 17.16814 | 5.61E-07 | 5.61E-07 | 5.56E-07 |
| | | KEGG_PATHWAY | hsa01130:Biosynthesis of antibiotics | 26 | 6.549118 | 1.67E-09 | P24752, Q | 202 | 212 | 6910 | 4.195311 | 3.74E-07 | 7.48E-08 | 2.13E-06 |
| | | KEGG_PATHWAY | hsa00020:Citrate cycle (TCA cycle) | 11 | 2.770781 | 6.28E-09 | P11498, P; | 202 | 30 | 6910 | 12.5429 | 1.41E-06 | 2.34E-07 | 8.01E-06 |
| Annotation Cluster 2 | Enrichment Score: 5.197965982291 9135 | GOTERM_MF_DIRECT | GO:0050660~flavin adenine dinucleotide binding | 13 | 3.274559 | 1.14E-08 | O95831, P; | 369 | 64 | 16881 | 9.292556 | 7.06E-06 | 2.35E-06 | 1.69E-05 |
| | | GOTERM_BP_DIRECT | GO:0033539~fatty acid beta-oxidation using acyl-CoA dehydrogena | 8 | 2.015113 | 6.15E-08 | P49748, P; | 371 | 18 | 16792 | 20.1162 | 1.05E-04 | 3.49E-05 | 1.04E-04 |
| | | GOTERM_MF_DIRECT | GO:0003995~acyl-CoA dehydrogenase activity | 7 | 1.763224 | 6.86E-07 | P49748, P; | 369 | 16 | 16881 | 20.01474 | 4.25E-04 | 8.49E-05 | 0.001018 |
| | | GOTERM_MF_DIRECT | GO:0009055~electron carrier activity | 12 | 3.02267 | 4.06E-06 | P49748, O | 369 | 90 | 16881 | 6.099729 | 0.002309 | 4.19E-04 | 0.00602 |
| | | INTERPRO | IPR013786:Acyl-CoA dehydrogenase/oxidase, N-terminal | 6 | 1.511335 | 4.31E-06 | P49748, P; | 388 | 13 | 18559 | 22.07653 | 0.003621 | 9.06E-04 | 0.006657 |
| | | INTERPRO | IPR006091:Acyl-CoA oxidase/dehydrogenase, central domain | 6 | 1.511335 | 9.71E-06 | P49748, P; | 388 | 15 | 18559 | 19.13299 | 0.008144 | 0.001362 | 0.015007 |
| | | INTERPRO | IPR009075:Acyl-CoA dehydrogenase/oxidase C-terminal | 6 | 1.511335 | 9.71E-06 | P49748, P; | 388 | 15 | 18559 | 19.13299 | 0.008144 | 0.001362 | 0.015007 |
| | | INTERPRO | IPR009100:Acyl-CoA dehydrogenase/oxidase | 6 | 1.511335 | 9.71E-06 | P49748, P; | 388 | 15 | 18559 | 19.13299 | 0.008144 | 0.001362 | 0.015007 |
| | | GOTERM_MF_DIRECT | GO:0052890~oxidoreductase activity, acting on the CH-CH group of | 6 | 1.511335 | 1.20E-05 | P49748, P; | 369 | 15 | 16881 | 18.29919 | 0.007412 | 9.29E-04 | 0.017824 |
| | | GOTERM_BP_DIRECT | GO:0055088~lipid homeostasis | 8 | 2.015113 | 2.00E-05 | P49748, P4 | 371 | 39 | 16792 | 9.284401 | 0.033353 | 0.004834 | 0.03365 |
| | | GOTERM_MF_DIRECT | GO:0000062~fatty-acyl-CoA binding | 7 | 1.763224 | 3.93E-04 | P49748, P; | 369 | 30 | 16881 | 10.67453 | 0.024027 | 0.002429 | 0.058258 |
| | | GOTERM_MF_DIRECT | GO:0016627~oxidoreductase activity, acting on the CH-CH group of | 5 | 1.259446 | 5.34E-04 | P49748, P; | 369 | 18 | 16881 | 12.70777 | 0.281361 | 0.025095 | 0.788558 |
| | | INTERPRO | IPR006089:Acyl-CoA dehydrogenase, conserved site | 4 | 1.007557 | 6.88E-04 | P49748, P; | 388 | 9 | 18559 | 21.25888 | 0.439969 | 0.030053 | 1.058396 |
| Annotation Cluster 3 | Enrichment Score: 5.056618457050607 | KEGG_PATHWAY | hsa00380:Tryptophan metabolism | 10 | 2.518892 | 1.56E-06 | P24752, Q | 202 | 40 | 6910 | 8.55198 | 3.49E-04 | 4.37E-05 | 0.001991 |
| | | KEGG_PATHWAY | hsa00310:Lysine degradation | 10 | 2.518892 | 1.55E-05 | P24752, P; | 202 | 52 | 6910 | 6.578446 | 0.003467 | 3.16E-04 | 0.019791 |
| | | KEGG_PATHWAY | hsa00071:Fatty acid degradation | 9 | 2.267003 | 3.21E-05 | P49748, P; | 202 | 44 | 6910 | 6.997075 | 0.007168 | 5.99E-04 | 0.04099 |
| Annotation Cluster 4 | Enrichment Score: 4.531021570326907 | GOTERM_BP_DIRECT | GO:0006635~fatty acid beta-oxidation | 10 | 2.518892 | 3.99E-07 | P49748, P; | 371 | 44 | 16792 | 10.28669 | 6.77E-04 | 1.35E-04 | 6.72E-04 |
| | | KEGG_PATHWAY | hsa00071:Fatty acid degradation | 9 | 2.267003 | 3.21E-05 | P49748, P; | 202 | 44 | 6910 | 6.997075 | 0.007168 | 5.99E-04 | 0.04099 |
| | | KEGG_PATHWAY | hsa01212:Fatty acid metabolism | 9 | 2.267003 | 6.19E-05 | P49748, P; | 202 | 48 | 6910 | 6.413985 | 0.013765 | 0.001066 | 0.079966 |

[Fig. 75]

| | | Category | Term | Count | % | PValue | Genes | |
|---|---|---|---|---|---|---|---|---|
| Annotation Cluster 1 | Enrichment Score: 9.89201150604489 | KEGG_PATHWAY | hsa01200:Carbon metabolism | 24 | 6.04534 | 7.82E-14 | P11498, P... | ① |
| | | KEGG_PATHWAY | hsa00020:Citrate cycle (TCA cycle) | 11 | 2.770781 | 6.28E-09 | P11498, P4... | ② |
| | | KEGG_PATHWAY | hsa00071:Fatty acid degradation | 9 | 2.267003 | 3.21E-05 | P49748, P... | ③ |
| Annotation Cluster 4 | Enrichment Score: 4.531021570326907 | GOTERM_BP_DIRECT | GO:0006635~fatty acid beta-oxidation | 10 | 2.518892 | 3.99E-07 | P49748, P... | ④ |
| | | KEGG_PATHWAY | hsa01212:Fatty acid metabolism | 9 | 2.267003 | 6.19E-05 | P49748, P... | ⑤ |
| Annotation Cluster 7 | Enrichment Score: 3.0657450055602973 | KEGG_PATHWAY | hsa00471:D-Glutamine and D-glutamate metabolism | 4 | 1.007557 | 9.49E-05 | P49448, P0... | ⑥ |
| | | KEGG_PATHWAY | hsa00220:Arginine biosynthesis | 5 | 1.259446 | 0.002334 | P49448, P0... | ⑦ |
| | | KEGG_PATHWAY | hsa00250:Alanine, aspartate and glutamate metabolism | 6 | 1.511335 | 0.00316 | P49448, P0... | ⑧ |
| | | KEGG_PATHWAY | hsa02010:ABC transporters | 6 | 1.511335 | 0.008563 | O14678, P... | ⑨ |
| | | KEGG_PATHWAY | hsa00190:Oxidative phosphorylation | 9 | 2.267003 | 0.039648 | P06576, P6... | |

EP 4 116 411 A1

132

[Fig. 76]

**Nitrogen metabolism**

Bile secretion
Pancreatic secretion

**Nitrogen metabolism**
**Arginine biosynthesis**
**Alanine, aspartate and glutamate metabolism**
D-Glutamine and D-glutamate metabolism
Metabolic pathways
Carbon metabolism

Carbon metabolism
Oxidative phosphorylation
**Glycolysis / Gluconeogenesis**
**Valine, leucine and isoleucine degradation (BCAA)**
**Pyruvate metabolism**
**Citrate cycle (TCA cycle)**
Fatty acid degradation
Fatty acid metabolism
Cysteine and methionine metabolism
beta-Alanine metabolism
**Alanine, aspartate and glutamate metabolism**
PPAR signaling pathway
**Nicotinate and nicotinamide metabolism**
**Glutathione metabolism**
Fatty acid biosynthesis

PPAR signaling pathway
Adipocytokine signaling pathway
Insulin resistance
AMPK signaling pathway

3  **Pathway emerging from the analysis of those
with a ratio greater than 27** ⋯⋯⋯⋯⋯⋯⋯⋯ Nitrogen metabolism

5  **Pathway emerging from comparison of mitochondria
after DAVID analysis** ⋯⋯ Carbon metabolism·
**Citrate cycle (TCA cycle)**
Fatty acid degradation
Fatty acid metabolism
D-Glutamine and D-glutamate metabolism
**Arginine biosynthesis**
**Alanine, aspartate and glutamate metabolism**
ABC transporters
Oxidative phosphorylation

[Fig. 77]

Nitrogen metabolism

EP 4 116 411 A1

[Fig. 78]

TCA cycle

Arginine biosynthesis

[Fig. 79]

EP 4 116 411 A1

[Fig. 80]

Glycolysis / Gluconeogenesis

Pyruvate metabolism

[Fig. 81]

EP 4 116 411 A1

[Fig. 82]

Valine, leucine and isoleucine degradation
(BCAA)

EP 4 116 411 A1

[Fig. 83]

Nicotinate and nicotinamide metabolism

[Fig. 84]

| Antigen | Manufacturer | Product Name · Product Number |
|---|---|---|
| human ATP1A1 = Na+ K+ ATPase | millipore | Anti-Na+/K+ ATPase α-1 Antibody, clone C464.6 05-369 |
| human ATP2B3 | abcam | Anti-Calcium Pump PMCA3 ATPase antibody (ab223787) |
| human AQP1 | abcam | Anti-Aquaporin 1 antibody [EPR11588(B)] (ab168387) |
| human AE1 | abcam | Anti-Band 3/AE 1 antibody [EPR1426] (ab108414) |
| human AE2 | invitrogen | SLC4A2 Polyclonal Antibody (Catalog # PA5-82675) |
| human NBCe1 | abcam | Anti-SLC4A4/NBC antibody (ab56215) |
| human SLC4A11 | (sigma)Atlas Antibodies | Anti-SLC4A11 Antibody (HPA018120) |
| human NHE1 | abcam | Anti-Sodium/Hydrogen Exchanger 1/NHE-1 antibody (ab67314) |
| human MCT1 | NOVUS(R&D) | MCT1/SLC16A1 Antibody (NBP1-59656) |
| human MCT8 | (sigma)Atlas Antibodies | Anti-SLC16A2 Antibody (HPA003353) |
| human MCT4 | Abcam | Anti-SLC16A3/MCT 4 antibody (ab234728) |
| human SLC25A42 | invitrogen | SLC25A42 Polyclonal Antibody (Cat #PA5-61975) |
| human CA2 | abcam | Anti-Carbonic anhydrase 2/CA2 antibody (ab191343) |
| human CA3 | abcam | Anti-Carbonic Anhydrase 3/CA3 antibody (ab175309) |
| human CA5B | abcam | Anti-Carbonic Anhydrase 5B/CA5B antibody (ab196800) |
| human CA12 | abcam | Anti-Carbonic Anhydrase 12/CA12 antibody (ab244309) |
| human ZO-1 | invitrogen | ZO-1 Monoclonal Antibody (ZO1-1A12) (#33-9100) |
| | invitrogen | ZO-1 Polyclonal Antibody (# 61-7300) |
| DAPI | Dojindo | D523 Cellstain® DAPI solution (wako code:340-07971) |
| 2nd Abs; anti-rab IgG dye-conjugated | invitrogen | Donkey anti-Rabbit IgG (H+L) Highly Cross-Adsorbed Secondary Antibody, Alexa Fluor Plus 488 (# A32790) |
| | invitrogen | Donkey anti-Rabbit IgG (H+L) Highly Cross-Adsorbed Secondary Antibody, Alexa Fluor Plus 647 (# A32795) |
| 2nd Abs; anti-mus IgG dye-conjugated | invitrogen | Donkey anti-Mouse IgG (H+L) Highly Cross-Adsorbed Secondary Antibody, Alexa Fluor Plus 488 (# A32766) |
| | invitrogen | Donkey anti-Mouse IgG (H+L) Highly Cross-Adsorbed Secondary Antibody, Alexa Fluor Plus 647 (# A32787) |

[Fig. 85]

ATP1A1 (Na+K+ATPase)

| Phase contrast | DAPI | ZO-1 | ATP1A1 |
|---|---|---|---|

① 

②

anti-ATP1A1 mus mAb (5 µg/mL)/Alexa647-conjugated anti-mus IgG (4 µg/mL)
Anti-ZO-1 rab pAb (2.5 µg/mL)/Alexa488-conjugated anti-rab IgG (4 µg/mL)

ATP1A1 = Na⁺-K⁺ ATPase
**Low expression in malignant cells**

[Fig. 86]

## AQP1(Na+K+ATPase)

### AQP1

① 

② 

## SLC4A11

### SLC4A11

**AQP1** : Highly expressed on cell membrane in standard cells
Not expressed in phase transition cells

**SLC4A11** : Highly expressed in standard cells

anti-AQP1 rab pAb (0.87 µg/mL)/Alexa647-conjugated anti-rab IgG (4 µg/mL)
Anti-ZO-1 mus mAb (2.5 µg/mL)/Alexa488-conjugated anti-mus IgG (4 µg/mL)

anti-SLC4A11 rab pAb (3 µg/mL)/Alexa647-conjugated anti-rab IgG (4 µg/mL)
Anti-ZO-1 mus mAb (2.5 µg/mL)/Alexa488-conjugated anti-mus IgG (4 µg/mL)

EP 4 116 411 A1

[Fig. 87]

# NHE1　　　　　　SLC25A42

NH1

NHE1 : Highly expressed in standard cells　　SLC25A42 : Expressed in standard cells

anti-NHE1 rab pAb (2 μg/mL)/Alexa647-conjugated anti-rab IgG (4 μg/mL)
Anti-ZO-1 mus mAb (2.5 μg/mL)/Alexa488-conjugated anti-mus IgG (4 μg/mL)

anti-SLC25A42 rab pAb (2 μg/mL)/Alexa647-conjugated anti-rab IgG (4 μg/mL)
Anti-ZO-1 mus mAb (2.5 μg/mL)/Alexa488-conjugated anti-mus IgG (4 μg/mL)

Organelle-Selective Localization of
Monocarboxylic Acid Transporter

[Fig. 88]

Standard Cells
MCT8   ZO-1   MCT1   MCT4   (reference) AQP

Non-Standard Cells

Mitochondria vs Cell Membrane

Nucleus vs Non-Expression   ( water efflux pump )

Cell Membrane vs Non-Expression

# #190719P2 ① Day42 (191108)

[Fig. 89]

#190719 P2 ① T25x2

cell number 950x10^4 cells
ECD 1900

pHi_BCECF-AM (n=32)

pH7.09±0.156

cHCECs#190719_P2①

EP 4 116 411 A1

[Fig. 90]

#190719P2 (Day40)_① (Nancy A+Y)well#2

x100

200 μm

#190802P3 Day40 (200115)

[Fig. 91]

#190802 P3 ② well1+2

Total cell number: 46.8 x 10^4 cells
(2 wells)
ECD: 46.8/2 x 10^4 / 190
= 1231 cells/mm^2

pHi_BCECF-AM (n=12)

pH7.35±0.228

cHCECs#190802P3_②

EP 4 116 411 A1

[Fig. 92]

#190802P3 Day40 Nancy A(+) Y(+) : 200115 　① well#1+2

pHi_BCECF-AM_cHCECs

[Fig. 93]

pH7.35 ± 0.228 (including outliers)

pH7.09 ± 0.156

□ standard cell #190719P2 (+A+Y)_Day40

□ non-standard cell #190802P3_(+A±Y+SB2+EGF)_Day35

【

【Statistical Processing_at Median Comparison】

If outliers are included,

Mann-Whitney U-test: p = 0.003966

EP 4 116 411 A1

[Fig. 94]

The cells below were passaged
to P3 (T-25) → P4 (24wp)
on Day39 and used.

C37 P3 Day39 (160126)

Day38 (160125) CPC photographing x200

96.7%   3.3%

0.0%   0.0%

0.0%

20.6%

77.6%

2.8%

14.4%   29.2%

75.0%

20.2%   0.6%

38.1%   18.3%

2.8%

ECD=
1789 cells/mm²

0.4%

EP 4 116 411 A1

[Fig. 95]

Collection of culture supernatant

— Cytokine measurement with BioPlex.

Day37 (20160303)

[Fig. 96]

a) Y(+), EGF(+), SB2(+)

b) D17～Y(-)

cell number
33.6x10^4cells/24w
p×1 well
ECD=1768
cells/mm²

cell number
30.6x10^4cells/24wp
×1 well
ECD=1611
cells/mm²

increase in CD44++, +++
compared to a)

# Day 37 (20160303)

[Fig. 97]

## c) D17〜 EGF(-)

cell number
38x10^4cells/24wp
×1 well
ECD=2000
cells/mm²
compared to a),
increase in CD44-
slightly low CD105

## d) D17〜 SB2(-)

cell number
32.1x10^4cells/24wp
×1 well
ECD=1689
cells/mm²

CD105 highly expressed
compared to a)

EP 4 116 411 A1

154

[Fig. 98]

**Other**

[Fig. 99]

※**Additives removed from** D17

**Other**
those with low value

**G-CSF**

**GM-CSF**

[Fig. 100]

**IL-15**

**Eotaxin**

※**Additives removed from** D17

**Involvement of MiR34a and CD44 expression and**

**mitochondrial energy metabolism control pathway**

of

[Fig. 101]

OXPHOS/ OCR ↑ by miR34a-5p introduction

C-Myc

Rock inhibitor

p53 → miR34 → CD44 → RhoA → Glycolysis

miR34a: via suppression of c-Myc expression · RhoA function for dedifferentiation suppression

miR-34a · Miz1 · Skp2 · c-Myc expression · Max · RhoA → Cell invasion

mitochondria-dependent oxidative respiration OXPHOS

mature differentiated cHCEC

EP 4 116 411 A1

[Fig. 102]

[Fig. 103]

lactic acid concentration gradient (osmotic pressure gradient)
→AQP1-mediated water exclusion → clinical pharmacological effect

regulation of mitochondrial function
→→→ clinical effect

clinical pharmacological effect

Opacity improvement

Hydrous edema improvement

Cell miniaturization

Densification

Epithelium                    Endothelium

corneal stroma    lactic ~13mM
                    acid
                    AQP1
2H+              H2O →
                    lactic acid ~7mM
2 lactic acids   MCTs    → H+
                    → lactic acid
Glucose              Glucose

Proliferate cells dissimilar to in vivo cells → Sustained release of cytokines by transfusion cells

Proliferate cells similar to in vivo cells → Reconstruction of corneal endothelial cells by transfusion cells

Reconstructed Monolayer Standard Cell Tissue

OXPHOS    GLYCOLYSIS
lactic acid    H2O    water efflux ↑
                    AQP1 ↑
lactic acid cncentration gradient ↑

Non-Standard Cell Adhesion Tissue

OXPHOS    GLYCOLYSIS
lactic acid    H2O    water efflux ↓
                    AQP1 ↓
lactic acid cncentration gradient ↓

lactic acid 13mM    lactic acid <7mM

lactic acid 13mM    lactic acid 10~13mM

EP 4 116 411 A1

[Fig. 104]

**Induction of differentiated or matured, human functional corneal endothelial cells through dedifferentiation pathway from somatic (stem) cells**

densification
opacity improvement
hydrous edema alleviation

Proliferative immature

CD44-/+
CD90-
CD166+

**differentiated mature cells**

maturation    differentiation

Rock inhibitor

EMT-stemness pathway release

**proliferative undifferentiated cells**

CD44++/+++
CD90+

differentiated mature

De-differentiation (-Y)

culture stress release
EGF not added

senescence pathway release

p38MAPK inhibitor

SB2

*Senescent Cell*

Basic research on differentiation process

[Fig. 105]

**Mitochondrial function is influenced by intracellular pH, and the differentiation/dedifferentiation state of cell is defined**

pHi Regulates Cell Sizes of cHCECs

pHi_BCECF-AM_①(n=32)

pHi 7.09±0.156

pHi_BCECF-AM_②(n=12)

pHi 7.48±0.0668

cHCECF#190802P2_②

Intracellular pH enhances mitochondrial glycolysis, and Citrate, which is transported from mitochondria to the nucleus, acetylates histones by ACLY localized in the nucleus, thus altering the mitochondrial molecular composition and protein composition and modifying the function

EP 4 116 411 A1

[Fig. 106]

cHCECs_#CR04 P3_Day41 (Nancy+Y)

[Fig. 107]

Expression of ion channels • monocarboxylic acid transporters in standard cells • non-standard cells

[Fig. 108]

# Immunoblotting: #191224S P4 Day46 & #200313 P1 Day74

[Fig. 109]

anti-IDH1 rab pAb (1:180 dilution, 5 μg/mL)
anti-IDH2 rab pAb (1:120 dilution, 5 μg/mL)
anti-ATP citrate lyase rab pAb (1:2000 dilution, 0.119 μg/mL)
anti-ACSS2 rab pAb (1:200 dilution, 5 μg/mL)
anti-GAPDH rab pAb (1:2000 dilution, 15 μg/mL)

cHCECs_#191224SP4 (Day46)_ ① (Nancy+Y)

【Cultured for Standard Cell】
#191224S P4 Day46 (+Y)

[Fig. 110]

x100

X100
PBS

Total cell number: 30.4 x 10⁴ cells

ECD: 1600 cells/mm²

Nancy + 10 μM Y-27632 + 20 μg/mL ascorbic acid

cHCECs_#200313 (Day74)_② (Nancy+SB4+EGF)

【Cultured for Non-Standard Cell】
#200313 P1 Day74 (+SB4,EGF)

[Fig. 111]

Total cell number: 14.8 x $10^4$ cells

ECD: 779 cells/mm$^2$

Nancy + 1 µM SB431542 + 5 ng/mL EGF + 20 µg/mL ascorbic acid

#191224S P4_Day42_① Nancy+Y

[Fig. 112]

Total cell number: 219 x $10^4$ cells
(1 well)

ECD: 2305 cells/mm$^2$

#191224S P4_Day42_② Nancy+SB4,EGF

[Fig. 113]

EP 4 116 411 A1

[Fig. 114]

HAT/HDAC activity measurement results

## INTERNATIONAL SEARCH REPORT

| International application No. |
| --- |
| PCT/JP2021/007490 |

**A. CLASSIFICATION OF SUBJECT MATTER**
C12N 5/071(2010.01)i; C12Q 1/04(2006.01)i; G01N 33/48(2006.01)i
FI: C12N5/071; C12Q1/04; G01N33/48 M
According to International Patent Classification (IPC) or to both national classification and IPC

**B. FIELDS SEARCHED**

Minimum documentation searched (classification system followed by classification symbols)
C12N5/071; C12Q1/04; G01N33/48

Documentation searched other than minimum documentation to the extent that such documents are included in the fields searched
| | |
| --- | --- |
| Published examined utility model applications of Japan | 1922–1996 |
| Published unexamined utility model applications of Japan | 1971–2021 |
| Registered utility model specifications of Japan | 1996–2021 |
| Published registered utility model applications of Japan | 1994–2021 |

Electronic data base consulted during the international search (name of data base and, where practicable, search terms used)
JSTPlus/JMEDPlus/JST7580 (JDreamIII); CAplus/MEDLINE/BIOSIS (STN)

**C. DOCUMENTS CONSIDERED TO BE RELEVANT**

| Category* | Citation of document, with indication, where appropriate, of the relevant passages | Relevant to claim No. |
| --- | --- | --- |
| X | WO 2017/141926 A1 (KYOTO PREFECTURAL PUBLIC UNIVERSITY CORPORATION) 24 August 2017 (2017-08-24) claims, examples, fig. 11, 16, 22, etc. | 1-7, 15-26, 31, 32 |
| Y | claims, examples, fig. 11, 16, 22, paragraph [0042], etc. | 1-32 |
| Y | CHEN, Shuyang et al., "Effects of epidermal growth factor on transforming growth factor-betal-induced epithelial-mesenchymal transition and potential mechanism in human corneal epithelial cells", Int. J. Ophthalmol., 18 January 2020, vol. 13, no. 1, pp. 11-20 abstract | 1-32 |

| ☐ Further documents are listed in the continuation of Box C. | ☒ See patent family annex. |
| --- | --- |

| * Special categories of cited documents: | |
| --- | --- |
| "A" document defining the general state of the art which is not considered to be of particular relevance | "T" later document published after the international filing date or priority date and not in conflict with the application but cited to understand the principle or theory underlying the invention |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| 15 April 2021 (15.04.2021) | 27 April 2021 (27.04.2021) |

| Name and mailing address of the ISA/ | Authorized officer |
| --- | --- |
| Japan Patent Office 3-4-3, Kasumigaseki, Chiyoda-ku, Tokyo 100-8915, Japan | |
| | Telephone No. |

Form PCT/ISA/210 (second sheet) (January 2015)

**INTERNATIONAL SEARCH REPORT**
Information on patent family members

| International application No. |
| --- |
| PCT/JP2021/007490 |

| Patent Documents referred in the Report | Publication Date | Patent Family | Publication Date |
| --- | --- | --- | --- |
| WO 2017/141926 A1 | 24 Aug. 2017 | JP 2019-510509 A claims, examples, fig. 11, 16, 22, paragraph [0042], etc. US 2019/0083543 A1 EP 3416658 A1 | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4678783 A
- JP 3421217 B
- WO 9528387 A
- WO 9920620 A
- WO 9961403 A
- WO 02076976 A
- WO 02076977 A
- WO 2002083175 A
- WO 02100833 A
- WO 03059913 A
- WO 03062227 A
- WO 2004009555 A
- WO 2004022541 A
- WO 2004108724 A
- WO 2005003101 A

- WO 2005039564 A
- WO 2005034866 A
- WO 2005037197 A
- WO 2005037198 A
- WO 2005035501 A
- WO 2005035503 A
- WO 2005035506 A
- WO 2005080394 A
- WO 2005103050 A
- WO 2006057270 A
- WO 2007026664 A
- WO 2017141926 A
- WO 2013051722 A
- JP 2020032139 A

**Non-patent literature cited in the description**

- **WIGHAM C ; HODSON S.** *Current Eye Research,* 1981, vol. 1, 37-41
- **HODSON S ; WIGHAM C.** *J Physiol.,* 1983, vol. 342, 409-419
- **HATOU S. ; YAMADA M. ; AKUNE Y. ; MOCHIZUKI H. ; SHIRAISHI A. ; JOKO T. ; NISHIDA T. ; TSUBOTA K.** *Investigative Ophthalmology & Visual Science,* 2010, vol. 51, 3935-3942
- **T. SOGA et al.** *Anal. Chem.,* 2002, vol. 74, 2233-2239
- *Anal. Chem.,* 2000, vol. 72, 1236-1241
- **T. SOGA et al.** *J. Proteome Res.,* 2003, vol. 2, 488-494
- **M. SUGIMOTO et al.** *Metabolomics,* 2009, vol. 6, 78-95
- **KOIZUMI N ; OKUMURA N ; KINOSHITA S.** *Experimental Eye Research,* 2012, vol. 95, 60-7

- **UENO M ; MATSUMURA M ; WATANABE K ; NAKAMURA T ; OSAKADA F ; TAKAHASHI M ; KAWASAKI H ; KINOSHITA S ; SASAI Y.** *Proc Natl Acad Sci USA.,* 2006, vol. 103 (25), 9554-9559
- *CellMetab.,* 03 March 2015, vol. 21 (3), 349-50
- *Trendsin Cell Biology,* June 2017, vol. 27 (6
- **SHEIKH et al.** *Nature Rev. Genetics,* 2019
- **LEUNG, T. et al.** *J. Biol. Chem.,* 1995, vol. 270, 29051-29054
- **ISHIZAKI, T. et al.** *The EMBO J.,* 1996, vol. 15 (8), 1885-1893
- **MIYAI T et al.** *Mol Vis.,* 2008, vol. 14, 942-50
- *Japanese Journal of Ophthalmology,* 2014, vol. 118, 81-83
- *Cell Metab.,* 03 March 2015, vol. 21 (3), 349-50
- *Trends inCell Biology,* June 2017, vol. 27 (6